# EUROPEAN PATENT APPLICATION

(11) **EP 4 144 862 A1**
(43) Date of publication of application: **08.03.2023**
(21) Application number: 21795650.7
(22) Date of filing: 28.04.2021
(51) Int. Cl.: C12Q 1/6809, C12N 15/12, C12Q 1/6813, G01N 33/50, G01N 33/53

(54) **METHOD FOR DETECTING ATOPIC DERMATITIS**

(30) Priority: 01.05.2020 JP 2020081470; 01.05.2020 JP 2020081473; 01.05.2020 JP 2020081503; 20.11.2020 JP 2020193411
(71) Applicant: Kao Corporation, Chuo-ku Tokyo 103-8210 (JP)
(72) Inventor: FUKAGAWA, Satoko, Haga-gun, Tochigi 321-3497 (JP); SHIMA, Kyoko, Haga-gun, Tochigi 321-3497 (JP); TAKADA, Naoto, Haga-gun, Tochigi 321-3497 (JP); INOUE, Takayoshi, Haga-gun, Tochigi 321-3497 (JP); ISHIKAWA, Junko, Haga-gun, Tochigi 321-3497 (JP); KUWANO, Tetsuya, Haga-gun, Tochigi 321-3497 (JP)
(74) Representative: Vossius & Partner Patentanwälte Rechtsanwälte mbB
(86) International application number: PCT/JP2021/017112
(87) International publication number: WO 2021/221138

(57) **Abstract**

Provided are a marker for detecting atopic dermatitis, and a method for detecting atopic dermatitis using the marker. The method for detecting atopic dermatitis in a test subject comprises a step of measuring an expression level of a gene or an expression product thereof contained in a biological sample collected from the test subject.

## Description

### Field of the Invention

The present invention relates to a method for detecting atopic dermatitis using an atopic dermatitis marker.

### Background of the Invention

Atopic dermatitis (hereinafter, also referred to as "AD") is an eczematous skin disease which develops mainly in people with atopic predisposition. Typical symptoms of atopic dermatitis are chronic and recurrent itchiness, eruption, erythema, and the like which occur bilaterally and symmetrically, as well as incomplete keratinization, decline in barrier function, dry skin, and the like. Most cases of atopic dermatitis occur in childhood, and children tend to outgrow atopic dermatitis. However, the number of adult or intractable atopic dermatitis cases has also increased in recent years.

Newborns/infants with genetic predisposition to allergy or atopy are known to develop various allergic diseases such as infantile eczema, atopic dermatitis, food allergy, bronchial asthma, and allergic rhinitis with age (allergy march). For such allergic diseases, the development of one disease is likely to trigger another allergic disease, and the treatment thereof is often prolonged. Hence, the development of an allergic disease reportedly needs to be suppressed at the stage of childhood.

The severity of atopic dermatitis is determined relying on observations with the naked eye under the current circumstances. There exist various items to be found, such as dryness symptoms, erythema, scaling, papule, excoriation, edema, scabbing, vesicle, erosion, and prurigo nodule. Severity Scoring of Atopic Dermatitis (SCORAD) or Eczema Area and Severity Index (EASI) is often used as items to be evaluated by dermatologists. However, these evaluation methods rely largely on the subjective views of evaluators.

As methods for detecting atopic dermatitis using biomarkers, the detection of peripheral blood eosinophil counts, total serum IgE values, LDH (lactate dehydrogenase) values, serum thymus and activation-regulated chemokine (TARC) values, or squamous cell carcinoma antigens 1 (SCCA1 or SerpinB3) and 2 (SCCA2 or SerpinB4) has been proposed (Non Patent Literatures 1, 2 and 3). However, these methods are invasive methods because they involve blood collection. For example, the detection of *Staphylococcus aureus* agrC mutation-dependent RNAIII gene in a skin bacterial flora (Patent Literature 1) has also been proposed, but this method does not always permit diagnosis of atopic dermatitis with sufficient accuracy.

AD detection based on biomarkers is particularly effective for children who have the difficulty in complaining of symptoms. On the other hand, the biomarkers for atopic dermatitis may differ in effectiveness depending on the age of a patient, for example, a pediatric or adult patient. For example, it has been reported on the serum TARC described above that the sensitivity and specificity of determination are reduced in pediatric subjects under the age of 2 compared with pediatric subjects at age 2 or over (Non Patent Literature 4). IL-18 in blood (Non Patent Literature 5) has been reported as a marker effective for the detection of childhood AD. Also, it has been reported that SerpinB4 in blood is effective for the detection of pediatric and adult AD (Non Patent Literatures 6 and 7). It has been reported that decrease in SerpinB12 level or increase in SerpinB3 level was observed in the stratum corneum collected from children with AD (Non Patent Literature 8). However, in this report, stratum corneum SerpinB4 was not detected as an AD-related protein.

Nucleic acids derived from the body can be extracted from body fluids such as blood, secretions, tissues, and the like. It has recently been reported that: RNA contained in skin surface lipids (SSL) can be used as a biological sample for analysis; and marker genes of the epidermis, the sweat gland, the hair follicle and the sebaceous gland can be detected from SSL (Patent Literature 2). It has also been reported that marker genes for atopic dermatitis can be detected from SSL (Patent Literature 3).

Various nucleic acid or protein markers have been isolated from skin tissues collected by biopsy or tape-stripped skin samples such as the stratum corneum. Non Patent Literatures 9 to 14 and Patent Literature 4 state that skin diseases or conditions were examined by applying a less sticky adhesive tape to the skin to noninvasively collect peptide markers such as interleukins (ILs), TNF-α, INF-y, and human β-defensin (hBD2) from the skin surface, and using the collected markers.

(Patent Literature 1) JP-A-2019-30272
(Patent Literature 2) WO 2018/008319
(Patent Literature 3) JP-A-2020-074769
(Patent Literature 4) WO 2014/144289
(Non Patent Literature 1) Allergy (2002) 57: 180-181
(Non Patent Literature 2) Ann Clin Biochem.(2012) 49: 277-84
(Non Patent Literature 3) The Japanese Journal of Dermatology (2018) 128: 2431-2502
(Non Patent Literature 4) Jpn. J. Pediatr. Allergy Clin. Immunol (2005) 19 (5): 744-757
(Non Patent Literature 5) Allergology International (2003) 52: 123-130
(Non Patent Literature 6) J Allergy Clin Immunol (2018) 141 (5): 1934-1936
(Non Patent Literature 7) Allergology International (2018) 67: 124-130
(Non Patent Literature 8) J Allergy Clin Immunol (2020) S0091-6749 (20): 30571-6
(Non Patent Literature 9) Skin Res Technol, 2001, 7 (4): 227-37
(Non Patent Literature 10) Skin Res Technol, 2002, 8 (3): 187-93
(Non Patent Literature 11) Med Devices (Auckl), 2016, 9: 409-417
(Non Patent Literature 12) Med Devices (Auckl), 2018, 11: 87-94
(Non Patent Literature 13) J Tissue Viability, 2019, 28 (1): 1-6
(Non Patent Literature 14) J Diabetes Res, doi/10.1155/2019/1973704

### Summary of the Invention

In one aspect, the present invention relates to the following A-1) to A-3).

A-1) A method for detecting adult atopic dermatitis in a test subject, comprising a step of measuring an expression level of at least one gene selected from the group of 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 or an expression product thereof in a biological sample collected from the test subject.

A-2) A test kit for detecting adult atopic dermatitis, the kit being used in a method according to A-1), and comprising an oligonucleotide which specifically hybridizes to the gene, or an antibody which recognizes an expression product of the gene.

A-3) A detection marker for adult atopic dermatitis comprising at least one gene selected from the group of 210 genes shown in Table A-b given below or an expression product thereof.

In another aspect, the present invention relates to the following B-1) to B-3).

B-1) A method for detecting childhood atopic dermatitis in a test subject, comprising a step of measuring an expression level of at least one gene selected from the group of 7 genes consisting of IMPDH2, ERI1, FBXW2, STK17B, TAGLN2, AMICA1 and HNRNPA1 or an expression product thereof in a biological sample collected from the test subject.

B-2) A test kit for detecting childhood atopic dermatitis, the kit being used in a method according to B-1), and comprising an oligonucleotide which specifically hybridizes to the gene, or an antibody which recognizes an expression product of the gene.

B-3) A detection marker for childhood atopic dermatitis comprising at least one gene selected from the group of genes shown in Tables B-b-1 and B-b-2 given below or an expression product thereof.

In a further alternative aspect, the present invention provides the following.

A method for preparing a protein marker for detecting atopic dermatitis, comprising collecting at least one protein selected from the group consisting of proteins shown in Tables C-1-1 to C-1-13 given below from skin surface lipids collected from a test subject.

A method for detecting atopic dermatitis in a test subject, comprising detecting at least one protein selected from the group consisting of proteins shown in Tables C-1-1 to C-1-13 given below from skin surface lipids collected from the test subject.

A protein marker for detecting atopic dermatitis comprising at least one protein selected from the group consisting of proteins shown in Tables C-2-1 to C-2-5 given below.

In a further alternative aspect, the present invention provides the following.

A method for detecting childhood atopic dermatitis in a child test subject, comprising a step of measuring an expression level of SerpinB4 protein in skin surface lipids collected from the test subject.

A test kit for detecting childhood atopic dermatitis, the kit being used in the method for detecting childhood atopic dermatitis, and comprising an antibody which recognizes SerpinB4 protein.

### Brief Description of Drawings

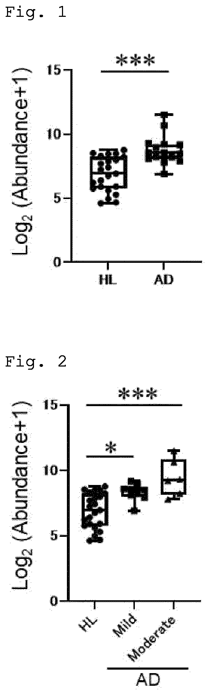

[Figure 1] Figure 1 is a box-and-whisker plot showing the expression level of SerpinB4 protein in SSL derived from the healthy site (face) of a healthy group (HL) of children and the eruption site (face) of an AD group (AD) of children. The drawing shows the plot of each data, in which the lowermost and uppermost ends of the whisker represent the minimum and maximum values, respectively, of the data, and the first quartile, the second quartile (median value), and the third quartile are indicated from the lower end of the box (the same applies to Figures 2 to 4 and 7 to 11 given below). ***: P < 0.001 (Student's t-test).
[Figure 2] Figure 2 is a box-and-whisker plot showing the expression level of SerpinB4 protein in SSL derived from the healthy site (face) of a healthy group (HL) of children and the eruption sites (face) of a mild AD group (Mild) and a moderate AD group (Moderate) of children. *: P < 0.05, ***: P < 0.001 (Tukey's test).
[Figure 3] Figure 3 is a box-and-whisker plot showing the expression level of SerpinB4 protein in SSL derived from the healthy site (back) of a healthy group (HL) of children and the non-eruption site (back) of an AD group (AD) of children. **: P < 0.01 (Student's t-test).
[Figure 4] Figure 4 is a box-and-whisker plot showing the expression level of SerpinB4 protein in SSL derived from the healthy site (back) of a healthy group (HL) of children and the non-eruption sites (back) of a mild AD group (Mild) and a moderate AD group (Moderate) of children. *: P < 0.05 (Tukey's test).
[Figure 5] Figure 5 shows an ROC curve of a SerpinB4 protein expression level in SSL derived from the healthy site (face) of a healthy group (HL) of children and the eruption site (face) of an AD group (AD) of children.
[Figure 6] Figure 6 shows an ROC curve of a SerpinB4 protein expression level in SSL derived from the healthy site (back) of a healthy group (HL) of children and the non-eruption site (back) of an AD group (AD) of children.
[Figure 7] Figure 7 is a box-and-whisker plot showing the expression level of SerpinB4 RNA in SSL derived from the healthy site (face) of a healthy group (HL) of children and the eruption site (face) of an AD group (AD) of children. n.s.: not significant (Student's t-test).
[Figure 8] Figure 8 is a box-and-whisker plot showing the expression level of SerpinB4 protein in SSL derived from the healthy site (face) of a healthy group (HL) of adults and the eruption site (face) of an AD group (AD) of adults. n.s.: not significant (Student's t-test).
[Figure 9] Figure 9 is a box-and-whisker plot showing the expression level of IL-18 protein in SSL derived from the healthy site (back) of a healthy group (HL) of children and the non-eruption site (back) of an AD group (AD) of children. n.s.: not significant (Student's t-test).
[Figure 10] Figure 10 is a box-and-whisker plot showing the expression level of SerpinB12 protein in SSL derived from the healthy site (face) of a healthy group (HL) of children and the eruption site (face) of an AD group (AD) of children. n.s.: not significant (Student's t-test).
[Figure 11] Figure 11 is a box-and-whisker plot showing the expression level of SerpinB12 protein in SSL derived from the healthy site (back) of a healthy group (HL) of children and the non-eruption site (back) of an AD group (AD) of children. n.s.: not significant (Student's t-test).

### Detailed Description of the Invention

All patent literatures, non patent literatures, and other publications cited herein are incorporated herein by reference in their entirety.

In the present specification, the term "nucleic acid" or "polynucleotide" means DNA or RNA. The DNA includes all of cDNA, genomic DNA, and synthetic DNA. The "RNA" includes all of total RNA, mRNA, rRNA, tRNA, non-coding RNA and synthetic RNA.

In the present specification, the "gene" encompasses double-stranded DNA including human genomic DNA as well as single-stranded DNA (positive strand) including cDNA, single-stranded DNA having a sequence complementary to the positive strand (complementary strand), and their fragments, and means those containing some biological information in sequence information on bases constituting DNA. The "gene" encompasses not only a "gene" represented by a particular nucleotide sequence but a nucleic acid encoding a congener (i.e., a homolog or an ortholog), a variant such as gene polymorphism, and a derivative thereof.

In the present specification, the gene capable of serving as an atopic dermatitis marker (marker for the detection of atopic dermatitis; hereinafter, also referred to as a "detection marker for atopic dermatitis" or a "marker for detecting atopic dermatitis") (hereinafter, this gene is also referred to as a "target gene") also encompasses a gene having a nucleotide sequence substantially identical to the nucleotide sequence of DNA constituting the gene as long as the gene is capable of serving as a biomarker for detecting atopic dermatitis. In this context, the nucleotide sequence substantially identical means a nucleotide sequence having 90% or higher, preferably 95% or higher, more preferably 98% or higher, further more preferably 99% or higher identity to the nucleotide sequence of DNA constituting the gene, for example, when searched using homology calculation algorithm NCBI BLAST under conditions of expectation value = 10; gap accepted; filtering = ON; match score = 1; and mismatch score = -3.

In the present specification, the "expression product" of a gene conceptually encompasses a transcription product and a translation product of the gene. The "transcription product" is RNA resulting from the transcription of the gene (DNA), and the "translation product" means a protein which is encoded by the gene and translationally synthesized on the basis of the RNA.

The names of genes disclosed in the preset specification follow Official Symbol described in NCBI ([www.ncbi.nlm.nih.gov/]). On the other hand, gene ontology (GO) follows Pathway ID. described in String ([string-db.org/]). The names of proteins disclosed in the present specification follow Gene Name or Protein Name described in UniProt ([https://www.uniprot.org/]).

In the present specification, the "feature" in machine learning is synonymous with an "explanatory variable". In the present specification, a gene and an expression product thereof for use in machine learning which are selected from markers for detecting atopic dermatitis are also collectively referred to as a "feature gene". In the present specification, a protein for use in machine learning which is selected from protein markers for detecting atopic dermatitis is also referred to as a "feature protein".

In the present specification, the "skin surface lipids (SSL)" refer to a lipid-soluble fraction present on skin surface, and is also called sebum. In general, SSL mainly contains secretions secreted from the exocrine gland such as the sebaceous gland in the skin, and is present on skin surface in the form of a thin layer that covers the skin surface. SSL is known to contain RNA expressed in skin cells (see Patent Literature 2).

In the present specification, the "skin" is a generic name for regions containing tissues such as the stratum corneum, the epidermis, the dermis, and the hair follicle as well as the sweat gland, the sebaceous gland and other glands, unless otherwise specified.

In the present specification, the "child" conceptually includes a "pediatric" individual before the start of secondary sex characteristics, specifically a 12-year-old or younger pediatric individual, in the broad sense, and preferably refers to a child from the age of 0 years to below school age, specifically, a 0- to 5-year-old child. In the present specification, the "adult" refers to a person that does not fall within the range of the "child" in the broad sense, and preferably refers to a person who has completed secondary sex characteristics. Specifically, the adult is preferably a person at age 16 or over, more preferably a person at age 20 or over.

The "atopic dermatitis" (AD) refers to a disease which has eczema with itch in principal pathogen and repeats exacerbation and remission. Most of AD patients reportedly have atopic predisposition. Examples of atopic predisposition include i) family history and/or previous medical history (any or a plurality of diseases among bronchial asthma, allergic rhinitis/ conjunctivitis, atopic dermatitis, and food allergy), or ii) a predisposition to easily produce an IgE antibody. Atopic dermatitis mostly develops in childhood, and children tend to outgrow atopic dermatitis. However, the number of adult atopic dermatitis cases has also increased in recent years. In the present specification, the atopic dermatitis encompasses childhood atopic dermatitis (childhood AD) which develops in childhood, and adult atopic dermatitis (adult AD) which develops in adults other than children.

Eruption of childhood AD is characterized by starting on the head or the face in infancy, often spreading down to the body trunk or the extremities, decreasing on the face in early childhood of age 1 or later, and appearing mostly on the neck and joints of the extremities. In recent years, childhood AD and adult AD have been reported to differ in that abnormal epidermal keratinization associated with chronic inflammatory abnormality is observed in adult AD compared with childhood AD (Journal of allergy and clinical immunology, 141 (6): 2094-2106, 2018), though it is uncertain due to a small number of reported cases.

The degree of progression (severity) of atopic dermatitis is classified into, for example, no symptoms, minor, mild (low grade), moderate (intermediate grade), and severe (high grade). The severity can be classified on the basis of, for example, a severity evaluation method described in Guidelines for the Management of Atopic Dermatitis (issued by Japanese Dermatological Association, The Japanese Journal of Dermatology, 128 (12): 2431-2502, 2018 (Heisei 30)). The Guidelines for the Management of Atopic Dermatitis describes some severity evaluation methods and states that severity classification methods with verified statistical reliability and validity for overall evaluation of severity are, for example, Atopic Dermatitis Severity Classification (The Japanese Journal of Dermatology, 111: 2023-2033 (2001); and The Japanese Journal of Dermatology, 108: 1491-1496 (1998)) provided by the Advisory Committee for Atopic Dermatitis Severity Classification of Japanese Dermatological Association, Severity Scoring of Atopic Dermatitis ("SCORAD"; Dermatology, 186: 23-31 (1993), and Eczema Area and Severity Index ("EASI"; Exp Dermatol, 10: 11-18 (2001)). Other severity classification methods described in the Guidelines for the Management of Atopic Dermatitis include evaluation of eruption severity, evaluation of pruritus, evaluation by patients, and evaluation of QOL. For example, EASI is a score from 0 to 72 which is calculated on the basis of scores based on four symptoms, erythema, edema/oozing/papule, excoriation, and lichenification, in each of the head and neck, the body trunk, the upper limbs, and the lower limbs as assessed sites, and the percentage (%) of areas with the four symptoms based on the whole assessed sites. As an example of severity classification based on the EASI scoring, the severity can be classified into "mild" when the EASI score is larger than 0 and smaller than 6, "moderate" when the EASI score is 6 or larger and smaller than 23, and "severe" when the EASI score is 23 or larger and 72 or smaller (Br J Dermatol, 177: 1316-1321 (2017)), though the severity classification is not limited thereto.

In the present specification, the "detection" of atopic dermatitis means to elucidate the presence or absence of atopic dermatitis. In the present specification, the "detection" of childhood atopic dermatitis means to elucidate the presence or absence of childhood atopic dermatitis.

In the present specification, the term "detection" may be used interchangeably with the term "test", "measurement", "determination", "evaluation" or "assistance of evaluation". In the present specification, the term "test", "measurement", "determination" or "evaluation" does not include any such action by a physician.

### (1. Detection marker for adult AD and method for detecting adult AD using same)

The present inventors collected SSL from adult AD patients and healthy adult subjects and exhaustively analyzed the expressed state of RNA contained in the SSL as sequence information, and consequently found that the expression levels of particular genes significantly differ therebetween, and AD can be detected on the basis of this index. Thus, one aspect of the present invention relates to a provision of a marker for detecting adult AD, and a method for detecting adult AD using the marker. The present invention enables adult AD to be conveniently and noninvasively detected early with high accuracy, sensitivity and specificity.

As shown in Examples mentioned later, 48 genes with increased expression and 75 genes with decreased expression (a total of 123 genes (Tables A-1-1 to A-1-3) were identified by extracting RNA which attained a corrected p value (FDR) of less than 0.05 in a likelihood ratio test in AD patients compared with healthy subjects using normalized count values obtained using DESeq2 (Love MI et al., Genome Biol. 2014) in data (read count values) on the expression level of RNA extracted from SSL of 14 healthy adult subjects and 29 adult AD patients. In the tables, genes represented by "UP" are genes whose expression level is increased in adult AD patients, and genes represented by "DOWN" are genes whose expression level is decreased in adult AD patients.

Thus, a gene selected from the group of these 123 genes or an expression product thereof is capable of serving as an adult atopic dermatitis marker for detecting adult AD. In the gene group, 107 genes (indicated by boldface with * added in Tables A-1-1 to A-1-3) are genes whose relation to adult AD have not been reported so far.

Feature gene extraction and prediction model construction were attempted using data on the expression level of every SSL-derived RNA (Log₂(RPM + 1) values of 7429 genes) from the test subjects as explanatory variables, the healthy subjects and the AD patients as objective variables, and random forest (Breiman L. Machine Learning (2001) 45; 5-32) as machine learning algorithm. As shown in Examples mentioned later, top 150 genes of variable importance based on Gini coefficient (Tables A-3-1 to A-3-4) were selected as feature genes, and prediction models were constructed using the genes. As a result, adult AD was found predictable.

Thus, a gene selected from the group of these 150 genes or an expression product thereof is capable of serving as a suitable adult atopic dermatitis marker for detecting adult AD. Among them, 127 genes (indicated by boldface with * added in Tables A-3-1 to A-3-4) are novel adult atopic dermatitis markers whose relation to AD has not been reported so far. As shown in Examples mentioned later, prediction models using these novel atopic dermatitis markers are also capable of predicting adult AD.

Prediction model construction was similarly attempted using data on the expression levels of the 123 genes described above which were differentially expressed between the healthy subjects and the AD patients, or 107 genes out of these genes (Log₂(RPM + 1) values), and using random forest. As a result, adult AD was found predictable in all the cases.

Feature genes were extracted (maximum number of trials: 1,000, p value: less than 0.01) using Boruta method (Kursa et al., Fundamental Informaticae (2010) 101; 271-286) as machine learning algorithm. As a result, 45 genes (Table A-4) were extracted as feature genes. As shown in Examples mentioned later, adult AD was found predictable with prediction models based on random forest using these genes.

Thus, a gene selected from the group of these 45 genes or an expression product thereof is capable of serving as a suitable adult atopic dermatitis marker for detecting adult AD. Among them, 39 genes (indicated by boldface with * added in Table A-4) are novel atopic dermatitis markers whose relation to AD has not been reported so far. As shown in Examples mentioned later, prediction models using these novel atopic dermatitis markers are also capable of predicting adult AD.

245 genes (Table A-a) which are the sum (AUBUC) of the group of 123 genes (A) shown in Tables A-1-1 to A-1-3 extracted by differential expression analysis, the group of 150 genes (B) shown in Tables A-3-1 to A-3-4 selected as feature genes by random forest, and the group of 45 genes (C) shown in Table A-4 selected as feature genes by Boruta method, as mentioned above, are adult atopic dermatitis markers. Among them, 210 genes (Table A-b) are novel adult atopic dermatitis markers.

**[Table A-a]**

| | | | | | | |
|---|---|---|---|---|---|---|
| ACAT1 | CDS1 | FABP7 | HMHA1 | MTSS1 | PSMA5 | SSH1 |
| ACO1 | CEP76 | FABP9 | IL17RA | MVP | PSMB4 | ST6GALNAC2 |
| ADAP2 | CETN2 | FAM108B1 | IL2RB | MYO6 | PTPN18 | TCHHL1 |
| AKAP17A | CHMP4C | FAM120A | ILF3 | NCOR2 | RAB11FIP5 | TEX2 |
| AKT1 | CISD1 | FAM190B | ISCA1 | NCS1 | RABL6 | TGFB1 |
| ANXA1 | COBLL1 | FAM26E | ITPRIPL2 | NDUFA4 | RAC1 | THBD |
| APOBR | COPS2 | FBXL17 | KIAA0146 | NIPSNAP3A | RAI14 | TM7SF2 |
| ARHGAP23 | COX6A1 | FBXL18 | KIAA0513 | NMRK1 | RASA4CP | TMC5 |
| ARHGAP24 | COX7B | FBXL6 | KLK5 | NPEPL1 | RB1CC1 | TMEM165 |
| ARHGAP29 | CREG1 | FBXO32 | KRT23 | NPR1 | RGS19 | TMEM222 |
| ARHGAP4 | CRISPLD2 | FDFT1 | KRT25 | NPR2 | RHOC | TMPRSS11E |
| ARL8A | CRTC2 | FIS1 | KRT71 | NR1D1 | RNPEPL1 | TNRC18 |
| ARRDC4 | CRY2 | FMN1 | LCE1D | NUDT16 | RORC | TPGS2 |
| ATOX1 | CSNK1G2 | FOSB | LCE2C | OAT | RPS6KB2 | TSTD1 |
| ATP12A | CSTB | FOXQ1 | LENG9 | OGFR | RRM1 | TTC39B |
| ATP5A1 | CTBP1 | FURIN | LEPREL1 | PADI1 | SAP30BP | TWSG1 |
| ATPIF1 | CTDSP1 | GABARAPL2 | LMNA | PALD1 | SCARB2 | TYK2 |
| ATXN7L3B | CTSB | GDE1 | LOC146880 | PARP4 | SFN | U2AF2 |
| BAX | CTSL2 | GIGYF1 | LOC152217 | PCDH1 | SH3BGRL2 | UNC13D |
| BCKDHB | CXCL16 | GLRX | LRP8 | PCSK7 | SHC1 | UQCRQ |
| BCRP3 | CYTH2 | GNA15 | LY6D | PCTP | SIRT6 | USP38 |
| BSG | DBNDD2 | GNB2 | LYNX1 | PDZK1 | SKP1 | VHL |
| C15orf23 | DBT | GPD1 | MAN2A2 | PHB | SLC12A9 | VOPP1 |
| C16orf70 | DGKA | GPNMB | MAPK3 | PINK1 | SLC25A16 | VPS4B |
| C17orf107 | DHX32 | GRASP | MAPKBP1 | PLAA | SLC25A33 | WBSCR16 |
| C19orf71 | DNASE1L1 | GRN | MARK2 | PLEKHG2 | SLC2A4RG | WDR26 |
| C1QB | DOPEY2 | GSDMA | MAZ | PLP2 | SLC31A1 | XKRX |
| C2CD2 | DPYSL3 | GSE1 | MECR | PMVK | SMAP2 | XPO5 |
| C4orf52 | DSTN | GTF2H2 | MEMO1 | PNPLA1 | SMARCD1 | ZC3H15 |
| CAMP | DUSP16 | HADHA | MINK1 | POLD4 | SNORA71C | ZC3H18 |
| CAPN1 | DYNLL1 | HBP1 | MIR548I1 | PPA1 | SNORA8 | ZFP36L2 |
| CARD18 | EFHD2 | HINT3 | MKNK2 | PPBP | SNORD17 | ZMIZ1 |
| CCDC88B | EHBP1L1 | HLA-B | MLL2 | PPP1R12C | SPDYE7P | ZNF335 |
| CCND3 | EIF1AD | HMGCL | MLL4 | PPP1R9B | SPINK5 | ZNF664 |
| CDK9 | EMP3 | HMGCS1 | MLLT11 | PRSS8 | SRF | ZNF706 |

**[Table A-b]**

| | | | | | | |
|---|---|---|---|---|---|---|
| ACAT1 | CEP76 | FABP7 | HMGCL | MLLT11 | PSMA5 | ST6GALNAC2 |
| ACO1 | CETN2 | FABP9 | HMHA1 | MTSS1 | PSMB4 | TEX2 |
| ADAP2 | CHMP4C | FAM108B1 | ILF3 | MVP | PTPN18 | TM7SF2 |
| AKAP17A | CISD1 | FAM120A | ISCA1 | MYO6 | RAB11FIP5 | TMC5 |
| APOBR | COBLL1 | FAM190B | ITPRIPL2 | NCOR2 | RABL6 | TMEM165 |
| ARHGAP23 | COPS2 | FAM26E | KIAA0146 | NCS1 | RAI14 | TMEM222 |
| ARHGAP24 | COX6A1 | FBXL17 | KIAA0513 | NDUFA4 | RASA4CP | TMPRSS11E |
| ARHGAP29 | COX7B | FBXL18 | KRT23 | NIPSNAP3A | RB1CC1 | TNRC18 |
| ARHGAP4 | CREG1 | FBXL6 | KRT25 | NMRK1 | RGS19 | TPGS2 |
| ARL8A | CRISPLD2 | FBXO32 | KRT71 | NPEPL1 | RHOC | TSTD1 |
| ARRDC4 | CRTC2 | FDFT1 | LCE1D | NR1D1 | RNPEPL1 | TTC39B |
| ATOX1 | CRY2 | FIS1 | LCE2C | NUDT16 | RPS6KB2 | TWSG1 |
| ATP12A | CSNK1G2 | FMN1 | LENG9 | OAT | RRM1 | U2AF2 |
| ATP5A1 | CSTB | FOSB | LEPREL1 | OGFR | SAP30BP | UNC13D |
| ATPIF1 | CTBP1 | FURIN | LMNA | PADI1 | SCARB2 | UQCRQ |
| ATXN7L3B | CTDSP1 | GABARAPL2 | LOC146880 | PALD1 | SH3BGRL2 | USP38 |
| BAX | CTSB | GDE1 | LOC152217 | PARP4 | SKP1 | VHL |
| BCKDHB | CYTH2 | GIGYF1 | LRP8 | PCSK7 | SLC12A9 | VOPP1 |
| BCRP3 | DBNDD2 | GLRX | LY6D | PCTP | SLC25A16 | VPS4B |
| C15orf23 | DBT | GNA15 | MAN2A2 | PDZK1 | SLC25A33 | WBSCR16 |
| C16orf70 | DGKA | GNB2 | MAPK3 | PHB | SLC2A4RG | WDR26 |
| C17orf107 | DHX32 | GPD1 | MAPKBP1 | PINK1 | SLC31A1 | XKRX |
| C19orf71 | DNASE1L1 | GRASP | MAZ | PLAA | SMAP2 | XPO5 |
| C1QB | DOPEY2 | GRN | MECR | PLEKHG2 | SMARCD1 | ZC3H15 |
| C2CD2 | DPYSL3 | GSDMA | MEMO1 | PLP2 | SNORA71C | ZC3H18 |
| C4orf52 | DSTN | GSE1 | MINK1 | PMVK | SNORA8 | ZFP36L2 |
| CARD18 | DUSP16 | GTF2H2 | MIR548I1 | POLD4 | SNORD17 | ZMIZ1 |
| CCDC88B | DYNLL1 | HADHA | MKNK2 | PPA1 | SPDYE7P | ZNF335 |
| CCND3 | EIF1AD | HBP1 | MLL2 | PPP1R12C | SRF | ZNF664 |
| CDS1 | EMP3 | HINT3 | MLL4 | PPP1R9B | SSH1 | ZNF706 |

17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 are common genes (A∩B∩C) among the group of 123 genes (A) shown in Tables A-1-1 to A-1-3 extracted by differential expression analysis, the group of 150 genes (B) shown in Tables A-3-1 to A-3-4 selected as feature genes by random forest, and the group of 45 genes (C) shown in Table A-4 selected as feature genes by Boruta method, as mentioned above, and are genes which have previously not been associated with AD (indicated by boldface with * added in each table). Thus, at least one gene selected from the group of these genes or an expression product thereof is particularly useful as a novel adult atopic dermatitis marker for detecting adult AD. These 17 genes are each capable of serving alone as an adult atopic dermatitis marker. It is preferred to use 2 or more, preferably 5 or more, more preferably 10 or more of these genes in combination, and it is even more preferred to use all the 17 genes in combination.

The method for detecting adult AD according to the present invention includes a step of measuring an expression level of a target gene which is, in one aspect, at least one gene selected from the group of 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 or an expression product thereof in a biological sample collected from an adult test subject.

Alternatively, a discriminant (prediction model) which discriminates between an AD patient and a healthy subject is constructed using measurement values of an expression level of the target gene or the expression product thereof derived from an adult AD patient and an expression level of the target gene or the expression product thereof derived from a healthy adult subject, and adult AD can be detected through the use of the discriminant. Thus, a prediction model capable of predicting adult AD can be constructed by using 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2, and 123 genes shown in Tables A-1-1 to A-1-3, 150 genes shown in Tables A-3-1 to A-3-4, or 45 genes shown in Table A-4, including the 17 genes, as feature genes.

In the case of preparing the discriminant which discriminates between an adult AD patient group and a healthy adult subject group, one or more, preferably 5 or more, more preferably 10 or more, even more preferably all the 17 genes are selected as feature genes from the group of 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2, and expression data on the gene(s) or expression product(s) thereof is used. In the case of selecting a plurality of genes, it is preferred to prepare the discriminant by selecting genes in a higher rank of variable importance in Tables A-3-1 to A-3-4 of these genes in order as feature genes. Further, adult AD may be detected according to a discriminant prepared by appropriately adding, to the expression data on the 17 genes, expression data on at least one, 5 or more, 10 or more, 20 or more or 50 or more genes or expression products thereof selected from the group consisting of genes other than the 17 genes among 245 genes shown in Table A-a, 123 genes shown in Tables A-1-1 to A-1-3, 150 genes shown in Tables A-3-1 to A-3-4 or 45 genes shown in Table A-4 described above. In the case of selecting gene(s) other than the 17 genes from the group consisting of 150 genes shown in Tables A-3-1 to A-3-4, the feature genes may be selected from the group consisting of genes in a higher rank of variable importance in order or from the group consisting of genes within top 50, preferably top 30 genes of variable importance. In the case of selecting gene(s) other than the 17 genes as feature genes, it is preferred to select feature genes from the group consisting of novel atopic dermatitis markers indicated by boldface with * added in Tables A-1-1 to A-1-3, Tables A-3-1 to A-3-4 and Table A-4.

Preferably, the discriminant using the 17 genes, 123 genes or 107 genes (indicated by boldface with * added in Tables A-1-1 to A-1-3) shown in Tables A-1-1 to A-1-3, 150 genes or 127 genes (indicated by boldface with * added in Tables A-3-1 to A-3-4) shown in Tables A-3-1 to A-3-4, or 45 genes or 39 genes (indicated by boldface with * added in Table A-4) shown in Table A-4 as feature genes can be mentioned.

In the present invention, preferably, the adult atopic dermatitis marker described above, selected from the group consisting of 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 or expression products thereof includes neither TMPRSS11E gene nor SPDYE7P gene. For example, in the case of measuring expression levels of the 17 genes or expression products thereof in the method for detecting adult AD according to the present invention, preferably, the expression levels of TMPRSS11E gene and SPDYE7P gene are measured neither alone nor in combination of only these genes.

In the present invention, preferably, the adult atopic dermatitis marker selected from the group consisting of 107 genes indicated by boldface with * added in Tables A-1-1 to A-1-3 or expression products thereof does not include 15 genes shown in Table A-5-a given below.

In the present invention, preferably, the adult atopic dermatitis marker selected from the group consisting of 127 genes indicated by boldface with * added in Tables A-3-1 to A-3-4 or expression products thereof does not include 8 genes shown in Table A-5-b given below.

In the present invention, preferably, the adult atopic dermatitis marker selected from the group consisting of 39 genes indicated by boldface with * added in Table A-4 or expression products thereof does not include 5 genes shown in Table A-5-c given below.

In the present invention, preferably, the adult atopic dermatitis marker selected from the group consisting of 210 genes shown in Table A-b or expression products thereof does not include 23 genes shown in Table A-5-d given below.

**[Table A-5-a]**

| | | | | | |
|---|---|---|---|---|---|
| ARHGAP24 | C16orf70 | CDS1 | CHMP4C | FBXO32 | GDE1 |
| ISCA1 | PADI1 | PDZK1 | PINK1 | RAI14 | SNORA8 |
| SPDYE7P | TMPRSS11E | TPGS2 | | | |

**[Table A-5-b]**

| | | | | | |
|---|---|---|---|---|---|
| FABP9 | LCE2C | MIR548I1 | NR1D1 | SH3BGRL2 | SNORA71C |
| SPDYE7P | TMPRSS11E | | | | |

**[Table A-5-c]**

| | | | | |
|---|---|---|---|---|
| KRT25 | KRT71 | MIR548I1 | SPDYE7P | TMPRSS11E |

**[Table A-5-d]**

| | | | | | |
|---|---|---|---|---|---|
| ARHGAP24 | C16orf70 | CDS1 | CHMP4C | FABP9 | FBXO32 |
| GDE1 | ISCA1 | KRT25 | KRT71 | LCE2C | MIR548I1 |
| NR1D1 | PAD\|1 | PDZK1 | PINK1 | RAI14 | SH3BGRL2 |
| SNORA71C | SNORA8 | SPDYE7P | TMPRSS11E | TPGS2 | |

Alternatively or additionally, in the present invention, preferably, the adult atopic dermatitis marker selected from the group consisting of 245 genes shown in Table A-a or expression products thereof does not include protein markers which are expression products of 13 genes shown in Table A-5-e given below. In the present invention, for example, preferably, the adult atopic dermatitis marker selected from the group consisting of 210 genes shown in Table A-b or expression products thereof does not include protein markers which are expression products of 9 genes shown in Table A-5-f given below.

**[Table A-5-e]**

| | | | | | |
|---|---|---|---|---|---|
| ANXA1 | CAMP | CARD18 | CRISPLD2 | DYNLL1 | EFHD2 |
| GLRX | GSDMA | KRT23 | KRT25 | LMNA | PSMB4 |
| SFN | | | | | |

**[Table A-5-f]**

| | | | | | |
|---|---|---|---|---|---|
| CARD18 | CRISPLD2 | DYNLL1 | GLRX | GSDMA | KRT23 |
| KRT25 | LMNA | PSMB4 | | | |

The biological sample used in the present invention can be a tissue or a biomaterial in which the expression of the gene of the present invention varies with the development or progression of atopic dermatitis. Examples thereof specifically include organs, the skin, blood, urine, saliva, sweat, stratum corneum, skin surface lipids (SSL), body fluids such as tissue exudates, serum, plasma and others prepared from blood, feces, and hair, and preferably include the skin, stratum corneum, and skin surface lipids (SSL), more preferably skin surface lipids (SSL). Examples of the site of the skin from which SSL is collected include, but are not particularly limited to, the skin at an arbitrary site of the body, such as the head, the face, the neck, the body trunk, and the limbs. A site having high secretion of sebum, for example, the facial skin, is preferred.

The adult test subject from whom the biological sample is collected is preferably a person in need of AD detection or a person suspected of developing AD and is preferably a person at age 16 or over, more preferably a person at age 20 or over, though not limited by sex and age.

### (2. Detection marker for childhood AD and method for detecting childhood AD using same)

The present inventors collected SSL from children having AD and children with healthy skin and no allergic predisposition and exhaustively analyzed the expressed state of RNA contained in the SSL as sequence information, and consequently found that the expression levels of particular genes significantly differ therebetween, and childhood AD can be detected on the basis of this index. Thus, another aspect of the present invention relates to a provision of a marker for detecting childhood AD, and a method for detecting childhood AD using the marker. The present invention enables childhood AD to be conveniently and noninvasively detected early with high accuracy, sensitivity and specificity.

As shown in Examples mentioned later, 61 genes with increased expression and 310 genes with decreased expression (a total of 371 genes (Tables B-1-1 to B-1-9) were identified by extracting RNA which attained a corrected p value (FDR) of less than 0.25 in a likelihood ratio test in children with AD compared with healthy children using normalized count values obtained using DESeq2 in data (read count values) on the expression level of RNA extracted from SSL of 28 healthy children and 25 children with AD. In the tables, genes represented by "UP" are genes whose expression level is increased in children with AD, and genes represented by "DOWN" are genes whose expression level is decreased in children with AD.

Thus, a gene selected from the group of these 371 genes or an expression product thereof is capable of serving as a childhood atopic dermatitis marker for detecting childhood AD. In the gene group, 318 genes (indicated by boldface with * added in Tables B-1-1 to B-1-9) are genes whose relation to AD have not been reported so far.

Feature gene extraction and prediction model construction were attempted using data on the expression level of every SSL-derived RNA (Log₂(RPM + 1) values of 3486 genes) detected from the test subjects as explanatory variables, the healthy children and the childhood AD patients as objective variables, and random forest as machine learning algorithm. As shown in Examples mentioned later, top 100 genes of variable importance based on Gini coefficient (Tables B-3-1 to B-3-3) were selected as feature genes, and childhood AD was found predictable with models using these genes.

Thus, a gene selected from the group of these 100 genes or an expression product thereof is capable of serving as a suitable childhood atopic dermatitis marker for detecting childhood AD. In the gene group, 92 genes (indicated by boldface with * added in Tables B-3-1 to B-3-3) are genes whose relation to AD has not been reported so far, and are thus novel childhood atopic dermatitis markers. As shown in Examples mentioned later, prediction models using these novel childhood atopic dermatitis markers are also capable of predicting childhood AD.

Prediction model construction was similarly attempted using data on the expression levels of the 371 genes described above which were differentially expressed between the healthy children and the children with AD, or 318 gene out of these genes (Log₂(RPM + 1) values), and using random forest. As a result, childhood AD was found predictable in all the cases.

Feature genes were extracted (maximum number of trials: 1,000, p value: less than 0.01) using Boruta method as machine learning algorithm. As a result, 9 genes (Table B-4) were extracted as feature genes. As shown in Examples mentioned later, childhood AD was found predictable with prediction models based on random forest using these genes.

Thus, a gene selected from the group of these 9 genes or an expression product thereof is capable of serving as a childhood atopic dermatitis marker for detecting childhood AD. In the gene group, 7 genes (indicated by boldface with * added in Table B-4) are genes whose relation to AD has not been reported so far, and are thus novel childhood atopic dermatitis markers. As shown in Examples mentioned later, prediction models using these novel childhood atopic dermatitis markers are also capable of predicting childhood AD.

All of 441 genes (Tables B-a-1 and B-a-2) which are the sum (A∪B∪C) of the group of 371 genes (A) shown in Tables B-1-1 to B-1-9 extracted by differential expression analysis, the group of 100 genes (B) shown in Tables B-3-1 to B-3-3 selected as feature genes by random forest, and the group of 9 genes (C) shown in Table B-4 selected as feature genes by Boruta method, as mentioned above, are childhood atopic dermatitis markers. Among them, 383 genes (Tables B-b-1 and B-b-2) are novel childhood atopic dermatitis markers.

**[Table B-a-1]**

| | | | | | |
|---|---|---|---|---|---|
| **DEFB1** | **RNF217** | **LCE2D** | **BNIP3** | **HSPA1B** | **TRIM29** |
| **AGR2** | **CA6** | **THRSP** | **PLA2G4E** | **PTK6** | **DGAT2** |
| **GAL** | **NTAN1** | **NR1D1** | **SLAMF7** | **DUSP16** | **ADIPOR1** |
| **CLU** | **CDKN2B** | **IRGQ** | **LCN2** | **SLPI** | **LCE2A** |
| **SPNS2** | **MARCKS** | **CYB5R1** | **C2orf54** | **FCHSD1** | **BASP1** |
| **H LA-A** | **RMND5B** | **FAM222B** | **PIK3AP1** | **SNX18** | **RASAL1** |
| **DNASE1L2** | **NCCRP1** | **DHCR7** | **ATMIN** | **RASA4CP** | **GIPC1** |
| **MEST** | **SLC15A1** | **CCL3** | **KIAA0513** | **CPEB4** | **CLTB** |
| **HES4** | **GBA2** | **FBXO32** | **GDPD3** | **RAB27A** | **UBIAD1** |
| **FAM108C1** | **SPAG1** | **CDSN** | **FAR2** | **AKTIP** | **BPGM** |
| **KRT79** | **KRT17** | **CARD18** | **KRT80** | **RGP1** | **LPCAT1** |
| **ARLSA** | **H1F0** | **MGST1** | **EPHX3** | **MIEN1** | **RANGAP1** |
| **ALDH3B2** | **RARG** | **WASL** | **LCE2C** | **SCD** | **PRSS22** |
| **CALML3** | **KLK11** | **TEX264** | **DNAJB1** | **VKORC1L1** | **CTSD** |
| **PLCD3** | **KRTAP4-9** | **LCE1C** | **NEDD4L** | **ABTB2** | **HIST3H2A** |
| **OXR1** | **SULT2B1** | **KLK13** | **POR** | **AATK** | **SMS** |
| **UNCSB** | **WIPI2** | **INPPL1** | **IRAK2** | **TUFT1** | **LGALS3** |
| **HSBP1L1** | **RUSC2** | **SORT1** | **KCTD11** | **MEA** | **TBC1D20** |
| **MARCH3** | **SMOX** | **STARDS** | **KRT8** | **HDAC7** | **SERINC2** |
| **ASPRV1** | **GCH1** | **TMEM189** | **SMPD3** | **PHLDA2** | **KCTD20** |
| **CRAT** | **MAPK13** | **A2M** | **CD48** | **TMED3** | **FAM188A** |
| **DMKN** | **MYZAP** | **LY6G6C** | **RSC1A1** | **PRR24** | **ASS1** |
| **PLB1** | **HS3ST6** | **ATP6V1C2** | **PLD3** | **SBSN** | **ZNF664** |
| **CDC34** | **KRTAP12-1** | **LYPDS** | **HN1L** | **HIST1H2BK** | **PPP2CB** |
| **FAM84B** | **PSORS1C2** | **BMP2** | **PGRMC2** | **SURF1** | **GOLGA4** |
| **CTSA** | **CIDEA** | **HIP1R** | **KDSR** | **DUSP14** | **ZRANB1** |
| **TSPAN6** | **DSP** | **S100A16** | **PPDPF** | **FAM214A** | **EHF** |
| **KRTAP5-5** | **C15orf62** | **C1orf21** | **LYPLA1** | **FAM102A** | **TSPAN14** |
| **SEPTS** | **DHCR24** | **KLHL21** | **SDCBP2** | **DNAJCS** | **KEAP1** |
| **MSMO1** | **KRT34** | **GAS7** | **ADIPOR2** | **TBC1D17** | **ABHD5** |
| **RRAD** | **PCDH1** | **LCE1F** | **SSFA2** | **SH3D21** | **NEU1** |
| **CHAC1** | **ZDHHC9** | **PARD6B** | **BCL2L1** | **MPZL3** | **OSBPL2** |
| **SLC40A1** | **GNG12** | **TM4SF1** | **ISG15** | **EPB41** | **RNF103** |
| **NIPAL2** | **CTNNBIP1** | **FOXO3** | **GTPBP2** | **UBAP1** | **FEM1B** |
| **SPTLC3** | **FAM 193 B** | **GDE1** | **DDHD1** | **LRP10** | **RANBP9** |
| **EPN3** | **ID1** | **SH3BP5L** | **GALNT1** | **PAPL** | **LOC100093631** |
| **KLK6** | **KRT86** | **MAL2** | **CRK** | **RALGDS** | **MAP1LC3A** |
| **KLH DC3** | **KRTAP3-1** | **SLC31A1** | **TMEM86A** | **SHB** | **PRDM1** |
| **SCYL1** | **NBR1** | **DBI** | **GPT2** | **PRPF38B** | **CDC42EP1** |
| **NPC1** | **ZFAND5** | **SH3BGRL3** | **PLIN2** | **ATPSH** | **CCM2** |
| **C6orf106** | **HSP90AA1** | **NDUFB11** | **FAM100B** | **BAX** | **RNF24** |
| **USP17L5** | **KIF1C** | **YWHAH** | **YPEL2** | **ALYREF** | **SRPK2** |
| **BNIP3L** | **CERK** | **CALR** | **MAP1LC3B2** | **PRMT1** | **LST1** |
| **EAF1** | **ATP6V1A** | **GSN** | **RLF** | **CTSC** | **INF2** |
| **MIR54811** | **PQLC1** | **SNORA31** | **KIAA0930** | **CYTIP** | **AMD1** |
| **JUP** | **CACUL1** | **CST3** | **UBE2R2** | **SNORA6** | **ITGAM** |
| **PEBP1** | **PRKCD** | **PDIA6** | **HK2** | **U2AF1** | **CAPG** |
| **HMOX1** | **STK10** | **ALDH2** | **USF2** | **VPS13C** | **VKORC1** |
| **CTSB** | **IER3** | **PPIB** | **PDIA3P** | **NBPF10** | **ACSL4** |
| **SQSTM1** | **HECA** | **TUBA1B** | **HNRNPUL1** | **ZNF430** | **CDC123** |
| **VAT1** | **DDIT4** | **ATP5J2** | **SEC61G** | **SPEN** | **SCARNA7** |
| **CYBASC3** | **TOLLIP** | **HLA-DPB1** | **DNAJB11** | **CIB1** | **RNASET2** |
| **EIF4EBP2** | **CHP1** | **RCC2** | **SDHD** | **TMEM33** | **C6orf62** |
| **ATG2A** | **LAMTOR3** | **AIM1** | **NDUFS7** | **NPEPPS** | **SLC39A8** |
| **RAD23B** | **KLF4** | **CSF1R** | **ECH1** | **SEC24D** | **ARHGAP9** |

**[Table B-a-2]**

| | | | | | |
|---|---|---|---|---|---|
| **DSTN** | **KCNQ1OT1** | **SYNGR2** | **CASS4** | **ARHGDIB** | **SCAP** |
| **TPRA1** | **CAST** | **TGFBI** | **IL7R** | **C10orf128** | **TMEM214** |
| **BICD2** | **CHMP5** | **DDOST** | **CLEC4A** | **TXN2** | **AMICA1** |
| **RNF11** | **TNIP1** | **TUBA1A** | **AREG** | **CISH** | **STK17B** |
| **ULK1** | **SIRPA** | **LGALS1** | **SNRPD1** | **YWHAG** | **HNRNPA1** |
| **SYTL1** | **GLRX** | **CD52** | **SLC7A11** | **LAMTOR4** | **TAGLN2** |
| **MGLL** | **NOTCH2NL** | **HLA-DMA** | **SNX8** | **CRCP** | |
| **WBP2** | **SLK** | **CCND2** | **IMPDH2** | **STT3A** | |
| **NUDT4** | **ZFP36L2** | **S100A4** | **ERI1** | **CRISPLD2** | |
| **PIM1** | **RAB21** | **TMX2** | **FBXW2** | **DEFB4B** | |
| **SYPL1** | **EIF5** | **HLA-DOA** | **PYCARD** | **CD93** | |
| **OTUDS** | **PRELID1** | **MMP12** | **CCL17** | **PLIN3** | |
| **IRAK1** | **SQRDL** | **CIITA** | **MED14** | **USMG5** | |
| **UPK3BL** | **SERP1** | **ADAM 19** | **HYOU1** | **LOC285359** | |
| **PTK2B** | **RAB7A** | **ANPEP** | **CTDSP1** | **SLC20A1** | |
| **MAPK3** | **ARF1** | **MAT2A** | **USP16** | **MSL1** | |
| **KRT23** | **NDUFA1** | **MRC1** | **TXNDC17** | **SLC11A2** | |
| **UBXN6** | **ENO1** | **CLEC10A** | **FBXW4** | **KHDRBS1** | |
| **ATP6V0C** | **H2AFY** | **CPVL** | **FBP1** | **CORO1B** | |
| **ZFAND6** | **GNB2L1** | **ATP2A2** | **ZNF91** | **ZFAND2A** | |
| **SIAH2** | **EIF3K** | **ABHD8** | **RBM17** | **DOK2** | |

**[Table B-b-1]**

| | | | | | |
|---|---|---|---|---|---|
| **AGR2** | **H1F0** | **LY6G6C** | **KDSR** | **TBC1D17** | **LOC100093631** |
| **SPNS2** | **RARG** | **ATP6V1C2** | **PPDPF** | **SH3D21** | **MAP1LC3A** |
| **DNASE1L2** | **KRTAP4-9** | **LYPDS** | **LYPLA1** | **MPZL3** | **PRDM1** |
| **MEST** | **SULT2B1** | **BMP2** | **SDCBP2** | **EPB41** | **SCYL1** |
| **HES4** | **WIPI2** | **HIP1R** | **ADIPOR2** | **UBAP1** | **NPC1** |
| **FAM108C1** | **RUSC2** | **S100A16** | **SSFA2** | **LRP10** | **C6orf106** |
| **KRT79** | **SMOX** | **C1orf21** | **ISG15** | **PAPL** | **USP17L5** |
| **ARL5A** | **GCH1** | **KLHL21** | **GTPBP2** | **RALGDS** | **BNIP3L** |
| **ALDH3B2** | **MAPK13** | **GAS7** | **DDHD1** | **TRIM29** | **EAF1** |
| **CALML3** | **MYZAP** | **LCE1F** | **GALNT1** | **ADIPOR1** | **MIR548I1** |
| **PLCD3** | **HS3ST6** | **PARD6B** | **CRK** | **LCE2A** | **JUP** |
| **OXR1** | **KRTAP12-1** | **TM4SF1** | **TMEM86A** | **BASP1** | **PEBP1** |
| **UNCSB** | **CIDEA** | **FOXO3** | **HSPA1B** | **RASAL1** | **CTSB** |
| **HSBP1L1** | **DSP** | **GDE1** | **PTK6** | **GIPC1** | **SQSTM1** |
| **MARCH3** | **C15orf62** | **SH3BP5L** | **DUSP16** | **CLTB** | **VAT1** |
| **CRAT** | **DHCR24** | **MAL2** | **FCHSD1** | **UBIAD1** | **CYBASC3** |
| **PLB1** | **KRT34** | **SLC31A1** | **SNX18** | **BPGM** | **EIF4EBP2** |
| **CDC34** | **ZDHHC9** | **BNIP3** | **RASA4CP** | **LPCAT1** | **ATG2A** |
| **FAM84B** | **GNG12** | **PLA2G4E** | **CPEB4** | **RANGAP1** | **RAD23B** |
| **TSPAN6** | **CTNNBIP1** | **SLAM F7** | **RAB27A** | **PRSS22** | **DSTN** |
| **KRTAP5-5** | **FAM 193 B** | **C2orf54** | **AKTIP** | **CTSD** | **TPRA1** |
| **SEPT5** | **ID1** | **PIK3AP1** | **RGP1** | **HIST3H2A** | **BICD2** |
| **MSMO1** | **KRT86** | **ATMIN** | **MIEN1** | **SMS** | **RNF11** |
| **RRAD** | **KRTAP3-1** | **KIAA0513** | **VKORC1L1** | **TBC1D20** | **ULK1** |
| **CHAC1** | **LCE2D** | **GDPD3** | **ABTB2** | **SERINC2** | **SYTL1** |
| **SLC40A1** | **THRSP** | **KRT80** | **AATK** | **KCTD20** | **MGLL** |
| **NIPAL2** | **NR1D1** | **EPHX3** | **TUFT1** | **FAM188A** | **WBP2** |
| **SPTLC3** | **IRGQ** | **LCE2C** | **MEA** | **ASS1** | **NUDT4** |
| **EPN3** | **CYB5R1** | **DNAJB1** | **HDAC7** | **ZNF664** | **PIM1** |
| **KLH DC3** | **FAM222B** | **NEDD4L** | **PHLDA2** | **PPP2CB** | **SYPL1** |
| **RNF217** | **DHCR7** | **IRAK2** | **TMED3** | **GOLGA4** | **OTUD5** |
| **NTAN1** | **FBXO32** | **KCTD11** | **PRR24** | **ZRANB1** | **IRAK1** |
| **CDKN2B** | **CARD18** | **KRT8** | **HIST1H2BK** | **TSPAN14** | **UPK3BL** |
| **MARCKS** | **MGST1** | **SMPD3** | **SURF1** | **NEU1** | **PTK2B** |
| **RMND5B** | **TEX264** | **RSC1A1** | **DUSP14** | **OSBPL2** | **MAPK3** |
| **NCCRP1** | **LCE1C** | **PLD3** | **FAM214A** | **RNF103** | **KRT23** |
| **GBA2** | **STARD5** | **HN1L** | **FAM102A** | **FEM1B** | **UBXN6** |
| **SPAG1** | **TMEM189** | **PGRMC2** | **DNAJC5** | **RANBP9** | **ATP6V0C** |
| **ZFAND6** | **SNORA31** | **SEC61G** | **SEC24D** | **STK17B** | **H2AFY** |
| **SIAH2** | **CST3** | **DNAJB11** | **ARHGDIB** | **HNRNPA1** | **GNB2L1** |
| **NBR1** | **PDIA6** | **SDHD** | **C10orf128** | **TAGLN2** | **EIF3K** |
| **ZFAND5** | **ALDH2** | **NDUFS7** | **TXN2** | **TNIP1** | **DBI** |
| **HSP90AA1** | **PPIB** | **ECH1** | **YWHAG** | **SIRPA** | **SH3BGRL3** |
| **KIF1C** | **TUBA1B** | **CASS4** | **LAMTOR4** | **GLRX** | **NDUFB11** |
| **CERK** | **ATP5J2** | **CLEC4A** | **CRCP** | **NOTCH2NL** | **YWHAH** |
| **ATP6V1A** | **RCC2** | **SNRPD1** | **STT3A** | **SLK** | **TMX2** |
| **PQLC1** | **AIM1** | **SLC7A11** | **CRISPLD2** | **ZFP36L2** | **HLA-DOA** |
| **CACUL1** | **SYNGR2** | **SNX8** | **DEFB4B** | **RAB21** | **CIITA** |
| **STK10** | **TGFBI** | **IMPDH2** | **CD93** | **EIF5** | **ADAM19** |
| **IER3** | **DDOST** | **ERI1** | **PLIN3** | **PRELID1** | **ANPEP** |
| **DDIT4** | **TUBA1A** | **FBXW2** | **USMG5** | **SQRDL** | **MAT2A** |
| **CHP1** | **CD52** | **MED14** | **LOC285359** | **SERP1** | **CPVL** |
| **LAMTOR3** | **HLA-DMA** | **HYOU1** | **SLC20A1** | **RAB7A** | **ATP2A2** |
| **KCNQ1OT1** | **CCND2** | **CTDSP1** | **MSL1** | **ARF1** | **ABHD8** |
| **CHMP5** | **S100A4** | **USP16** | **SLC11A2** | **NDUFA1** | **GPT2** |

**[Table B-b-2]**

| | | |
|---|---|---|
| **PLIN2** | **TXNDC17** | **CAPG** |
| **FAM100B** | **FBXW4** | **VKORC1** |
| **YPEL2** | **FBP1** | **ACSL4** |
| **MAP1LC3B2** | **ZNF91** | **CDC123** |
| **RLF** | **RBM17** | **SCARNA7** |
| **KIAA0930** | **PRPF38B** | **RNASET2** |
| **UBE2R2** | **ATP5H** | **C6orf62** |
| **HK2** | **BAX** | **SLC39A8** |
| **USF2** | **ALYREF** | **ARHGAP9** |
| **PDIA3P** | **PRMT1** | **TMEM214** |
| **HNRNPUL1** | **CTSC** | **AMICA1** |
| **KHDRBS1** | **CYTIP** | |
| **CORO1B** | **SNORA6** | |
| **ZFAND2A** | **U2AF1** | |
| **CDC42EP1** | **VPS13C** | |
| **CCM2** | **NBPF10** | |
| **RNF24** | **ZNF430** | |
| **SRPK2** | **SPEN** | |
| **LST1** | **CIB1** | |
| **INF2** | **TMEM33** | |
| **AMD1** | **NPEPPS** | |

7 genes consisting of IMPDH2, ERI1, FBXW2, STK17B, TAGLN2, AMICA1 and HNRNPA1 are common genes (BnC) between the group of 100 genes (B) described in Tables B-3-1 to B-3-3 selected as feature genes by random forest, and the group of 9 genes (C) shown in Table B-4 selected as feature genes by Boruta method, as mentioned above, and are genes which have previously not been associated with AD (indicated by boldface with * added in each table). Thus, at least one gene selected from the group of these genes or an expression product thereof is particularly useful as a novel childhood atopic dermatitis marker for detecting childhood AD.

Among them, IMPDH2, ERI1 and FBXW2 are genes (A∩B∩C) also included in the group of 371 genes (A) described in Tables B-1-1 to B-1-9 extracted by differential expression analysis as mentioned above, and are therefore more preferred novel childhood atopic dermatitis markers.

These 7 genes are each capable of serving alone as a childhood atopic dermatitis marker. It is preferred to use 2 or more, preferably 4 or more, more preferably 6 or more of these genes in combination, and it is even more preferred to use all the 7 genes in combination.

23 genes consisting of ABHD8, GPT2, PLIN2, FAM100B, YPEL2, MAP1LC3B2, RLF, KIAA0930, UBE2R2, HK2, USF2, PDIA3P, HNRNPUL1, SEC61G, DNAJB11, SDHD, NDUFS7, ECH1, CASS4, CLEC4A, SNRPD1, SLC7A11 and SNX8 are included in common moieties between the group of 371 genes (A) described in Tables B-1-1 to B-1-9 extracted by differential expression analysis and the group of 100 genes (B) described in Tables B-3-1 to B-3-3 selected as feature genes by random forest, as mentioned above, and are genes whose relation to AD has previously not been reported except for the genes IMPDH2, ERI1 and FBXW2. Thus, at least one gene selected from the group of these genes or an expression product thereof is also useful as a novel childhood atopic dermatitis marker for detecting childhood AD.

The method for detecting childhood AD according to the present invention includes a step of measuring an expression level of a target gene which is, in one aspect, at least one gene selected from the group of 7 genes consisting of IMPDH2, ERI1, FBXW2, STK17B, TAGLN2, AMICA1 and HNRNPA1 or an expression product thereof in a biological sample collected from a test subject.

Alternatively, a discriminant (prediction model) which discriminates between a child with AD and a healthy child is constructed using measurement values of an expression level of the target gene or the expression product thereof derived from a child with AD and an expression level of the target gene or the expression product thereof derived from a healthy child, and childhood AD can be detected through the use of the discriminant. Thus, a prediction model capable of predicting childhood AD can be constructed by using 7 genes consisting of IMPDH2, ERI1, FBXW2, STK17B, TAGLN2, AMICA1 and HNRNPA1, and 100 genes shown in Tables B-3-1 to B-3-3 or 9 genes shown in Table B-4, including the 7 genes, or 371 genes shown in Tables B-1-1 to B-1-9 as feature genes.

In the case of preparing the discriminant which discriminates between a children group with childhood AD and a healthy children group, one or more, preferably 5 or more, more preferably all the 7 genes are selected as target genes from the group of 7 genes consisting of IMPDH2, ERI1, FBXW2, STK17B, TAGLN2, AMICA1 and HNRNPA1, and expression data on the gene(s) or expression product(s) thereof is used. In the case of selecting a plurality of genes, it is preferred to prepare the discriminant by selecting genes in a higher rank of variable importance in Tables B-3-1 to B-3-3 of these genes in order as feature genes. Further, childhood AD may be detected according to a discriminant prepared by appropriately adding, to the expression data on the 7 genes, expression data on at least one, 5 or more, 10 or more, 20 or more or 50 or more genes or expression products thereof selected from the group consisting of genes other than the 7 genes among 441 genes shown in Table B-a described above, 100 genes shown in Tables B-3-1 to B-3-3, 9 genes shown in Table B-4, or 371 genes shown in Tables B-1-1 to B-1-9. In the case of selecting gene(s) other than the 7 genes from the group consisting of 100 genes shown in Tables B-3-1 to B-3-3, the feature genes may be selected from the group consisting of genes in a higher rank of variable importance in order or from the group consisting of genes within top 50, preferably top 30 genes of variable importance. In the case of selecting gene(s) other than the 7 genes as feature genes, it is preferred to select feature genes from the group consisting of novel atopic dermatitis markers indicated by boldface with * added in Tables B-1-1 to B-1-9, Tables B-3-1 to B-3-3 and Table B-4.

In the case of adding 371 genes shown in B-1-1 to B-1-9, the discriminant may be prepared by appropriately adding expression data on at least one gene selected from the group of 25 genes consisting of ABHD8, GPT2, PLIN2, FAM100B, YPEL2, MAP1LC3B2, RLF, KIAA0930, UBE2R2, HK2, USF2, PDIA3P, HNRNPUL1, SEC61G, DNAJB11, SDHD, NDUFS7, ECH1, CASS4, IL7R, CLEC4A, AREG, SNRPD1, SLC7A11 and SNX8 among the 371 genes, preferably at least one, 5 or more, 10 or more, or 20 or more genes with higher variable importance among these genes in Tables B-3-1 to B-3-3, or expression products thereof, in addition to the 7 genes consisting of IMPDH2, ERI1, FBXW2, STK17B, TAGLN2, AMICA1 and HNRNPA1, as target genes. These 25 genes are genes included in common moieties between the group of 371 genes (A) described in Tables B-1-1 to B-1-9 extracted by differential expression analysis and the group of 100 genes (B) described in Tables B-3-1 to B-3-3 selected as feature genes by random forest, as mentioned above.

Preferably, the discriminant using the 7 genes, 371 genes or 318 genes (indicated by boldface with * added in Tables B-1-1 to B-1-9) shown in Tables B-1-1 to B-1-9, 100 genes or 92 genes (indicated by boldface with * added in Tables B-3-1 to B-3-3) shown in Tables B-3-1 to B-3-3, or 9 genes shown in Table B-4 as feature genes can be mentioned.

More preferably, the discriminant using the 7 genes, 100 genes or 92 genes (indicated by boldface with * added in Tables B-3-1 to B-3-3) shown in Tables B-3-1 to B-3-3, or 9 genes shown in Table B-4 as feature genes can be mentioned.

The biological sample used in the present invention can be a tissue or a biomaterial in which the expression of the gene of the present invention varies with the development or progression of atopic dermatitis. Examples thereof specifically include organs, the skin, blood, urine, saliva, sweat, stratum corneum, skin surface lipids (SSL), body fluids such as tissue exudates, serum, plasma and others prepared from blood, feces, and hair, and preferably include the skin, stratum corneum, and skin surface lipids (SSL), more preferably skin surface lipids (SSL). Examples of the site of the skin from which SSL is collected include, but are not particularly limited to, the skin at an arbitrary site of the body, such as the head, the face, the neck, the body trunk, and the limbs. A site having high secretion of sebum, for example, the facial skin, is preferred.

The test subject from whom the biological sample is collected is not particularly limited by sex, race, and the like, as long as the test subject is a child. A child in need of AD detection or a child suspected of developing AD is preferred.

In the present invention, preferably, the childhood atopic dermatitis marker selected from the group consisting of 316 genes indicated by boldface with * added in Tables B-1-1 to B-1-9 or expression products thereof does not include 46 genes shown in Table B-5-a given below.

In the present invention, preferably, the childhood atopic dermatitis marker selected from the group consisting of 383 genes shown in Tables B-b-1 and B-b-2 or expression products thereof does not include 46 genes shown in Table B-5-a given below.

**[Table B-5-a]**

| | | | | | |
|---|---|---|---|---|---|
| ABTB2 | AGR2 | ASS1 | BMP2 | C15orf62 | CDC34 |
| CHAC1 | DHCR24 | FAM84B | FBXO32 | GDE1 | HIST3H2A |
| HS3ST6 | HSBP1L1 | IER3 | KCNQ1OT1 | KCTD11 | KRT8 |
| KRTAP12-1 | KRTAP5-5 | LCE1C | LCE1F | LCE2A | LCE2C |
| LCE2D | LY6G6C | LYPLA1 | MAL2 | MAPK13 | MGST1 |
| MIR548I1 | NCCRP1 | NEDD4L | NR1D1 | PARD6B | PLA2G4E |
| PLCD3 | PPDPF | RSC1A1 | SERINC2 | SLC40A1 | SMS |
| TMEM189 | UBAP1 | USP17L5 | WIPI2 | | |

Alternatively or additionally, in the present invention, preferably, the childhood atopic dermatitis marker selected from the group consisting of 441 genes shown in Tables B-a-1 and B-a-2 or expression products thereof does not include a protein marker which is an expression product of at least one gene selected from the group of 37 genes shown in Table B-5-b given below.

Alternatively or additionally, in the present invention, preferably, the childhood atopic dermatitis marker selected from the group consisting of 383 genes shown in Tables B-b-1 and B-b-2 or expression products thereof does not include a protein marker which is an expression product of at least one gene selected from the group of 22 genes shown in Table B-5-c given below.

**[Table B-5-b]**

| | | | | | |
|---|---|---|---|---|---|
| A2M | ARHGDIB | ASPRV1 | CALR | CAPG | CARD18 |
| CRISPLD2 | CTSA | DBI | DNAJB1 | DSP | ENO1 |
| GLRX | GSN | HLA-DPB1 | ITGAM | JUP | KLK13 |
| KLK6 | KRT23 | KRT79 | LCN2 | LGALS1 | LGALS3 |
| LY6G6C | NCCRP1 | PDIA6 | PLD3 | PPIB | PYCARD |
| RAB27A | SBSN | SYNGR2 | TAGLN2 | TRIM29 | YWHAG |
| YWHAH | | | | | |

**[Table B-5-c]**

| | | | | | |
|---|---|---|---|---|---|
| ARHGDIB | CAPG | CARD18 | CRISPLD2 | DBI | DNAJB1 |
| DSP | GLRX | JUP | KRT23 | KRT79 | LY6G6C |
| NCCRP1 | PDIA6 | PLD3 | PPIB | RAB27A | SYNGR2 |
| TAGLN2 | TRIM29 | YWHAG | YWHAH | | |

### (3. Protein marker for detecting AD and method for detecting AD using same)

The present inventors further found that SSL contains proteins useful for the detection of AD. These proteins can be used as protein markers for detecting AD. A biological sample for detecting AD in a test subject and a protein marker contained therein can be collected by a convenient and low invasive or noninvasive approach of collecting SSL from the skin surface of the test subject.

Thus, a further alternative aspect of the present invention relates to a method for low invasively or noninvasively preparing a protein marker for detecting AD from a test subject, and a method for detecting AD using the protein marker. According to the present invention, a protein marker for detecting AD can be collected from a test subject by a convenient and low invasive or noninvasive approach, or AD can be detected using the marker. Thus, the present invention enables AD to be diagnosed in various test subjects including children, in whom collection of a biological sample in an invasive manner was not easy. Furthermore, the method of the present invention is capable of contributing to the early diagnosis and treatment of childhood and adult AD.

Thus, in one aspect, the present invention provides a protein marker for detecting AD. In another aspect, the present invention provides a method for preparing a protein marker for detecting AD. The method includes collecting a target protein marker for detecting AD from SSL collected from a test subject. In an alternative aspect, the present invention provides a method for detecting AD. The method includes detecting the protein marker for detecting AD from SSL collected from a test subject.

As shown in Examples mentioned later, 418 SSL-derived proteins shown in Tables C-1-1 to C-1-13 are proteins whose abundance in SSL significantly differs in AD patients compared with healthy subjects. A prediction model constructed by machine learning using the abundances of these proteins in SSL as features is capable of predicting AD. Thus, the SSL-derived proteins shown in Tables C-1-1 to C-1-13 can be used as protein markers for AD detecting. Among the proteins shown in Tables C-1-1 to C-1-13, 147 proteins shown in Tables C-2-1 to C-2-5 are, as shown in Examples mentioned later, novel protein markers for detecting AD whose relation to AD has not been reported so far. More specifically, the SSL-derived proteins shown in Tables C-1-1 to C-1-13 include 200 proteins shown in Tables C-4-1 to C-4-6 and 283 proteins shown in Tables C-5-1 to C-5-9, as mentioned later.

65 proteins shown in Tables C-3-1 to C-3-2 are common proteins between the proteins shown in Tables C-4-1 to C-4-6 and the proteins shown in Tables C-5-1 to C-5-9, as mentioned later, and can be preferably used as protein markers for detecting AD.

**[Table C-1-1]**

| Gene name | Protein name |
|---|---|
| A1BG | Alpha-1B-glycoprotein |
| A2M | Alpha-2-macroglobulin |
| ACP5 | Tartrate-resistant acid phosphatase type 5 |
| ACTB | Actin, cytoplasmic 1 |
| ACTR2 | Actin-related protein 2 |
| AFM | Afamin |
| AGRN | Agrin |
| AGT | Angiotensinogen |
| AHNAK | Neuroblast differentiation-associated protein AHNAK |
| AHSG | Alpha-2-HS-glycoprotein |
| AKR1A1 | Aldo-keto reductase family 1 member A1 |
| ALB | Serum albumin |
| ALDH3A1 | Aldehyde dehydrogenase, dimeric NADP-preferring |
| ALDOA | Fructose-bisphosphate aldolase A |
| AMBP | Protein AMBP |
| ANXA1 | Annexin A1 |
| ANXA11 | Annexin A11 |
| ANXA2 | Annexin A2 |
| ANXA3 | Annexin A3 |
| ANXA6 | Annexin A6 |
| APCS | Serum amyloid P-component |
| APOA1 | Apolipoprotein A-I |
| APOA2 | Apolipoprotein A-II |
| APOB | Apolipoprotein B-100 |
| APOC1 | Apolipoprotein C-I |
| APOH | Beta-2-glycoprotein 1 |
| ARF6 | ADP-ribosylation factor 6 |
| ARHGDIB | Rho GDP-dissociation inhibitor 2 |
| ARPC2 | Actin-related protein 2/3 complex subunit 2 |
| ARPC3 | Actin-related protein 2/3 complex subunit 3 |
| ASPRV1 | Retroviral-like aspartic protease 1 |
| ATP1B1 | Sodium/potassium-transporting ATPase subunit beta-1 |
| ATP5PO | ATP synthase subunit O, mitochondrial |
| AZGP1 | Zinc-alpha-2-glycoprotein |

**[Table C-1-2]**

| Gene name | Protein name |
|---|---|
| AZU1 | Azurocidin |
| B2M | Beta-2-microglobulin |
| BPI | Bactericidal permeability-increasing protein |
| BST1 | ADP-ribosyl cyclase/cyclic ADP-ribose hydrolase 2 |
| BTF3 | Transcription factor BTF3 |
| C1QA | Complement C1q subcomponent subunit A |
| C1QC | Complement C1q subcomponent subunit C |
| C1S | Complement C1s subcomponent |
| C3 | Complement C3 |
| C4A | Complement C4-A |
| C4BPA | C4b-binding protein alpha chain |
| C7 | Complement component C7 |
| CA2 | Carbonic anhydrase 2 |
| CALR | Calreticulin |
| CAMP | Cathelicidin antimicrobial peptide |
| CANX | Calnexin |
| CAP1 | Adenylyl cyclase-associated protein 1 |
| CAPG | Macrophage-capping protein |
| CAPZA1 | F-actin-capping protein subunit alpha-1 |
| CARD18 | Caspase recruitment domain-containing protein 18 |
| CASP14 | Caspase-14 |
| CBR1 | Carbonyl reductase [NADPH] 1 |
| CCAR2 | Cell cycle and apoptosis regulator protein 2 |
| CCT3 | T-complex protein 1 subunit gamma |
| CCT6A | T-complex protein 1 subunit zeta |
| CDC42 | Cell division control protein 42 homolog |
| CDH23 | Cadherin-23 |
| CEACAM5 | Carcinoembryonic antigen-related cell adhesion molecule 5 |
| CFB | Complement factor B |
| CFH | Complement factor H |
| CFI | Complement factor I |
| CFL1 | Cofilin-1 |
| CKMT1A | Creatine kinase U-type, mitochondrial |
| CLEC3B | Tetranectin |

**[Table C-1-3]**

| Gene name | Protein name |
|---|---|
| CLIC1 | Chloride intracellular channel protein 1 |
| CORO1A | Coronin-1A |
| COTL1 | Coactosin-like protein |
| CP | Ceruloplasmin |
| CPNE3 | Copine-3 |
| CPQ | Carboxypeptidase Q |
| CRISP3 | Cysteine-rich secretory protein 3 |
| CRISPLD2 | Cysteine-rich secretory protein LCCL domain-containing 2 |
| CRNN | Cornulin |
| CTSA | Lysosomal protective protein |
| CTSG | Cathepsin G |
| DAG1 | Dystroglycan |
| DBI | Acyl-CoA-binding protein |
| DCD | Dermcidin |
| DDB1 | DNA damage-binding protein 1 |
| DDX10 | Probable ATP-dependent RNA helicase DDX10 |
| DDX55 | ATP-dependent RNA helicase DDX55 |
| DEFA3 | Neutrophil defensin 3 |
| DERA | Deoxyribose-phosphate aldolase |
| DHRS11 | Dehydrogenase/reductase SDR family member 11 |
| DHX36 | ATP-dependent DNA/RNA helicase DHX36 |
| DLD | Dihydrolipoyl dehydrogenase, mitochondrial |
| DNAAF1 | Dynein assembly factor 1, axonemal |
| DNAJB1 | DnaJ homolog subfamily B member 1 |
| DSC1 | Desmocollin-1 |
| DSC3 | Desmocollin-3 |
| DSP | Desmoplakin |
| DYNLL1 | Dynein light chain 1, cytoplasmic |
| ECM1 | Extracellular matrix protein 1 |
| EEF1A1 | Elongation factor 1-alpha 1 |
| EEF2 | Elongation factor 2 |
| EFHD2 | EF-hand domain-containing protein D2 |
| EFNA1 | Ephrin-A1 |
| EIF3I | Eukaryotic translation initiation factor 3 subunit I |

**[Table C-1-4]**

| Gene name | Protein name |
|---|---|
| EIF4A2 | Eukaryotic initiation factor 4A-II |
| EIF5A | Eukaryotic translation initiation factor 5A-1 |
| EIF6 | Eukaryotic translation initiation factor 6 |
| ELANE | Neutrophil elastase |
| ENO1 | Alpha-enolase |
| EPPK1 | Epiplakin |
| EPS8L1 | Epidermal growth factor receptor kinase substrate 8-like protei n 1 |
| EPX | Eosinophil peroxidase |
| ERP29 | Endoplasmic reticulum resident protein 29 |
| EVPL | Envoplakin |
| EZR | Ezrin |
| F2 | Prothrombin |
| F5 | Coagulation factor V |
| FABP5 | Fatty acid-binding protein 5 |
| FAU | 40S ribosomal protein S30 |
| FBXO6 | F-box only protein 6 |
| FGA | Fibrinogen alpha chain |
| FGB | Fibrinogen beta chain |
| FGG | Fibrinogen gamma chain |
| FLG2 | Filaggrin-2 |
| FLNB | Filamin-B |
| FN1 | Fibronectin |
| G6PD | Glucose-6-phosphate 1-dehydrogenase |
| GARS1 | Glycine--tRNA ligase |
| GART | Trifunctional purine biosynthetic protein adenosine-3 |
| GBA | Lysosomal acid glucosylceramidase |
| GC | Vitamin D-binding protein |
| GCA | Grancalcin |
| GDI2 | Rab GDP dissociation inhibitor beta |
| GLRX | Glutaredoxin-1 |
| GM2A | Ganglioside GM2 activator |
| GMPR2 | GMP reductase 2 |
| GNAI2 | Guanine nucleotide-binding protein G |
| GPI | Glucose-6-phosphate isomerase |

**[Table C-1-5]**

| Gene name | Protein name |
|---|---|
| GPLD1 | Phosphatidylinositol-glycan-specific phospholipase D |
| GPT | Alanine aminotransferase 1 |
| GSDMA | Gasdermin-A |
| GSN | Gelsolin |
| GSTP1 | Glutathione S-transferase P |
| H1-0 | Histone H1.0 |
| H1-3 | Histone H1.3 |
| H1-5 | Histone H1.5 |
| H2AC11 | Histone H2A type 1 |
| H2AC4 | Histone H2A type 1-B/E |
| H2AZ1 | Histone H2A.Z |
| H2BC12 | Histone H2B type 1-K |
| H3C1 | Histone H3.1 |
| H4C1 | Histone H4 |
| HBA1 | Hemoglobin subunit alpha |
| HBB | Hemoglobin subunit beta |
| HK3 | Hexokinase-3 |
| HLA-DPB1 | HLA class II histocompatibility antigen, DP beta 1 chain |
| HLA-DRB1 | HLA class II histocompatibility antigen, DRB1 beta chain |
| HM13 | Minor histocompatibility antigen H13 |
| HMGA1 | High mobility group protein HMG-I/HMG-Y |
| HMGB1 | High mobility group protein B1 |
| HMGB2 | High mobility group protein B2 |
| HNRNPA2B1 | Heterogeneous nuclear ribonucleoproteins A2/B1 |
| HNRNPD | Heterogeneous nuclear ribonucleoprotein D0 |
| HNRNPK | Heterogeneous nuclear ribonucleoprotein K |
| HNRNPR | Heterogeneous nuclear ribonucleoprotein R |
| HP | Haptoglobin |
| HPX | Hemopexin |
| HRG | Histidine-rich glycoprotein |
| HSD17B4 | Peroxisomal multifunctional enzyme type 2 |
| HSPA1A | Heat shock 70 kDa protein 1A |
| HSPA5 | Endoplasmic reticulum chaperone BiP |
| HSPA9 | Stress-70 protein, mitochondrial |

**[Table C-1-6]**

| Gene name | Protein name |
|---|---|
| HSPB1 | Heat shock protein beta-1 |
| HSPE1 | 10 kDa heat shock protein, mitochondrial |
| IDH2 | Isocitrate dehydrogenase [NADP], mitochondrial |
| IGHG1 | Immunoglobulin heavy constant gamma 1 |
| IGHG2 | Immunoglobulin heavy constant gamma 2 |
| IGHG3 | Immunoglobulin heavy constant gamma 3 |
| IGHG4 | Immunoglobulin heavy constant gamma 4 |
| IGHM | Immunoglobulin heavy constant mu |
| IGHV1-46 | Immunoglobulin heavy variable 1-46 |
| IGHV3-30 | Immunoglobulin heavy variable 3-30 |
| IGHV3-33 | Immunoglobulin heavy variable 3-33 |
| IGHV3-7 | Immunoglobulin heavy variable 3-7 |
| IGKC | Immunoglobulin kappa constant |
| IGKV1-5 | Immunoglobulin kappa variable 1-5 |
| IGKV3-11 | Immunoglobulin kappa variable 3-11 |
| IGKV3-20 | Immunoglobulin kappa variable 3-20 |
| IGKV4-1 | Immunoglobulin kappa variable 4-1 |
| IGLV1-51 | Immunoglobulin lambda variable 1-51 |
| IL36G | Interleukin-36 gamma |
| IMPA2 | Inositol monophosphatase 2 |
| ITGAM | Integrin alpha-M |
| ITGB2 | Integrin beta-2 |
| ITIH1 | Inter-alpha-trypsin inhibitor heavy chain H1 |
| ITIH2 | Inter-alpha-trypsin inhibitor heavy chain H2 |
| ITIH4 | Inter-alpha-trypsin inhibitor heavy chain H4 |
| JCHAIN | Immunoglobulin J chain |
| JUP | Junction plakoglobin |
| KLK10 | Kallikrein-10 |
| KLK13 | Kallikrein-13 |
| KLK6 | Kallikrein-6 |
| KLK7 | Kallikrein-7 |
| KLK9 | Kallikrein-9 |
| KLKB1 | Plasma kallikrein |
| KNG1 | Kininogen-1 |

**[Table C-1-7]**

| Gene name | Protein name |
|---|---|
| KRT13 | Keratin, type I cytoskeletal 13 |
| KRT15 | Keratin, type I cytoskeletal 15 |
| KRT23 | Keratin, type I cytoskeletal 23 |
| KRT25 | Keratin, type I cytoskeletal 25 |
| KRT77 | Keratin, type II cytoskeletal 1b |
| KRT79 | Keratin, type II cytoskeletal 79 |
| KRTAP2-3 | Keratin-associated protein 2-3 |
| KV310 | Ig kappa chain V-III region VH |
| LACRT | Extracellular glycoprotein lacritin |
| LAMP2 | Lysosome-associated membrane glycoprotein 2 |
| LCN1 | Lipocalin-1 |
| LCN15 | Lipocalin-15 |
| LCN2 | Neutrophil gelatinase-associated lipocalin |
| LCP1 | Plastin-2 |
| LDHA | L-lactate dehydrogenase A chain |
| LGALS1 | Galectin-1 |
| LGALS3 | Galectin-3 |
| LGALS7 | Galectin-7 |
| LGALSL | Galectin-related protein |
| LMNA | Prelamin-A/C |
| LPO | Lactoperoxidase |
| LRG1 | Leucine-rich alpha-2-glycoprotein |
| LTF | Lactotransferrin |
| LY6G6C | Lymphocyte antigen 6 complex locus protein G6c |
| LYZ | Lysozyme C |
| MACROH2A1 | Core histone macro-H2A.1 |
| MAST4 | Microtubule-associated serine/threonine-protein kinase 4 |
| MDH2 | Malate dehydrogenase, mitochondrial |
| ME1 | NADP-dependent malic enzyme |
| MGST2 | Microsomal glutathione S-transferase 2 |
| MIF | Macrophage migration inhibitory factor |
| MMGT1 | Membrane magnesium transporter 1 |
| MMP9 | Matrix metalloproteinase-9 |
| MNDA | Myeloid cell nuclear differentiation antigen |

**[Table C-1-8]**

| Gene name | Protein name |
|---|---|
| MPO | Myeloperoxidase |
| MSLN | Mesothelin |
| MSN | Moesin |
| MTAP | S-methyl-5'-thioadenosine phosphorylase |
| MUC5AC | Mucin-5AC |
| MUCL1 | Mucin-like protein 1 |
| MYH1 | Myosin-1 |
| MYH14 | Myosin-14 |
| MYH9 | Myosin-9 |
| MYL12B | Myosin regulatory light chain 12B |
| MYL6 | Myosin light polypeptide 6 |
| NAMPT | Nicotinamide phosphoribosyltransferase |
| NAPA | Alpha-soluble NSF attachment protein |
| NCCRP1 | F-box only protein 50 |
| NDUFB6 | NADH dehydrogenase [ubiquinone] 1 beta subcomplex subunit 6 |
| NME1 | Nucleoside diphosphate kinase A |
| NME2 | Nucleoside diphosphate kinase B |
| NPC2 | NPC intracellular cholesterol transporter 2 |
| OPRPN | Opiorphin prepropeptide |
| ORM1 | Alpha-1-acid glycoprotein 1 |
| P4HB | Protein disulfide-isomerase |
| PCBP1 | Poly(rC)-binding protein 1 |
| PDIA3 | Protein disulfide-isomerase A3 |
| PDIA6 | Protein disulfide-isomerase A6 |
| PFN1 | Profilin-1 |
| PGAM1 | Phosphoglycerate mutase 1 |
| PGK1 | Phosphoglycerate kinase 1 |
| PHB2 | Prohibitin-2 |
| PI3 | Elafin |
| PKM | Pyruvate kinase PKM |
| PLD3 | 5'-3' exonuclease PLD3 |
| PLEC | Plectin |
| PLG | Plasminogen |
| PLS3 | Plastin-3 |

**[Table C-1-9]**

| Gene name | Protein name |
|---|---|
| PLTP | Phospholipid transfer protein |
| PNP | Purine nucleoside phosphorylase |
| POF1B | Protein POF1B |
| POLR3A | DNA-directed RNA polymerase III subunit RPC1 |
| POM121 | Nuclear envelope pore membrane protein POM 121 |
| PON1 | Serum paraoxonase/arylesterase 1 |
| PPIA | Peptidyl-prolyl cis-trans isomerase A |
| PPIB | Peptidyl-prolyl cis-trans isomerase B |
| PPL | Periplakin |
| PRDX2 | Peroxiredoxin-2 |
| PRDX6 | Peroxiredoxin-6 |
| PRR4 | Proline-rich protein 4 |
| PRSS27 | Serine protease 27 |
| PSMA1 | Proteasome subunit alpha type-1 |
| PSMB1 | Proteasome subunit beta type-1 |
| PSMB2 | Proteasome subunit beta type-2 |
| PSMB3 | Proteasome subunit beta type-3 |
| PSMB4 | Proteasome subunit beta type-4 |
| PSMB5 | Proteasome subunit beta type-5 |
| PSMD14 | 26S proteasome non-ATPase regulatory subunit 14 |
| PSME2 | Proteasome activator complex subunit 2 |
| PYCARD | Apoptosis-associated speck-like protein containing a CARD |
| PYGL | Glycogen phosphorylase, liver form |
| RAB10 | Ras-related protein Rab-10 |
| RAB1A | Ras-related protein Rab-1A |
| RAB1B | Ras-related protein Rab-1B |
| RAB27A | Ras-related protein Rab-27A |
| RAC2 | Ras-related C3 botulinum toxin substrate 2 |
| RAD9B | Cell cycle checkpoint control protein RAD9B |
| RALY | RNA-binding protein Raly |
| RAN | GTP-binding nuclear protein Ran |
| RANBP1 | Ran-specific GTPase-activating protein |
| RARRES1 | Retinoic acid receptor responder protein 1 |
| RDH12 | Retinol dehydrogenase 12 |

**[Table C-1-10]**

| Gene name | Protein name |
|---|---|
| RECQL | ATP-dependent DNA helicase Q1 |
| REEP5 | Receptor expression-enhancing protein 5 |
| RETN | Resistin |
| RNASE3 | Eosinophil cationic protein |
| RP1BL | Ras-related protein Rap-1b-like protein |
| RPL10A | 60S ribosomal protein L10a |
| RPL12 | 60S ribosomal protein L12 |
| RPL13 | 60S ribosomal protein L13 |
| RPL14 | 60S ribosomal protein L14 |
| RPL15 | 60S ribosomal protein L15 |
| RPL18A | 60S ribosomal protein L18a |
| RPL22 | 60S ribosomal protein L22 |
| RPL26 | 60S ribosomal protein L26 |
| RPL29 | 60S ribosomal protein L29 |
| RPL30 | 60S ribosomal protein L30 |
| RPL31 | 60S ribosomal protein L31 |
| RPL4 | 60S ribosomal protein L4 |
| RPL5 | 60S ribosomal protein L5 |
| RPL6 | 60S ribosomal protein L6 |
| RPL7 | 60S ribosomal protein L7 |
| RPL8 | 60S ribosomal protein L8 |
| RPS11 | 40S ribosomal protein S11 |
| RPS13 | 40S ribosomal protein S13 |
| RPS14 | 40S ribosomal protein S14 |
| RPS16 | 40S ribosomal protein S16 |
| RPS17 | 40S ribosomal protein S17 |
| RPS19 | 40S ribosomal protein S19 |
| RPS23 | 40S ribosomal protein S23 |
| RPS25 | 40S ribosomal protein S25 |
| RPS27A | Ubiquitin-40S ribosomal protein S27a |
| RPS6 | 40S ribosomal protein S6 |
| RPS9 | 40S ribosomal protein S9 |
| RPSA | 40S ribosomal protein SA |
| RTCB | RNA-splicing ligase RtcB homolog |

**[Table C-1-11]**

| Gene name | Protein name |
|---|---|
| S100A10 | Protein S100-A10 |
| S100A11 | Protein S100-A11 |
| S100A14 | Protein S100-A14 |
| S100A6 | Protein S100-A6 |
| S100A7 | Protein S100-A7 |
| S100A8 | Protein S100-A8 |
| SAMD4A | Protein Smaug homolog 1 |
| SBSN | Suprabasin |
| SCEL | Sciellin |
| SCGB1D2 | Secretoglobin family 1D member 2 |
| SCGB2A1 | Mammaglobin-B |
| SCGB2A2 | Mammaglobin-A |
| SEPTIN8 | Septin-8 |
| SEPTIN9 | Septin-9 |
| SERBP1 | Plasminogen activator inhibitor 1 RNA-binding protein |
| SERPINA1 | Alpha-1-antitrypsin |
| SERPINA3 | Alpha-1-antichymotrypsin |
| SERPINA4 | Kallistatin |
| SERPINB1 | Leukocyte elastase inhibitor |
| SERPINB13 | Serpin B13 |
| SERPINB3 | Serpin B3 |
| SERPINB4 | Serpin B4 |
| SERPINB5 | Serpin B5 |
| SERPINC1 | Antithrombin-III |
| SERPIND1 | Heparin cofactor 2 |
| SERPINF1 | Pigment epithelium-derived factor |
| SERPINF2 | Alpha-2-antiplasmin |
| SERPING1 | Plasma protease C1 inhibitor |
| SFN | 14-3-3 protein sigma |
| SFPQ | Splicing factor, proline- and glutamine-rich |
| SLURP2 | Secreted Ly-6/uPAR domain-containing protein 2 |
| SNRPD3 | Small nuclear ribonucleoprotein Sm D3 |
| SPRR1B | Cornifin-B |
| SPRR2D | Small proline-rich protein 2D |

**[Table C-1-12]**

| Gene name | Protein name |
|---|---|
| SPRR2F | Small proline-rich protein 2F |
| SRSF2 | Serine/arginine-rich splicing factor 2 |
| SRSF3 | Serine/arginine-rich splicing factor 3 |
| STS | Steryl-sulfatase |
| SUB1 | Activated RNA polymerase II transcriptional coactivator p15 |
| SUMO3 | Small ubiquitin-related modifier 3 |
| SYNGR2 | Synaptogyrin-2 |
| TACSTD2 | Tumor-associated calcium signal transducer 2 |
| TAGLN2 | Transgelin-2 |
| TALDO1 | Transaldolase |
| TASOR2 | Protein TASOR 2 |
| TF | Serotransferrin |
| TGM1 | Protein-glutamine gamma-glutamyltransferase K |
| THBS1 | Thrombospondin-1 |
| TIMP1 | Metalloproteinase inhibitor 1 |
| TIMP2 | Metalloproteinase inhibitor 2 |
| TKT | Transketolase |
| TMED5 | Transmembrane emp24 domain-containing protein 5 |
| TMSL3 | Thymosin beta-4-like protein 3 |
| TNNI3K | Serine/threonine-protein kinase TNNI3K |
| TPD52L2 | Tumor protein D54 |
| TPM3 | Tropomyosin alpha-3 chain |
| TPP1 | Tripeptidyl-peptidase 1 |
| TPT1 | Translationally-controlled tumor protein |
| TRIM29 | Tripartite motif-containing protein 29 |
| TTR | Transthyretin |
| TUBB | Tubulin beta chain |
| TUBB2A | Tubulin beta-2A chain |
| TUBB4B | Tubulin beta-4B chain |
| UBE2N | Ubiquitin-conjugating enzyme E2 N |
| UGP2 | UTP--glucose-1-phosphate uridylyltransferase |
| VDAC1 | Voltage-dependent anion-selective channel protein 1 |
| VIM | Vimentin |
| VSIG10L | V-set and immunoglobulin domain-containing protein 10-like |

**[Table C-1-13]**

| Gene name | Protein name |
|---|---|
| VTN | Vitronectin |
| WDR1 | WD repeat-containing protein 1 |
| WFDC12 | WAP four-disulfide core domain protein 12 |
| WFDC5 | WAP four-disulfide core domain protein 5 |
| YWHAE | 14-3-3 protein epsilon |
| YWHAG | 14-3-3 protein gamma |
| YWHAH | 14-3-3 protein eta |
| YWHAZ | 14-3-3 protein zeta/delta |
| ZN F236 | Zinc finger protein 236 |
| ZNF292 | Zinc finger protein 292 |

**[Table C-2-1]**

| Gene name | Protein name |
|---|---|
| CCAR2 | Cell cycle and apoptosis regulator protein 2 |
| CKMT1A | Creatine kinase U-type, mitochondrial |
| DDX10 | Probable ATP-dependent RNA helicase DDX10 |
| DDX55 | ATP-dependent RNA helicase DDX55 |
| DYNLL1 | Dynein light chain 1, cytoplasmic |
| EIF3I | Eukaryotic translation initiation factor 3 subunit I |
| EIF5A | Eukaryotic translation initiation factor 5A-1 |
| GMPR2 | GMP reductase 2 |
| H1-0 | Histone H1.0 |
| H2AC4 | Histone H2A type 1-B/E |
| HNRNPR | Heterogeneous nuclear ribonucleoprotein R |
| IGKV3-11 | Immunoglobulin kappa variable 3-11 |
| IGLV1-51 | Immunoglobulin lambda variable 1-51 |
| IMPA2 | Inositol monophosphatase 2 |
| KRTAP2-3 | Keratin-associated protein 2-3 |
| MMGT1 | Membrane magnesium transporter 1 |
| MYH14 | Myosin-14 |
| RAD9B | Cell cycle checkpoint control protein RAD9B |
| REEP5 | Receptor expression-enhancing protein 5 |
| RP1BL | Ras-related protein Rap-1b-like protein |
| RPL6 | 60S ribosomal protein L6 |
| RTCB | RNA-splicing ligase RtcB homolog |
| SYNGR2 | Synaptogyrin-2 |
| TASOR2 | Protein TASOR 2 |
| TMED5 | Transmembrane emp24 domain-containing protein 5 |
| TPD52L2 | Tumor protein D54 |
| VSIG10L | V-set and immunoglobulin domain-containing protein 10-like |
| ZN F236 | Zinc finger protein 236 |
| GARS1 | Glycine--tRNA ligase |
| H3C1 | Histone H3.1 |
| H 1-5 | Histone H1.5 |
| H2AZ1 | Histone H2A.Z |
| H2AC11 | Histone H2A type 1 |
| H2BC12 | Histone H2B type 1-K |

**[Table C-2-2]**

| Gene name | Protein name |
|---|---|
| LGALSL | Galectin-related protein |
| KV310 | Ig kappa chain V-III region VH |
| ATP5PO | ATP synthase subunit O, mitochondrial |
| DERA | Deoxyribose-phosphate aldolase |
| PRR4 | Proline-rich protein 4 |
| AKR1A1 | Aldo-keto reductase family 1 member A1 |
| BTF3 | Transcription factor BTF3 |
| CCT6A | T-complex protein 1 subunit zeta |
| CPNE3 | Copine-3 |
| DNAAF1 | Dynein assembly factor 1, axonemal |
| EIF4A2 | Eukaryotic initiation factor 4A-II |
| EPS8L1 | Epidermal growth factor receptor kinase substrate 8-like protei n 1 |
| ERP29 | Endoplasmic reticulum resident protein 29 |
| GART | Trifunctional purine biosynthetic protein adenosine-3 |
| GDI2 | Rab GDP dissociation inhibitor beta |
| HM13 | Minor histocompatibility antigen H13 |
| IGHV1-46 | Immunoglobulin heavy variable 1-46 |
| IGKV1-5 | Immunoglobulin kappa variable 1-5 |
| IGKV4-1 | Immunoglobulin kappa variable 4-1 |
| MAST4 | Microtubule-associated serine/threonine-protein kinase 4 |
| MDH2 | Malate dehydrogenase, mitochondrial |
| MYH1 | Myosin-1 |
| NCCRP1 | F-box only protein 50 |
| PCBP1 | Poly(rC)-binding protein 1 |
| POM121 | Nuclear envelope pore membrane protein POM 121 |
| PSMB3 | Proteasome subunit beta type-3 |
| RAB10 | Ras-related protein Rab-10 |
| RAB1B | Ras-related protein Rab-1B |
| RECQL | ATP-dependent DNA helicase Q1 |
| RPL10A | 60S ribosomal protein L10a |
| RPL12 | 60S ribosomal protein L12 |
| RPL29 | 60S ribosomal protein L29 |
| RPS14 | 40S ribosomal protein S14 |
| RPS23 | 40S ribosomal protein S23 |

**[Table C-2-3]**

| Gene name | Protein name |
|---|---|
| RPS25 | 40S ribosomal protein S25 |
| RPS27A | Ubiquitin-40S ribosomal protein S27a |
| SAMD4A | Protein Smaug homolog 1 |
| SEPTIN8 | Septin-8 |
| SEPTIN9 | Septin-9 |
| SERBP1 | Plasminogen activator inhibitor 1 RNA-binding protein |
| SFPQ | Splicing factor, proline- and glutamine-rich |
| SNRPD3 | Small nuclear ribonucleoprotein Sm D3 |
| TAGLN2 | Transgelin-2 |
| TMSL3 | Thymosin beta-4-like protein 3 |
| TNNI3K | Serine/threonine-protein kinase TNNI3K |
| ZNF292 | Zinc finger protein 292 |
| WDR1 | WD repeat-containing protein 1 |
| ARPC3 | Actin-related protein 2/3 complex subunit 3 |
| BST1 | ADP-ribosyl cyclase/cyclic ADP-ribose hydrolase 2 |
| CAPZA1 | F-actin-capping protein subunit alpha-1 |
| CCT3 | T-complex protein 1 subunit gamma |
| COTL1 | Coactosin-like protein |
| CRISPLD2 | Cysteine-rich secretory protein LCCL domain-containing 2 |
| GPLD1 | Phosphatidylinositol-glycan-specific phospholipase D |
| IGKV3-20 | Immunoglobulin kappa variable 3-20 |
| MACROH2A1 | Core histone macro-H2A.1 |
| MYL6 | Myosin light polypeptide 6 |
| NDUFB6 | NADH dehydrogenase [ubiquinone] 1 beta subcomplex subuni t 6 |
| PDIA6 | Protein disulfide-isomerase A6 |
| PGAM1 | Phosphoglycerate mutase 1 |
| POLR3A | DNA-directed RNA polymerase III subunit RPC1 |
| PSMB1 | Proteasome subunit beta type-1 |
| PSMB5 | Proteasome subunit beta type-5 |
| PSMD14 | 26S proteasome non-ATPase regulatory subunit 14 |
| RAB1A | Ras-related protein Rab-1A |
| RANBP1 | Ran-specific GTPase-activating protein |
| RDH12 | Retinol dehydrogenase 12 |
| RPL14 | 60S ribosomal protein L14 |

**[Table C-2-4]**

| Gene name | Protein name |
|---|---|
| SRSF3 | Serine/arginine-rich splicing factor 3 |
| SUB1 | Activated RNA polymerase II transcriptional coactivator p15 |
| TRIM29 | Tripartite motif-containing protein 29 |
| TUBB4B | Tubulin beta-4B chain |
| CPQ | Carboxypeptidase Q |
| FLNB | Filamin-B |
| RPS9 | 40S ribosomal protein S9 |
| RPL8 | 60S ribosomal protein L8 |
| A1BG | Alpha-1B-glycoprotein |
| ARHGDIB | Rho GDP-dissociation inhibitor 2 |
| CDH23 | Cadherin-23 |
| EIF6 | Eukaryotic translation initiation factor 6 |
| FBXO6 | F-box only protein 6 |
| HSD17B4 | Peroxisomal multifunctional enzyme type 2 |
| IGHV3-30 | Immunoglobulin heavy variable 3-30 |
| IGHV3-33 | Immunoglobulin heavy variable 3-33 |
| IGHV3-7 | Immunoglobulin heavy variable 3-7 |
| ITIH2 | Inter-alpha-trypsin inhibitor heavy chain H2 |
| LCN15 | Lipocalin-15 |
| LY6G6C | Lymphocyte antigen 6 complex locus protein G6c |
| PLD3 | 5'-3' exonuclease PLD3 |
| POF1B | Protein POF1B |
| PSMA1 | Proteasome subunit alpha type-1 |
| RPL15 | 60S ribosomal protein L15 |
| RPL30 | 60S ribosomal protein L30 |
| RPL31 | 60S ribosomal protein L31 |
| RPS17 | 40S ribosomal protein S17 |
| TUBB2A | Tubulin beta-2A chain |
| HK3 | Hexokinase-3 |
| MTAP | S-methyl-5'-thioadenosine phosphorylase |
| RALY | RNA-binding protein Raly |
| RPL4 | 60S ribosomal protein L4 |
| RPL7 | 60S ribosomal protein L7 |
| TPP1 | Tripeptidyl-peptidase 1 |

**[Table C-2-5]**

| Gene name | Protein name |
|---|---|
| DHRS11 | Dehydrogenase/reductase SDR family member 11 |
| HNRNPA2B1 | Heterogeneous nuclear ribonucleoproteins A2/B1 |
| ITIH1 | Inter-alpha-trypsin inhibitor heavy chain H1 |
| LACRT | Extracellular glycoprotein lacritin |
| PRSS27 | Serine protease 27 |
| PSMB2 | Proteasome subunit beta type-2 |
| PSME2 | Proteasome activator complex subunit 2 |
| RPS16 | 40S ribosomal protein S16 |
| CAP1 | Adenylyl cyclase-associated protein 1 |
| CTSA | Lysosomal protective protein |
| DLD | Dihydrolipoyl dehydrogenase, mitochondrial |

**[Table C-3-1]**

| Gene name | Protein name |
|---|---|
| H1-5 | Histone H1.5 |
| MYL6 | Myosin light polypeptide 6 |
| POF1B | Protein POF1B |
| LCN2 | Neutrophil gelatinase-associated lipocalin |
| YWHAG | 14-3-3 protein gamma |
| PGAM1 | Phosphoglycerate mutase 1 |
| LDHA | L-lactate dehydrogenase A chain |
| ERP29 | Endoplasmic reticulum resident protein 29 |
| CFB | Complement factor B |
| AMBP | Protein AMBP |
| PFN1 | Profilin-1 |
| TF | Serotransferrin |
| ACTB | Actin, cytoplasmic 1 |
| IGHG1 | Immunoglobulin heavy constant gamma 1 |
| ORM1 | Alpha-1-acid glycoprotein 1 |
| GSN | Gelsolin |
| FGA | Fibrinogen alpha chain |
| APOH | Beta-2-glycoprotein 1 |
| CP | Ceruloplasmin |
| ASPRV1 | Retroviral-like aspartic protease 1 |
| GPI | Glucose-6-phosphate isomerase |
| APOA1 | Apolipoprotein A-I |
| KNG1 | Kininogen-1 |
| FGB | Fibrinogen beta chain |
| H4C1 | Histone H4 |
| SBSN | Suprabasin |
| VTN | Vitronectin |
| APOA2 | Apolipoprotein A-II |
| CBR1 | Carbonyl reductase [NADPH] 1 |
| MYL12B | Myosin regulatory light chain 12B |
| PDIA3 | Protein disulfide-isomerase A3 |
| SERPINB5 | Serpin B5 |
| PLG | Plasminogen |
| CAPG | Macrophage-capping protein |

**[Table C-3-2]**

| Gene name | Protein name |
|---|---|
| PSMA1 | Proteasome subunit alpha type-1 |
| ELANE | Neutrophil elastase |
| IGHG3 | Immunoglobulin heavy constant gamma 3 |
| ALB | Serum albumin |
| CTSG | Cathepsin G |
| VIM | Vimentin |
| APCS | Serum amyloid P-component |
| KRT15 | Keratin, type I cytoskeletal 15 |
| A2M | Alpha-2-macroglobulin |
| CALR | Calreticulin |
| CASP14 | Caspase-14 |
| HSPE1 | 10 kDa heat shock protein, mitochondrial |
| RNASE3 | Eosinophil cationic protein |
| CORO1A | Coronin-1A |
| TAGLN2 | Transgelin-2 |
| F2 | Prothrombin |
| P4HB | Protein disulfide-isomerase |
| RAN | GTP-binding nuclear protein Ran |
| GC | Vitamin D-binding protein |
| FGG | Fibrinogen gamma chain |
| AHSG | Alpha-2-HS-glycoprotein |
| DCD | Dermcidin |
| PPIA | Peptidyl-prolyl cis-trans isomerase A |
| KLK10 | Kallikrein-10 |
| MIF | Macrophage migration inhibitory factor |
| MYH9 | Myosin-9 |
| CFL1 | Cofilin-1 |
| H 1-3 | Histone H1.3 |
| ARHGDIB | Rho GDP-dissociation inhibitor 2 |
| SCGB2A2 | Mammaglobin-A |
| CA2 | Carbonic anhydrase 2 |

The proteins shown in Tables C-4-1 to C-4-6 include proteins shown in Tables C-7-1 to C-7-4, Table C-8, Tables C-11-1 to C-11-4, Tables C-12-1 to C-12-4 and Table C-13 shown in Examples mentioned later. The proteins shown in Tables C-5-1 to C-5-9 include proteins shown in Tables C-9-1 to C-9-7, Tables C-10-1 and C-10-2, Tables C-14-1 to C-14-7, Tables C-15-1 to C-15-4 and Table C-16 shown in Examples mentioned later.

As shown in Examples mentioned later, proteins which were extracted from SSL of healthy children and children with AD and produced a quantitative value in 75% or more test subjects in the group of either healthy children or children with AD were analyzed for their quantitative values. As a result, 116 proteins whose abundance ratio was increased to 1.5 or more times (p ≤ 0.05) (Tables C-7-1 to C-7-4), and 12 proteins whose abundance ratio was decreased to 0.75 or less times (p ≤ 0.05) (Table C-8) were identified in the children with AD compared with the healthy children. Likewise, proteins which were extracted from SSL of adult healthy subjects and adult AD patients 2 and produced a quantitative value in 75% or more test subjects in the group of either healthy subjects or AD patients were analyzed for their quantitative values. As a result, 205 proteins whose abundance ratio was increased to 1.5 or more times (p ≤ 0.05) (Tables C-9-1 to C-9-7), and 37 proteins whose abundance ratio was decreased to 0.75 or less times (p ≤ 0.05) (Tables C-10-1 and C-10-2) were identified in the AD patients compared with the healthy subjects.

Thus, in one embodiment, the method for detecting AD according to the present invention includes detecting AD on the basis of an amount of any of the protein markers for detecting AD in SSL (e.g., a marker concentration in SSL) of a test subject.

For example, on the basis of the concentration of at least one protein marker shown in Tables C-7-1 to C-7-4, Table C-8, Tables C-9-1 to C-9-7 and Tables C-10-1 and C-10-2 in SSL of a test subject, whether or not the test subject from whom the SSL is derived has AD (in other words, whether or not the SSL is derived from a test subject having AD) can be determined. In the method for detecting AD according to the present invention, any one of or any two or more in combination of the proteins shown in Tables C-7-1 to C-7-4, Table C-8, Tables C-9-1 to C-9-7 and Tables C-10-1 and C-10-2 can be used as a protein marker for detecting AD. For example, whether or not a test subject has AD can be determined by measuring the concentration of the at least one marker (target marker) in SSL of the test subject, and comparing the measured concentration of the marker with that of a healthy group. The healthy group to be compared is a healthy group of adults for detecting adult AD and a healthy group of children for detecting childhood AD.

When the target marker is at least one protein selected from the group consisting of proteins shown in Tables C-7-1 to C-7-4 and Tables C-9-1 to C-9-7, the test subject can be determined as having AD if the concentration of the target marker in the test subject is higher than that in a healthy group. The test subject can be determined as having AD, for example, if the concentration of the target marker in the test subject is statistically significantly higher than that in a healthy group. The test subject can be determined as having AD, for example, if the concentration of the target marker in the test subject is preferably 110% or more, more preferably 120% or more, further more preferably 150% or more, of that in a healthy group. In the case of using two or more protein markers for detecting AD as target markers, AD in the test subject can be detected on the basis of whether or not a given proportion, for example, 50% or more, preferably 70% or more, more preferably 90% or more, further more preferably 100%, of the target markers satisfy the criteria mentioned above.

When the target marker is at least one protein selected from the group consisting of proteins shown in Table C-8 and Tables C-10-1 and C-10-2, the test subject can be determined as having AD if the concentration of the target marker in the test subject is lower than that in a healthy group. The test subject can be determined as having AD, for example, if the concentration of the target marker in the test subject is statistically significantly lower than that in a healthy group. The test subject can be determined as having AD, for example, if the concentration of the target marker in the test subject is preferably 90% or less, more preferably 80% or less, further more preferably 75% or less, of that in a healthy group. In the case of using two or more protein markers for detecting AD as target markers, AD in the test subject can be detected on the basis of whether or not a given proportion, for example, 50% or more, preferably 70% or more, more preferably 90% or more, further more preferably 100%, of the target markers satisfy the criteria mentioned above.

The healthy group can be a population having no AD. If necessary, the population constituting the healthy group may be selected depending on the nature of the test subject. For example, when the test subject is a child, a healthy children population can be used as the healthy group. Alternatively, when the test subject is an adult, a healthy adult population can be used as the healthy group. The concentration of the protein marker for detecting AD in the healthy group can be measured by procedures mentioned later, as in measurement for the test subject. Preferably, the concentration of the marker in the healthy group is measured in advance. More preferably, the concentrations of all the markers shown in Tables C-7-1 to C-7-4, Table C-8, Tables C-9-1 to C-9-7 and Tables C-10-1 and C-10-2 in the healthy group are measured in advance.

Alternatively, at least one protein selected from the group consisting of proteins shown in Tables C-7-1 to C-7-4 and Tables C-9-1 to C-9-7, and at least one protein selected from the group consisting of proteins shown in Table C-8 and Tables C-10-1 and C-10-2 may be used in combination as target markers. The criteria for detecting AD are the same as above.

In one embodiment of the method for detecting AD according to the present invention, when the test subject is a child, the target marker is preferably at least one selected from the group consisting of protein markers for detecting AD shown in Tables C-7-1 to C-7-4 and Table C-8; and when the test subject is an adult, the target marker is preferably at least one selected from the group consisting of protein markers for detecting AD shown in Tables C-9-1 to C-9-7 and Tables C-10-1 and C-10-2.

Other preferred examples of the protein marker for detecting AD for children include 127 proteins shown in Tables C-11-1 to C-11-4 given below. The proteins shown in Tables C-11-1 to C-11-4 are proteins whose abundance ratio was increased to 1.5 or more times (p ≤ 0.05) or decreased to 0.75 or less times (p ≤ 0.05) in children with AD compared with healthy children among proteins which were extracted from SSL of healthy children and children with AD and produced a quantitative value in 75% or more of all test subjects. Other preferred examples of the protein marker for detecting AD for adults include 220 proteins shown in Tables C-14-1 to C-14-7 given below. The proteins shown in Tables C-14-1 to C-14-7 are proteins whose abundance ratio was increased to 1.5 or more times (p ≤ 0.05) or decreased to 0.75 or less times (p ≤ 0.05) in AD patients compared with healthy subjects among proteins which were extracted from SSL of adult healthy subjects and adult AD patients and produced a quantitative value in 75% or more of all test subjects.

Thus, in another embodiment of the method for detecting AD according to the present invention, when the test subject is a child, the target marker is preferably at least one selected from the group consisting of protein markers for detecting AD shown in Tables C-11-1 to C-11-4; and when the test subject is an adult, the target marker is preferably at least one selected from the group consisting of protein markers for detecting AD shown in Tables C-14-1 to C-14-7. Alternatively, when the test subject includes both a child and an adult, at least one protein selected from the group consisting of proteins shown in Tables C-11-1 to C-11-4, and at least one protein selected from the group consisting of proteins shown in Tables C-14-1 to C-14-7 may be used in combination as target markers.

In a further embodiment, the method for detecting AD according to the present invention includes detecting AD on the basis of a prediction model constructed through the use of an amount of any of the protein markers for detecting AD in SSL (e.g., the concentration of marker in SSL) of a test subject.

As shown in Examples mentioned later, detection model construction was attempted using proteins of Tables C-11-1 to C-11-4 which were differentially expressed between healthy children and children with AD as feature proteins, quantitative data thereon (Log₂(Abundance + 1) values) as explanatory variables, healthy children and children with AD as objective variables, and random forest as machine learning algorithm. Childhood AD was found predictable with the constructed prediction models. As shown in Examples mentioned later, adult AD was also found predictable with prediction models similarly constructed in proteins of Tables C-14-1 to C-14-7 which were differentially expressed between adult healthy subjects and adult AD patients. Accordingly, in one embodiment of the method for detecting AD according to the present invention, the test subject is a child, and the target marker is any of 127 proteins shown in Tables C-11-1 to C-11-4. In another embodiment of the method for detecting AD according to the present invention, the test subject is an adult, and the target marker is any of 220 proteins shown in Tables C-14-1 to C-14-7.

As shown in Examples mentioned later, feature protein extraction and prediction model construction were attempted using healthy children and children with AD as test subjects, quantitative data on SSL-derived proteins from the test subjects (Log₂(Abundance + 1) values) as explanatory variables, healthy children and children with AD as objective variables, and random forest as machine learning algorithm. Top 140 proteins of variable importance based on Gini coefficient (Tables C-12-1 to C-12-4) calculated in the process of model construction were selected as feature proteins, and prediction models were constructed using the proteins. Childhood AD was found predictable with the constructed prediction models. As shown in Examples mentioned later, feature protein extraction and prediction model construction were similarly attempted using healthy subjects (adults) and AD patients (adults) as test subjects, and quantitative data on SSL-derived proteins from the test subjects (Log₂(Abundance + 1) values). Top 110 proteins of variable importance based on Gini coefficient (Tables C-15-1 to C-15-4) were selected as feature proteins, and prediction models were constructed using the proteins. Adult AD was found predictable with the constructed prediction models. Accordingly, in one embodiment of the method for detecting AD according to the present invention, the test subject is a child, and the target marker is any of 140 proteins shown in Tables C-12-1 to C-12-4. In another embodiment of the method for detecting AD according to the present invention, the test subject is an adult, and the target marker is any of 110 proteins shown in Tables C-15-1 to C-15-4.

As shown in Examples mentioned later, feature proteins were extracted (maximum number of trials: 1,000, p value: less than 0.01) using healthy children and children with AD as test subjects, quantitative data on SSL-derived proteins from the test subjects (Log₂(Abundance + 1) values) as explanatory variables, healthy children and children with AD as objective variables, and Boruta method as machine learning algorithm. 35 proteins (Table C-13) were extracted as feature proteins. Childhood AD was found predictable with prediction models constructed by random forest using quantitative data on these proteins as features. As shown in Examples mentioned later, feature proteins were similarly extracted using healthy subjects (adults) and AD patients (adults) as test subjects, and quantitative data on SSL-derived proteins from the test subjects (Log₂(Abundance + 1) values) as explanatory variables. 24 proteins (Table C-16) were extracted as feature proteins. Adult AD was found predictable with prediction models similarly constructed by random forest using these proteins. Accordingly, in an alternative embodiment of the method for detecting AD according to the present invention, the test subject is a child, and the protein marker for detecting AD is any of 35 proteins shown in Table C-13. In an alternative embodiment of the method for detecting AD according to the present invention, the test subject is an adult, and the protein marker for detecting AD is any of 24 proteins shown in Table C-16.

Among the protein markers for detecting AD mentioned above, a sum set (A∪B∪C) of 130 proteins (A) included in any of Tables C-7-1 to C-7-4, Table C-8 and Tables C-11-1 to C-11-4 extracted by differential expression analysis, 140 proteins (B) shown in Tables C-12-1 to C-12-4 selected as feature proteins by random forest, and 35 proteins (C) shown in Table C-13 selected as feature proteins by Boruta method are 200 proteins shown in Tables C-4-1 to C-4-6. At least one protein selected from the group consisting of proteins shown in Tables C-4-1 to C-4-6 is used as a preferred marker for detecting childhood AD in the present invention. Childhood AD can be detected by comparing an amount of the at least one protein between a test subject and a healthy group. Alternatively, childhood AD can be detected on the basis of a prediction model constructed by using the at least one protein as a feature protein.

**[Table C-4-1]**

| Gene name | Protein name |
|---|---|
| KLK6 | Kallikrein-6 |
| H1-5 | Histone H1.5 |
| RPL29 | 60S ribosomal protein L29 |
| EIF4A2 | Eukaryotic initiation factor 4A-II |
| MYL6 | Myosin light polypeptide 6 |
| POF1B | Protein POF1B |
| LCN2 | Neutrophil gelatinase-associated lipocalin |
| YWHAG | 14-3-3 protein gamma |
| HNRNPA2B1 | Heterogeneous nuclear ribonucleoproteins A2/B1 |
| S100A11 | Protein S100-A11 |
| IL36G | Interleukin-36 gamma |
| MNDA | Myeloid cell nuclear differentiation antigen |
| SERPINB4 | Serpin B4 |
| RAB1A | Ras-related protein Rab-1A |
| PGAM1 | Phosphoglycerate mutase 1 |
| CLEC3B | Tetranectin |
| PLEC | Plectin |
| MYH14 | Myosin-14 |
| LDHA | L-lactate dehydrogenase A chain |
| LGALS7 | Galectin-7 |
| NME1 | Nucleoside diphosphate kinase A |
| ERP29 | Endoplasmic reticulum resident protein 29 |
| LACRT | Extracellular glycoprotein lacritin |
| CFB | Complement factor B |
| H2AC4 | Histone H2A type 1-B/E |
| LGALSL | Galectin-related protein |
| HSPA5 | Endoplasmic reticulum chaperone BiP |
| SERPINB3 | Serpin B3 |
| AMBP | Protein AMBP |
| PFN1 | Profilin-1 |
| PSMB5 | Proteasome subunit beta type-5 |
| DSC3 | Desmocollin-3 |
| TF | Serotransferrin |
| GCA | Grancalcin |

**[Table C-4-2]**

| Gene name | Protein name |
|---|---|
| ACTB | Actin, cytoplasmic 1 |
| KRT23 | Keratin, type I cytoskeletal 23 |
| IGHG1 | Immunoglobulin heavy constant gamma 1 |
| ORM1 | Alpha-l-acid glycoprotein 1 |
| SCGB1D2 | Secretoglobin family 1D member 2 |
| RECQL | ATP-dependent DNA helicase Q1 |
| RPL26 | 60S ribosomal protein L26 |
| GSN | Gelsolin |
| FGA | Fibrinogen alpha chain |
| APOH | Beta-2-glycoprotein 1 |
| CP | Ceruloplasmin |
| TKT | Transketolase |
| FLNB | Filamin-B |
| PSMB1 | Proteasome subunit beta type-1 |
| GBA | Lysosomal acid glucosylceramidase |
| RPL30 | 60S ribosomal protein L30 |
| ASPRV1 | Retroviral-like aspartic protease 1 |
| GPI | Glucose-6-phosphate isomerase |
| APOA1 | Apolipoprotein A-I |
| MMGT1 | Membrane magnesium transporter 1 |
| KLK13 | Kallikrein-13 |
| H2AC11 | Histone H2A type 1 |
| RPS27A | Ubiquitin-40S ribosomal protein S27a |
| KNG1 | Kininogen-1 |
| FGB | Fibrinogen beta chain |
| HSPB1 | Heat shock protein beta-1 |
| H4C1 | Histone H4 |
| SCEL | Sciellin |
| SBSN | Suprabasin |
| VTN | Vitronectin |
| FABP5 | Fatty acid-binding protein 5 |
| RPL22 | 60S ribosomal protein L22 |
| APOA2 | Apolipoprotein A-II |
| SPRR1B | Cornifin-B |

**[Table C-4-3]**

| Gene name | Protein name |
|---|---|
| MSLN | Mesothelin |
| RARRES1 | Retinoic acid receptor responder protein 1 |
| CBR1 | Carbonyl reductase [NADPH] 1 |
| MYL12B | Myosin regulatory light chain 12B |
| ENO1 | Alpha-enolase |
| ITGAM | Integrin alpha-M |
| ANXA2 | Annexin A2 |
| PDIA3 | Protein disulfide-isomerase A3 |
| DSP | Desmoplakin |
| SLURP2 | Secreted Ly-6/uPAR domain-containing protein 2 |
| DYNLL1 | Dynein light chain 1, cytoplasmic |
| LYZ | Lysozyme C |
| SERPINB5 | Serpin B5 |
| LAMP2 | Lysosome-associated membrane glycoprotein 2 |
| LCN15 | Lipocalin-15 |
| PLG | Plasminogen |
| DSC1 | Desmocollin-1 |
| CAPG | Macrophage-capping protein |
| PSMA1 | Proteasome subunit alpha type-1 |
| YWHAZ | 14-3-3 protein zeta/delta |
| MUC5AC | Mucin-5AC |
| JCHAIN | Immunoglobulin J chain |
| ELANE | Neutrophil elastase |
| PCBP1 | Poly(rC)-binding protein 1 |
| TPM3 | Tropomyosin alpha-3 chain |
| S100A10 | Protein S100-A10 |
| IGHG3 | Immunoglobulin heavy constant gamma 3 |
| LTF | Lactotransferrin |
| ALB | Serum albumin |
| RAB10 | Ras-related protein Rab-10 |
| CRISP3 | Cysteine-rich secretory protein 3 |
| VSIG10L | V-set and immunoglobulin domain-containing protein 10-like |
| WFDC5 | WAP four-disulfide core domain protein 5 |
| CPNE3 | Copine-3 |

**[Table C-4-4]**

| Gene name | Protein name |
|---|---|
| CTSG | Cathepsin G |
| VIM | Vimentin |
| RPSA | 40S ribosomal protein SA |
| ANXA3 | Annexin A3 |
| IGHM | Immunoglobulin heavy constant mu |
| MDH2 | Malate dehydrogenase, mitochondrial |
| APCS | Serum amyloid P-component |
| CARD18 | Caspase recruitment domain-containing protein 18 |
| CAP1 | Adenylyl cyclase-associated protein 1 |
| AZGP1 | Zinc-alpha-2-glyco protein |
| NPC2 | NPC intracellular cholesterol transporter 2 |
| KRT13 | Keratin, type I cytoskeletal 13 |
| TGM1 | Protein-glutamine gamma-glutamyltransferase K |
| JUP | Junction plakoglobin |
| EVPL | Envoplakin |
| GDI2 | Rab GDP dissociation inhibitor beta |
| RPL14 | 60S ribosomal protein L14 |
| SPRR2F | Small proline-rich protein 2F |
| KRT15 | Keratin, type I cytoskeletal 15 |
| PRDX2 | Peroxiredoxin-2 |
| PNP | Purine nucleoside phosphorylase |
| S100A6 | Protein S100-A6 |
| PGK1 | Phosphoglycerate kinase 1 |
| CKMT1A | Creatine kinase U-type, mitochondrial |
| AHNAK | Neuroblast differentiation-associated protein AHNAK |
| A2M | Alpha-2-macroglobulin |
| PRSS27 | Serine protease 27 |
| CALR | Calreticulin |
| TALDO1 | Transaldolase |
| CASP14 | Caspase-14 |
| KLK9 | Kallikrein-9 |
| HSPE1 | 10 kDa heat shock protein, mitochondrial |
| S100A14 | Protein S100-A14 |
| HLA-DPB1 | HLA class II histocompatibility antigen, DP beta 1 chain |

**[Table C-4-5]**

| Gene name | Protein name |
|---|---|
| B2M | Beta-2-microglobulin |
| PKM | Pyruvate kinase PKM |
| RNASE3 | Eosinophil cationic protein |
| KRTAP2-3 | Keratin-associated protein 2-3 |
| CORO1A | Coronin-1A |
| TAGLN2 | Transgelin-2 |
| EEF1A1 | Elongation factor 1-alpha 1 |
| SPRR2D | Small proline-rich protein 2D |
| ALDOA | Fructose-bisphosphate aldolase A |
| RPS11 | 40S ribosomal protein S11 |
| F2 | Prothrombin |
| DDX10 | Probable ATP-dependent RNA helicase DDX10 |
| LMNA | Prelamin-A/C |
| SFN | 14-3-3 protein sigma |
| VDAC1 | Voltage-dependent anion-selective channel protein 1 |
| S100A7 | Protein S100-A7 |
| S100A8 | Protein S100-A8 |
| ECM1 | Extracellular matrix protein 1 |
| EIF5A | Eukaryotic translation initiation factor 5A-1 |
| LY6G6C | Lymphocyte antigen 6 complex locus protein G6c |
| NCCRP1 | F-box only protein 50 |
| PI3 | Elafin |
| HLA-DRB1 | HLA class II histocompatibility antigen, DRB1 beta chain |
| P4HB | Protein disulfide-isomerase |
| GPLD1 | Phosphatidylinositol-glycan-specific phospholipase D |
| CLIC1 | Chloride intracellular channel protein 1 |
| ARF6 | ADP-ribosylation factor 6 |
| SNRPD3 | Small nuclear ribonucleoprotein Sm D3 |
| RAN | GTP-binding nuclear protein Ran |
| GC | Vitamin D-binding protein |
| CDH23 | Cadherin-23 |
| FGG | Fibrinogen gamma chain |
| AHSG | Alpha-2-HS-glycoprotein |
| EEF2 | Elongation factor 2 |

**[Table C-4-6]**

| Gene name | Protein name |
|---|---|
| WFDC12 | WAP four-disulfide core domain protein 12 |
| DCD | Dermcidin |
| PPIA | Peptidyl-prolyl cis-trans isomerase A |
| KLK7 | Kallikrein-7 |
| PPL | Periplakin |
| KLK10 | Kallikrein-10 |
| MUCL1 | Mucin-like protein 1 |
| MIF | Macrophage migration inhibitory factor |
| EIF6 | Eukaryotic translation initiation factor 6 |
| MYH9 | Myosin-9 |
| SERPINA3 | Alpha-1-antichymotrypsin |
| EPPK1 | Epiplakin |
| HSD17B4 | Peroxisomal multifunctional enzyme type 2 |
| GM2A | Ganglioside GM2 activator |
| RPL15 | 60S ribosomal protein L15 |
| RPL31 | 60S ribosomal protein L31 |
| CFL1 | Cofilin-1 |
| H1-3 | Histone H1.3 |
| ARHGDIB | Rho GDP-dissociation inhibitor 2 |
| SCGB2A2 | Mammaglobin-A |
| LCN1 | Lipocalin-1 |
| SCGB2A1 | Mammaglobin-B |
| BST1 | ADP-ribosyl cyclase/cyclic ADP-ribose hydrolase 2 |
| PRR4 | Proline-rich protein 4 |
| SAMD4A | Protein Smaug homolog 1 |
| POLR3A | DNA-directed RNA polymerase III subunit RPC1 |
| SERPINB13 | Serpin B13 |
| CA2 | Carbonic anhydrase 2 |
| IGHG4 | Immunoglobulin heavy constant gamma 4 |
| RPS13 | 40S ribosomal protein S13 |

Among the proteins shown in Tables C-4-1 to C-4-6 mentioned above, 23 proteins consisting of POF1B (Protein POF1B), MNDA (Myeloid cell nuclear differentiation antigen), SERPINB4 (Serpin B4), CLEC3B (Tetranectin), PLEC (Plectin), LGALS7 (Galectin-7), H2AC4 (Histone H2A type 1-B/E), SERPINB3 (Serpin B3), AMBP (Protein AMBP), PFN1 (Profilin-1), DSC3 (Desmocollin-3), IGHG1 (Immunoglobulin heavy constant gamma 1), ORM1 (Alpha-1-acid glycoprotein 1), RECQL (ATP-dependent DNA helicase Q1), RPL26 (60S ribosomal protein L26), KLK13 (Kallikrein-13), RPL22 (60S ribosomal protein L22), APOA2 (Apolipoprotein A-II), SERPINB5 (Serpin B5), LCN15 (Lipocalin-15), IGHG3 (Immunoglobulin heavy constant gamma 3), CAP1 (Adenylyl cyclase-associated protein 1) and SPRR2F (Small proline-rich protein 2F) are common proteins among the proteins (A), (B) and (C) described above. At least one protein selected from the group consisting of these 23 proteins are used as a more preferred marker for detecting childhood AD in the present invention. Childhood AD can be detected by comparing an amount of the at least one protein between a test subject (child) and a healthy group (children). Alternatively, childhood AD can be detected on the basis of a prediction model constructed by using the at least one protein as a feature protein.

In a preferred embodiment of the method for detecting childhood AD according to the present invention, at least one, preferably 2 or more, more preferably 5 or more, further more preferably 10 or more, further more preferably all the proteins selected from the group consisting of the 23 proteins are quantified from SSL collected from of a child test subject. In the present invention, the at least one protein selected from the group consisting of the 23 proteins as well as at least one protein selected from the group consisting of 200 proteins shown in Tables C-4-1 to C-4-6 given below (except for the 23 proteins) may be quantified. For example, the at least one protein selected from the group consisting of the 23 proteins as well as at least one protein selected from the group consisting of 127 proteins shown in Tables C-11-1 to C-11-4 (except for the 23 proteins), at least one protein selected from the group consisting of 140 proteins shown in Tables C-12-1 to C-12-4 (except for the 23 proteins), and/or at least one protein selected from the group consisting of 35 proteins shown in Table C-13 (except for the 23 proteins) may be quantified. In this respect, in the case of selecting a protein from Tables C-11-1 to C-11-4, a protein with higher significance of differential expression (e.g., a smaller p value) may be preferentially selected. In the case of selecting a protein from Tables C-12-1 to C-12-4, a protein in a higher rank of variable importance may be preferentially selected, or the protein may be selected from the group of top 50, preferably top 30 proteins of variable importance. Childhood AD can be detected by comparing an amount of the at least one protein as described above between a test subject (child) and a healthy group (children). Alternatively, childhood AD can be detected on the basis of a prediction model constructed by using the at least one protein as described above as a feature protein.

Among the protein markers for detecting AD mentioned above, a sum set (D∪E∪F) of 242 proteins (D) shown in Tables C-9-1 to C-9-7, Tables C-10-1 and C-10-2 and Tables C-14-1 to C-14-7 extracted by differential expression analysis, 110 proteins (E) shown in Tables C-15-1 to C-15-4 selected as feature proteins by random forest, and 24 proteins (F) shown in Table C-16 selected as feature proteins by Boruta method are 283 proteins shown in Tables C-5-1 to C-5-9. At least one protein selected from the group consisting of proteins shown in Tables C-5-1 to C-5-9 is used as a preferred protein marker for detecting adult AD in the present invention. Adult AD can be detected by comparing an amount of the at least one protein between a test subject (adult) and a healthy group (adults). Alternatively, adult AD can be detected on the basis of a prediction model constructed by using the at least one protein as a feature protein.

**[Table C-5-1]**

| Gene name | Protein name |
|---|---|
| LGALS3 | Galectin-3 |
| SERPINB1 | Leukocyte elastase inhibitor |
| HMGB2 | High mobility group protein B2 |
| GC | Vitamin D-binding protein |
| TF | Serotransferrin |
| ITIH4 | Inter-alpha-trypsin inhibitor heavy chain H4 |
| ALB | Serum albumin |
| HPX | Hemopexin |
| TTR | Transthyretin |
| DERA | Deoxyribose-phosphate aldolase |
| SERPINA1 | Alpha-1-antitrypsin |
| VTN | Vitronectin |
| APOA1 | Apolipoprotein A-I |
| NAPA | Alpha-soluble NSF attachment protein |
| APOB | Apolipoprotein B-100 |
| IGHV1-46 | Immunoglobulin heavy variable 1-46 |
| MSN | Moesin |
| CFB | Complement factor B |
| EZR | Ezrin |
| ERP29 | Endoplasmic reticulum resident protein 29 |
| PLG | Plasminogen |
| CP | Ceruloplasmin |
| KV310 | Ig kappa chain V-III region VH |
| AMBP | Protein AMBP |
| FN1 | Fibronectin |
| F2 | Prothrombin |
| DDX55 | ATP-dependent RNA helicase DDX55 |
| PPIA | Peptidyl-prolyl cis-trans isomerase A |
| PRDX6 | Peroxiredoxin-6 |
| H2AZ1 | Histone H2A.Z |
| A2M | Alpha-2-macroglobulin |
| AHSG | Alpha-2-HS-glycoprotein |
| IGHG3 | Immunoglobulin heavy constant gamma 3 |
| A1BG | Alpha-1B-glycoprotein |

**[Table C-5-2]**

| Gene name | Protein name |
|---|---|
| ITIH1 | Inter-alpha-trypsin inhibitor heavy chain H1 |
| FGG | Fibrinogen gamma chain |
| C4BPA | C4b-binding protein alpha chain |
| SERPINF2 | Alpha-2-antiplasmin |
| GSN | Gelsolin |
| CEACAM5 | Carcinoembryonic antigen-related cell adhesion molecule 5 |
| HRG | Histidine-rich glycoprotein |
| CFH | Complement factor H |
| SERPIND1 | Heparin cofactor 2 |
| KNG1 | Kininogen-1 |
| P4HB | Protein disulfide-isomerase |
| VIM | Vimentin |
| SERPINB5 | Serpin B5 |
| RNASE3 | Eosinophil cationic protein |
| MMP9 | Matrix metalloproteinase-9 |
| G6PD | Glucose-6-phosphate 1-dehydrogenase |
| C3 | Complement C3 |
| IGHG1 | Immunoglobulin heavy constant gamma 1 |
| ORM1 | Alpha-1-acid glycoprotein 1 |
| SERPING1 | Plasma protease C1 inhibitor |
| CFL1 | Cofilin-1 |
| H4C1 | Histone H4 |
| FGB | Fibrinogen beta chain |
| HMGB1 | High mobility group protein B1 |
| C4A | Complement C4-A |
| CFI | Complement factor I |
| GPT | Alanine aminotransferase 1 |
| IGKC | Immunoglobulin kappa constant |
| FGA | Fibrinogen alpha chain |
| APCS | Serum amyloid P-component |
| PGAM1 | Phosphoglycerate mutase 1 |
| PDIA3 | Protein disulfide-isomerase A3 |
| CDC42 | Cell division control protein 42 homolog |
| HBB | Hemoglobin subunit beta |

**[Table C-5-3]**

| Gene name | Protein name |
|---|---|
| RPS17 | 40S ribosomal protein S17 |
| ELANE | Neutrophil elastase |
| GNAI2 | Guanine nucleotide-binding protein G |
| IGHV3-7 | Immunoglobulin heavy variable 3-7 |
| GSTP1 | Glutathione S-transferase P |
| MYH9 | Myosin-9 |
| PYCARD | Apoptosis-associated speck-like protein containing a CARD |
| ARPC3 | Actin-related protein 2/3 complex subunit 3 |
| C1QC | Complement C1q subcomponent subunit C |
| IGKV4-1 | Immunoglobulin kappa variable 4-1 |
| DBI | Acyl-CoA-binding protein |
| H2BC12 | Histone H2B type 1-K |
| SUMO3 | Small ubiquitin-related modifier 3 |
| FAU | 40S ribosomal protein S30 |
| RPL8 | 60S ribosomal protein L8 |
| TPT1 | Translationally-controlled tumor protein |
| AZU1 | Azurocidin |
| PFN1 | Profilin-1 |
| C1QA | Complement C1q subcomponent subunit A |
| TUBB | Tubulin beta chain |
| HNRNPD | Heterogeneous nuclear ribonucleoprotein D0 |
| TPD52L2 | Tumor protein D54 |
| TUBB2A | Tubulin beta-2A chain |
| TAGLN2 | Transgelin-2 |
| SERPINF1 | Pigment epithelium-derived factor |
| WDR1 | WD repeat-containing protein 1 |
| HBA1 | Hemoglobin subunit alpha |
| ARPC2 | Actin-related protein 2/3 complex subunit 2 |
| ITIH2 | Inter-alpha-trypsin inhibitor heavy chain H2 |
| RPS14 | 40S ribosomal protein S14 |
| RAN | GTP-binding nuclear protein Ran |
| H 1-5 | Histone H1.5 |
| CTSG | Cathepsin G |
| H3C1 | Histone H3.1 |

**[Table C-5-4]**

| Gene name | Protein name |
|---|---|
| SUB1 | Activated RNA polymerase II transcriptional coactivator p15 |
| MYL6 | Myosin light polypeptide 6 |
| IGKV1-5 | Immunoglobulin kappa variable 1-5 |
| RP1BL | Ras-related protein Rap-1b-like protein |
| ACTB | Actin, cytoplasmic 1 |
| ANXA1 | Annexin A1 |
| TUBB4B | Tubulin beta-4B chain |
| YWHAE | 14-3-3 protein epsilon |
| YWHAH | 14-3-3 protein eta |
| PPIB | Peptidyl-prolyl cis-trans isomerase B |
| NME2 | Nucleoside diphosphate kinase B |
| IGKV3-11 | Immunoglobulin kappa variable 3-11 |
| CAMP | Cathelicidin antimicrobial peptide |
| RAC2 | Ras-related C3 botulinum toxin substrate 2 |
| SRSF3 | Serine/arginine-rich splicing factor 3 |
| GPI | Glucose-6-phosphate isomerase |
| AGT | Angiotensinogen |
| MIF | Macrophage migration inhibitory factor |
| PYGL | Glycogen phosphorylase, liver form |
| TACSTD2 | Tumor-associated calcium signal transducer 2 |
| IGHV3-33 | Immunoglobulin heavy variable 3-33 |
| RPL6 | 60S ribosomal protein L6 |
| LGALS1 | Galectin-1 |
| PLS3 | Plastin-3 |
| RETN | Resistin |
| MACROH2A1 | Core histone macro-H2A.1 |
| IGKV3-20 | Immunoglobulin kappa variable 3-20 |
| EPS8L1 | Epidermal growth factor receptor kinase substrate 8-like protei n 1 |
| CORO1A | Coronin-1A |
| RPS19 | 40S ribosomal protein S19 |
| ANXA6 | Annexin A6 |
| PON1 | Serum paraoxonase/arylesterase 1 |
| APOA2 | Apolipoprotein A-II |
| ARHGDIB | Rho GDP-dissociation inhibitor 2 |

**[Table C-5-5]**

| Gene name | Protein name |
|---|---|
| MYL12B | Myosin regulatory light chain 12B |
| HSPA1A | Heat shock 70 kDa protein 1A |
| BTF3 | Transcription factor BTF3 |
| AKR1A1 | Aldo-keto reductase family 1 member A1 |
| UGP2 | UTP--glucose-1-phosphate uridylyltransferase |
| LCP1 | Plastin-2 |
| LCN2 | Neutrophil gelatinase-associated lipocalin |
| UBE2N | Ubiquitin-conjugating enzyme E2 N |
| COTL1 | Coactosin-like protein |
| RALY | RNA-binding protein Raly |
| DEFA3 | Neutrophil defensin 3 |
| NAMPT | Nicotinamide phosphoribosyltransferase |
| IGHG2 | Immunoglobulin heavy constant gamma 2 |
| H1-3 | Histone H1.3 |
| ALDH3A1 | Aldehyde dehydrogenase, dimeric NADP-preferring |
| C1S | Complement C1s subcomponent |
| ACTR2 | Actin-related protein 2 |
| TNNI3K | Serine/threonine-protein kinase TNNI3K |
| AFM | Afamin |
| ASPRV1 | Retroviral-like aspartic protease 1 |
| CAPZA1 | F-actin-capping protein subunit alpha-1 |
| MPO | Myeloperoxidase |
| CANX | Calnexin |
| CBR1 | Carbonyl reductase [NADPH] 1 |
| DNAJB1 | DnaJ homolog subfamily B member 1 |
| RTCB | RNA-splicing ligase RtcB homolog |
| CAPG | Macrophage-capping protein |
| H1-0 | Histone H1.0 |
| RPL4 | 60S ribosomal protein L4 |
| TRIM29 | Tripartite motif-containing protein 29 |
| EFNA1 | Ephrin-A1 |
| HNRNPK | Heterogeneous nuclear ribonucleoprotein K |
| CALR | Calreticulin |
| IGLV1-51 | Immunoglobulin lambda variable 1-51 |

**[Table C-5-6]**

| Gene name | Protein name |
|---|---|
| RPS6 | 40S ribosomal protein S6 |
| LPO | Lactoperoxidase |
| TMSL3 | Thymosin beta-4-like protein 3 |
| SERPINA4 | Kallistatin |
| EFHD2 | EF-hand domain-containing protein D2 |
| SEPTIN8 | Septin-8 |
| RAB27A | Ras-related protein Rab-27A |
| RPS23 | 40S ribosomal protein S23 |
| RPS9 | 40S ribosomal protein S9 |
| YWHAG | 14-3-3 protein gamma |
| TMED5 | Transmembrane emp24 domain-containing protein 5 |
| HNRNPR | Heterogeneous nuclear ribonucleoprotein R |
| HK3 | Hexokinase-3 |
| SBSN | Suprabasin |
| SRSF2 | Serine/arginine-rich splicing factor 2 |
| LDHA | L-lactate dehydrogenase A chain |
| IGHV3-30 | Immunoglobulin heavy variable 3-30 |
| LRG1 | Leucine-rich alpha-2-glycoprotein |
| SEPTIN9 | Septin-9 |
| RPL12 | 60S ribosomal protein L12 |
| CCT6A | T-complex protein 1 subunit zeta |
| RPL18A | 60S ribosomal protein L18a |
| THBS1 | Thrombospondin-1 |
| C7 | Complement component C7 |
| DAG1 | Dystroglycan |
| APOC1 | Apolipoprotein C-I |
| RPL10A | 60S ribosomal protein L10a |
| ITGB2 | Integrin beta-2 |
| CA2 | Carbonic anhydrase 2 |
| RPS25 | 40S ribosomal protein S25 |
| RAB1B | Ras-related protein Rab-1B |
| PSMD14 | 26S proteasome non-ATPase regulatory subunit 14 |
| PSME2 | Proteasome activator complex subunit 2 |
| RPL5 | 60S ribosomal protein L5 |

**[Table C-5-7]**

| Gene name | Protein name |
|---|---|
| BPI | Bactericidal permeability-increasing protein |
| RAD9B | Cell cycle checkpoint control protein RAD9B |
| FLG2 | Filaggrin-2 |
| DHX36 | ATP-dependent DNA/RNA helicase DHX36 |
| MGST2 | Microsomal glutathione S-transferase 2 |
| GSDMA | Gasdermin-A |
| TPP1 | Tripeptidyl-peptidase 1 |
| F5 | Coagulation factor V |
| KRT77 | Keratin, type II cytoskeletal 1b |
| STS | Steryl-sulfatase |
| MYH1 | Myosin-1 |
| PLD3 | 5'-3' exonuclease PLD3 |
| SCGB2A2 | Mammaglobin-A |
| PSMB4 | Proteasome subunit beta type-4 |
| CCAR2 | Cell cycle and apoptosis regulator protein 2 |
| PSMB3 | Proteasome subunit beta type-3 |
| PSMA1 | Proteasome subunit alpha type-1 |
| DHRS11 | Dehydrogenase/reductase SDR family member 11 |
| POM121 | Nuclear envelope pore membrane protein POM 121 |
| HSPE1 | 10 kDa heat shock protein, mitochondrial |
| FBXO6 | F-box only protein 6 |
| GART | Trifunctional purine biosynthetic protein adenosine-3 |
| DCD | Dermcidin |
| CRNN | Cornulin |
| SYNGR2 | Synaptogyrin-2 |
| PHB2 | Prohibitin-2 |
| DLD | Dihydrolipoyl dehydrogenase, mitochondrial |
| ME1 | NADP-dependent malic enzyme |
| IDH2 | Isocitrate dehydrogenase [NADP], mitochondrial |
| IMPA2 | Inositol monophosphatase 2 |
| HMGA1 | High mobility group protein HMG-I/HMG-Y |
| KRT15 | Keratin, type I cytoskeletal 15 |
| PLTP | Phospholipid transfer protein |
| SFPQ | Splicing factor, proline- and glutamine-rich |

**[Table C-5-8]**

| Gene name | Protein name |
|---|---|
| GMPR2 | GMP reductase 2 |
| ZNF236 | Zinc finger protein 236 |
| TIMP2 | Metalloproteinase inhibitor 2 |
| ZNF292 | Zinc finger protein 292 |
| HP | Haptoglobin |
| TASOR2 | Protein TASOR 2 |
| CCT3 | T-complex protein 1 subunit gamma |
| SERBP1 | Plasminogen activator inhibitor 1 RNA-binding protein |
| PDIA6 | Protein disulfide-isomerase A6 |
| GLRX | Glutaredoxin-1 |
| GARS1 | Glycine--tRNA ligase |
| KRT25 | Keratin, type I cytoskeletal 25 |
| CPQ | Carboxypeptidase Q |
| KRT79 | Keratin, type II cytoskeletal 79 |
| TIMP1 | Metalloproteinase inhibitor 1 |
| KLK10 | Kallikrein-10 |
| CTSA | Lysosomal protective protein |
| POF1B | Protein POF1B |
| HM13 | Minor histocompatibility antigen H13 |
| DDB1 | DNA damage-binding protein 1 |
| HSPA9 | Stress-70 protein, mitochondrial |
| RPL13 | 60S ribosomal protein L13 |
| ACP5 | Tartrate-resistant acid phosphatase type 5 |
| AGRN | Agrin |
| MTAP | S-methyl-5'-thioadenosine phosphorylase |
| CRISPLD2 | Cysteine-rich secretory protein LCCL domain-containing 2 |
| PSMB2 | Proteasome subunit beta type-2 |
| ANXA11 | Annexin A11 |
| MAST4 | Microtubule-associated serine/threonine-protein kinase 4 |
| ATP5PO | ATP synthase subunit O, mitochondrial |
| EIF3I | Eukaryotic translation initiation factor 3 subunit I |
| RPS16 | 40S ribosomal protein S16 |
| DNAAF1 | Dynein assembly factor 1, axonemal |
| RANBP1 | Ran-specific GTPase-activating protein |

**[Table C-5-9]**

| Gene name | Protein name |
|---|---|
| APOH | Beta-2-glycoprotein 1 |
| REEP5 | Receptor expression-enhancing protein 5 |
| RPL7 | 60S ribosomal protein L7 |
| ATP1B1 | Sodium/potassium-transporting ATPase subunit beta-1 |
| CASP14 | Caspase-14 |
| RDH12 | Retinol dehydrogenase 12 |
| SERPINC1 | Antithrombin-III |
| KLKB1 | Plasma kallikrein |
| EPX | Eosinophil peroxidase |
| OPRPN | Opiorphin prepropeptide |
| NDUFB6 | NADH dehydrogenase [ubiquinone] 1 beta subcomplex subunit 6 |

Among the proteins shown in Tables C-5-1 to C-5-9 mentioned above, 19 proteins consisting of SERPINB1 (Leukocyte elastase inhibitor), TTR (Transthyretin), DHX36 (ATP-dependent DNA/RNA helicase DHX36), ITIH4 (Inter-alpha-trypsin inhibitor heavy chain H4), GC (Vitamin D-binding protein), ALB (Serum albumin), SERPING1 (Plasma protease C1 inhibitor), DDX55 (ATP-dependent RNA helicase DDX55), IGHV1-46 (Immunoglobulin heavy variable 1-46), EZR (Ezrin), VTN (Vitronectin), AHSG (Alpha-2-HS-glycoprotein), HPX (Hemopexin), PPIA (Peptidyl-prolyl cis-trans isomerase A), KNG1 (Kininogen-1), FN1 (Fibronectin), PLG (Plasminogen), PRDX6 (Peroxiredoxin-6) and FLG2 (Filaggrin-2) are common proteins among the proteins (D), (E) and (F) described above. At least one protein selected from the group consisting of these 19 proteins are used as a more preferred marker for detecting adult AD in the present invention. Adult AD can be detected by comparing an amount of the at least one protein between a test subject (adult) and a healthy group (adults). Alternatively, adult AD can be detected on the basis of a prediction model constructed by using the at least one protein as a feature protein.

In a preferred embodiment of the method for detecting adult AD according to the present invention, at least one, preferably 2 or more, more preferably 5 or more, further more preferably 10 or more, further more preferably all the proteins selected from the group consisting of the 19 proteins are quantified from SSL collected from an adult test subject. In the present invention, the at least one protein selected from the group consisting of the 19 proteins as well as at least one protein selected from the group consisting of 283 proteins shown in Tables C-5-1 to C-5-9 given below (except for the 19 proteins) may be quantified. For example, the at least one protein selected from the group consisting of the 19 proteins as well as at least one protein selected from the group consisting of 220 proteins shown in Tables C-14-1 to C-14-7 (except for the 19 proteins), at least one protein selected from the group consisting of 110 proteins shown in Tables C-15-1 to C-15-4 (except for the 19 proteins), and/or at least one protein selected from the group consisting of 24 proteins shown in Table C-16 (except for the 19 proteins) may be quantified. In this respect, in the case of selecting a protein from Tables C-14-1 to C-14-7, the protein may be preferentially selected from the group consisting of protein with higher significance of differential expression (e.g., a smaller p value. In the case of selecting a protein from Tables C-15-1 to C-15-4, the protein may be preferentially selected from the group consisting of proteins in a higher rank of variable importance, or from the group consisting of proteins within top 50, preferably top 30 of variable importance. Adult AD can be detected by comparing an amount of the at least one protein between a test subject and a healthy group. Alternatively, adult AD can be detected on the basis of a prediction model constructed by using the at least one protein as a feature protein.

In the method for preparing a protein marker for detecting AD and the method for detecting AD using the same according to the present invention, the test subject is not limited by sex and age and can include infants to adults. Preferably, the test subject is a human who needs or desires detection of AD. The test subject is, for example, a human suspected of developing AD.

In one embodiment, the method for preparing a protein marker for detecting AD and the method for detecting AD using the same according to the present invention may further include collecting SSL from a test subject. Examples of the site of the skin from which SSL is collected include the skin at an arbitrary site of the body, such as the head, the face, the neck, the body trunk, and the limbs, and preferably include the skin at a site having AD-like symptoms such as eczema or dryness.

### (4. Method for detecting childhood AD using SerpinB4)

The present inventors found that: the expression level of SerpinB4 protein is increased in SSL collected from children having AD; and childhood AD can be detected by using the SerpinB4 protein as an index. Thus, a further aspect of the present invention relates to a method for detecting childhood AD using SerpinB4 as an SSL-derived protein marker for detecting childhood AD. The present invention enables childhood AD to be detected by a convenient and noninvasive approach.

In the present specification, "SerpinB4", which is also referred to as squamous cell carcinoma antigen 2 (SCCA-2) or leupin, refers to a protein belonging to the serine protease inhibitor (Serpin) family. SerpinB4 protein is registered under P48594 in UniProt.

In the present specification, the "detecting childhood AD" using a SerpinB4 marker encompasses to elucidate the presence (with symptoms) or absence (without symptoms) of childhood AD defined above as well as to elucidate the degree of progression, i.e., "mild (low grade)", "moderate (intermediate grade)" and "severe (high grade)", of childhood AD, preferably to detect each of "no symptom", "mild" and "moderate".

As shown in Examples mentioned later, protein expression analysis in SSL collected from the face (healthy sites for healthy children and eruption sites (including eruption) for children with AD) was conducted on healthy children and children with AD. As a result, the expression level of SerpinB4 protein was significantly increased in the children with AD. Also, the expression of SerpinB4 protein in SSL collected from the face of healthy children, children with mild AD and children with moderate AD was examined. As a result, the expression level of SerpinB4 protein was increased in a manner dependent on the severity of AD. The expression of SerpinB4 protein in SSL collected from the back (healthy sites for healthy children and non-eruption sites (including no eruption) for children with AD) of healthy children and children with AD was further examined. As a result, the expression level of SerpinB4 protein in SSL was increased not only at the eruption sites but at the non-eruption sites in the children with AD.

By contrast, SerpinB4 RNA in SSL did not differ in expression level between healthy children and children with AD. As for adults, SerpinB4 protein in SSL did not differ in expression level between healthy subjects and AD patients.

Since IL-18 protein in blood and SerpinB12 protein in the stratum corneum are known as AD markers (Non Patent Literatures 5 and 8), the expression of IL-18 protein and SerpinB12 protein in SSL of children with AD was examined. As a result, as shown in Examples mentioned later, neither IL-18 protein nor SerpinB12 protein in SSL differed in expression level between healthy children and children with AD.

These results indicate that SerpinB4 protein in SSL is useful as a childhood AD marker for detecting childhood AD. Considering that: SSL which can be noninvasively collected is an important biological sample source for children; and in the case of using SSL as a biological sample, SerpinB4 RNA or a marker protein known in the art such as IL-18 and SerpinB12 cannot be used as a childhood AD marker, SerpinB4 protein in SSL, which can be used as a childhood AD marker, is unexpected and is very useful.

Thus, the present invention provides a method for detecting childhood AD. The method for detecting childhood AD according to the present invention includes a step of measuring an expression level of SerpinB4 protein in SSL collected from a child test subject.

In the method for detecting AD according to the present invention, an expression level of SerpinB4 in SSL collected from a test subject (child test subject; the same applies to the description below in this section) is measured, and childhood AD is detected on the basis of the expression level. In one example, the detection is performed by comparing the measured expression level of SerpinB4 with a reference value. More specifically, the presence or absence of childhood AD or a degree of progression thereof in a test subject can be detected by comparing the expression level of SerpinB4 in SSL in the test subject with a reference value.

The "reference value" can be arbitrarily set depending on the purpose of detection, and the like. Examples of the "reference value" include the expression level of SerpinB4 protein in SSL in a healthy child. For example, a statistic (e.g., a mean) of the expression level of SerpinB4 protein in SSL measured from a healthy children population can be used as the expression level in a healthy child. Depending on the purpose of detection, the expression level of SerpinB4 protein in SSL in a child with mild AD or a child with moderate AD may be used as the "reference value".

In one embodiment, the presence or absence of childhood AD is detected by comparing the expression level of the SerpinB4 protein in SSL in the test subject with the reference value based on the healthy children population mentioned above. In one example, whether or not the expression level of SerpinB4 protein in SSL in the test subject is higher than the reference value based on the healthy children population mentioned above is determined. In this context, the test subject can be determined as having childhood AD when the expression level of the test subject is higher than the reference value.

In another embodiment, the degree of progression of childhood AD is detected by comparing the expression level of SerpinB4 protein in SSL in the test subject with the reference value based on the healthy children population mentioned above and a reference value based on a population of children with mild or moderate AD. In one example, whether or not the expression level of SerpinB4 protein in SSL in the test subject is higher than the respective reference values is determined. For example, the test subject can be determined as having moderate AD when the expression level of SerpinB4 protein in SSL in the test subject is higher than the reference value based on the healthy children population and is equivalent to or higher than the reference value based on the children population with moderate AD. Alternatively, the test subject can be determined as having mild AD when the expression level of SerpinB4 protein in SSL in the test subject is higher than the reference value based on the healthy children population but is lower than the reference value based on the children population with moderate AD.

In the embodiments described above, provided that the expression level of SerpinB4 protein in SSL in the test subject is, for example, preferably 110% or more, more preferably 120% or more, further more preferably 150% or more, of the reference value, it can be confirmed that the expression level of SerpinB4 protein in SSL in the test subject is "higher" than the reference value. Alternatively, whether or not the expression level of SerpinB4 protein in SSL in the test subject is higher than the reference value can be confirmed by using, for example, mean + 2SD, mean + SD, mean + 1/2SD, or mean + 1/3SD of expression level of SerpinB4 protein in SSL of a healthy children population or a children population with AD (e.g., mild or moderate AD) as the reference value.

Another example of the "reference value" includes a cutoff value determined on the basis of the expression level of SerpinB4 protein in SSL measured from children populations including healthy children and children with AD. The cutoff value can be determined by various statistical analysis approaches. Examples thereof include a cutoff value based on an ROC curve (receiver operatorating characteristic curve) analysis. The ROC curve can be prepared by determining the probability (%) of producing positive results in positive patients (TPF: true position fraction, sensitivity) and the probability (%) of producing negative results in negative patients (specificity) about the expression level of SerpinB4 protein in SSL measured from the children populations, and plotting the sensitivity against [100 - specificity] (FPF: false position fraction). A point to be adopted as the cutoff value in the ROC curve can be determined depending on the severity of the disease, the positioning of test, and other various conditions. In general, in order to enhance both sensitivity and specificity (bring them closer to 100%), the cutoff value is set to an expression level at a point closest to (0,100) on the ROC curve with the true positive fraction (sensitivity) on the ordinate (Y axis) against the false positive fraction on the abscissa (X axis), or an expression level at a point where ["true positive (sensitivity)" - "false positive (100 - specificity)"] is maximized (Youden index).

Thus, in a further alternative embodiment of the present invention, the degree of progression of childhood AD is detected by comparing the expression level of SerpinB4 protein in SSL in the test subject with the reference value based on the cutoff value mentioned above. In one example, whether or not the expression level of SerpinB4 protein in SSL in the test subject is higher than the reference value based on the cutoff value mentioned above is determined. In this context, the test subject can be determined as having childhood AD when the expression level of the test subject is higher than the reference value.

In the present invention, the test subject from whom SSL is collected is not particularly limited by sex, race, and the like, as long as the test subject is a child. Preferred examples of the test subject include children in need of atopic dermatitis detection, and children suspected of developing atopic dermatitis.

In one embodiment, the method of the present invention may further include collecting SSL from a test subject. The site of the skin from which SSL is collected in the test subject can include the skin of the head, the face, the neck, the body trunk, the limbs, or the like, and is not particularly limited. The site from which SSL is collected may or may not be a site which manifests AD symptoms of the skin, and may be, for example, an eruption site or a non-eruption site.

### (5. Preparation and detection of marker for detecting AD)

### 1) Preparation of SSL

Any approach for use in the collection or removal of SSL from the skin can be adopted for the collection of SSL from the skin of a test subject. Preferably, an SSL-absorbent material or an SSL-adhesive material mentioned later, or a tool for scraping off SSL from the skin can be used. The SSL-absorbent material or the SSL-adhesive material is not particularly limited as long as the material has affinity for SSL. Examples thereof include polypropylene and pulp. More detailed examples of the procedure of collecting SSL from the skin include a method of allowing SSL to be absorbed to a sheet-like material such as an oil blotting paper or an oil blotting film, a method of allowing SSL to adhere to a glass plate, a tape, or the like, and a method of collecting SSL by scraping with a spatula, a scraper, or the like. In order to improve the adsorbability of SSL, an SSL-absorbent material impregnated in advance with a solvent having high lipid solubility may be used. On the other hand, the SSL-absorbent material preferably has a low content of a solvent having high water solubility or water because the adsorption of SSL to a material containing the solvent having high water solubility or water is inhibited. The SSL-absorbent material is preferably used in a dry state.

SSL collected from the test subject may be immediately used or may be preserved for a given period. The collected SSL is preferably preserved under low-temperature conditions as rapidly as possible after collection in order to minimize the degradation of contained RNA or proteins. The temperature conditions for the preservation of SSL according to the present invention can be 0°C or lower and are preferably from -20 ± 20°C to -80 ± 20°C, more preferably from -20 ± 10°C to - 80 ± 10°C, further more preferably from -20 ± 20°C to -40 ± 20°C, further more preferably from -20 ± 10°C to -40 ± 10°C, further more preferably -20 ± 10°C, further more preferably -20 ± 5°C. The period of preservation of the RNA-containing SSL under the low-temperature conditions is not particularly limited and is preferably 12 months or shorter, for example, 6 hours or longer and 12 months or shorter, more preferably 6 months or shorter, for example, 1 day or longer and 6 months or shorter, further more preferably 3 months or shorter, for example, 3 days or longer and 3 months or shorter.

### 2) Measurement of expression level of gene or expression product thereof

In the present invention, examples of a measurement object for the expression level of a target gene or an expression product thereof include cDNA artificially synthesized from RNA, DNA encoding the RNA, a protein encoded by the RNA, a molecule which interacts with the protein, a molecule which interacts with the RNA, and a molecule which interacts with the DNA. In this context, examples of the molecule which interacts with the RNA, the DNA or the protein include DNA, RNA, proteins, polysaccharides, oligosaccharides, monosaccharides, lipids, fatty acids, and their phosphorylation products, alkylation products, and sugar adducts, and complexes of any of them. The expression level comprehensively means the expression level (expressed amount) or activity of the gene or the expression product.

In a preferred aspect, in the method of the present invention, SSL is used as a biological sample. In one aspect, in the method of the present invention, the expression level of RNA contained in SSL is analyzed. Specifically, RNA is converted to cDNA through reverse transcription, followed by the measurement of the cDNA or an amplification product thereof.

In the extraction of RNA from SSL, a method which is usually used in RNA extraction or purification from a biological sample, for example, phenol/chloroform method, AGPC (acid guanidinium thiocyanate-phenol-chloroform extraction) method, a method using a column such as TRIzol(R), RNeasy(R), or QIAzol(R), a method using special magnetic particles coated with silica, a method using magnetic particles for solid phase reversible immobilization, or extraction with a commercially available RNA extraction reagent such as ISOGEN can be used.

In the reverse transcription, primers which target particular RNA to be analyzed may be used, and random primers are preferably used for more comprehensive nucleic acid preservation and analysis. In the reverse transcription, common reverse transcriptase or reverse transcription reagent kit can be used. Highly accurate and efficient reverse transcriptase or reverse transcription reagent kit is suitably used. Examples thereof include M-MLV reverse transcriptase and its modified forms, and commercially available reverse transcriptase or reverse transcription reagent kits, for example, PrimeScript(R) Reverse Transcriptase series (Takara Bio Inc.) and SuperScript(R) Reverse Transcriptase series (Thermo Fisher Scientific, Inc.). SuperScript(R) III Reverse Transcriptase, SuperScript(R) VILO cDNA Synthesis kit (both from Thermo Fisher Scientific, Inc.), and the like are preferably used.

The temperature of extension reaction in the reverse transcription is adjusted to preferably 42°C ± 1°C, more preferably 42°C ± 0.5°C, further more preferably 42°C ± 0.25°C, while its reaction time is adjusted to preferably 60 minutes or longer, more preferably from 80 to 120 minutes.

In the case of using RNA, cDNA or DNA as a measurement object, the method for measuring the expression level can be selected from nucleic acid amplification methods typified by PCR using DNA primers which hybridize thereto, real-time RT-PCR, multiplex PCR, SmartAmp, and LAMP, hybridization using a nucleic acid probe which hybridizes thereto (DNA chip, DNA microarray, dot blot hybridization, slot blot hybridization, Northern blot hybridization, and the like), a method of determining a nucleotide sequence (sequencing), and combined methods thereof.

In PCR, one particular DNA to be analyzed may be amplified using a primer pair which targets the particular DNA, or a plurality of particular DNAs may be amplified at the same time using a plurality of primer pairs. Preferably, the PCR is multiplex PCR. The multiplex PCR is a method of amplifying a plurality of gene regions at the same time by using a plurality of primer pairs at the same time in a PCR reaction system. The multiplex PCR can be carried out using a commercially available kit (e.g., Ion AmpliSeq Transcriptome Human Gene Expression Kit; Life Technologies Japan Ltd.).

The temperature of annealing and extension reaction in the PCR depends on the primers used and therefore cannot be generalized. In the case of using the multiplex PCR kit described above, the temperature is preferably 62°C ± 1°C, more preferably 62°C ± 0.5°C, further more preferably 62°C ± 0.25°C. Thus, preferably, the annealing and the extension reaction are performed by one step in the PCR. The time of the step of the annealing and the extension reaction can be adjusted depending on the size of DNA to be amplified, and the like, and is preferably from 14 to 18 minutes. Conditions for denaturation reaction in the PCR can be adjusted depending on DNA to be amplified, and are preferably from 95 to 99°C and from 10 to 60 seconds. The reverse transcription and the PCR using the temperatures and the times as described above can be carried out using a thermal cycler which is generally used for PCR.

The reaction product obtained by the PCR is preferably purified by the size separation of the reaction product. By the size separation, the PCR reaction product of interest can be separated from the primers and other impurities contained in the PCR reaction solution. The size separation of DNA can be performed using, for example, a size separation column, a size separation chip, or magnetic beads which can be used in size separation. Preferred examples of the magnetic beads which can be used in size separation include magnetic beads for solid phase reversible immobilization (SPRI) such as Ampure XP.

The purified PCR reaction product may be subjected to further treatment necessary for conducting subsequent quantitative analysis. For example, for DNA sequencing, the purified PCR reaction product may be prepared into an appropriate buffer solution, the PCR primer regions contained in DNA amplified by PCR may be cleaved, and an adaptor sequence may be further added to the amplified DNA. For example, the purified PCR reaction product can be prepared into a buffer solution, and the removal of the PCR primer sequences and adaptor ligation can be performed for the amplified DNA. If necessary, the obtained reaction product can be amplified to prepare a library for quantitative analysis. These operations can be performed, for example, using 5 × VILO RT Reaction Mix attached to Superscript(R) VILO cDNA Synthesis kit (Life Technologies Japan Ltd.), 5 × Ion AmpliSeq HiFi Mix attached to Ion AmpliSeq Transcriptome Human Gene Expression Kit (Life Technologies Japan Ltd.), and Ion AmpliSeq Transcriptome Human Gene Expression Core Panel according to a protocol attached to each kit.

In the case of measuring the expression level of a target gene or a nucleic acid derived therefrom by use of Northern blot hybridization, for example, probe DNA is first labeled with a radioisotope, a fluorescent material, or the like. Subsequently, the obtained labeled DNA is allowed to hybridize to biological sample-derived RNA transferred to a nylon membrane or the like in accordance with a routine method. Then, the formed duplex of the labeled DNA and the RNA can be measured by detecting a signal derived from the label.

In the case of measuring the expression level of a target gene or a nucleic acid derived therefrom by use of RT-PCR, for example, cDNA is first prepared from biological sample-derived RNA in accordance with a routine method. This cDNA is used as a template, and a pair of primers (a positive strand which binds to the cDNA (- strand) and an opposite strand which binds to a + strand) prepared so as to be able to amplify the target gene of the present invention is allowed to hybridize thereto. Then, PCR is performed in accordance with a routine method, and the obtained amplified double-stranded DNA is detected. In the detection of the amplified double-stranded DNA, for example, a method of detecting labeled double-stranded DNA produced by the PCR using primers labeled in advance with RI, a fluorescent material, or the like can be used.

In the case of measuring the expression level of a target gene or a nucleic acid derived therefrom by use of a DNA microarray, for example, an array in which at least one nucleic acid (cDNA or DNA) derived from the target gene of the present invention is immobilized on a support is used. Labeled cDNA or cRNA prepared from mRNA is allowed to bind onto the microarray, and the expression level of the mRNA can be measured by detecting the label on the microarray. The nucleic acid to be immobilized on the array can be a nucleic acid which specifically hybridizes (i.e., substantially only to the nucleic acid of interest) under stringent conditions, and may be, for example, a nucleic acid having the whole sequence of the target gene of the present invention or may be a nucleic acid consisting of a partial sequence thereof. In this context, examples of the "partial sequence" include nucleic acids consisting of at least 15 to 25 bases. In this context, examples of the stringent conditions can usually include washing conditions on the order of "1 × SSC, 0.1% SDS, and 37°C". Examples of the more stringent hybridization conditions can include conditions on the order of "0.5 × SSC, 0.1% SDS, and 42°C". Examples of the much more stringent hybridization conditions can include conditions on the order of "0.1 × SSC, 0.1% SDS, and 65°C". The hybridization conditions are described in, for example, J. Sambrook et al., Molecular Cloning: A Laboratory Manual, Third Edition, Cold Spring Harbor Laboratory Press (2001).

In the case of measuring the expression level of a target gene or a nucleic acid derived therefrom by sequencing, examples thereof include analysis using a next-generation sequencer (e.g., Ion S5/XL system, Life Technologies Japan Ltd.). RNA expression can be quantified on the basis of the number of reads (read count) prepared by the sequencing.

The probe or the primers for use in the measurement described above, which correspond to the primers for specifically recognizing and amplifying the target gene of the present invention or a nucleic acid derived therefrom, or the probe for specifically detecting the RNA or the nucleic acid derived therefrom, can be designed on the basis of a nucleotide sequence constituting the target gene. In this context, the phrase "specifically recognize" means that a detected product or an amplification product can be confirmed to be the gene or the nucleic acid derived therefrom in such a way that, for example, substantially only the target gene of the present invention or the nucleic acid derived therefrom can be detected in Northern blot, or, for example, substantially only the nucleic acid is amplified in RT-PCR.

Specifically, an oligonucleotide containing a given number of nucleotides complementary to DNA consisting of a nucleotide sequence constituting the target gene of the present invention, or a complementary strand thereof can be used. In this context, the "complementary strand" refers to one strand of double-stranded DNA consisting of A:T (U for RNA) and/or G:C base pairs with respect to the other strand. The term "complementary" is not limited to the case of being a completely complementary sequence in a region with the given number of consecutive nucleotides, and may have preferably 80% or higher, more preferably 90% or higher, further more preferably 95% or higher, even more preferably 98% or higher identity of the nucleotide sequence. The identity of the nucleotide sequence can be determined by algorithm such as BLAST described above.

For use as a primer, the oligonucleotide may achieve specific annealing and strand extension. Examples thereof usually include oligonucleotides having a strand length of 10 or more bases, preferably 15 or more bases, more preferably 20 or more bases, and 100 or less bases, preferably 50 or less bases, more preferably 35 or less bases. For use as a probe, the oligonucleotide may achieve specific hybridization. An oligonucleotide can be used which has at least a portion or the whole of the sequence of DNA (or a complementary strand thereof) consisting of a nucleotide sequence constituting the target gene of the present invention, and has a strand length of, for example, 10 or more bases, preferably 15 or more bases, and, for example, 100 or less bases, preferably 50 or less bases, more preferably 25 or less bases.

In this context, the "oligonucleotide" can be DNA or RNA and may be synthetic or natural. The probe for use in hybridization is usually labeled for use.

In the case of measuring a translation product (protein) of the target gene of the present invention, a molecule which interacts with the protein, a molecule which interacts with the RNA, or a molecule which interacts with the DNA, a method such as protein chip analysis, immunoassay (e.g., ELISA), mass spectrometry (e.g., LC-MS/MS and MALDI-TOF/MS), one-hybrid method (PNAS 100, 12271-12276 (2003)), or two-hybrid method (Biol. Reprod. 58, 302-311 (1998)) can be used and can be appropriately selected depending on the measurement object.

For example, in the case of using the protein as a measurement object, the measurement is carried out by contacting an antibody against the expression product of the present invention with a biological sample, detecting a protein in the sample bound with the antibody, and measuring the level thereof. For example, according to Western blot, the antibody described above is used as a primary antibody, and an antibody which binds to the primary antibody and which is labeled with, for example, a radioisotope, a fluorescent material or an enzyme is used as a secondary antibody so that the primary antibody is labeled, followed by the measurement of a signal derived from such a labeling material using a radiation meter, a fluorescence detector, or the like.

The antibody against the translation product may be a polyclonal antibody or a monoclonal antibody. These antibodies can be produced in accordance with a method known in the art. Specifically, the polyclonal antibody may be produced by using a protein which has been expressed in *E. coli* or the like and purified in accordance with a routine method, or synthesizing a partial polypeptide of the protein in accordance with a routine method, and immunizing a nonhuman animal such as a house rabbit therewith, followed by obtainment from the serum of the immunized animal in accordance with a routine method.

On the other hand, the monoclonal antibody can be obtained from hybridoma cells prepared by immunizing a nonhuman animal such as a mouse with a protein which has been expressed in *E. coli* or the like and purified in accordance with a routine method, or a partial polypeptide of the protein, and fusing the obtained spleen cells with myeloma cells. Alternatively, the monoclonal antibody may be prepared by use of phage display (Griffiths, A.D.; Duncan, A.R., Current Opinion in Biotechnology, Volume 9, Number 1, February 1998, pp. 102-108 (7)).

In this way, the expression level of the target gene of the present invention or the expression product thereof in a biological sample collected from a test subject is measured, and AD is detected on the basis of the expression level. In one embodiment, the detection is specifically performed by comparing the measured expression level of the target gene of the present invention or the expression product thereof with a control level.

Examples of the "control level" include an expression level of the target gene or the expression product thereof in a healthy subject. The expression level of the healthy subject may be a statistic (e.g., a mean) of the expression level of the gene or the expression product thereof measured from a healthy subject population. For a plurality of target genes, it is preferred to determine a standard expression level in each individual gene or expression product thereof. The healthy subject for use in the calculation of the control level is a healthy subject of an adult for detecting adult AD and a healthy subject of a child for detecting childhood AD.

In the case of analyzing expression levels of a plurality of target genes by sequencing, as described above, read count values which are data on expression levels, RPM values which normalize the read count values for difference in the total number of reads among samples, values obtained by the conversion of the RPM values to logarithmic values to base 2 (Log₂RPM values) or logarithmic values to base 2 plus integer 1 (Log₂(RPM + 1) values), or normalized count values obtained using DESeq2 or logarithmic values to base 2 plus integer 1 (Log₂(count + 1) values) are preferably used as an index. Also, values calculated by, for example, fragments per kilobase of exon per million reads mapped (FPKM), reads per kilobase of exon per million reads mapped (RPKM), or transcripts per million (TPM) which are general quantitative values of RNA-seq may be used. Further, signal values obtained by microarray method or corrected values thereof may be used. In the case of analyzing an expression level of only a particular target gene by RT-PCR or the like, an analysis method of converting the expression level of the target gene to a relative expression level based on the expression level of a housekeeping gene (relative quantification), or an analysis method of quantifying an absolute copy number using a plasmid containing a region of the target gene (absolute quantification) is preferred. A copy number obtained by digital PCR may be used.

The detection of AD according to the present invention may be performed through an increase and/or decrease in the expression level of the target gene of the present invention or the expression product thereof. In this case, the expression level of the target gene or the expression product thereof in a biological sample derived from a test subject is compared with a reference value of the gene or the expression product thereof. The reference value can be appropriately determined on the basis of a statistical numeric value, such as a mean or standard deviation, of the expression level based on standard data obtained in advance on the expression level of this target gene or expression product thereof in a healthy subject. The healthy subject for use in the calculation of the reference value is a healthy subject of an adult for detecting adult AD and a healthy subject of a child for detecting childhood AD.

### 3) Measurement of protein marker

In the method for preparing a protein marker for detecting AD and the method for detecting AD using the same according to the present invention, a method which is usually used in protein extraction or purification from a biological sample can be used in the extraction of the protein from SSL. For example, an extraction method with water, a phosphate-buffered saline solution, or a solution containing a surfactant such as Triton X-100 or Tween 20, or a protein extraction method with a commercially available protein extraction reagent or kit such as M-PER buffer (Thermo Fisher Scientific, Inc.), MPEX PTS Reagent (GL Sciences Inc.), QIAzol Lysis Reagent (Qiagen N.V.), or EasyPep(TM) Mini MS Sample Prep Kit (Thermo Fisher Scientific, Inc.) can be used.

The extracted SSL-derived protein is capable of containing at least one protein marker for detecting AD mentioned above. The SSL-derived protein may be immediately used in AD detection or may be preserved under usual protein preservation conditions until use in the AD detection.

The concentration of the protein marker for detecting AD in SSL can be measured by use of a usual protein detection or quantification method such as ELISA, immunostaining, fluorescent method, electrophoresis, chromatography, or mass spectrometry. Among them, mass spectrometry such as LC-MS/MS is preferred. In the concentration measurement, the detection or quantification of at least one target protein marker can be carried out in accordance with usual procedures using the SSL-derived protein as a sample. The concentration of the target marker to be calculated may be a concentration based on the absolute amount of the target marker in SSL or may be a relative concentration with respect to other standard substances or total protein in SSL.

In the method for detecting AD using SerpinB4, the expression level of SerpinB4 protein may be measured by measuring the amount or activity of SerpinB4 protein itself or by using an antibody against SerpinB4. Alternatively, the amount or activity of a molecule which interacts with the SerpinB4 protein, for example, another protein, a saccharide, a lipid, a fatty acid, or any of their phosphorylation products, alkylation products, and sugar adducts, or a complex of any of them, may be measured. The expression level of SerpinB4 protein to be calculated may be a value based on the absolute amount of the SerpinB4 protein in SSL or may be a relative value with respect to other standard substances or total protein in SSL, and is preferably a relative value with respect to human-derived total protein.

As an approach of measuring the expression level of SerpinB4 protein, a usual protein detection or quantification method such as Western blot, protein chip analysis, immunoassay (e.g., ELISA), chromatography, mass spectrometry (e.g., LC-MS/MS and MALDI-TOF/MS), one-hybrid method (PNAS, 100: 12271-12276 (2003)), or two-hybrid method (Biol. Reprod. 58: 302-311 (1998)) can be used. The expression level of SerpinB4 protein can be measured, for example, by contacting an antibody against SerpinB4 protein with a protein sample derived from SSL, and detecting a protein in the sample bound with the antibody. For example, according to Western blot, the antibody described above is used as a primary antibody, and an antibody which binds to the primary antibody and which is labeled with, for example, a radioisotope, a fluorescent material or an enzyme is used as a secondary antibody so that the primary antibody is labeled, followed by the measurement of a signal derived from such a labeling material using a radiation meter, a fluorescence detector, or the like. The primary antibody may be a polyclonal antibody or a monoclonal antibody. Commercially available products can be used as these antibodies. Also, the antibodies can be produced in accordance with a method known in the art. Specifically, the polyclonal antibody may be produced by using a protein which has been expressed in *E. coli* or the like and purified in accordance with a routine method, or synthesizing a partial polypeptide of the protein in accordance with a routine method, and immunizing a nonhuman animal such as a house rabbit therewith, followed by obtainment from the serum of the immunized animal in accordance with a routine method. On the other hand, the monoclonal antibody can be obtained from hybridoma cells prepared by immunizing a nonhuman animal such as a mouse with a protein which has been expressed in *E. coli* or the like and purified in accordance with a routine method, or a partial polypeptide of the protein, and fusing the obtained spleen cells with myeloma cells. Alternatively, the monoclonal antibody may be prepared by use of phage display (Current Opinion in Biotechnology, 9 (1) : 102-108 (1998)).

### (6. Construction of prediction model for detecting AD)

The detection of AD based on a prediction model will be described. In one example, in the case of detecting adult AD as described in the above section 1. or detecting childhood AD as described in the above section 2., a discriminant (prediction model) which discriminates between an AD patient and a healthy subject is constructed by using measurement values of an expression level of a target gene or an expression product thereof derived from an AD patient (adult or child) and an expression level of the target gene or the expression product thereof derived from a healthy subject (adult or child) as teacher samples, and a cutoff value (reference value) which discriminates between the AD patient and the healthy subject is determined on the basis of the discriminant. In the preparation of the discriminant, dimensional compression is performed by principal component analysis (PCA), and a principal component can be used as an explanatory variable. The presence or absence of AD in a test subject can be evaluated by similarly measuring a level of the target gene or the expression product thereof from a biological sample collected from the test subject, substituting the obtained measurement value into the discriminant, and comparing the results obtained from the discriminant with the reference value.

In another example, in the case of detecting AD using a protein marker as described in the above section 3., a discriminant (prediction model) which discriminates between an AD patient (adult or child) and a healthy subject (adult or child) is constructed by machine learning algorithm using an amount of the protein marker for detecting AD as an explanatory variable and the presence or absence of AD as an objective variable. AD can be detected through the use of the discriminant. The amount (concentration) of the marker may be an absolute value or a relative value and may be normalized. In one embodiment, a discriminant (prediction model) which discriminates between an AD patient and a healthy subject is constructed by using a quantitative value of the target marker derived from SSL of an AD patient and a quantitative value of the target marker derived from SSL of the healthy subject as teacher samples, and a cutoff value (reference value) which discriminates the AD patient and the healthy subject is determined on the basis of the discriminant. Subsequently, the presence or absence of AD in a test subject can be detected by measuring an amount of the target marker from SSL collected from the test subject, substituting the obtained measurement value into the discriminant, and comparing the results obtained from the discriminant with the reference value.

Variables for use in the construction of the discriminant are an explanatory variable and an objective variable. For example, an expression level of a target gene or an expression product thereof selected by a method described below, or an expression level (e.g., a concentration in SSL) of a protein marker for detecting AD can be used as the explanatory variable. For example, whether the sample is derived from a healthy subject or derived from an AD patient (the presence or absence of AD) can be used as the objective variable.

For feature selection, statistically significant difference between two groups for discrimination, for example, an expression level of a gene whose expression level significantly differs between two groups (differentially expressed gene) or an expression product thereof (e.g., a differentially expressed protein) can be used. Further, a feature gene may be extracted by use of an approach known in the art such as algorithm for use in machine learning, and an expression level thereof can be used. For example, an expression level of a gene or an expression product thereof (e.g., a protein) with high variable importance in random forest given below can be used, or a feature gene or a feature protein is extracted using "Boruta" package of R language, and an expression level thereof can be used.

Algorithm known in the art such as algorithm for use in machine learning can be used as the algorithm in the construction of the discriminant. Examples of the machine learning algorithm include random forest, linear kernel support vector machine (SVM linear), rbf kernel support vector machine (SVM rbf), neural network, generalized linear model, regularized linear discriminant analysis, and regularized logistic regression. A predictive value is calculated by inputting data for the verification of the constructed prediction model, and a model which attains the predictive value most compatible with an actually measured value, for example, a model which attains the largest accuracy, can be selected as the optimum prediction model. Further, recall, precision, and an F value which is a harmonic mean thereof are calculated from a prediction value and an actually measured value, and a model having the largest F value can be selected as the optimum prediction model.

In the case of using random forest algorithm in the construction of the discriminant, an estimate error rate (OOB error rate) for unknown data can be calculated as an index for the precision of the prediction model (Breiman L. Machine Learning (2001) 45; 5-32). In the random forest, a classifier called decision tree is prepared by randomly extracting samples of approximately 2/3 of the number of samples from all samples with duplication accepted in accordance with an approach called bootstrap method. In this respect, a sample which has not been extracted is called out of bug (OOB). An objective variable of OOB can be predicted using one decision tree and compared with an accurate label to calculate an error rate thereof (OOB error rate in the decision tree). Similar operation is repetitively performed 500 times, and a value which corresponds to a mean OOB error rate in 500 decision trees can be used as an OOB error rate of a model of the random forest.

The number of decision trees (ntree value) to construct the model of the random forest is 500 for default and can be changed, if necessary, to an arbitrary number. The number of variables (mtry value) for use in the preparation of the sample discriminant in one decision tree is a value which corresponds to the square root of the number of explanatory variables for default and can be changed, if necessary, to any value from one to the total number of explanatory variables. A "caret" package of R language can be used in the determination of the mtry value. Random forest is designated as the method of the "caret" package, and eight trials of the mtry value are made. For example, a mtry value which attains the largest accuracy can be selected as the optimum mtry value. The number of trials of the mtry value can be changed, if necessary, to an arbitrary number of trials.

In the case of using random forest algorithm in the construction of the discriminant, the importance of the explanatory variable used in model construction can be converted into a numeric value (variable importance). For example, the amount of decrease in Gini coefficient (mean decrease Gini) can be used as a value of the variable importance.

The method for determining the cutoff value (reference value) is not particularly limited, and the value can be determined in accordance with an approach known in the art. The value can be determined from, for example, an ROC (receiver operating characteristic) curve prepared using the discriminant. In the ROC curve, the probability (%) of producing positive results in positive patients (sensitivity) is plotted on the ordinate against a value (false positive rate) of 1 minus the probability (%) of producing negative results in negative patients (specificity) on the abscissa. As for "true positive (sensitivity)" and "false positive (1 - specificity)" shown in the ROC curve, a value at which "true positive (sensitivity)" - "false positive (1 - specificity)" is maximized (Youden index) can be used as the cutoff value (reference value).

In the case of using data on a large number of proteins in the construction of the prediction model, the data may be compressed, if necessary, by principal component analysis (PCA), followed by the construction of the prediction model. For example, dimensional compression is performed by principal component analysis on quantitative values of the protein, and a principal component can be used as an explanatory variable for the construction of the prediction model.

### (7. Kit for detecting AD)

The test kit for detecting AD according to the present invention contains a test reagent for measuring an expression level of the target gene of the present invention or an expression product thereof in a biological sample separated from a patient. Specific examples thereof include a reagent for nucleic acid amplification and hybridization containing an oligonucleotide (e.g., a primer for PCR) which specifically binds (hybridizes) to the target gene of the present invention or a nucleic acid derived therefrom, and a reagent for immunoassay containing an antibody which recognizes an expression product (protein) of the target gene of the present invention. The oligonucleotide, the antibody, or the like contained in the kit can be obtained by a method known in the art as mentioned above. The test kit can contain, in addition to the antibody or the nucleic acid, a labeling reagent, a buffer solution, a chromogenic substrate, a secondary antibody, a blocking agent, an instrument necessary for a test, a control reagent for use as a positive control or a negative control, a tool for collecting a biological sample (e.g., an oil blotting film for collecting SSL), and the like.

The present invention also provides a test kit for detecting childhood AD which can be used in the method for detecting childhood AD using SerpinB4 protein described above. In one embodiment, the kit has a reagent or an instrument for measuring an expression level of SerpinB4 protein. The kit may have, for example, a reagent (e.g., a reagent for immunoassay) for quantifying SerpinB4 protein. Preferably, the kit contains an antibody which recognizes SerpinB4 protein. The antibody contained in the kit can be obtained as a commercially available product or by a method known in the art as mentioned above. The kit may contain, in addition to the antibody, a labeling reagent, a buffer solution, a chromogenic substrate, a secondary antibody, a blocking agent, an instrument necessary for a test, and a control reagent for use as a positive control or a negative control. Preferably, the kit further has an index or a guidance for evaluating an expression level of SerpinB4 protein. The kit may have, for example, a guidance which describes a reference value of the expression level of SerpinB4 protein for detecting AD. The kit may further have an SSL collection device (e.g., the SSL-absorbent material or the SSL-adhesive material described above), a reagent for extracting a protein from a biological sample, a preservative or a container for preservation for a sample collection device after biological sample collection, and the like.

The following substances, production methods, use, methods, and the like will be further disclosed herein as exemplary embodiments of the present invention. However, the present invention is not limited to these embodiments.

[A-1] A method for detecting adult atopic dermatitis in an adult test subject, comprising a step of measuring an expression level of at least one gene selected from the group of 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 or an expression product thereof in a biological sample collected from the test subject.

[A-2] The method according to [A-1], wherein preferably, the expression level of the gene or the expression product thereof is measured as an expression level of mRNA.

[A-3] The method according to [A-1] or [A-2], wherein preferably, the gene or the expression product thereof is RNA contained in skin surface lipids of the test subject.

[A-4] The method according to any one of [A-1] to [A-3], wherein preferably, the presence or absence of adult atopic dermatitis is evaluated by comparing the measurement value of the expression level with a reference value of the gene or the expression product thereof.

[A-5] The method according to any one of [A-1] to [A-3], wherein preferably, the presence or absence of adult atopic dermatitis in the test subject is evaluated by the following steps: preparing a discriminant which discriminates between the atopic dermatitis patient and the healthy subject by using measurement values of an expression level of the gene or the expression product thereof derived from an adult atopic dermatitis patient and an expression level of the gene or the expression product thereof derived from an adult healthy subject as teacher samples;, substituting the measurement value of the expression level of the gene or the expression product thereof obtained from the biological sample collected from the test subject into the discriminant; and comparing the obtained results with a reference value.

[A-6] The method according to [A-5], wherein preferably, algorithm in construction of the discriminant is random forest, linear kernel support vector machine, rbf kernel support vector machine, neural network, generalized linear model, regularized linear discriminant analysis, or regularized logistic regression.

[A-7] The method according to [A-5] or [A-6], wherein preferably, expression levels of all the genes of the group of 17 genes or expression products thereof are measured.

[A-8] The method according to any one of [A-5] to [A-7], wherein preferably, expression levels of the at least one gene selected from the group of 17 genes as well as at least one gene selected from the group of 123 genes shown in Tables A-1-1 to A-1-3 given below, 150 genes shown in Tables A-3-1 to A-3-4 given below, or 45 genes shown in Table A-4 except for the 17 genes, or expression products thereof are measured.

[A-9] The method according to [A-8], wherein preferably, the 150 genes shown in Tables A-3-1 to A-3-4 given below are feature genes extracted by use of random forest.

[A-10] The method according to [A-8], wherein preferably, the 45 genes shown in Table A-4 given below are feature genes extracted by use of Boruta method.

[A-11] Use of at least one selected from the group consisting of the following 17 genes: MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 and expression products of the genes derived from a biological sample collected from an adult test subject, as a detection marker for adult atopic dermatitis.

[A-12] The use according to [A-11], wherein preferably, the genes or the expression products thereof are mRNA contained in skin surface lipids collected from the test subject.

[A-13] The use according to [A-11] or [A-12], wherein preferably, the at least one gene selected from the group of 17 genes or the expression product thereof as well as at least one gene selected from the group of 123 genes shown in Tables A-1-1 to A-1-3 given below, 150 genes shown in Tables A-3-1 to A-3-4 given below, or 45 genes shown in Table A-4 except for the 17 genes or an expression product thereof is used.

[A-14] A test kit for detecting adult atopic dermatitis, the kit being used in the method according to any one of [A-1] to [A-10], and comprising an oligonucleotide which specifically hybridizes to the gene or a nucleic acid derived therefrom, or an antibody which recognizes an expression product of the gene.

[A-15] A marker for detecting adult atopic dermatitis comprising at least one gene selected from the group of 210 genes shown in Table A-b described above or an expression product thereof.

[A-16] A marker for detecting adult atopic dermatitis comprising at least one gene selected from the group of 187 genes shown in the following Table A-c or an expression product thereof.

**[Table A-c]**

| | | | | | | |
|---|---|---|---|---|---|---|
| ACAT1 | CISD1 | FAM120A | KIAA0146 | NMRK1 | RRM1 | VOPP1 |
| ACO1 | COBLL1 | FAM190B | KIAA0513 | NPEPL1 | SAP30BP | VPS4B |
| ADAP2 | COPS2 | FAM26E | KRT23 | NUDT16 | SCARB2 | WBSCR1 6 |
| AKAP17A | COX6A1 | FBXL17 | LCE1D | OAT | SKP1 | WDR26 |
| APOBR | COX7B | FBXL18 | LENG9 | OGFR | SLC12A9 | XKRX |
| ARHGAP2 3 | CREG1 | FBXL6 | LEPREL1 | PALD1 | SLC25A16 | XPO5 |
| ARHGAP2 9 | CRISPLD2 | FDFT1 | LMNA | PARP4 | SLC25A33 | ZC3H15 |
| ARHGAP4 | CRTC2 | FIS1 | LOC146880 | PCSK7 | SLC2A4RG | ZC3H18 |
| ARL8A | CRY2 | FMN1 | LOC152217 | PCTP | SLC31A1 | ZFP36L2 |
| ARRDC4 | CSNK1G2 | FOSB | LRP8 | PHB | SMAP2 | ZMIZ1 |
| ATOX1 | CSTB | FURIN | LY6D | PLAA | SMARCD1 | ZNF335 |
| ATP12A | CTBP1 | GABARAPL 2 | MAN2A2 | PLEKHG2 | SNORD17 | ZNF664 |
| ATP5A1 | CTDSP1 | GIGYF1 | MAPK3 | PLP2 | SRF | ZNF706 |
| ATPIF1 | CTSB | GLRX | MAPKBP1 | PMVK | SSH1 | |
| ATXN7L3B | CYTH2 | GNA15 | MAZ | POLD4 | ST6GALNAC 2 | |
| BAX | DBNDD2 | GNB2 | MECR | PPA1 | TEX2 | |
| BCKDHB | DBT | GPD1 | MEMO1 | PPP1R12C | TM7SF2 | |
| BCRP3 | DGKA | GRASP | MINK1 | PPP1R9B | TMC5 | |
| C15orf23 | DHX32 | GRN | MKNK2 | PSMA5 | TMEM165 | |
| C17orf107 | DNASE1L 1 | GSDMA | MLL2 | PSMB4 | TMEM222 | |
| C19orf71 | DOPEY2 | GSE1 | MLL4 | PTPN18 | TNRC18 | |
| C1QB | DPYSL3 | GTF2H2 | MLLT11 | RAB11FIP 5 | TSTD1 | |
| C2CD2 | DSTN | HADHA | MTSS1 | RABL6 | TTC39B | |
| C4orf52 | DUSP16 | HBP1 | MVP | RASA4CP | TWSG1 | |
| CARD18 | DYNLL1 | HINT3 | MY06 | RB1CC1 | U2AF2 | |
| CCDC88B | EIF1AD | HMGCL | NCOR2 | RGS19 | UNC13D | |
| CCND3 | EMP3 | HMHA1 | NCS1 | RHOC | UQCRQ | |
| CEP76 | FABP7 | ILF3 | NDUFA4 | RNPEPL1 | USP38 | |
| CETN2 | FAM108B 1 | ITPRIPL2 | NIPSNAP3 A | RPS6KB2 | VHL | |

[A-17] The marker according to [A-15] or [A-16], wherein preferably, the marker is at least one gene selected from the group of 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 or an expression product thereof.

[A-18] The marker according to [A-17], wherein preferably, the marker is at least one gene selected from the group of 15 genes consisting of MECR, RASA4CP, ARRDC4, EIF1AD, FDFT1, ZNF706, TEX2, RPS6KB2, CTBP1, ZNF335, DGKA, PPP1R9B, DNASE1L1, GNB2 and CSNK1G2 or an expression product thereof.

[B-1] A method for detecting childhood atopic dermatitis in a child test subject, comprising a step of measuring an expression level of at least one gene selected from the group of 7 genes consisting of IMPDH2, ERI1, FBXW2, STK17B, TAGLN2, AMICA1 and HNRNPA1 or an expression product thereof in a biological sample collected from the test subject.

[B-2] The method according to [B-1], wherein preferably, the method comprises at least measuring an expression level of a gene selected from the group of 3 genes consisting of IMPDH2, ERI1 and FBXW2 or an expression product thereof.

[B-3] The method according to [B-1] or [B-2], wherein preferably, the expression level of the gene or the expression product thereof is measured as an expression level of mRNA.

[B-4] The method according to any one of [B-1] to [B-3], wherein preferably, the gene or the expression product thereof is RNA contained in skin surface lipids of the test subject.

[B-5] The method according to any one of [B-1] to [B-4], wherein preferably, the presence or absence of childhood atopic dermatitis is evaluated by comparing the measurement value of the expression level with a reference value of the gene or the expression product thereof.

[B-6] The method according to any one of [B-1] to [B-4], wherein preferably, the presence or absence of childhood atopic dermatitis in the test subject is evaluated by the following steps: preparing a discriminant which discriminates between the child with atopic dermatitis and the healthy child by using measurement values of an expression level of the gene or the expression product thereof derived from a child with atopic dermatitis and an expression level of the gene or the expression product thereof derived from a healthy child as teacher samples; substituting the measurement value of the expression level of the gene or the expression product thereof obtained from the biological sample collected from the test subject into the discriminant; and comparing the obtained results with a reference value.

[B-7] The method according to [B-6], wherein preferably, algorithm in construction of the discriminant is random forest, linear kernel support vector machine, rbf kernel support vector machine, neural network, generalized linear model, regularized linear discriminant analysis, or regularized logistic regression.

[B-8] The method according to [B-6] or [B-7], wherein preferably, expression levels of all the genes of the group of 7 genes or expression products thereof are measured.

[B-9] The method according to any one of [B-6] to [B-8], wherein preferably, expression levels of the at least one gene selected from the group of 7 genes as well as at least one gene selected from the group of 100 genes shown in Tables B-3-1 to B-3-3 given below or 9 genes shown in Table B-4 except for the 7 genes, or expression products thereof are measured.

[B-10] The method according to [B-9], wherein preferably, the 100 genes shown in Tables B-3-1 to B-3-3 given below are feature genes extracted by use of random forest.

[B-11] The method according to [B-9], wherein preferably, the 9 genes shown in Table B-4 given below are feature genes extracted by use of Boruta method.

[B-12] The method according to any one of [B-6] to [B-8], wherein preferably, expression levels of the at least one gene selected from the group of 7 genes as well as at least one gene selected from the group of 371 genes shown in Tables B-1-1 to B-1-9 given below except for the 7 genes, or expression products thereof are measured.

[B-13] The method according to [B-11] or [B-12], wherein preferably, expression levels of the at least one gene selected from the group of 7 genes as well as at least one gene selected from the following group of 25 genes or expression products thereof are measured:
ABHD8, GPT2, PLIN2, FAM100B, YPEL2, MAP1LC3B2, RLF, KIAA0930, UBE2R2, HK2, USF2, PDIA3P, HNRNPUL1, SEC61G, DNAJB11, SDHD, NDUFS7, ECH1, CASS4, IL7R, CLEC4A, AREG, SNRPD1, SLC7A11 and SNX8.

[B-14] Use of at least one selected from the group consisting of the following 7 genes: IMPDH2, ERI1, FBXW2, STK17B, TAGLN2, AMICA1 and HNRNPA1 and expression products of the genes derived from a biological sample collected from a child test subject, as a marker for detecting childhood atopic dermatitis.

[B-15] The use according to [B-14], wherein preferably, the genes or the expression products thereof are mRNA contained in skin surface lipids collected from the test subject.

[B-16] The use according to [B-14] or [B-15], wherein preferably, the at least one gene selected from the group of 7 genes or the expression product thereof as well as at least one gene selected from the groups of 371 genes shown in Tables B-1-1 to B-1-9 given below, 100 genes shown in Tables B-3-1 to B-3-3 given below, and 9 genes shown in Table B-4 except for the 7 genes or an expression product thereof is used.

[B-17] A test kit for detecting childhood atopic dermatitis, the kit being used in a method according to any one of [B-1] to [B-13], and comprising an oligonucleotide which specifically hybridizes to the gene or a nucleic acid derived therefrom, or an antibody which recognizes an expression product of the gene.

[B-18] A marker for detecting childhood atopic dermatitis comprising at least one gene selected from the group of 383 genes shown in Tables B-b-1 and B-b-2 described above or an expression product thereof.

[B-19] A marker for detecting childhood atopic dermatitis comprising at least one gene selected from the group of 337 genes shown in the following Tables B-c-1 and B-c-2 or an expression product thereof.

**[Table B-c-1]**

| | | | | | | |
|---|---|---|---|---|---|---|
| AATK | ATP6V1C2 | CHMP5 | DDIT4 | FAM193B | HIP1R | KLHDC3 |
| ABHD8 | BASP1 | CHP1 | DDOST | FAM214A | HIST1H2BK | KLHL21 |
| ACSL4 | BAX | CIB1 | DEFB4B | FAM222B | HK2 | KRT23 |
| ADAM19 | BICD2 | CIDEA | DHCR7 | FBP1 | HLA-DMA | KRT34 |
| ADIPOR1 | BNIP3 | CIITA | DNAJB1 | FBXW2 | HLA-DOA | KRT79 |
| ADIPOR2 | BNIP3L | CLEC4A | DNAJB11 | FBXW4 | HN1L | KRT80 |
| AIM1 | BPGM | CLTB | DNAJC5 | FCHSD1 | HNRNPA1 | KRT86 |
| AKTIP | C10orf128 | CORO1B | DNASE1L2 | FEM1B | HNRNPUL1 | KRTAP3-1 |
| ALDH2 | C1orf21 | CPEB4 | DSP | FOXO3 | HSP90AA1 | KRTAP4-9 |
| ALDH3B2 | C2orf54 | CPVL | DSTN | GALNT1 | HSPA1B | LAMTOR3 |
| ALYREF | C6orf106 | CRAT | DUSP14 | GAS7 | HYOU1 | LAMTOR4 |
| AMD1 | C6orf62 | CRCP | DUSP16 | GBA2 | ID1 | LOC100093631 |
| AMICA1 | CACUL1 | CRISPLD2 | EAF1 | GCH1 | IMPDH2 | LOC285359 |
| ANPEP | CALML3 | CRK | ECH1 | GDPD3 | INF2 | LPCAT1 |
| ARF1 | CAPG | CST3 | EIF3K | GIPC1 | IRAK1 | LRP10 |
| ARHGAP9 | CARD18 | CTDSP1 | EIF4EBP2 | GLRX | IRAK2 | LST1 |
| ARHGDIB | CASS4 | CTNNBIP1 | EIF5 | GNB2L1 | IRGQ | LYPD5 |
| ARL5A | CCM2 | CTSB | EPB41 | GNG12 | ISG15 | MAP1LC3A |
| ATG2A | CCND2 | CTSC | EPHX3 | GOLGA4 | JUP | MAP1LC3B2 |
| ATMIN | CD52 | CTSD | EPN3 | GPT2 | KCTD20 | MAPK3 |
| ATP2A2 | CD93 | CYB5R1 | ERI1 | GTPBP2 | KDSR | MARCH3 |
| ATP5H | CDC123 | CYBASC3 | FAM100B | H1F0 | KHDRBS1 | MARCKS |
| ATP5J2 | CDC42EP1 | CYTIP | FAM102A | H2AFY | KIAA0513 | MAT2A |
| ATP6V0C | CDKN2B | DBI | FAM108C1 | HDAC7 | KIAA0930 | MEA |
| ATP6V1A | CERK | DDHD1 | FAM188A | HES4 | KIF1C | MED14 |

**[Table B-c-2]**

| | | | | | | |
|---|---|---|---|---|---|---|
| MEST | PDIA6 | RAD23B | SDHD | SPAG1 | TMED3 | USP16 |
| MGLL | PEBP1 | RALGDS | SEC24D | SPEN | TMEM214 | VAT1 |
| MIEN1 | PGRMC2 | RANBP9 | SEC61G | SPNS2 | TMEM33 | VKORC1 |
| MPZL3 | PHLDA2 | RANGAP1 | SEPT5 | SPTLC3 | TMEM86A | VKORC1L1 |
| MSL1 | PIK3AP1 | RARG | SERP1 | SQRDL | TMX2 | VPS13C |
| MSMO1 | PIM1 | RASA4CP | SH3BGRL3 | SQSTM1 | TNIP1 | WBP2 |
| MYZAP | PLB1 | RASAL1 | SH3BP5L | SRPK2 | TPRA1 | YPEL2 |
| NBPF10 | PLD3 | RBM17 | SH3D21 | SSFA2 | TRIM29 | YWHAG |
| NBR1 | PLIN2 | RCC2 | SIAH2 | STARD5 | TSPAN14 | YWHAH |
| NDUFA1 | PLIN3 | RGP1 | SIRPA | STK10 | TSPAN6 | ZDHHC9 |
| NDUFB11 | PPIB | RLF | SLAMF7 | STK17B | TUBA1A | ZFAND2A |
| NDUFS7 | PPP2CB | RMND5B | SLC11A2 | STT3A | TUBA1B | ZFAND5 |
| NEU1 | PQLC1 | RNASET2 | SLC20A1 | SULT2B1 | TUFT1 | ZFAND6 |
| NIPAL2 | PRDM1 | RNF103 | SLC31A1 | SURF1 | TXN2 | ZFP36L2 |
| NOTCH2NL | PRELID1 | RNF11 | SLC39A8 | SYNGR2 | TXNDC17 | ZNF430 |
| NPC1 | PRMT1 | RNF217 | SLC7A11 | SYPL1 | U2AF1 | ZNF664 |
| NPEPPS | PRPF38B | RN F24 | SLK | SYTL1 | UBE2R2 | ZNF91 |
| NTAN1 | PRR24 | RRAD | SMOX | TAGLN2 | UBIAD1 | ZRANB1 |
| NUDT4 | PRSS22 | RUSC2 | SMPD3 | TBC1D17 | UBXN6 | |
| OSBPL2 | PTK2B | S100A16 | SNORA31 | TBC1D20 | ULK1 | |
| OTUD5 | PTK6 | S100A4 | SNORA6 | TEX264 | UNC5B | |
| OXR1 | RAB21 | SCARNA7 | SNRPD1 | TGFBI | UPK3BL | |
| PAPL | RAB27A | SCYL1 | SNX18 | THRSP | USF2 | |
| PDIA3P | RAB7A | SDCBP2 | SNX8 | TM4SF1 | USMG5 | |

[B-20] The marker according to [B-18] or [B-19], wherein preferably, the marker is at least one gene selected from the group of 7 genes consisting of IMPDH2, ERI1, FBXW2, STK17B, TAGLN2, AMICA1 and HNRNPA1 or an expression product thereof.

[B-21] The marker according to [B-18] or [B-19], wherein preferably, the marker is at least one gene selected from the group of 23 genes consisting of ABHD8, GPT2, PLIN2, FAM100B, YPEL2, MAP1LC3B2, RLF, KIAA0930, UBE2R2, HK2, USF2, PDIA3P, HNRNPUL1, SEC61G, DNAJB11, SDHD, NDUFS7, ECH1, CASS4, CLEC4A, SNRPD1, SLC7A11 and SNX8 or an expression product thereof.

[C-1] A method for preparing a protein marker for detecting atopic dermatitis, comprising collecting at least one protein selected from the group consisting of proteins shown in Tables C-1-1 to C-1-13 described above from skin surface lipids collected from a test subject.

[C-2] A method for detecting atopic dermatitis in a test subject, comprising detecting at least one protein selected from the group consisting of proteins shown in Tables C-1-1 to C-1-13 described above from skin surface lipids collected from the test subject.

[C-3] The method according to [C-1] or [C-2], wherein preferably, the at least one protein is
at least one protein selected from the group consisting of proteins shown in Tables C-2-1 to C-2-5, or
at least one protein selected from the group consisting of proteins shown in Tables C-3-1 to C-3-2.

[C-4] The method according to [C-1] or [C-2], wherein
the test subject is preferably a child, and
the at least one protein
is preferably at least one protein selected from the group consisting of proteins shown in Tables C-4-1 to C-4-6,
is more preferably at least one protein selected from the group consisting of proteins shown in Tables C-7-1 to C-7-4 and Table C-8,
is further more preferably at least one protein selected from the group consisting of proteins shown in Tables C-11-1 to C-11-4, at least one protein selected from the group consisting of proteins shown in Tables C-12-1 to C-12-4, or at least one protein selected from the group consisting of proteins shown in Table C-13,
further more preferably comprises at least one protein selected from the group consisting of POF1B, MNDA, SERPINB4, CLEC3B, PLEC, LGALS7, H2AC4, SERPINB3, AMBP, PFN1, DSC3, IGHG1, ORM1, RECQL, RPL26, KLK13, RPL22, APOA2, SERPINB5, LCN15, IGHG3, CAP1 and SPRR2F, and
is further more preferably a combination of at least one protein selected from the group consisting of POF1B, MNDA, SERPINB4, CLEC3B, PLEC, LGALS7, H2AC4, SERPINB3, AMBP, PFN1, DSC3, IGHG1, ORM1, RECQL, RPL26, KLK13, RPL22, APOA2, SERPINB5, LCN15, IGHG3, CAP1 and SPRR2F, and at least one other protein selected from the group consisting of proteins shown in Tables C-11-1 to C-11-4, Tables C-12-1 to C-12-4 and Table C-13.

[C-5] The method according to [C-1] or [C-2], wherein
the test subject is preferably an adult, and
the at least one protein
is preferably at least one protein selected from the group consisting of proteins shown in Tables C-5-1 to C-5-9,
is more preferably at least one protein selected from the group consisting of proteins shown in Tables C-9-1 to C-9-7 and Tables C-10-1 and C-10-2,
is further more preferably at least one protein selected from the group consisting of proteins shown in Tables C-14-1 to C-14-7, at least one protein selected from the group consisting of proteins shown in Tables C-15-1 to C-15-4, or at least one protein selected from the group consisting of proteins shown in Table C-16,
further more preferably comprises at least one protein selected from the group consisting of SERPINB1, TTR, DHX36, ITIH4, GC, ALB, SERPING1, DDX55, IGHV1-46, EZR, VTN, AHSG, HPX, PPIA, KNG1, FN1, PLG, PRDX6 and FLG2, and
is further more preferably a combination of at least one protein selected from the group consisting of SERPINB1, TTR, DHX36, ITIH4, GC, ALB, SERPING1, DDX55, IGHV1-46, EZR, VTN, AHSG, HPX, PPIA, KNG1, FN1, PLG, PRDX6 and FLG2, and at least one other protein selected from the group consisting of proteins shown in Tables C-14-1 to C-14-7, Tables C-15-1 to C-15-4 and Table C-16.

[C-6] The method according to [C-2], wherein
the test subject is preferably a child,
the at least one protein is preferably at least one protein selected from the group consisting of proteins shown in Tables C-7-1 to C-7-4, and
the method preferably comprises detecting the test subject as having atopic dermatitis when a concentration of the at least one protein is increased as compared with a healthy children group.

[C-7] The method according to [C-2], wherein
the test subject is preferably a child,
the at least one protein is preferably at least one protein selected from the group consisting of proteins shown in Table C-8, and
the method preferably comprises detecting the test subject as having atopic dermatitis when a concentration of the at least one protein is decreased as compared with a healthy children group.

[C-8] The method according to [C-2], wherein
the test subject is preferably an adult
the at least one protein is preferably at least one protein selected from the group consisting of proteins shown in Tables C-9-1 to C-9-7, and
the method preferably comprises detecting the test subject as having atopic dermatitis when a concentration of the at least one protein is increased as compared with a healthy adult group.

[C-9] The method according to [C-2], wherein
the test subject is preferably an adult
the at least one protein is preferably at least one protein selected from the group consisting of proteins shown in Tables C-10-1 and C-10-2, and
the method preferably comprises detecting the test subject as having atopic dermatitis when a concentration of the at least one protein is decreased as compared with a healthy adult group.

[C-10] The method according to any one of [C-2] to [C-5], wherein the method
preferably comprises detecting AD on the basis of a prediction model constructed by using a concentration of the at least one protein as an explanatory variable and the presence or absence of AD as an objective variable, and
more preferably comprises detecting AD on the basis of a cutoff value which discriminates between an atopic dermatitis patient and a healthy subject, wherein the cutoff value is calculated from a discriminant which discriminates between the atopic dermatitis patient and the healthy subject, the discriminant being constructed by using a concentration of the at least one protein derived from the atopic dermatitis patient and a concentration of the protein derived from the healthy subject as teacher samples, and the presence or absence of atopic dermatitis in the test subject is evaluated by substituting a concentration of the at least one protein obtained from skin surface lipids of the test subject into the discriminant, and comparing the obtained results with the cutoff value.

[C-11] The method according to any one of [C-2] to [C-10], wherein preferably, skin surface lipids derived from a test subject having atopic dermatitis or suspected of developing atopic dermatitis are detected.

[C-12] The method according to [C-11], wherein
the test subject is preferably a child,
the at least one protein is preferably at least one protein selected from the group consisting of proteins shown in Tables C-7-1 to C-7-4, and
the method preferably comprises detecting the skin surface lipids as being derived from a test subject having atopic dermatitis or suspected of developing atopic dermatitis when a concentration of the at least one protein is increased as compared with a healthy children group.

[C-13] The method according to [C-11], wherein
the test subject is preferably a child,
the at least one protein is preferably at least one protein selected from the group consisting of proteins shown in Table C-8, and
the method preferably comprises detecting the skin surface lipids as being derived from a test subject having atopic dermatitis or suspected of developing atopic dermatitis when a concentration of the at least one protein is decreased as compared with a healthy children group.

[C-14] The method according to [C-11], wherein
the test subject is preferably an adult,
the at least one protein is preferably at least one protein selected from the group consisting of proteins shown in Tables C-9-1 to C-9-7, and
the method preferably comprises detecting the skin surface lipids as being derived from a test subject having atopic dermatitis or suspected of developing atopic dermatitis when a concentration of the at least one protein is increased as compared with a healthy adult group.

[C-15] The method according to [C-11], wherein
the test subject is preferably an adult,
the at least one protein is preferably at least one protein selected from the group consisting of proteins shown in Tables C-10-1 and C-10-2, and
the method preferably comprises detecting the skin surface lipids as being derived from a test subject having atopic dermatitis or suspected of developing atopic dermatitis when a concentration of the at least one protein is decreased as compared with a healthy adult group.

[C-16] The method according to any one of [C-1] to [C-15], further comprising collecting skin surface lipids from the test subject.

[C-17] A protein marker for detecting atopic dermatitis comprising at least one protein selected from the group consisting of proteins shown in Tables C-1-1 to C-1-13 described above.

[C-18] The marker according to [C-17], wherein preferably, the at least one protein is
at least one protein selected from the group consisting of proteins shown in Tables C-2-1 to C-2-5, or
at least one protein selected from the group consisting of proteins shown in Tables C-3-1 to C-3-2.

[C-19] The marker according to [C-17], wherein
the marker is preferably a marker for detecting childhood atopic dermatitis, and
the at least one protein is
preferably at least one protein selected from the group consisting of proteins shown in Tables C-7-1 to C-7-4 and Table C-8,
more preferably at least one protein selected from the group consisting of proteins shown in Tables C-11-1 to C-11-4,
further more preferably at least one protein selected from the group consisting of proteins shown in Tables C-4-1 to C-4-6,
further more preferably at least one protein selected from the group consisting of POF1B, MNDA, SERPINB4, CLEC3B, PLEC, LGALS7, H2AC4, SERPINB3, AMBP, PFN1, DSC3, IGHG1, ORM1, RECQL, RPL26, KLK13, RPL22, APOA2, SERPINB5, LCN15, IGHG3, CAP1 and SPRR2F.

[C-20] The marker according to [C-17], wherein
the marker is preferably a marker for detecting adult atopic dermatitis, and
the at least one protein is
preferably at least one protein selected from the group consisting of proteins shown in Tables C-9-1 to C-9-7 and Tables C-10-1 and C-10-2,
more preferably at least one protein selected from the group consisting of proteins shown in Tables C-14-1 to C-14-7,
further more preferably at least one protein selected from the group consisting of proteins shown in Tables C-5-1 to C-5-9,
further more preferably at least one protein selected from the group consisting of SERPINB1, TTR, DHX36, ITIH4, GC, ALB, SERPING1, DDX55, IGHV1-46, EZR, VTN, AHSG, HPX, PPIA, KNG1, FN1, PLG, PRDX6 and FLG2.

[C-21] Use of at least one protein selected from the group consisting of proteins shown in Tables C-1-1 to C-1-13 described above as a marker for detecting atopic dermatitis.

[C-22] Use of at least one protein selected from the group consisting of proteins shown in Tables C-1-1 to C-1-13 described above in the production of a protein marker for detecting atopic dermatitis.

[C-23] The use according to [C-21] or [C-22], wherein preferably, the at least one protein is
at least one protein selected from the group consisting of proteins shown in Tables C-2-1 to C-2-5, or
at least one protein selected from the group consisting of proteins shown in Tables C-3-1 to C-3-2.

[C-24] The use according to [C-21] or [C-22], wherein
the marker is preferably a marker for detecting childhood atopic dermatitis, and
the at least one protein is
preferably at least one protein selected from the group consisting of proteins shown in Tables C-7-1 to C-7-4 and Table C-8,
more preferably at least one protein selected from the group consisting of proteins shown in Tables C-11-1 to C-11-4,
further more preferably at least one protein selected from the group consisting of proteins shown in Tables C-4-1 to C-4-6,
further more preferably at least one protein selected from the group consisting of POF1B, MNDA, SERPINB4, CLEC3B, PLEC, LGALS7, H2AC4, SERPINB3, AMBP, PFN1, DSC3, IGHG1, ORM1, RECQL, RPL26, KLK13, RPL22, APOA2, SERPINB5, LCN15, IGHG3, CAP1 and SPRR2F.

[C-25] The use according to [C-21] or [C-22], wherein
the marker is preferably a marker for detecting adult atopic dermatitis, and
the at least one protein is
preferably at least one protein selected from the group consisting of proteins shown in Tables C-9-1 to C-9-7 and Tables C-10-1 and C-10-2,
more preferably at least one protein selected from the group consisting of proteins shown in Tables C-14-1 to C-14-7,
further more preferably at least one protein selected from the group consisting of proteins shown in Tables C-5-1 to C-5-9,
further more preferably at least one protein selected from the group consisting of SERPINB1, TTR, DHX36, ITIH4, GC, ALB, SERPING1, DDX55, IGHV1-46, EZR, VTN, AHSG, HPX, PPIA, KNG1, FN1, PLG, PRDX6 and FLG2.

[D-1] A method for detecting childhood atopic dermatitis in a child test subject, comprising a step of measuring an expression level of SerpinB4 protein in skin surface lipids collected from the test subject.

[D-2] The method according to [D-1], preferably, further comprising detecting the presence or absence of childhood atopic dermatitis, or a degree of progression thereof by comparing the measurement value of the expression level of SerpinB4 protein with a reference value.

[D-3] The method according to [D-2], wherein preferably, the detection of the degree of progression of childhood atopic dermatitis is detection of mild or moderate atopic dermatitis.

[D-4] The method according to any one of [D-1] to [D-3], wherein preferably, the child is a 0- to 5-year-old child.

[D-5] The method according to any one of [D-1] to [D-4], preferably, further comprising collecting skin surface lipids from the test subject.

[D-6] A test kit for detecting childhood atopic dermatitis, the kit being used in a method according to any one of [D-1] to [D-5], and comprising an antibody which recognizes SerpinB4 protein.

[D-7] Use of SerpinB4 protein in skin surface lipids collected from a child test subject for detecting childhood atopic dermatitis.

[D-8] The use according to [D-7], preferably, for detecting the presence or absence of childhood atopic dermatitis, or a degree of progression thereof.

[D-9] The use according to [D-8], wherein preferably, the detection of the degree of progression of childhood atopic dermatitis is detection of mild or moderate atopic dermatitis.

[D-10] The use according to any one of [D-7] to [D-9] preferably, the child is a 0- to 5-year-old child.

### Examples

Hereinafter, the present invention will be described in more detail with reference to Examples. However, the present invention is not limited by these examples.

### Example A-1 Detection of differentially expressed gene related to atopic dermatitis in RNA extracted from SSL

### 1) SSL collection

14 healthy adult subjects (HL) (from 25 to 57 years old, male) and 29 adults having atopic skin (AD) (from 23 to 56 years old, male) were selected as test subjects. The test subjects with atopic dermatitis were each diagnosed as having eruption at least on the face area and having mild or moderate atopic dermatitis in terms of severity by a dermatologist. Sebum was collected from the whole face (including an eruption site for the AD patients) of each test subject using an oil blotting film (5 × 8 cm, made of polypropylene, 3M Company). Then, the oil blotting film was transferred to a vial and preserved at -80°C for approximately 1 month until use in RNA extraction.

### 2) RNA preparation and sequencing

The oil blotting film of the above section 1) was cut into an appropriate size, and RNA was extracted using QIAzol Lysis Reagent (Qiagen N.V.) in accordance with the attached protocol. On the basis of the extracted RNA, cDNA was synthesized through reverse transcription at 42°C for 90 minutes using Superscript VILO cDNA Synthesis kit (Life Technologies Japan Ltd.). The primers used for reverse transcription reaction were random primers attached to the kit. A library containing DNA derived from 20802 genes was prepared by multiplex PCR from the obtained cDNA. The multiplex PCR was performed using Ion AmpliSeq Transcriptome Human Gene Expression Kit (Life Technologies Japan Ltd.) under conditions of [99°C, 2 min → (99°C, 15 sec -> 62°C, 16 min) × 20 cycles -> 4°C, hold]. The obtained PCR product was purified with Ampure XP (Beckman Coulter Inc.), followed by buffer reconstitution, primer sequence digestion, adaptor ligation, purification, and amplification to prepare a library. The prepared library was loaded on Ion 540 Chip and sequenced using Ion S5/XL system (Life Technologies Japan Ltd.).

### 3) Data analysis

### i) Data used

Data (read count values) on the expression level of RNA derived from the test subjects measured in the above section 2) was normalized by use of an approach called DESeq2. However, only 7429 genes which produced expression level data without missing values in 90% or more sample test subjects among the expression level data from all the sample test subjects were used in analysis given below. In the analysis, normalized count values obtained by use of an approach called DESeq2 were used.

### ii) RNA expression analysis

On the basis of the SSL-derived RNA expression levels (normalized count values) of the healthy subjects and AD measured in the above section i), RNA which attained a corrected p value (FDR) of less than 0.05 in a likelihood ratio test in AD compared with the healthy subjects (differentially expressed gene) was identified. As a result, the expression of 75 RNAs was decreased (DOWN) in AD, and the expression of 48 RNAs was increased (UP) in AD (Tables A-1-1 to A-1-3).

**[Table A-1-1]**

| | Gene Symbol | log2(FoldChange) | FDR | Regulation |
|---|---|---|---|---|
| * | **ACAT1** | -1.08533 | 0.03109 | DOWN |
| * | **ARHGAP24** | -1.98798 | 0.02314 | DOWN |
| * | **ARHGAP29** | -1.22671 | 0.02314 | DOWN |
| * | **ARRDC4** | -1.16199 | 0.02956 | DOWN |
| * | **ATP5A1** | -0.84424 | 0.02782 | DOWN |
| * | **ATPIF1** | -1.48084 | 0.03179 | DOWN |
| * | **BCKDHB** | -1.38255 | 0.02956 | DOWN |
| * | **C15orf23** | -1.20994 | 0.04823 | DOWN |
| * | **C16orf70** | -1.22700 | 0.04791 | DOWN |
| * | **C4orf52** | -1.15134 | 0.04522 | DOWN |
| * | **CDS1** | -1.97382 | 0.02314 | DOWN |
| * | **CEP76** | -1.29082 | 0.02946 | DOWN |
| * | **CETN2** | -1.04482 | 0.02956 | DOWN |
| * | **CHMP4C** | -1.26781 | 0.02314 | DOWN |
| * | **COBLL1** | -1.41045 | 0.02314 | DOWN |
| * | **COPS2** | -0.53728 | 0.04823 | DOWN |
| * | **COX6A1** | -0.58517 | 0.02678 | DOWN |
| * | **COX7B** | -0.60501 | 0.02314 | DOWN |
| * | **CREG1** | -1.60383 | 0.03889 | DOWN |
| | CTSL2 | -1.31488 | 0.03464 | DOWN |
| * | **DBT** | -1.26046 | 0.01247 | DOWN |
| * | **DHX32** | -0.92977 | 0.03678 | DOWN |
| * | **DPYSL3** | -1.25879 | 0.03889 | DOWN |
| * | **EIF1AD** | -0.99475 | 0.03277 | DOWN |
| * | **FABP7** | -2.32742 | 0.02314 | DOWN |
| * | **FAM26E** | -1.48483 | 0.02314 | DOWN |
| * | **FBXL17** | -1.83949 | 0.03639 | DOWN |
| * | **FBXO32** | -1.29629 | 0.02800 | DOWN |
| * | **FDFT1** | -0.92847 | 0.03669 | DOWN |
| * | **FIS1** | -0.78645 | 0.03464 | DOWN |
| * | **FMN1** | -1.67297 | 0.03277 | DOWN |
| | FOXQ1 | -1.56465 | 0.04242 | DOWN |
| * | **GDE1** | -1.24003 | 0.02314 | DOWN |
| * | **GLRX** | -0.87673 | 0.02862 | DOWN |
| * | **GSDMA** | -1.43665 | 0.02832 | DOWN |
| * | **HADHA** | -0.89711 | 0.02314 | DOWN |
| * | **HBP1** | -1.09167 | 0.03922 | DOWN |
| * | **HINT3** | -1.36273 | 0.02862 | DOWN |
| * | **HMGCL** | -1.12701 | 0.02314 | DOWN |
| | **HMGCS1** | -1.05483 | 0.02826 | DOWN |
| * | **ISCA1** | -1.16275 | 0.03901 | DOWN |

**[Table A-1-2]**

| | | | | |
|---|---|---|---|---|
| * | **MAPKBP1** | -1.05065 | 0.02862 | DOWN |
| * | **MECR** | -1.62760 | 0.01247 | DOWN |
| * | **MLLT11** | -1.87795 | 0.02314 | DOWN |
| * | **MYO6** | -1.31978 | 0.02314 | DOWN |
| * | **NDUFA4** | -0.67215 | 0.03678 | DOWN |
| | NPR2 | -1.48136 | 0.02314 | DOWN |
| * | **PADI1** | -1.78745 | 0.02314 | DOWN |
| * | **PCTP** | -1.15559 | 0.02314 | DOWN |
| * | **PDZK1** | -1.45245 | 0.02826 | DOWN |
| * | **PINK1** | -1.74630 | 0.01247 | DOWN |
| * | **PMVK** | -1.08518 | 0.02862 | DOWN |
| | PNPLA1 | -1.49296 | 0.02721 | DOWN |
| * | **PPA1** | -0.92154 | 0.02314 | DOWN |
| * | **PSMA5** | -0.58569 | 0.03678 | DOWN |
| * | **RAI14** | -1.43072 | 0.03678 | DOWN |
| * | **RASA4CP** | -1.36595 | 0.02314 | DOWN |
| * | **RB1CC1** | -0.95244 | 0.02826 | DOWN |
| | RORC | -1.53822 | 0.03615 | DOWN |
| * | **RPS6KB2** | -1.03893 | 0.04986 | DOWN |
| * | **RRM1** | -1.19718 | 0.03889 | DOWN |
| * | **SLC25A16** | -1.42379 | 0.03678 | DOWN |
| * | **SLC31A1** | -1.13960 | 0.03926 | DOWN |
| | SPINK5 | -1.46883 | 0.04823 | DOWN |
| * | **TEX2** | -1.12592 | 0.03889 | DOWN |
| * | **TMC5** | -1.84795 | 0.02862 | DOWN |
| * | **TMPRSS11E** | -1.11373 | 0.03901 | DOWN |
| * | **TPGS2** | -1.67682 | 0.02314 | DOWN |
| * | **TSTD1** | -0.96556 | 0.02603 | DOWN |
| * | **UQCRQ** | -0.80236 | 0.03889 | DOWN |
| * | **WBSCR16** | -1.79812 | 0.02314 | DOWN |
| * | **XKRX** | -1.39190 | 0.02314 | DOWN |
| * | **ZC3H15** | -0.72586 | 0.04792 | DOWN |
| * | **ZNF664** | -1.05672 | 0.02314 | DOWN |
| * | **ZNF706** | -0.92443 | 0.03678 | DOWN |
| * | **ADAP2** | 1.03743 | 0.04823 | UP |
| | ANXA1 | 1.12224 | 0.02982 | UP |
| * | **APOBR** | 0.85042 | 0.02314 | UP |
| * | **ARHGAP4** | 1.18905 | 0.02826 | UP |
| * | **C19orf71** | 1.69039 | 0.03615 | UP |
| * | **C1QB** | 1.29287 | 0.03678 | UP |
| | CAPN1 | 0.87723 | 0.02314 | UP |

**[Table A-1-3]**

| | | | | |
|---|---|---|---|---|
| * | **CCDC88B** | 1.09586 | 0.02314 | UP |
| * | **CCND3** | 0.87706 | 0.02862 | UP |
| * | **CRTC2** | 1.32316 | 0.02314 | UP |
| * | **CSNK1G2** | 0.87945 | 0.03889 | UP |
| * | **CTBP1** | 1.26144 | 0.01247 | UP |
| * | **DGKA** | 1.17078 | 0.02314 | UP |
| * | **DNASE1L1** | 1.13695 | 0.03615 | UP |
| | EFHD2 | 0.83078 | 0.04242 | UP |
| | EHBP1L1 | 1.04466 | 0.03277 | UP |
| * | **FAM120A** | 0.48177 | 0.03615 | UP |
| * | **FOSB** | 1.21823 | 0.02786 | UP |
| * | **GIGYF1** | 1.14204 | 0.03889 | UP |
| * | **GNB2** | 0.64265 | 0.03678 | UP |
| * | **GRASP** | 1.62097 | 0.02314 | UP |
| | HLA-B | 7.00492 | 0.02284 | UP |
| * | **KIAA0146** | 2.04960 | 0.02826 | UP |
| * | **LMNA** | 0.86976 | 0.02894 | UP |
| * | **LOC146880** | 0.88138 | 0.03277 | UP |
| | MARK2 | 1.12583 | 0.03987 | UP |
| * | **MINK1** | 0.94470 | 0.03179 | UP |
| * | **MTSS1** | 1.43861 | 0.02314 | UP |
| * | **MVP** | 0.68340 | 0.04564 | UP |
| * | **NCOR2** | 0.96150 | 0.02314 | UP |
| * | **NPEPL1** | 0.95309 | 0.04242 | UP |
| | NPR1 | 1.80891 | 0.03889 | UP |
| * | **NUDT16** | 1.25760 | 0.03889 | UP |
| * | **PCSK7** | 0.97945 | 0.03464 | UP |
| * | **PLP2** | 1.07700 | 0.02678 | UP |
| * | **PPP1R12C** | 0.98301 | 0.02314 | UP |
| * | **PPP1R9B** | 0.94437 | 0.02314 | UP |
| | RAC1 | 0.38603 | 0.03922 | UP |
| * | **RHOC** | 0.94634 | 0.03615 | UP |
| * | **SNORA8** | 1.09004 | 0.02314 | UP |
| * | **SNORD17** | 0.79644 | 0.03889 | UP |
| * | **SPDYE7P** | 1.26833 | 0.02314 | UP |
| | TGFB1 | 0.74610 | 0.03370 | UP |
| * | **TNRC18** | 0.99095 | 0.02314 | UP |
| * | **UNC13D** | 1.30904 | 0.03109 | UP |
| * | **VOPP1** | 0.84946 | 0.02314 | UP |
| * | **ZFP36L2** | 0.72030 | 0.03370 | UP |
| * | **ZNF335** | 1.10574 | 0.01247 | UP |

123 genes shown in Tables A-1-1 to A-1-3 were searched for a biological process (BP) by gene ontology (GO) enrichment analysis using the public database STRING. As a result, 27 BPs related to the gene group with decreased expression in the AD patients were obtained and found to include a term related to lipid metabolism or amino acid metabolism (Table A-2), and 4 BPs related to the gene group with increased expression were obtained and found to include a term related to leucocyte activation, or the like (Table A-2). On the other hand, 107 genes (indicated by boldface with * added in each table) among 123 genes shown in Tables A-1-1 to A-1-3 described above were confirmed to be capable of serving as novel atopic dermatitis markers because there was not previous report suggesting their relation to atopic dermatitis.

**[Table A-2]**

| ID | Term description (Biological process) | FDR | Regulation |
|---|---|---|---|
| GO:0006091 | generation of precursor metabolites and energy | 0.0005 | DOWN |
| GO:0044281 | small molecule metabolic process | 0.0220 | DOWN |
| GO:0006629 | lipid metabolic process | 0.0227 | DOWN |
| GO:0007005 | mitochondrion organization | 0.0227 | DOWN |
| GO:0008299 | isoprenoid biosynthetic process | 0.0227 | DOWN |
| GO:0009081 | branched-chain amino acid metabolic process | 0.0227 | DOWN |
| GO:0009083 | branched-chain amino acid catabolic process | 0.0227 | DOWN |
| GO:0009117 | nucleotide metabolic process | 0.0227 | DOWN |
| GO:0009150 | purine ribonucleotide metabolic process | 0.0227 | DOWN |
| GO:0019637 | organophosphate metabolic process | 0.0227 | DOWN |
| GO:0022900 | electron transport chain | 0.0227 | DOWN |
| GO:0036314 | response to sterol | 0.0227 | DOWN |
| GO:0044242 | cellular lipid catabolic process | 0.0227 | DOWN |
| GO:0044255 | cellular lipid metabolic process | 0.0227 | DOWN |
| GO:0055086 | nucleobase-containing small molecule metabolic process | 0.0227 | DOWN |
| GO:0055114 | oxidation-reduction process | 0.0227 | DOWN |
| GO:1903533 | regulation of protein targeting | 0.0227 | DOWN |
| GO:1900425 | negative regulation of defense response to bacterium | 0.0290 | DOWN |
| GO:0010822 | positive regulation of mitochondrion organization | 0.0302 | DOWN |
| GO:0022904 | respiratory electron transport chain | 0.0364 | DOWN |
| GO:0000422 | autophagy of mitochondrion | 0.0372 | DOWN |
| GO:0006119 | oxidative phosphorylation | 0.0372 | DOWN |
| GO:0006695 | cholesterol biosynthetic process | 0.0372 | DOWN |
| GO:0045540 | regulation of cholesterol biosynthetic process | 0.0372 | DOWN |
| GO:0046503 | glycerolipid catabolic process | 0.0372 | DOWN |
| GO:0046951 | ketone body biosynthetic process | 0.0372 | DOWN |
| GO:0019218 | regulation of steroid metabolic process | 0.0431 | DOWN |
| GO:0001775 | cell activation | 0.0254 | UP |
| GO:0045321 | leukocyte activation | 0.0254 | UP |
| GO:0002694 | regulation of leukocyte activation | 0.0449 | UP |
| GO:0048771 | tissue remodeling | 0.0449 | UP |

Example A-2 Construction of discriminant model using gene with high variable importance in random forest 1) Data used

Data (read count values) on the expression level of SSL-derived RNA from the test subjects was obtained in the same manner as in Example A-1 and converted to RPM values which normalized the read count values for difference in the total number of reads among samples. However, only 7429 genes which produced expression level data without missing values in 90% or more samples in all the samples were used in analysis given below. In the construction of machine learning models, logarithmic values to base 2 plus integer 1 (Log₂(RPM + 1) values) were used in order to approximate the RPM values, which followed negative binominal distribution, to normal distribution.

### 2) Selection of feature gene

In order to select feature genes using random forest algorithm, the Log₂(RPM + 1) values of 7429 genes which produced expression level data without missing values in 90% or more samples in all the samples were used as explanatory variables, and the healthy subjects (HL) and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and top 150 genes of variable importance based on Gini coefficient were calculated (Tables A-3-1 to A-3-4). These 150 genes or 127 genes (indicated by boldface with * added in each table) whose relation to atopic dermatitis had not been reported so far were selected as feature genes.

**[Table A-3-1]**

| | Rank | Gene Symbol | Mean Decrease Gini |
|---|---|---|---|
| * | 1 | **TMPRSS11E** | 0.204087 |
| * | 2 | **CTBP1** | 0.187037 |
| * | 3 | **C19orf71** | 0.149372 |
| * | 4 | **CTDSP1** | 0.141099 |
| * | 5 | **NCS1** | 0.139139 |
| * | 6 | **FDFT1** | 0.129546 |
| * | 7 | **FBXL6** | 0.118753 |
| | 8 | IL17RA | 0.117211 |
| * | 9 | **ZNF335** | 0.112427 |
| * | 10 | **ZNF706** | 0.111978 |
| | 11 | PPBP | 0.101680 |
| * | 12 | **BCRP3** | 0.101202 |
| * | 13 | **GNA15** | 0.100816 |
| * | 14 | **RHOC** | 0.100750 |
| * | 15 | **TTC39B** | 0.098869 |
| * | 16 | **PCSK7** | 0.096900 |
| * | 17 | **ARRDC4** | 0.096863 |
| * | 18 | **LOC152217** | 0.096284 |
| * | 19 | **RNPEPL1** | 0.095320 |
| * | 20 | **EIF1AD** | 0.093756 |
| | 21 | SIRT6 | 0.092836 |
| * | 22 | **VOPP1** | 0.091970 |
| * | 23 | **SPDYE7P** | 0.089451 |
| * | 24 | **ARL8A** | 0.088270 |
| * | 25 | **LENG9** | 0.087649 |
| * | 26 | **DNASE1L1** | 0.087504 |
| * | 27 | **NIPSNAP3A** | 0.085475 |
| * | 28 | **SRF** | 0.083433 |
| * | 29 | **RB1CC1** | 0.082409 |
| * | 30 | **PTPN18** | 0.077605 |
| * | 31 | **RAB11FIP5** | 0.076648 |
| * | 32 | **MIR548I1** | 0.075200 |
| * | 33 | **AKAP17A** | 0.071995 |
| * | 34 | **NMRK1** | 0.071131 |
| * | 35 | **LCE2C** | 0.070540 |
| * | 36 | **PPP1R9B** | 0.069973 |
| * | 37 | **NPEPL1** | 0.069559 |
| * | 38 | **ST6GALNAC2** | 0.066441 |

**[Table A-3-2]**

| | | | |
|---|---|---|---|
| * | 39 | **PALD1** | 0.065745 |
| * | 40 | **SLC12A9** | 0.061805 |
| | 41 | CAPN1 | 0.059985 |
| * | 42 | **MECR** | 0.059949 |
| * | 43 | **TEX2** | 0.058748 |
| * | 44 | **PPP1R12C** | 0.058420 |
| * | 45 | **SLC2A4RG** | 0.058353 |
| * | 46 | **DGKA** | 0.058266 |
| * | 47 | **TMEM222** | 0.057258 |
| * | 48 | **CSNK1G2** | 0.057078 |
| * | 49 | **CYTH2** | 0.056003 |
| * | 50 | **DOPEY2** | 0.055810 |
| | 51 | GPNMB | 0.055471 |
| * | 52 | **C2CD2** | 0.054456 |
| | 53 | ANXA1 | 0.054326 |
| * | 54 | **OAT** | 0.053253 |
| * | 55 | **SKP1** | 0.052479 |
| * | 56 | **CISDl** | 0.052319 |
| * | 57 | **OGFR** | 0.052175 |
| | 58 | TCHHL1 | 0.052092 |
| * | 59 | **TWSG1** | 0.050930 |
| * | 60 | **ARHGAP23** | 0.050450 |
| * | 61 | **FABP9** | 0.050425 |
| * | 62 | **GSDMA** | 0.049977 |
| | 63 | HMGCS1 | 0.049842 |
| * | 64 | **SH3BGRL2** | 0.049557 |
| * | 65 | **DSTN** | 0.049485 |
| * | 66 | **SLC25A33** | 0.048103 |
| * | 67 | **ATOX1** | 0.048013 |
| * | 68 | **MINK1** | 0.047908 |
| * | 69 | **WDR26** | 0.047882 |
| | 70 | SFN | 0.047672 |
| * | 71 | **RGS19** | 0.047523 |
| * | 72 | **CSTB** | 0.047345 |
| * | 73 | **MAZ** | 0.047219 |
| * | 74 | **GABARAPL2** | 0.047181 |
| * | 75 | **CARD18** | 0.047149 |
| * | 76 | **HMHA1** | 0.047113 |

**[Table A-3-3]**

| | | | |
|---|---|---|---|
| * | 77 | **ACO1** | 0.046927 |
| * | 78 | **COX6A1** | 0.046810 |
| * | 79 | **BAX** | 0.046506 |
| * | 80 | **ATXN7L3B** | 0.045629 |
| * | 81 | **XPO5** | 0.045495 |
| * | 82 | **RASA4CP** | 0.045352 |
| * | 83 | **FIS1** | 0.044891 |
| * | 84 | **ATP12A** | 0.044206 |
| | 85 | LYNX1 | 0.044191 |
| * | 86 | **CRISPLD2** | 0.043741 |
| * | 87 | **PSMB4** | 0.043307 |
| * | 88 | **VHL** | 0.043307 |
| * | 89 | **KRT23** | 0.043276 |
| * | 90 | **MAN2A2** | 0.043058 |
| * | 91 | **MLL2** | 0.042563 |
| | 92 | IL2RB | 0.042522 |
| | 93 | PCDH1 | 0.042469 |
| * | 94 | **MLLT11** | 0.041846 |
| * | 95 | **SAP30BP** | 0.040434 |
| * | 96 | **LY6D** | 0.040427 |
| | 97 | CAMP | 0.040185 |
| * | 98 | **COX7B** | 0.040067 |
| * | 99 | **COPS2** | 0.039721 |
| * | 100 | **MKNK2** | 0.039231 |
| * | 101 | **NR1D1** | 0.038569 |
| * | 102 | **GRN** | 0.038385 |
| | 103 | CXCL16 | 0.038156 |
| * | 104 | **SSH1** | 0.037729 |
| | 105 | AKT1 | 0.037578 |
| * | 106 | **CRTC2** | 0.037339 |
| * | 107 | **KIAA0513** | 0.037080 |
| * | 108 | **ZFP36L2** | 0.037044 |
| * | 109 | **MVP** | 0.036872 |
| * | 110 | **SMARCD1** | 0.036582 |
| * | 111 | **HINT3** | 0.036333 |
| * | 112 | **ZC3H18** | 0.036219 |
| | 113 | CDK9 | 0.036007 |
| * | 114 | RPS6KB2 | 0.035977 |

**[Table A-3-4]**

| | | | |
|---|---|---|---|
| * | 115 | **FURIN** | 0.035848 |
| * | 116 | **FAM108B1** | 0.035848 |
| | 117 | SHC1 | 0.035686 |
| * | 118 | **SCARB2** | 0.035283 |
| * | 119 | **LCE1D** | 0.035208 |
| * | 120 | **ILF3** | 0.034809 |
| * | 121 | **PLAA** | 0.034438 |
| * | 122 | **MEMO1** | 0.034307 |
| * | 123 | **LEPREL1** | 0.034003 |
| | 124 | THBD | 0.033427 |
| * | 125 | **RABL6** | 0.033283 |
| | 126 | PRSS8 | 0.033115 |
| * | 127 | **FAM190B** | 0.032669 |
| * | 128 | **FBXL18** | 0.032483 |
| * | 129 | **POLD4** | 0.032417 |
| * | 130 | **PHB** | 0.032271 |
| * | 131 | **LRP8** | 0.032085 |
| * | 132 | **MLL4** | 0.031603 |
| * | 133 | **GSE1** | 0.031507 |
| * | 134 | **DBNDD2** | 0.031053 |
| | 135 | TGFB1 | 0.030916 |
| | 136 | TYK2 | 0.030700 |
| * | 137 | **C17orfl07** | 0.030475 |
| | 138 | BSG | 0.030191 |
| * | 139 | **EMP3** | 0.030165 |
| * | 140 | **CTSB** | 0.030136 |
| * | 141 | **DUSP16** | 0.030029 |
| * | 142 | **TM7SF2** | 0.029959 |
| * | 143 | **GTF2H2** | 0.029515 |
| * | 144 | **TMEM165** | 0.029070 |
| * | 145 | **CRY2** | 0.029054 |
| * | 146 | **PARP4** | 0.028779 |
| * | 147 | **SNORA71C** | 0.028744 |
| * | 148 | **GNB2** | 0.028466 |
| * | 149 | **ITPRIPL2** | 0.028286 |
| | 150 | RAC1 | 0.028231 |

### 3) Model construction

The Loga(RPM + 1) values of the 150 genes or the 127 genes were used as explanatory variables, and HL and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an estimate error rate (OOB error rate) was calculated. As a result, the OOB error rate was 6.98% in the model using the 150 genes and was 6.98% in the model using the 127 genes.

### Example A-3 Construction of discriminant model using differentially expressed gene

### 1) Data used

Data (read count values) on the expression level of SSL-derived RNA from the test subjects was obtained in the same manner as in Example A-1 and converted to RPM values which normalized the read count values for difference in the total number of reads among samples. In the construction of machine learning models, logarithmic values to base 2 plus integer 1 (Log₂(RPM + 1) values) were used in order to approximate the RPM values, which followed negative binominal distribution, to normal distribution.

### 2) Selection of feature gene

123 genes whose expression significantly differed in AD compared with the healthy subjects (HL) (Tables A-1-1 to A-1-3) in Example A-1, or 107 genes (indicated by boldface with * added in each table) whose relation to atopic dermatitis had not been reported so far were selected as feature genes.

### 3) Model construction

The Log₂(RPM + 1) values of the 123 genes or the 107 genes were used as explanatory variables, and HL and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the OOB error rate was 13.95% in the model using the 123 genes and was 13.95% in the model using the 107 genes.

### Example A-4 Construction of discriminant model using feature gene extracted by Boruta method

### 1) Data used

Data (read count values) on the expression level of SSL-derived RNA from the test subjects was obtained in the same manner as in Example A-1 and converted to RPM values which normalized the read count values for difference in the total number of reads among samples. However, only 7429 genes which produced expression level data without missing values in 90% or more samples in all the samples were used in analysis given below. In the construction of machine learning models, logarithmic values to base 2 plus integer 1 (Log₂(RPM + 1) values) were used in order to approximate the RPM values, which followed negative binominal distribution, to normal distribution.

### 2) Selection of feature gene

The Log₂(RPM + 1) values of 7429 genes which produced expression level data without missing values in 90% or more samples in all the samples were used as explanatory variables, and the healthy subjects (HL) and AD were used as objective variables. Algorithm in the "Boruta" package of R language was carried out. The maximum number of trials was set to 1,000, and 45 genes which attained a p value of less than 0.01 were calculated (Table A-4). These 45 genes or 39 genes (indicated by boldface with * added in Table A-4) whose relation to atopic dermatitis had not been reported so far were selected as feature genes.

**[Table A-4]**

| | Gene Symbol | | | Gene Symbol |
|---|---|---|---|---|
| * | **ARRDC4** | | * | **PLEKHG2** |
| * | **BCRP3** | | * | **PMVK** |
| | CAPN1 | | * | **PPA1** |
| * | **CCDC88B** | | | PPBP |
| * | **CSNK1G2** | | * | **PPP1R9B** |
| * | **CTBP1** | | * | **RASA4CP** |
| * | **CTDSP1** | | * | **RGS19** |
| * | **DGKA** | | * | **RPS6KB2** |
| * | **DNASE1L1** | | | SIRT6 |
| * | **DYNLL1** | | * | **SKP1** |
| * | **EIF1AD** | | * | **SMAP2** |
| * | **FDFT1** | | * | **SPDYE7P** |
| * | **GNA15** | | * | **SSH1** |
| * | **GNB2** | | * | **TEX2** |
| * | **GPD1** | | * | **TMPRSS11E** |
| | HMGCS1 | | * | **TTC39B** |
| | IL2RB | | * | **U2AF2** |
| | KLK5 | | * | **USP38** |
| * | **KRT25** | | * | **VPS4B** |
| * | **KRT71** | | * | **ZMIZ1** |
| * | **MAPK3** | | * | **ZNF335** |
| * | **MECR** | | * | **ZNF706** |
| * | **MIR548I1** | | | |

### 3) Model construction

The Loga(RPM + 1) values of the 45 genes or the 39 genes were used as explanatory variables, and HL and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the OOB error rate was 6.98% in the model using the 45 genes and was 9.3% in the model using the 39 genes.

Example A-5 Construction of discriminant model based on feature gene duplicately used in plurality of Examples

### 1) Data used

Data (read count values) on the expression level of SSL-derived RNA from the test subjects was obtained in the same manner as in Example A-1 and converted to RPM values which normalized the read count values for difference in the total number of reads among samples. In the construction of machine learning models, logarithmic values to base 2 plus integer 1 (Log₂(RPM + 1) values) were used in order to approximate the RPM values, which followed negative binominal distribution, to normal distribution.

### 2) Selection of feature gene

Among the feature genes used in Examples A-2 to A-4, the genes used in all of Examples A-2 to A-4 were 19 genes, MECR, RASA4CP, HMGCS1, ARRDC4, EIF1AD, FDFT1, ZNF706, TEX2, TMPRSS11E, RPS6KB2, CTBP1, ZNF335, CAPN1, DGKA, PPP1R9B, SPDYE7P, DNASE1L1, GNB2, and CSNK1G2 (Table A-5). Among these 19 genes, 17 genes (indicated by boldface with * added in Table A-5) whose relation to atopic dermatitis had not been reported so far were selected as feature genes.

### 3) Model construction

The Logz(RPM + 1) values of the 17 genes were used as explanatory variables, and HL and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the OOB error rate was 6.98%.

**[Table A-5]**

| | Gene Symbol |
|---|---|
| * | **ARRDC4** |
| | CAPN1 |
| * | **CSNK1G2** |
| * | **CTBP1** |
| * | **DGKA** |
| * | **DNASE1L1** |
| * | **EIF1AD** |
| * | **FDFT1** |
| * | **GNB2** |
| | HMGCS1 |
| * | **MECR** |
| * | **PPP1R9B** |
| * | **RASA4CP** |
| * | **RPS6KB2** |
| * | **SPDYE7P** |
| * | **TEX2** |
| * | **TMPRSS11E** |
| * | **ZNF335** |
| * | **ZNF706** |

### Example B-1 Detection of differentially expressed gene related to childhood atopic dermatitis in RNA extracted from SSL

### 1) SSL collection

28 children with healthy skin (HL) (from 6 months after birth to 5 years old, male and female) and 25 children with atopic dermatitis (AD) (from 6 months after birth to 5 years old, male and female) were selected as test subjects. The children with atopic dermatitis were each diagnosed as having eruption on the whole face and having low grade or intermediate grade atopic dermatitis in terms of severity by a dermatologist. Sebum was collected from the whole face (including an eruption site for AD) of each test subject using an oil blotting film (5 × 8 cm, made of polypropylene, 3M Company). Then, the oil blotting film was transferred to a vial and preserved at -80°C for approximately 1 month until use in RNA extraction.

### 2) RNA preparation and sequencing

The oil blotting film of the above section 1) was cut into an appropriate size, and RNA was extracted using QIAzol Lysis Reagent (Qiagen N.V.) in accordance with the attached protocol. On the basis of the extracted RNA, cDNA was synthesized through reverse transcription at 42°C for 90 minutes using Superscript VILO cDNA Synthesis kit (Life Technologies Japan Ltd.). The primers used for reverse transcription reaction were random primers attached to the kit. A library containing DNA derived from 20802 genes was prepared by multiplex PCR from the obtained cDNA. The multiplex PCR was performed using Ion AmpliSeq Transcriptome Human Gene Expression Kit (Life Technologies Japan Ltd.) under conditions of [99°C, 2 min → (99°C, 15 sec -> 62°C, 16 min) × 20 cycles -> 4°C, hold]. The obtained PCR product was purified with Ampure XP (Beckman Coulter Inc.), followed by buffer reconstitution, primer sequence digestion, adaptor ligation, purification, and amplification to prepare a library. The prepared library was loaded on Ion 540 Chip and sequenced using Ion S5/XL system (Life Technologies Japan Ltd.).

### 3) Data analysis

### i) Data used

Data (read count values) on the expression level of RNA derived from the test subjects measured in the above section 2) was normalized by use of an approach called DESeq2. However, only 3486 genes which produced expression level data without missing values in 90% or more sample test subjects among the expression level data from all the sample test subjects were used in analysis given below. In the analysis, normalized count values obtained by use of an approach called DESeq2 were used.

### ii) RNA expression analysis

On the basis of the SSL-derived RNA expression levels (normalized count values) of the healthy subjects and AD measured in the above section i), RNA which attained a corrected p value (FDR) of less than 0.25 in a likelihood ratio test (differentially expressed gene) in AD compared with the healthy subjects was identified. As a result, the expression of 310 RNAs was decreased (DOWN), and the expression of 61 RNAs was increased (UP) (Tables B-1-1 to B-1-9).

**[Table B-1-1]**

| | Gene symbol | log2(FoldChange ) | FDR | Regulation |
|---|---|---|---|---|
| | DEFB1 | -3.00 | 0.00 | DOWN |
| * | **AGR2** | -2.86 | 0.01 | DOWN |
| | GAL | -2.69 | 0.00 | DOWN |
| | CLU | -2.67 | 0.00 | DOWN |
| * | **SPNS2** | -2.66 | 0.00 | DOWN |
| | H LA-A | -2.63 | 0.01 | DOWN |
| * | **DNASE1L2** | -2.47 | 0.01 | DOWN |
| * | **MEST** | -2.45 | 0.01 | DOWN |
| * | **HES4** | -2.37 | 0.02 | DOWN |
| * | **FAM 108C1** | -2.35 | 0.01 | DOWN |
| * | **KRT79** | -2.34 | 0.01 | DOWN |
| * | **ARL5A** | -2.30 | 0.00 | DOWN |
| * | **ALDH3B2** | -2.27 | 0.01 | DOWN |
| * | **CALML3** | -2.22 | 0.01 | DOWN |
| * | **PLCD3** | -2.19 | 0.01 | DOWN |
| * | **OXR1** | -2.17 | 0.01 | DOWN |
| * | **ABHD8** | -2.16 | 0.02 | DOWN |
| * | **UNC5B** | -2.14 | 0.01 | DOWN |
| * | **HSBP1L1** | -2.13 | 0.02 | DOWN |
| * | **MARCH3** | -2.11 | 0.01 | DOWN |
| | ASPRV1 | -2.11 | 0.02 | DOWN |
| * | **CRAT** | -2.11 | 0.01 | DOWN |
| | DMKN | -2.09 | 0.03 | DOWN |
| * | **PLB1** | -2.09 | 0.03 | DOWN |
| * | **CDC34** | -2.08 | 0.00 | DOWN |
| * | **FAM84B** | -2.06 | 0.03 | DOWN |
| | CTSA | -2.06 | 0.00 | DOWN |
| * | **TSPAN6** | -2.03 | 0.04 | DOWN |
| * | **GPT2** | -2.02 | 0.04 | DOWN |
| * | **KRTAP5-5** | -2.02 | 0.06 | DOWN |
| * | **SEPT5** | -1.99 | 0.03 | DOWN |
| * | **MSMO1** | -1.98 | 0.01 | DOWN |
| * | **RRAD** | -1.97 | 0.01 | DOWN |
| * | **CHAC1** | -1.93 | 0.02 | DOWN |
| * | **SLC40A1** | -1.92 | 0.02 | DOWN |
| * | **NIPAL2** | -1.90 | 0.02 | DOWN |
| * | **SPTLC3** | -1.89 | 0.08 | DOWN |
| * | **EPN3** | -1.88 | 0.03 | DOWN |
| | KLK6 | -1.85 | 0.03 | DOWN |
| * | **KLHDC3** | -1.85 | 0.03 | DOWN |
| * | **RNF217** | -1.76 | 0.08 | DOWN |
| | CA6 | -1.75 | 0.09 | DOWN |

**[Table B-1-2]**

| | Gene symbol | log2(FoldChange) | FDR | Regulation |
|---|---|---|---|---|
| * | **NTAN1** | -1.74 | 0.03 | DOWN |
| * | **CDKN2B** | -1.73 | 0.02 | DOWN |
| * | **PLIN2** | -1.73 | 0.01 | DOWN |
| * | **MARCKS** | -1.72 | 0.01 | DOWN |
| * | **RMND5B** | -1.72 | 0.06 | DOWN |
| * | **NCCRP1** | -1.72 | 0.02 | DOWN |
| | SLC15A1 | -1.72 | 0.10 | DOWN |
| * | **GBA2** | -1.71 | 0.01 | DOWN |
| * | **SPAG1** | -1.71 | 0.06 | DOWN |
| | KRT17 | -1.71 | 0.01 | DOWN |
| * | **H1F0** | -1.71 | 0.02 | DOWN |
| * | **RARG** | -1.70 | 0.07 | DOWN |
| | KLK11 | -1.70 | 0.10 | DOWN |
| * | **KRTAP4-9** | -1.70 | 0.15 | DOWN |
| * | **SULT2B1** | -1.70 | 0.04 | DOWN |
| * | **WIPI2** | -1.69 | 0.01 | DOWN |
| * | **RUSC2** | -1.69 | 0.08 | DOWN |
| * | **SMOX** | -1.69 | 0.07 | DOWN |
| * | **GCH1** | -1.68 | 0.10 | DOWN |
| * | **MAPK13** | -1.67 | 0.01 | DOWN |
| * | **MYZAP** | -1.67 | 0.10 | DOWN |
| * | **HS3ST6** | -1.66 | 0.11 | DOWN |
| * | **KRTAP12-1** | -1.65 | 0.12 | DOWN |
| | PSORS1C2 | -1.65 | 0.07 | DOWN |
| * | **CIDEA** | -1.65 | 0.15 | DOWN |
| * | **DSP** | -1.65 | 0.08 | DOWN |
| * | **C15orf62** | -1.64 | 0.10 | DOWN |
| * | **DHCR24** | -1.61 | 0.07 | DOWN |
| * | **KRT34** | -1.61 | 0.25 | DOWN |
| | PCDH1 | -1.61 | 0.10 | DOWN |
| * | **ZDHHC9** | -1.59 | 0.08 | DOWN |
| * | **GNG12** | -1.59 | 0.16 | DOWN |
| * | **CTNNBIP1** | -1.59 | 0.02 | DOWN |
| * | **FAM193B** | -1.58 | 0.08 | DOWN |
| * | **ID1** | -1.58 | 0.07 | DOWN |
| * | **KRT86** | -1.57 | 0.18 | DOWN |
| * | **KRTAP3-1** | -1.57 | 0.17 | DOWN |
| * | **LCE2D** | -1.56 | 0.09 | DOWN |
| * | **THRSP** | -1.56 | 0.15 | DOWN |
| * | **NR1D1** | -1.56 | 0.09 | DOWN |
| * | **IRGQ** | -1.55 | 0.10 | DOWN |
| * | **CYB5R1** | -1.55 | 0.04 | DOWN |

**[Table B-1-3]**

| | Gene symbol | log2(FoldChange) | FDR | Regulation |
|---|---|---|---|---|
| * | **FAM222B** | -1.54 | 0.07 | DOWN |
| * | **DHCR7** | -1.53 | 0.07 | DOWN |
| | CCL3 | -1.53 | 0.10 | DOWN |
| * | **FBXO32** | -1.52 | 0.15 | DOWN |
| | CDSN | -1.52 | 0.10 | DOWN |
| * | **CARD18** | -1.52 | 0.15 | DOWN |
| * | **MGST1** | -1.52 | 0.15 | DOWN |
| | WASL | -1.51 | 0.07 | DOWN |
| * | **TEX264** | -1.51 | 0.08 | DOWN |
| * | **LCE1C** | -1.50 | 0.08 | DOWN |
| | KLK13 | -1.50 | 0.19 | DOWN |
| | INPPL1 | -1.50 | 0.03 | DOWN |
| | SORT1 | -1.50 | 0.03 | DOWN |
| * | **STARD5** | -1.49 | 0.10 | DOWN |
| * | **TMEM189** | -1.49 | 0.01 | DOWN |
| | A2M | -1.49 | 0.12 | DOWN |
| * | **LY6G6C** | -1.47 | 0.19 | DOWN |
| * | **ATP6V1C2** | -1.47 | 0.10 | DOWN |
| * | **LYPDS** | -1.46 | 0.15 | DOWN |
| * | **BMP2** | -1.46 | 0.15 | DOWN |
| * | **HIP1R** | -1.45 | 0.09 | DOWN |
| * | **S100A16** | -1.45 | 0.08 | DOWN |
| * | **Clorf21** | -1.44 | 0.12 | DOWN |
| * | **KLHL21** | -1.44 | 0.10 | DOWN |
| * | **GAS7** | -1.43 | 0.01 | DOWN |
| * | **LCE1F** | -1.43 | 0.10 | DOWN |
| * | **PARD6B** | -1.42 | 0.20 | DOWN |
| * | **TM4SF1** | -1.42 | 0.08 | DOWN |
| * | **FOXO3** | -1.42 | 0.02 | DOWN |
| * | **GDE1** | -1.42 | 0.09 | DOWN |
| * | **SH3BP5L** | -1.40 | 0.10 | DOWN |
| * | **MAL2** | -1.40 | 0.13 | DOWN |
| * | **SLC31A1** | -1.40 | 0.03 | DOWN |
| * | **BNIP3** | -1.40 | 0.05 | DOWN |
| * | **FAM100B** | -1.39 | 0.01 | DOWN |
| * | **PLA2G4E** | -1.38 | 0.15 | DOWN |
| * | **SLAMF7** | -1.38 | 0.23 | DOWN |
| | LCN2 | -1.38 | 0.18 | DOWN |
| * | **C2orf54** | -1.38 | 0.15 | DOWN |
| * | **PIK3AP1** | -1.37 | 0.10 | DOWN |
| * | **ATMIN** | -1.37 | 0.07 | DOWN |
| * | **KIAA0513** | -1.37 | 0.14 | DOWN |

**[Table B-1-4]**

| | Gene symbol | log2(FoldChange) | FDR | Regulation |
|---|---|---|---|---|
| * | **GDPD3** | -1.36 | 0.15 | DOWN |
| | FAR2 | -1.35 | 0.09 | DOWN |
| * | **KRT80** | -1.35 | 0.13 | DOWN |
| * | **EPHX3** | -1.35 | 0.21 | DOWN |
| * | **LCE2C** | -1.35 | 0.17 | DOWN |
| * | **DNAJB1** | -1.34 | 0.04 | DOWN |
| * | **NEDD4L** | -1.34 | 0.20 | DOWN |
| | POR | -1.34 | 0.06 | DOWN |
| * | **IRAK2** | -1.33 | 0.14 | DOWN |
| * | **KCTD11** | -1.33 | 0.21 | DOWN |
| * | **KRT8** | -1.32 | 0.23 | DOWN |
| * | **SMPD3** | -1.32 | 0.16 | DOWN |
| | CD48 | -1.32 | 0.10 | DOWN |
| * | **RSC1A1** | -1.32 | 0.10 | DOWN |
| * | **PLD3** | -1.31 | 0.08 | DOWN |
| * | **HN1L** | -1.30 | 0.10 | DOWN |
| * | **PGRMC2** | -1.30 | 0.21 | DOWN |
| * | **KDSR** | -1.30 | 0.10 | DOWN |
| * | **PPDPF** | -1.30 | 0.01 | DOWN |
| * | **LYPLA1** | -1.29 | 0.08 | DOWN |
| * | **SDCBP2** | -1.29 | 0.15 | DOWN |
| * | **ADIPOR2** | -1.29 | 0.08 | DOWN |
| * | **SSFA2** | -1.29 | 0.02 | DOWN |
| | BCL2L1 | -1.29 | 0.01 | DOWN |
| * | **YPEL2** | -1.28 | 0.10 | DOWN |
| * | **ISG15** | -1.28 | 0.24 | DOWN |
| * | **GTPBP2** | -1.28 | 0.07 | DOWN |
| * | **DDHD1** | -1.27 | 0.18 | DOWN |
| * | **GALNT1** | -1.27 | 0.07 | DOWN |
| * | **CRK** | -1.26 | 0.16 | DOWN |
| * | **TMEM86A** | -1.26 | 0.21 | DOWN |
| * | **HSPA1B** | -1.26 | 0.08 | DOWN |
| * | **PTK6** | -1.25 | 0.24 | DOWN |
| * | **DUSP16** | -1.25 | 0.03 | DOWN |
| | SLPI | -1.25 | 0.10 | DOWN |
| * | **FCHSD1** | -1.24 | 0.08 | DOWN |
| * | **SNX18** | -1.24 | 0.22 | DOWN |
| * | **RASA4CP** | -1.24 | 0.18 | DOWN |
| * | **CPEB4** | -1.23 | 0.01 | DOWN |
| * | **RAB27A** | -1.23 | 0.05 | DOWN |
| * | **AKTIP** | -1.23 | 0.16 | DOWN |
| * | **RGP1** | -1.23 | 0.15 | DOWN |

**[Table B-1-5]**

| | Gene symbol | log2(FoldChange) | FDR | Regulation |
|---|---|---|---|---|
| * | **MIEN1** | -1.23 | 0.05 | DOWN |
| | SCD | -1.23 | 0.14 | DOWN |
| * | **VKORC1L1** | -1.22 | 0.18 | DOWN |
| * | **ABTB2** | -1.22 | 0.10 | DOWN |
| * | **AATK** | -1.22 | 0.23 | DOWN |
| * | **TUFT1** | -1.22 | 0.24 | DOWN |
| * | **MEA1** | -1.21 | 0.10 | DOWN |
| * | **HDAC7** | -1.21 | 0.18 | DOWN |
| * | **PHLDA2** | -1.21 | 0.03 | DOWN |
| * | **MAP1LC3B2** | -1.20 | 0.01 | DOWN |
| * | **TMED3** | -1.20 | 0.16 | DOWN |
| | PRR24 | -1.19 | 0.05 | DOWN |
| | SBSN | -1.19 | 0.21 | DOWN |
| * | **HIST1H2BK** | -1.19 | 0.08 | DOWN |
| * | **SURF1** | -1.19 | 0.19 | DOWN |
| * | **DUSP14** | -1.19 | 0.24 | DOWN |
| * | **FAM214A** | -1.19 | 0.09 | DOWN |
| * | **FAM102A** | -1.17 | 0.21 | DOWN |
| * | **DNAJCS** | -1.17 | 0.07 | DOWN |
| * | **TBC1D17** | -1.17 | 0.10 | DOWN |
| * | **SH3D21** | -1.16 | 0.17 | DOWN |
| * | **MPZL3** | -1.16 | 0.08 | DOWN |
| * | **EPB41** | -1.16 | 0.24 | DOWN |
| * | **UBAP1** | -1.16 | 0.18 | DOWN |
| * | **LRP10** | -1.16 | 0.02 | DOWN |
| * | **PAPL** | -1.15 | 0.19 | DOWN |
| * | **RALGDS** | -1.15 | 0.15 | DOWN |
| | SHB | -1.15 | 0.20 | DOWN |
| * | **TRIM29** | -1.15 | 0.21 | DOWN |
| | DGAT2 | -1.14 | 0.10 | DOWN |
| * | **ADIPOR1** | -1.14 | 0.01 | DOWN |
| * | **LCE2A** | -1.14 | 0.23 | DOWN |
| * | **BASP1** | -1.13 | 0.09 | DOWN |
| * | **RASAL1** | -1.12 | 0.20 | DOWN |
| * | **GIPC1** | -1.12 | 0.18 | DOWN |
| * | **CLTB** | -1.11 | 0.02 | DOWN |
| * | **UBIAD1** | -1.11 | 0.22 | DOWN |
| * | **BPGM** | -1.11 | 0.23 | DOWN |
| * | **LPCAT1** | -1.10 | 0.24 | DOWN |
| * | **RANGAP1** | -1.10 | 0.10 | DOWN |
| * | **RLF** | -1.09 | 0.24 | DOWN |
| * | **PRSS22** | -1.09 | 0.20 | DOWN |

**[Table B-1-6]**

| | Gene symbol | log2(FoldChange) | FDR | Regulation |
|---|---|---|---|---|
| * | **CTSD** | -1.09 | 0.15 | DOWN |
| * | **KIAA0930** | -1.09 | 0.06 | DOWN |
| * | **HIST3H2A** | -1.09 | 0.24 | DOWN |
| * | **SMS** | -1.09 | 0.23 | DOWN |
| | LGALS3 | -1.09 | 0.01 | DOWN |
| * | **TBC1D20** | -1.08 | 0.10 | DOWN |
| * | **SERINC2** | -1.08 | 0.15 | DOWN |
| * | **KCTD20** | -1.07 | 0.25 | DOWN |
| * | **FAM188A** | -1.07 | 0.25 | DOWN |
| * | **ASS1** | -1.07 | 0.24 | DOWN |
| * | **ZNF664** | -1.07 | 0.08 | DOWN |
| * | **UBE2R2** | -1.07 | 0.01 | DOWN |
| * | **PPP2CB** | -1.07 | 0.10 | DOWN |
| * | **GOLGA4** | -1.06 | 0.10 | DOWN |
| * | **ZRANB1** | -1.05 | 0.11 | DOWN |
| | EHF | -1.05 | 0.24 | DOWN |
| * | **TSPAN14** | -1.04 | 0.10 | DOWN |
| * | **HK2** | -1.04 | 0.16 | DOWN |
| | KEAP1 | -1.04 | 0.24 | DOWN |
| | ABHD5 | -1.04 | 0.18 | DOWN |
| * | **NEU1** | -1.03 | 0.24 | DOWN |
| * | **OSBPL2** | -1.03 | 0.10 | DOWN |
| * | **RNF103** | -1.02 | 0.07 | DOWN |
| * | **FEM1B** | -1.02 | 0.14 | DOWN |
| * | **RANBP9** | -1.02 | 0.08 | DOWN |
| * | **LOC100093631** | -1.02 | 0.14 | DOWN |
| * | **MAP1LC3A** | -1.02 | 0.06 | DOWN |
| * | **PRDM1** | -1.01 | 0.05 | DOWN |
| * | **SCYL1** | -1.01 | 0.14 | DOWN |
| * | **NPC1** | -1.01 | 0.10 | DOWN |
| * | **C6orf106** | -1.01 | 0.03 | DOWN |
| * | **USP17L5** | -1.00 | 0.22 | DOWN |
| * | **BNIP3L** | -0.99 | 0.02 | DOWN |
| * | **EAF1** | -0.99 | 0.10 | DOWN |
| * | **MIR548I1** | -0.99 | 0.15 | DOWN |
| * | **JUP** | -0.97 | 0.18 | DOWN |
| * | **PEBP1** | -0.97 | 0.13 | DOWN |
| | HMOX1 | -0.96 | 0.02 | DOWN |
| * | **CTSB** | -0.96 | 0.06 | DOWN |
| * | **SQSTM1** | -0.96 | 0.08 | DOWN |
| * | **VAT1** | -0.96 | 0.13 | DOWN |
| * | **CYBASC3** | -0.95 | 0.18 | DOWN |

**[Table B-1-7]**

| | Gene symbol | log2(FoldChange) | FDR | Regulation |
|---|---|---|---|---|
| * | **EIF4EBP2** | -0.95 | 0.05 | DOWN |
| * | **ATG2A** | -0.94 | 0.15 | DOWN |
| * | **RAD23B** | -0.93 | 0.09 | DOWN |
| * | **DSTN** | -0.93 | 0.10 | DOWN |
| * | **TPRA1** | -0.93 | 0.15 | DOWN |
| * | **BICD2** | -0.93 | 0.16 | DOWN |
| * | **RNF11** | -0.93 | 0.09 | DOWN |
| * | **ULK1** | -0.92 | 0.18 | DOWN |
| * | **SYTL1** | -0.91 | 0.21 | DOWN |
| * | **MGLL** | -0.91 | 0.08 | DOWN |
| * | **WBP2** | -0.90 | 0.13 | DOWN |
| * | **NUDT4** | -0.90 | 0.22 | DOWN |
| * | **USF2** | -0.89 | 0.06 | DOWN |
| * | **PIM1** | -0.88 | 0.10 | DOWN |
| * | **SYPL1** | -0.88 | 0.20 | DOWN |
| * | **OTUD5** | -0.88 | 0.14 | DOWN |
| * | **IRAK1** | -0.87 | 0.23 | DOWN |
| * | **UPK3BL** | -0.86 | 0.18 | DOWN |
| * | **PTK2B** | -0.84 | 0.15 | DOWN |
| * | **MAPK3** | -0.84 | 0.10 | DOWN |
| * | **KRT23** | -0.83 | 0.17 | DOWN |
| * | **UBXN6** | -0.83 | 0.19 | DOWN |
| * | **ATP6V0C** | -0.82 | 0.07 | DOWN |
| * | **ZFAND6** | -0.81 | 0.06 | DOWN |
| * | **SIAH2** | -0.81 | 0.18 | DOWN |
| * | **NBR1** | -0.80 | 0.15 | DOWN |
| * | **ZFAND5** | -0.80 | 0.08 | DOWN |
| * | **HSP90AA1** | -0.80 | 0.24 | DOWN |
| * | **KIF1C** | -0.78 | 0.25 | DOWN |
| * | **CERK** | -0.78 | 0.09 | DOWN |
| * | **ATP6V1A** | -0.78 | 0.22 | DOWN |
| * | **PQLC1** | -0.78 | 0.13 | DOWN |
| * | **CACUL1** | -0.77 | 0.20 | DOWN |
| | PRKCD | -0.76 | 0.18 | DOWN |
| * | **STK10** | -0.76 | 0.18 | DOWN |
| * | **IER3** | -0.75 | 0.24 | DOWN |
| | HECA | -0.74 | 0.18 | DOWN |
| * | **DDIT4** | -0.74 | 0.16 | DOWN |
| | TOLLIP | -0.72 | 0.16 | DOWN |
| * | **CHP1** | -0.72 | 0.08 | DOWN |
| * | **LAMTOR3** | -0.69 | 0.25 | DOWN |
| | KLF4 | -0.68 | 0.09 | DOWN |

**[Table B-1-8]**

| | Gene symbol | log2(FoldChange) | FDR | Regulation |
|---|---|---|---|---|
| * | **KCNQ1OT1** | -0.68 | 0.18 | DOWN |
| | CAST | -0.68 | 0.21 | DOWN |
| * | **CHMP5** | -0.66 | 0.22 | DOWN |
| * | **TNIP1** | -0.65 | 0.18 | DOWN |
| * | **SIRPA** | -0.65 | 0.09 | DOWN |
| * | **GLRX** | -0.61 | 0.10 | DOWN |
| * | **NOTCH2NL** | -0.60 | 0.19 | DOWN |
| * | **SLK** | -0.59 | 0.18 | DOWN |
| * | **ZFP36L2** | -0.59 | 0.10 | DOWN |
| * | **RAB21** | -0.58 | 0.15 | DOWN |
| * | **EIF5** | -0.57 | 0.18 | DOWN |
| * | **PRELID1** | -0.57 | 0.24 | DOWN |
| * | **SQRDL** | -0.56 | 0.19 | DOWN |
| * | **SERP1** | -0.53 | 0.24 | DOWN |
| * | **RAB7A** | -0.44 | 0.15 | DOWN |
| * | **ARF1** | -0.37 | 0.18 | DOWN |
| * | **NDUFA1** | 0.38 | 0.21 | UP |
| | ENO1 | 0.45 | 0.19 | UP |
| * | **H2AFY** | 0.45 | 0.19 | UP |
| * | **GNB2L1** | 0.50 | 0.19 | UP |
| * | **EIF3K** | 0.54 | 0.19 | UP |
| * | **DBI** | 0.58 | 0.19 | UP |
| * | **SH3BGRL3** | 0.58 | 0.15 | UP |
| * | **PDIA3P** | 0.60 | 0.18 | UP |
| * | **NDUFB11** | 0.69 | 0.23 | UP |
| * | **YWHAH** | 0.69 | 0.08 | UP |
| | CALR | 0.70 | 0.18 | UP |
| | GSN | 0.70 | 0.08 | UP |
| * | **SNORA31** | 0.71 | 0.21 | UP |
| * | **CST3** | 0.71 | 0.21 | UP |
| * | **HNRNPUL1** | 0.71 | 0.20 | UP |
| * | **PDIA6** | 0.72 | 0.22 | UP |
| * | **ALDH2** | 0.72 | 0.22 | UP |
| * | **PPIB** | 0.73 | 0.07 | UP |
| * | **TUBA1B** | 0.73 | 0.15 | UP |
| * | **SEC61G** | 0.75 | 0.19 | UP |
| * | **ATP5J2** | 0.77 | 0.15 | UP |
| | HLA-DPB1 | 0.81 | 0.14 | UP |
| * | **RCC2** | 0.81 | 0.19 | UP |
| * | **AIM1** | 0.81 | 0.21 | UP |
| * | **DNAJB11** | 0.83 | 0.07 | UP |
| | CSF1R | 0.83 | 0.15 | UP |

**[Table B-1-9]**

| | Gene symbol | log2(FoldChange) | FDR | Regulation |
|---|---|---|---|---|
| * | **SYNGR2** | 0.86 | 0.23 | UP |
| * | **SDHD** | 0.86 | 0.09 | UP |
| * | **TGFBI** | 0.89 | 0.07 | UP |
| * | **NDUFS7** | 0.90 | 0.21 | UP |
| * | **DDOST** | 0.90 | 0.15 | UP |
| * | **TUBA1A** | 0.91 | 0.02 | UP |
| * | **ECH1** | 0.92 | 0.25 | UP |
| * | **IMPDH2** | 0.94 | 0.20 | UP |
| * | **CASS4** | 0.95 | 0.15 | UP |
| | LGALS1 | 0.95 | 0.08 | UP |
| | IL7R | 0.95 | 0.18 | UP |
| * | **CD52** | 0.96 | 0.13 | UP |
| * | **HLA-DMA** | 0.96 | 0.08 | UP |
| * | **CCND2** | 0.98 | 0.22 | UP |
| * | **S100A4** | 0.99 | 0.08 | UP |
| * | **ERI1** | 1.00 | 0.22 | UP |
| * | **FBXW2** | 1.00 | 0.23 | UP |
| | PYCARD | 1.02 | 0.13 | UP |
| * | **TMX2** | 1.04 | 0.20 | UP |
| * | **HLA-DOA** | 1.04 | 0.24 | UP |
| | MMP12 | 1.06 | 0.15 | UP |
| * | **CIITA** | 1.11 | 0.24 | UP |
| * | **ADAM 19** | 1.11 | 0.18 | UP |
| * | **ANPEP** | 1.11 | 0.08 | UP |
| * | **MAT2A** | 1.14 | 0.08 | UP |
| * | **CLEC4A** | 1.17 | 0.08 | UP |
| | MRC1 | 1.20 | 0.14 | UP |
| | AREG | 1.21 | 0.09 | UP |
| * | **SNRPD1** | 1.24 | 0.14 | UP |
| * | **SLC7A11** | 1.28 | 0.08 | UP |
| | CLEC10A | 1.29 | 0.15 | UP |
| * | **CPVL** | 1.29 | 0.10 | UP |
| * | **SNX8** | 1.37 | 0.09 | UP |
| * | **ATP2A2** | 1.43 | 0.08 | UP |
| | CCL17 | 1.59 | 0.07 | UP |

371 genes shown in Tables B-1-1 to B-1-9 were searched for a biological process (BP) by gene ontology (GO) enrichment analysis using the public database STRING. As a result, 144 BPs related to the gene group with decreased expression in the AD patients were obtained and found to include a term related to cell death, keratinization, immune response (neutrophil and leukocyte degranulation), myeloid cell activation, or lipid metabolism (Tables B-2-1 to B-2-4). 44 BPs related to the gene group with increased expression were obtained and found to include a term related to immune response to exogenous antigens, or the like (Table B-2-4). On the other hand, 318 genes (indicated by boldface with * added in each table) among 371 genes shown in Tables B-1-1 to B-1-9 described above were confirmed to be capable of serving as novel atopic dermatitis markers because there was not previous report suggesting their relation to atopic dermatitis.

**[Table B-2-1]**

| #term ID | term description | FDR | Regulation |
|---|---|---|---|
| GO:0009056 | catabolic process | 1.75E-07 | DOWN |
| GO:0008219 | cell death | 2.57E-07 | DOWN |
| GO:0012501 | programmed cell death | 2.57E-07 | DOWN |
| GO:0044248 | cellular catabolic process | 3.42E-07 | DOWN |
| GO:0030855 | epithelial cell differentiation | 3.86E-07 | DOWN |
| GO:0031424 | keratinization | 9.73E-07 | DOWN |
| GO:0016192 | vesicle-mediated transport | 1.68E-06 | DOWN |
| GO:1901565 | organonitrogen compound catabolic process | 1.68E-06 | DOWN |
| GO:0030216 | keratinocyte differentiation | 1.91E-06 | DOWN |
| GO:0030163 | protein catabolic process | 2.58E-06 | DOWN |
| GO:1901575 | organic substance catabolic process | 2.61E-06 | DOWN |
| GO:0009913 | epidermal cell differentiation | 2.73E-06 | DOWN |
| GO:1901564 | organonitrogen compound metabolic process | 2.73E-06 | DOWN |
| GO:0006629 | lipid metabolic process | 6.61E-06 | DOWN |
| GO:0045055 | regulated exocytosis | 7.10E-06 | DOWN |
| GO:0043588 | skin development | 1.40E-05 | DOWN |
| GO:0036230 | granulocyte activation | 4.69E-05 | DOWN |
| GO:0006915 | apoptotic process | 4.76E-05 | DOWN |
| GO:0043299 | leukocyte degranulation | 5.04E-05 | DOWN |
| GO:0002275 | myeloid cell activation involved in immune response | 6.99E-05 | DOWN |
| GO:0002444 | myeloid leukocyte mediated immunity | 6.99E-05 | DOWN |
| GO:0043312 | neutrophil degranulation | 6.99E-05 | DOWN |
| GO:0044257 | cellular protein catabolic process | 7.59E-05 | DOWN |
| GO:0006914 | autophagy | 8.17E-05 | DOWN |
| GO:0002274 | myeloid leukocyte activation | 9.35E-05 | DOWN |
| GO:0002252 | immune effector process | 0.0001 | DOWN |
| GO:0009057 | macromolecule catabolic process | 0.0001 | DOWN |
| GO:0046903 | secretion | 0.00014 | DOWN |
| GO:0002443 | leukocyte mediated immunity | 0.00015 | DOWN |
| GO:0032940 | secretion by cell | 0.00019 | DOWN |
| GO:0002366 | leukocyte activation involved in immune response | 0.00027 | DOWN |
| GO:1901701 | cellular response to oxygen-containing compound | 0.00028 | DOWN |
| GO:0070268 | cornification | 0.00032 | DOWN |
| GO:0060429 | epithelium development | 0.00054 | DOWN |
| GO:0051603 | proteolysis involved in cellular protein catabolic process | 0.00056 | DOWN |
| GO:1901700 | response to oxygen-containing compound | 0.00068 | DOWN |
| GO:0070887 | cellular response to chemical stimulus | 0.00087 | DOWN |
| GO:0044265 | cellular macromolecule catabolic process | 0.0012 | DOWN |
| GO:0048731 | system development | 0.0018 | DOWN |
| GO:0060548 | negative regulation of cell death | 0.002 | DOWN |
| GO:0043069 | negative regulation of programmed cell death | 0.0022 | DOWN |
| GO:1903428 | positive regulation of reactive oxygen species biosynthetic process | 0.0024 | DOWN |
| GO:0009894 | regulation of catabolic process | 0.0026 | DOWN |
| GO:0046890 | regulation of lipid biosynthetic process | 0.0026 | DOWN |
| GO:0019216 | regulation of lipid metabolic process | 0.003 | DOWN |
| GO:0097164 | ammonium ion metabolic process | 0.0032 | DOWN |
| GO:0043066 | negative regulation of apoptotic process | 0.0036 | DOWN |

**[Table B-2-2]**

| #term ID | term description | FDR | Regulatio n |
|---|---|---|---|
| GO:0010033 | response to organic substance | 0.0037 | DOWN |
| GO:0043393 | regulation of protein binding | 0.0037 | DOWN |
| GO:0032502 | developmental process | 0.0041 | DOWN |
| GO:0031329 | regulation of cellular catabolic process | 0.0043 | DOWN |
| GO:0007275 | multicellular organism development | 0.0047 | DOWN |
| GO:0016236 | macroautophagy | 0.0048 | DOWN |
| GO:0034599 | cellular response to oxidative stress | 0.0048 | DOWN |
| GO:0051707 | response to other organism | 0.0048 | DOWN |
| GO:0000422 | autophagy of mitochondrion | 0.005 | DOWN |
| GO:0010941 | regulation of cell death | 0.0057 | DOWN |
| GO:0019538 | protein metabolic process | 0.0058 | DOWN |
| GO:0045321 | leukocyte activation | 0.0058 | DOWN |
| GO:0009987 | cellular process | 0.0061 | DOWN |
| GO:0042542 | response to hydrogen peroxide | 0.0062 | DOWN |
| GO:0097327 | response to antineoplastic agent | 0.0062 | DOWN |
| GO:2000377 | regulation of reactive oxygen species metabolic process | 0.0063 | DOWN |
| GO:0044267 | cellular protein metabolic process | 0.0066 | DOWN |
| GO:0071396 | cellular response to lipid | 0.0066 | DOWN |
| GO:0002376 | immune system process | 0.0067 | DOWN |
| GO:0048856 | anatomical structure development | 0.0067 | DOWN |
| GO:0071345 | cellular response to cytokine stimulus | 0.0067 | DOWN |
| GO:0006665 | sphingolipid metabolic process | 0.0068 | DOWN |
| GO:0010821 | regulation of mitochondrion organization | 0.0087 | DOWN |
| GO:0008152 | metabolic process | 0.009 | DOWN |
| GO:0051246 | regulation of protein metabolic process | 0.009 | DOWN |
| GO:2000379 | positive regulation of reactive oxygen species metabolic process | 0.009 | DOWN |
| GO:0019941 | modification-dependent protein catabolic process | 0.0097 | DOWN |
| GO:0006810 | transport | 0.0114 | DOWN |
| GO:0034097 | response to cytokine | 0.0114 | DOWN |
| GO:0044419 | interspecies interaction between organisms | 0.0115 | DOWN |
| GO:0009896 | positive regulation of catabolic process | 0.0117 | DOWN |
| GO:0043067 | regulation of programmed cell death | 0.0117 | DOWN |
| GO:1901214 | regulation of neuron death | 0.0117 | DOWN |
| GO:0016241 | regulation of macroautophagy | 0.0118 | DOWN |
| GO:0090083 | regulation of inclusion body assembly | 0.0118 | DOWN |
| GO:0009888 | tissue development | 0.0126 | DOWN |
| GO:0042221 | response to chemical | 0.0126 | DOWN |
| GO:0006508 | proteolysis | 0.0153 | DOWN |
| GO:0006979 | response to oxidative stress | 0.0153 | DOWN |
| GO:0032768 | regulation of monooxygenase activity | 0.0154 | DOWN |
| GO:0016042 | lipid catabolic process | 0.0159 | DOWN |
| GO:0030154 | cell differentiation | 0.0159 | DOWN |
| GO:0033036 | macromolecule localization | 0.0159 | DOWN |
| GO:0042981 | regulation of apoptotic process | 0.0159 | DOWN |
| GO:0051234 | establishment of localization | 0.0159 | DOWN |
| GO:0001775 | cell activation | 0.0163 | DOWN |
| GO:0071310 | cellular response to organic substance | 0.0163 | DOWN |

**[Table B-2-3]**

| #term ID | term description | FDR | Regulation |
|---|---|---|---|
| GO:0006796 | phosphate-containing compound metabolic process | 0.0164 | DOWN |
| GO:0006511 | ubiquitin-dependent protein catabolic process | 0.0177 | DOWN |
| GO:0018149 | peptide cross-linking | 0.0177 | DOWN |
| GO:0032870 | cellular response to hormone stimulus | 0.0177 | DOWN |
| GO:0048513 | animal organ development | 0.0177 | DOWN |
| GO:0048869 | cellular developmental process | 0.0177 | DOWN |
| GO:0035690 | cellular response to drug | 0.0187 | DOWN |
| GO:0008637 | apoptotic mitochondrial changes | 0.0188 | DOWN |
| GO:0044255 | cellular lipid metabolic process | 0.019 | DOWN |
| GO:0006464 | cellular protein modification process | 0.0191 | DOWN |
| GO:0010917 | negative regulation of mitochondrial membrane potential | 0.0191 | DOWN |
| GO:0071447 | cellular response to hydroperoxide | 0.0191 | DOWN |
| GO:0007033 | vacuole organization | 0.0202 | DOWN |
| GO:0048519 | negative regulation of biological process | 0.0219 | DOWN |
| GO:0051098 | regulation of binding | 0.0219 | DOWN |
| GO:0006066 | alcohol metabolic process | 0.0243 | DOWN |
| GO:0007041 | lysosomal transport | 0.0243 | DOWN |
| GO:0010243 | response to organonitrogen compound | 0.0243 | DOWN |
| GO:0010506 | regulation of autophagy | 0.0243 | DOWN |
| GO:0044403 | symbiont process | 0.0243 | DOWN |
| GO:0045429 | positive regulation of nitric oxide biosynthetic process | 0.0243 | DOWN |
| GO:1904407 | positive regulation of nitric oxide metabolic process | 0.0243 | DOWN |
| GO:0048523 | negative regulation of cellular process | 0.0248 | DOWN |
| GO:0019221 | cytokine-mediated signaling pathway | 0.0252 | DOWN |
| GO:0071417 | cellular response to organonitrogen compound | 0.0252 | DOWN |
| GO:0051179 | localization | 0.0277 | DOWN |
| GO:0050999 | regulation of nitric-oxide synthase activity | 0.0297 | DOWN |
| GO:0000302 | response to reactive oxygen species | 0.0311 | DOWN |
| GO:0043433 | negative regulation of DNA-binding transcription factor activity | 0.0321 | DOWN |
| GO:0009725 | response to hormone | 0.0333 | DOWN |
| GO:0032268 | regulation of cellular protein metabolic process | 0.0356 | DOWN |
| GO:1901615 | organic hydroxy compound metabolic process | 0.0356 | DOWN |
| GO:0031331 | positive regulation of cellular catabolic process | 0.0375 | DOWN |
| GO:0043523 | regulation of neuron apoptotic process | 0.0375 | DOWN |
| GO:0097237 | cellular response to toxic substance | 0.0375 | DOWN |
| GO:0003335 | corneocyte development | 0.0385 | DOWN |
| GO:0008333 | endosome to lysosome transport | 0.0385 | DOWN |
| GO:0009636 | response to toxic substance | 0.0385 | DOWN |
| GO:0034395 | regulation of transcription from RNA polymerase II promoter in response to iron | 0.0385 | DOWN |
| GO:0071383 | cellular response to steroid hormone stimulus | 0.0385 | DOWN |
| GO:0071495 | cellular response to endogenous stimulus | 0.0385 | DOWN |
| GO:0071985 | multivesicular body sorting pathway | 0.0385 | DOWN |
| GO:0009617 | response to bacterium | 0.0395 | DOWN |
| GO:0033993 | response to lipid | 0.0397 | DOWN |
| GO:0010823 | negative regulation of mitochondrion organization | 0.0403 | DOWN |
| GO:0070498 | interleukin-1-mediated signaling pathway | 0.0434 | DOWN |
| GO:0009395 | phospholipid catabolic process | 0.0456 | DOWN |
| GO:0000045 | autophagosome assembly | 0.0464 | DOWN |

**[Table B-2-4]**

| #term ID | term description | FDR | Regulation |
|---|---|---|---|
| GO:0051248 | negative regulation of protein metabolic process | 0.0464 | DOWN |
| GO:0031663 | lipopolysaccharide-mediated signaling pathway | 0.0499 | DOWN |
| GO:0006955 | immune response | 0.0045 | UP |
| GO:0001775 | cell activation | 0.0387 | UP |
| GO:0002376 | immune system process | 0.0387 | UP |
| GO:0002478 | antigen processing and presentation of exogenous peptide antigen | 0.0387 | UP |
| GO:0002501 | peptide antigen assembly with MHC protein complex | 0.0387 | UP |
| GO:0002586 | regulation of antigen processing and presentation of peptide antigen via MHC class II | 0.0387 | UP |
| GO:0006091 | generation of precursor metabolites and energy | 0.0387 | UP |
| GO:0006119 | oxidative phosphorylation | 0.0387 | UP |
| GO:0006897 | endocytosis | 0.0387 | UP |
| GO:0009150 | purine ribonucleotide metabolic process | 0.0387 | UP |
| GO:0009167 | purine ribonucleoside monophosphate metabolic process | 0.0387 | UP |
| GO:0009205 | purine ribonucleoside triphosphate metabolic process | 0.0387 | UP |
| GO:0009987 | cellular process | 0.0387 | UP |
| GO:0010033 | response to organic substance | 0.0387 | UP |
| GO:0010713 | negative regulation of collagen metabolic process | 0.0387 | UP |
| GO:0016043 | cellular component organization | 0.0387 | UP |
| GO:0022409 | positive regulation of cell-cell adhesion | 0.0387 | UP |
| GO:0022900 | electron transport chain | 0.0387 | UP |
| GO:0030155 | regulation of cell adhesion | 0.0387 | UP |
| GO:0032981 | mitochondrial respiratory chain complex I assembly | 0.0387 | UP |
| GO:0034097 | response to cytokine | 0.0387 | UP |
| GO:0042921 | glucocorticoid receptor signaling pathway | 0.0387 | UP |
| GO:0045087 | innate immune response | 0.0387 | UP |
| GO:0045785 | positive regulation of cell adhesion | 0.0387 | UP |
| GO:0046034 | ATP metabolic process | 0.0387 | UP |
| GO:0046907 | intracellular transport | 0.0387 | UP |
| GO:0050863 | regulation of T cell activation | 0.0387 | UP |
| GO:0051234 | establishment of localization | 0.0387 | UP |
| GO:0055114 | oxidation-reduction process | 0.0387 | UP |
| GO:0070887 | cellular response to chemical stimulus | 0.0387 | UP |
| GO:0071310 | cellular response to organic substance | 0.0387 | UP |
| GO:0071345 | cellular response to cytokine stimulus | 0.0387 | UP |
| GO:0071346 | cellular response to interferon-gamma | 0.0387 | UP |
| GO:0071353 | cellular response to interleukin-4 | 0.0387 | UP |
| GO:0071840 | cellular component organization or biogenesis | 0.0387 | UP |
| GO:0090197 | positive regulation of chemokine secretion | 0.0387 | UP |
| GO:0008284 | positive regulation of cell population proliferation | 0.0403 | UP |
| GO:0045454 | cell redox homeostasis | 0.0406 | UP |
| GO:0050764 | regulation of phagocytosis | 0.0416 | UP |
| GO:0006810 | transport | 0.042 | UP |
| GO:0045321 | leukocyte activation | 0.042 | UP |
| GO:0016192 | vesicle-mediated transport | 0.0426 | UP |
| GO:0061024 | membrane organization | 0.0442 | UP |
| GO:0051641 | cellular localization | 0.0479 | UP |

### Example B-2 Construction of discriminant model using gene with high variable importance in random forest

### 1) Data used

Data (read count values) on the expression level of SSL-derived RNA from the test subjects was obtained in the same manner as in Example B-1 and converted to RPM values which normalized the read count values for difference in the total number of reads among samples. However, only 3486 genes which produced expression level data without missing values in 90% or more samples in all the samples were used in analysis given below. In the construction of machine learning models, logarithmic values to base 2 plus integer 1 (Log₂(RPM + 1) values) were used in order to approximate the RPM values, which followed negative binominal distribution, to normal distribution.

### 2) Selection of feature gene

In order to select feature genes using random forest algorithm, the Loga(RPM + 1) values of 3486 genes which produced expression level data without missing values in 90% or more samples in all the samples were used as explanatory variables, and the healthy subjects (HL) and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and top 100 genes of variable importance based on Gini coefficient were calculated (Tables B-3-1 to B-3-3). These 100 genes or 92 genes (indicated by boldface with * added in each table) whose relation to atopic dermatitis had not been reported so far were selected as feature genes.

**[Table B-3-1]**

| | Rank | Gene Symbol | Mean Decrease Gini |
|---|---|---|---|
| * | 1 | **AMICA1** | 2.055595121 |
| * | 2 | **FBXW2** | 1.353802031 |
| | 3 | PYCARD | 1.033739223 |
| * | 4 | **STK17B** | 0.978510839 |
| | 5 | DNAJB11 | 0.71656419 |
| * | 6 | **ERI1** | 0.538724844 |
| * | 7 | **ECH1** | 0.534257071 |
| * | 8 | **MED14** | 0.482331688 |
| * | 9 | **HYOU1** | 0.291317096 |
| | 10 | MAP1LC3B2 | 0.291025256 |
| | 11 | IL7R | 0.285395284 |
| * | 12 | **CTDSP1** | 0.25256621 |
| * | 13 | **USP16** | 0.199302177 |
| * | 14 | **HNRNPA1** | 0.193749323 |
| | 15 | CCL17 | 0.192148161 |
| * | 16 | **UBE2R2** | 0.18276738 |
| * | 17 | **SDHD** | 0.182089394 |
| | 18 | AREG | 0.181766398 |
| * | 19 | **TXNDC17** | 0.180982681 |
| * | 20 | **FBXW4** | 0.17987884 |
| * | 21 | **FBP1** | 0.171270238 |
| * | 22 | **FAM100B** | 0.16614037 |
| * | 23 | **PDIA3P** | 0.162448803 |
| * | 24 | **ZN F91** | 0.157466471 |
| * | 25 | **RBM17** | 0.156733289 |
| * | 26 | **PRPF38B** | 0.152730954 |
| * | 27 | **ATP5H** | 0.150590128 |
| * | 28 | **BAX** | 0.148159853 |
| * | 29 | **ALYREF** | 0.147856883 |
| * | 30 | **HK2** | 0.140603185 |
| * | 31 | **PRMT1** | 0.131508716 |
| * | 32 | **CTSC** | 0.131417162 |
| * | 33 | **SNRPD1** | 0.126019405 |
| * | 34 | **TAGLN2** | 0.124762576 |
| * | 35 | **CYTIP** | 0.124343512 |
| * | 36 | **CASS4** | 0.112113307 |
| * | 37 | **SNORA6** | 0.107783969 |

**[Table B-3-2]**

| | Rank | Gene Symbol | Mean Decrease Gini |
|---|---|---|---|
| * | 38 | **U2AF1** | 0.10599447 |
| * | 39 | **VPS13C** | 0.105087046 |
| * | 40 | **SNX8** | 0.104683402 |
| * | 41 | **NBPF10** | 0.103533939 |
| * | 42 | **ZNF430** | 0.102006549 |
| * | 43 | **SPEN** | 0.099173466 |
| * | 44 | **CIB1** | 0.098863699 |
| * | 45 | **TMEM33** | 0.09050211 |
| * | 46 | **NPEPPS** | 0.089495443 |
| * | 47 | **SEC24D** | 0.08717598 |
| * | 48 | **SLC7A11** | 0.085648698 |
| * | 49 | **ARHGDIB** | 0.083273024 |
| * | 50 | **C10orf128** | 0.081392728 |
| * | 51 | **HNRNPUL1** | 0.079931673 |
| * | 52 | **TXN2** | 0.079583971 |
| | 53 | CISH | 0.079051797 |
| * | 54 | **YWHAG** | 0.078687752 |
| * | 55 | **GPT2** | 0.077532431 |
| * | 56 | **KIAA0930** | 0.075420923 |
| * | 57 | **LAMTOR4** | 0.074586405 |
| * | 58 | **CRCP** | 0.073002526 |
| * | 59 | **CLEC4A** | 0.071813857 |
| * | 60 | **STT3A** | 0.069062315 |
| * | 61 | **CRISPLD2** | 0.068308483 |
| * | 62 | **DEFB4B** | 0.067951618 |
| * | 63 | **CD93** | 0.06784085 |
| * | 64 | **PLIN3** | 0.066833805 |
| * | 65 | **USMG5** | 0.066696653 |
| * | 66 | **LOC285359** | 0.066466571 |
| * | 67 | **SLC20A1** | 0.06630307 |
| * | 68 | **MSL1** | 0.065687379 |
| * | 69 | **SLC11A2** | 0.065021055 |
| * | 70 | **KHDRBS1** | 0.064634857 |
| * | 71 | **ABHD8** | 0.063676494 |
| * | 72 | **CORO1B** | 0.062873503 |
| * | 73 | **ZFAND2A** | 0.061802381 |
| | 74 | DOK2 | 0.061523251 |

**[Table B-3-3]**

| | Rank | Gene Symbol | Mean Decrease Gini |
|---|---|---|---|
| * | 75 | **PLIN2** | 0.060826061 |
| * | 76 | **CDC42EP1** | 0.060499775 |
| * | 77 | **CCM2** | 0.057445175 |
| * | 78 | **RNF24** | 0.055689918 |
| * | 79 | **SRPK2** | 0.054119769 |
| * | 80 | **LST1** | 0.052995793 |
| * | 81 | **YPEL2** | 0.052300229 |
| * | 82 | **INF2** | 0.051988691 |
| * | 83 | **AMD1** | 0.051853831 |
| | 84 | ITGAM | 0.051474063 |
| * | 85 | **IMPDH2** | 0.050981003 |
| * | 86 | **CAPG** | 0.050832747 |
| * | 87 | **VKORC1** | 0.050813812 |
| * | 88 | **ACSL4** | 0.050136541 |
| * | 89 | **CDC123** | 0.04843141 |
| * | 90 | **SCARNA7** | 0.048153862 |
| * | 91 | **RNASET2** | 0.047675382 |
| * | 92 | **RLF** | 0.046521947 |
| * | 93 | **C6orf62** | 0.046410655 |
| * | 94 | **SLC39A8** | 0.046281482 |
| * | 95 | **ARHGAP9** | 0.044962677 |
| * | 96 | **NDUFS7** | 0.04437666 |
| * | 97 | **SEC61G** | 0.044157826 |
| | 98 | SCAP | 0.043471551 |
| * | 99 | **TMEM214** | 0.043214673 |
| * | 100 | **USF2** | 0.042867138 |

### 3) Model construction

The Logz(RPM + 1) values of the 100 genes or the 92 genes were used as explanatory variables, and HL and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an estimate error rate (OOB error rate) was calculated. As a result, the OOB error rate was 9.43% in the model using the 100 genes and was 13.21% in the model using the 92 genes.

### Example B-3 Construction of discriminant model using differentially expressed gene

### 1) Data used

Data (read count values) on the expression level of SSL-derived RNA from the test subjects was obtained in the same manner as in Example B-1 and converted to RPM values which normalized the read count values for difference in the total number of reads among samples. In the construction of machine learning models, logarithmic values to base 2 plus integer 1 (Log₂(RPM + 1) values) were used in order to approximate the RPM values, which followed negative binominal distribution, to normal distribution.

### 2) Selection of feature gene

371 genes whose expression significantly differed in AD compared with the healthy subjects (HL) (Tables B-1-1 to B-1-9) in Example B-2, or 318 genes (indicated by boldface with * added in each table) whose relation to atopic dermatitis had not been reported so far were selected as feature genes.

### 3) Model construction

The Logz(RPM + 1) values of the 371 genes or the 318 genes were used as explanatory variables, and HL and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the OOB error rate was 26.42% in the model using the 371 genes and was 30.19% in the model using the 318 genes.

### Example B-4 Construction of discriminant model using feature gene extracted by Boruta method

### 1) Data used

Data (read count values) on the expression level of SSL-derived RNA from the test subjects was obtained in the same manner as in Example B-1 and converted to RPM values which normalized the read count values for difference in the total number of reads among samples. However, only 3486 genes which produced expression level data without missing values in 90% or more samples in all the samples were used in analysis given below. In the construction of machine learning models, logarithmic values to base 2 plus integer 1 (Log₂(RPM + 1) values) were used in order to approximate the RPM values, which followed negative binominal distribution, to normal distribution.

### 2) Selection of feature gene

The Logz(RPM + 1) values of 3486 genes which produced expression level data without missing values in 90% or more samples in all the samples were used as explanatory variables, and the healthy subjects (HL) and AD were used as objective variables. Algorithm in the "Boruta" package of R language was carried out. The maximum number of trials was set to 1,000, and 9 genes which attained a p value of less than 0.01 were calculated (Table B-4). The 9 genes shown in Table B-4 or 7 genes (indicated by boldface with * added in Table B-4) whose relation to atopic dermatitis had not been reported so far were selected as feature genes.

**[Table B-4]**

| | Gene Symbol |
|---|---|
| | CCL17 |
| | PYCARD |
| * | **IMPDH2** |
| * | **ERI1** |
| * | **FBXW2** |
| * | **STK17B** |
| * | **TAGLN2** |
| * | **AMICA1** |
| * | **HNRNPA1** |

### 3) Model construction

The Loga(RPM + 1) values of the 9 genes or the 7 genes were used as explanatory variables, and HL and AD were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the OOB error rate was 9.43% in the model using the 9 genes and was 15.09% in the model using the 7 genes.

### Example C-1 Identification of differentially expressed protein related to atopic dermatitis using child SSL-derived protein

### 1) Test subject and SSL collection

23 healthy children (from 6 months to 5 years old, male and female) (healthy group) and 16 children with atopic dermatitis (children with AD) (from 6 months to 5 years old, male and female) (AD group) were selected as test subjects. For the recruiting of the children with AD, children with AD who satisfied the UKWP criteria under parent's judgement were gathered, and patients from whom a parent's consent was obtained by informed consent were selected. A dermatologist performed systemic skin observation and interview as to the selected children with AD, and diagnosed AD on the basis of Guidelines for the Management of Atopic Dermatitis. Among the children with AD who were thus diagnosed with AD, children who manifested symptoms such as mild or higher AD-like eczema or dryness on the face were selected as test subjects on the basis of the severity assessment criteria described in Guidelines for the Management of Atopic Dermatitis. Sebum was collected from the whole face (including an eruption site for the children with AD) of each test subject using an oil blotting film (5 × 8 cm, made of polypropylene, 3M Company). The oil blotting film was transferred to a glass vial and preserved at -80°C for approximately 1 month until use in protein extraction.

### 2) Protein preparation

The oil blotting film of the above section 1) was cut into an appropriate size, and protein precipitates were obtained using QIAzol Lysis Reagent (Qiagen N.V.) in accordance with the attached protocol. Proteins were dissolved from the obtained protein precipitates with a solubilizing solution using MPEX PTS Reagent (GL Sciences Inc.) in accordance with the attached protocol, and then digested with trypsin to obtain a peptide solution. The obtained peptide solution was dried under reduced pressure (35°C) and then dissolved in an aqueous solution containing 0.1% formic acid and 2% acetonitrile. Peptide concentrations in the solution were measured using a microplate reader (Corona Electric Co., Ltd.) in accordance with the protocol of Pierce(TM) Quantitative Fluorometric Peptide Assay (Thermo Fisher Scientific, Inc.). A peptide solution from one child with AD from whom a necessary amount of peptides could not be obtained was excluded from samples for analysis given below. For LC-MS/MS analysis, quantitative values of proteins were calculated by analysis with constant peptide concentrations applied to a MS apparatus.

### 3) LC-MS/MS analysis and data analysis

Each sample peptide solution obtained in the above section 2) was analyzed by LC-MS/MS under conditions of the following Table C-6.

**[Table C-6]**

| | System and parameter |
|---|---|
| LC | nanoAcquity UPLC (Waters) |
| Trap column | nanoEase Xbridge BEH 130 C18, 0.3 mm × 50 mm, 5 µm |
| Column | nanoAcquity BEH 130 C18, 0.1 mm × 100 mm, 1.7 µm, 40°C |
| Solution A | 0.1% Formic acid, water |
| Solution B | 0.1% Formic acid, 80% acetonitrile |
| Flow rate | 0.4-0.5 µL/min |
| Injection volume | 4 µL. |
| Gradient | B5% (0-5 min) -> B50% (125 min) → B95% (126-150 min) |
| MS system | Q-Exactive plus (ThermoFisher Scientific) |
| Collision | HCD |
| Top N MSMS | 15 |
| Detection | nanoESI, Positive polarty, Spray voltage: 1,800V, |
| Capillary temp | 250°C |

The spectral data obtained by LC-MS/MS analysis was analyzed using Proteome Discoverer ver. 2.2 (Thermo Fisher Scientific, Inc.). For protein identification, a reference database was Swiss Prot and was searched using Mascot database search (Matrix Science) with Taxonomy set to Homo sapiens. In the search, Enzyme was set to Trypsin; Missed cleavage was set to 2; Dynamic modifications were set to Oxidation (M), Acetyl (N-term), and Acetyl (Protein N-term); and Static Modifications were set to Carbamidomethyl (C). Peptides which satisfied a false discovery rate (FDR) of p < 0.01 were to be searched for. The identified proteins were subjected to label free quantification (LFQ) based on precursor ions. Quantitative values of proteins were calculated from the peak intensity of precursor ions derived from the peptides, and peak intensity equal to or lower than a detection limit was regarded as a missing value. Protein abundance ratios were calculated using the summed abundance based method, p values which indicate the significance of difference in abundance among groups were calculated using ANOVA (individual based, t study).

### 4) Results

Among the identified proteins, proteins having a false discovery rate (FDR) of 0.1 or more were excluded from analysis objects. 533 types of proteins which produced a calculated quantitative value without missing values in 75% or more test subjects in either the healthy group or the AD group were extracted as analysis objects. 116 proteins whose abundance ratio was increased to 1.5 time or more (p ≤ 0.05) (Tables C-7-1 to C-7-4), and 12 proteins whose abundance ratio was decreased to 0.75 times or less (p ≤ 0.05) (Table C-8) in the AD group compared with the healthy group were identified.

**[Table C-7-1]**

| Gene name | Protein name | Fold change | p-value |
|---|---|---|---|
| LGALS7 | Galectin-7 | 4.38 | 1.9E-05 |
| SERPINB4 | Serpin B4 | 3.10 | 4.6E-05 |
| TAGLN2 | Transgelin-2 | 2.41 | 2.3E-04 |
| IGHG3 | Immunoglobulin heavy constant gamma 3 | 2.40 | 8.1E-04 |
| RECQL | ATP-dependent DNA helicase Q1 | 2.36 | 1.1E-03 |
| RPL22 | 60S ribosomal protein L22 | 2.31 | 7.7E-04 |
| RPL26 | 60S ribosomal protein L26 | 2.26 | 6.0E-04 |
| EEF1A1 | Elongation factor 1-alpha 1 | 2.13 | 3.4E-04 |
| SERPINB5 | Serpin B5 | 2.07 | 8.2E-04 |
| APOH | Beta-2-glycoprotein 1 | 2.05 | 1.0E-03 |
| LMNA | Prelamin-A/C | 2.01 | 9.4E-04 |
| HSPA5 | Endoplasmic reticulum chaperone BiP | 1.69 | 8.7E-04 |
| CLEC3B | Tetranectin | 1.67 | 1.2E-03 |
| SPRR2D | Small proline-rich protein 2D | 3.37 | 1.4E-03 |
| SERPINB3 | Serpin B3 | 2.28 | 1.5E-03 |
| CAP1 | Adenylyl cyclase-associated protein 1 | 2.10 | 1.6E-03 |
| IGHG1 | Immunoglobulin heavy constant gamma 1 | 2.17 | 1.6E-03 |
| ALDOA | Fructose-bisphosphate aldolase A | 1.58 | 1.7E-03 |
| SFN | 14-3-3 protein sigma | 2.57 | 2.0E-03 |
| DYNLL1 | Dynein light chain 1, cytoplasmic | 1.57 | 2.0E-03 |
| APOA2 | Apolipoprotein A-II | 2.87 | 2.1E-03 |
| S100A10 | Protein S100-A10 | 2.21 | 2.2E-03 |
| SPRR2F | Small proline-rich protein 2F | 2.60 | 2.2E-03 |
| RPS11 | 40S ribosomal protein S11 | 3.34 | 2.4E-03 |
| DSC3 | Desmocollin-3 | 2.15 | 2.5E-03 |
| POF1B | Protein POF1B | 3.87 | 2.9E-03 |
| APOA1 | Apolipoprotein A-I | 2.98 | 2.9E-03 |
| HNRNPA2B1 | Heterogeneous nuclear ribonucleoproteins A2/B1 | 2.72 | 3.0E-03 |
| VDAC1 | Voltage-dependent anion-selective channel protein 1 | 2.07 | 3.1E-03 |
| S100A7 | Protein S100-A7 | 2.63 | 3.2E-03 |
| KLK6 | Kallikrein-6 | 1.75 | 3.2E-03 |
| S100A8 | Protein S100-A8 | 1.53 | 3.2E-03 |
| VTN | Vitronectin | 2.14 | 3.8E-03 |

**[Table C-7-2]**

| Gene name | Protein name | Fold change | p-value |
|---|---|---|---|
| HSPB1 | Heat shock protein beta-1 | 1.82 | 4.1E-03 |
| KLK13 | Kallikrein-13 | 2.50 | 4.4E-03 |
| PLG | Plasminogen | 2.48 | 4.5E-03 |
| ECM1 | Extracellular matrix protein 1 | 2.39 | 4.5E-03 |
| EIF5A | Eukaryotic translation initiation factor 5A-1 | 1.77 | 4.6E-03 |
| PGAM1 | Phosphoglycerate mutase 1 | 1.70 | 4.7E-03 |
| SBSN | Suprabasin | 1.68 | 5.3E-03 |
| MYH14 | Myosin-14 | 2.60 | 5.7E-03 |
| WFDC5 | WAP four-disulfide core domain protein 5 | 2.18 | 6.4E-03 |
| ASPRV1 | Retroviral-like aspartic protease 1 | 3.59 | 6.6E-03 |
| CA2 | Carbonic anhydrase 2 | 5.03 | 7.9E-03 |
| IGHG4 | Immunoglobulin heavy constant gamma 4 | 2.18 | 8.2E-03 |
| LY6G6C | Lymphocyte antigen 6 complex locus protein G6c | 1.56 | 8.5E-03 |
| AHNAK | Neuroblast differentiation-associated protein AHNAK | 2.96 | 8.6E-03 |
| AMBP | Protein AMBP | 2.11 | 9.0E-03 |
| IL36G | Interleukin-36 gamma | 2.19 | 9.3E-03 |
| NCCRP1 | F-box only protein 50 | 1.92 | 9.4E-03 |
| YWHAZ | 14-3-3 protein zeta/delta | 1.71 | 0.010 |
| RPL30 | 60S ribosomal protein L30 | 1.70 | 0.010 |
| H1-5 | Histone H1.5 | 4.94 | 0.011 |
| PI3 | Elafin | 2.32 | 0.011 |
| HLA-DRB1 | HLA class II histocompatibility antigen, DRB1 beta chain | 2.58 | 0.012 |
| EIF4A2 | Eukaryotic initiation factor 4A-II | 2.84 | 0.013 |
| PLEC | Plectin | 1.84 | 0.013 |
| P4HB | Protein disulfide-isomerase | 2.11 | 0.013 |
| VIM | Vimentin | 1.95 | 0.014 |
| GPLD1 | Phosphatidylinositol-glycan-specific phospholipase D | 1.82 | 0.015 |
| F2 | Prothrombin | 2.41 | 0.015 |
| CAPG | Macrophage-capping protein | 2.43 | 0.016 |
| TF | Serotransferrin | 2.34 | 0.017 |
| MYL6 | Myosin light polypeptide 6 | 2.04 | 0.017 |
| PDIA3 | Protein disulfide-isomerase A3 | 1.95 | 0.018 |

**[Table C-7-3]**

| Gene name | Protein name | Fold change | p-value |
|---|---|---|---|
| CLIC1 | Chloride intracellular channel protein 1 | 1.77 | 0.017 |
| GDI2 | Rab GDP dissociation inhibitor beta | 1.70 | 0.018 |
| ARF6 | ADP-ribosylation factor 6 | 1.67 | 0.017 |
| SNRPD3 | Small nuclear ribonucleoprotein Sm D3 | 1.54 | 0.018 |
| S100A11 | Protein S100-A11 | 1.67 | 0.019 |
| FABP5 | Fatty acid-binding protein 5 | 2.09 | 0.020 |
| H2AC4 | Histone H2A type 1-B/E | 2.03 | 0.021 |
| RAN | GTP-binding nuclear protein Ran | 1.75 | 0.021 |
| GC | Vitamin D-binding protein | 1.70 | 0.021 |
| CDH23 | Cadherin-23 | 1.79 | 0.022 |
| LGALSL | Galectin-related protein | 1.69 | 0.022 |
| LDHA | L-lactate dehydrogenase A chain | 2.62 | 0.025 |
| FGG | Fibrinogen gamma chain | 2.21 | 0.024 |
| PFN1 | Profilin-1 | 2.04 | 0.024 |
| DSP | Desmoplakin | 1.67 | 0.025 |
| AHSG | Alpha-2-HS-glycoprotein | 2.39 | 0.025 |
| EEF2 | Elongation factor 2 | 2.20 | 0.025 |
| WFDC12 | WAP four-disulfide core domain protein 12 | 1.87 | 0.025 |
| ALB | Serum albumin | 1.90 | 0.026 |
| PKM | Pyruvate kinase PKM | 1.88 | 0.026 |
| CALR | Calreticulin | 1.84 | 0.026 |
| YWHAG | 14-3-3 protein gamma | 1.75 | 0.027 |
| DCD | Dermcidin | 1.53 | 0.027 |
| PPIA | Peptidyl-prolyl cis-trans isomerase A | 1.54 | 0.027 |
| KLK7 | Kallikrein-7 | 1.73 | 0.028 |
| PPL | Periplakin | 1.52 | 0.028 |
| KLK10 | Kallikrein-10 | 1.60 | 0.028 |
| ORM1 | Alpha-1-acid glycoprotein 1 | 2.00 | 0.029 |
| MUCL1 | Mucin-like protein 1 | 1.93 | 0.031 |
| MIF | Macrophage migration inhibitory factor | 1.52 | 0.031 |
| SCGB1D2 | Secretoglobin family 1D member 2 | 2.26 | 0.032 |
| EIF6 | Eukaryotic translation initiation factor 6 | 1.56 | 0.032 |
| MYH9 | Myosin-9 | 1.87 | 0.033 |

**[Table C-7-4]**

| Gene name | Protein name | Fold change | p-value |
|---|---|---|---|
| RPS13 | 40S ribosomal protein S13 | 1.51 | 0.034 |
| SERPINA3 | Alpha-1-antichymotrypsin | 1.75 | 0.034 |
| EPPK1 | Epiplakin | 3.50 | 0.035 |
| CP | Ceruloplasmin | 2.72 | 0.035 |
| FLNB | Filamin-B | 1.66 | 0.035 |
| HSD17B4 | Peroxisomal multifunctional enzyme type 2 | 1.61 | 0.035 |
| GM2A | Ganglioside GM2 activator | 1.56 | 0.039 |
| RPL15 | 60S ribosomal protein L15 | 1.82 | 0.040 |
| MNDA | Myeloid cell nuclear differentiation antigen | 2.17 | 0.040 |
| RPL31 | 60S ribosomal protein L31 | 1.62 | 0.043 |
| CFL1 | Cofilin-1 | 1.83 | 0.045 |
| GBA | Lysosomal acid glucosylceramidase | 1.66 | 0.046 |
| H1-3 | Histone H1.3 | 1.92 | 0.048 |
| ARHGDIB | Rho GDP-dissociation inhibitor 2 | 1.80 | 0.048 |
| SCGB2A2 | Mammaglobin-A | 1.82 | 0.049 |
| APCS | Serum amyloid P-component | 1.77 | 0.049 |
| ANXA3 | Annexin A3 | 1.83 | 0.049 |
| ERP29 | Endoplasmic reticulum resident protein 29 | 1.58 | 0.050 |

**[Table C-8]**

| Gene name | Protein name | Fold change | p-value |
|---|---|---|---|
| SERPINB13 | Serpin B13 | 0.62 | 5.6E-03 |
| POLR3A | DNA-directed RNA polymerase III subunit RPC1 | 0.45 | 0.011 |
| JCHAIN | Immunoglobulin J chain | 0.69 | 0.028 |
| LTF | Lactotransferrin | 0.45 | 0.030 |
| SAMD4A | Protein Smaug homolog 1 | 0.46 | 0.030 |
| LCN15 | Lipocalin-15 | 0.14 | 0.033 |
| LYZ | Lysozyme C | 0.63 | 0.040 |
| PRR4 | Proline-rich protein 4 | 0.51 | 0.040 |
| BST1 | ADP-ribosyl cyclase/cyclic ADP-ribose hydrolase 2 | 0.68 | 0.041 |
| SCGB2A1 | Mammaglobin-B | 0.40 | 0.042 |
| LACRT | Extracellular glycoprotein lacritin | 0.57 | 0.046 |
| LCN1 | Lipocalin-1 | 0.42 | 0.048 |

### Example C-2 Identification of differentially expressed protein related to atopic dermatitis using adult SSL-derived protein

### 1) Test subject and SSL collection

18 healthy subjects (from 20 to 59 years old, male) (healthy group) and 26 atopic dermatitis patients (AD patients) (from 20 to 59 years old, male) (AD group) were selected as test subjects. A consent was obtained from the test subjects by informed consent. The test subjects of the AD group were each diagnosed with mild or moderate atopic dermatitis in terms of severity by a dermatologist, and were selected as persons who manifested symptoms such as mild or higher AD-like eczema or dryness on the face. Sebum was collected from the whole face (including an eruption site for the AD patients) of each test subject using an oil blotting film (5 × 8 cm, made of polypropylene, 3M Company). The oil blotting film was transferred to a vial and preserved at -80°C for approximately 1 month until use in protein extraction.

### 2) Protein preparation

Peptide concentrations were measured by the same procedures as in Example C-1 except that the peptide solution was obtained using EasyPep(TM) Mini MS Sample Prep Kit (Thermo Fisher Scientific, Inc.) instead of MPEX PTS Reagent (GL Sciences Inc.) in accordance with the attached protocol.

### 3) LC-MS/MS analysis and data analysis

Protein analysis and data analysis were conducted using the same conditions and procedures as in Example C-1.

### 4) Results

Among the identified proteins, proteins having a false discovery rate (FDR) of 0.1 or more were excluded from analysis objects. 1075 types of proteins which produced a calculated quantitative value without missing values in 75% or more test subjects in either the healthy group or the AD group were extracted as analysis objects. One AD patient for which many missing values were observed in the quantitative values of proteins was excluded from analysis. 205 proteins whose abundance ratio was increased to 1.5 times or more (p ≤ 0.05) (Tables C-9-1 to C-9-7), and 37 proteins whose abundance ratio was decreased to 0.75 time or less (p ≤ 0.05) (Tables C-10-1 and C-10-2) in the AD group compared with the healthy group were identified.

**[Table C-9-1]**

| Gene name | Protein names | Fold change | p-value |
|---|---|---|---|
| LGALS3 | Galectin-3 | >1000 | - |
| SERPINB1 | Leukocyte elastase inhibitor | 1.92 | 4.0E-06 |
| HMGB2 | High mobility group protein B2 | 2.57 | 1.5E-05 |
| GC | Vitamin D-binding protein | 2.49 | 2.5E-05 |
| TF | Serotransferrin | 2.47 | 2.8E-05 |
| ITIH4 | Inter-alpha-trypsin inhibitor heavy chain H4 | 3.11 | 3.0E-05 |
| ALB | Serum albumin | 2.62 | 3.5E-05 |
| HPX | Hemopexin | 2.20 | 3.5E-05 |
| TTR | Transthyretin | 3.20 | 3.9E-05 |
| DERA | Deoxyribose-phosphate aldolase | 3.56 | 4.0E-05 |
| SERPINA1 | Alpha-1-antitrypsin | 1.67 | 6.0E-05 |
| VTN | Vitronectin | 2.39 | 7.6E-05 |
| APOA1 | Apolipoprotein A-I | 3.36 | 1.2E-04 |
| NAPA | Alpha-soluble NSF attachment protein | 3.62 | 1.4E-04 |
| APOB | Apolipoprotein B-100 | 2.78 | 1.4E-04 |
| IGHV1-46 | Immunoglobulin heavy variable 1-46 | 2.16 | 1.5E-04 |
| MSN | Moesin | 2.66 | 1.9E-04 |
| CFB | Complement factor B | 2.63 | 1.9E-04 |
| EZR | Ezrin | 1.54 | 2.0E-04 |
| ERP29 | Endoplasmic reticulum resident protein 29 | 2.84 | 2.0E-04 |
| PLG | Plasminogen | 1.91 | 2.2E-04 |
| CP | Ceruloplasmin | 2.96 | 2.2E-04 |
| KV310 | Ig kappa chain V-III region VH | 2.18 | 2.5E-04 |
| AMBP | Protein AMBP | 1.86 | 2.7E-04 |
| FN1 | Fibronectin | 2.46 | 3.0E-04 |
| F2 | Prothrombin | 2.84 | 3.1E-04 |
| DDX55 | ATP-dependent RNA helicase DDX55 | 2.34 | 3.2E-04 |
| PPIA | Peptidyl-prolyl cis-trans isomerase A | 2.88 | 3.3E-04 |
| PRDX6 | Peroxiredoxin-6 | 2.31 | 3.9E-04 |
| H2AZ1 | Histone H2A.Z | 1.81 | 4.2E-04 |
| A2M | Alpha-2-macroglobulin | 3.22 | 4.3E-04 |
| AHSG | Alpha-2-HS-glycoprotein | 3.20 | 4.5E-04 |
| IGHG3 | Immunoglobulin heavy constant gamma 3 | 1.77 | 4.8E-04 |

**[Table C-9-2]**

| Gene name | Protein names | Fold change | p-value |
|---|---|---|---|
| A1BG | Alpha-1B-glycoprotein | 1.71 | 5.0E-04 |
| ITIH1 | Inter-alpha-trypsin inhibitor heavy chain H1 | 3.20 | 5.3E-04 |
| FGG | Fibrinogen gamma chain | 1.96 | 5.4E-04 |
| C4BPA | C4b-binding protein alpha chain | 2.80 | 5.5E-04 |
| SERPINF2 | Alpha-2-antiplasmin | 1.77 | 5.5E-04 |
| GSN | Gelsolin | 1.78 | 5.8E-04 |
| CEACAM5 | Carcinoembryonic antigen-related cell adhesion molecule 5 | 1.77 | 6.0E-04 |
| HRG | Histidine-rich glycoprotein | 1.85 | 6.1E-04 |
| CFH | Complement factor H | 2.04 | 6.5E-04 |
| SERPIND1 | Heparin cofactor 2 | 2.22 | 7.2E-04 |
| KNG1 | Kininogen-1 | 2.53 | 7.4E-04 |
| P4HB | Protein disulfide-isomerase | 2.30 | 8.0E-04 |
| VIM | Vimentin | 2.80 | 9.0E-04 |
| SERPINB5 | Serpin B5 | 1.89 | 9.9E-04 |
| RNASE3 | Eosinophil cationic protein | 4.33 | 9.9E-04 |
| MMP9 | Matrix metalloproteinase-9 | 3.88 | 1.0E-03 |
| G6PD | Glucose-6-phosphate 1-dehydrogenase | 2.71 | 1.0E-03 |
| C3 | Complement C3 | 2.70 | 1.0E-03 |
| IGHG1 | Immunoglobulin heavy constant gamma 1 | 1.76 | 1.1E-03 |
| ORM1 | Alpha-1-acid glycoprotein 1 | 2.80 | 1.1E-03 |
| SERPING1 | Plasma protease C1 inhibitor | 5.91 | 1.2E-03 |
| CFL1 | Cofilin-1 | 1.95 | 1.3E-03 |
| H4C1 | Histone H4 | 2.44 | 1.3E-03 |
| FGB | Fibrinogen beta chain | 2.49 | 1.3E-03 |
| HMGB1 | High mobility group protein B1 | 4.45 | 1.4E-03 |
| C4A | Complement C4-A | 1.63 | 1.5E-03 |
| CFI | Complement factor I | 2.61 | 1.6E-03 |
| GPT | Alanine aminotransferase 1 | 2.89 | 1.6E-03 |
| IGKC | Immunoglobulin kappa constant | 2.64 | 1.7E-03 |
| FGA | Fibrinogen alpha chain | 2.41 | 1.7E-03 |
| APCS | Serum amyloid P-component | 2.08 | 1.8E-03 |
| PGAM1 | Phosphoglycerate mutase 1 | 2.30 | 1.9E-03 |
| PDIA3 | Protein disulfide-isomerase A3 | 2.55 | 1.9E-03 |

**[Table C-9-3]**

| Gene name | Protein names | Fold change | p-value |
|---|---|---|---|
| CDC42 | Cell division control protein 42 homolog | 2.01 | 2.0E-03 |
| HBB | Hemoglobin subunit beta | 8.71 | 2.1E-03 |
| RPS17 | 40S ribosomal protein S17 | 2.17 | 2.2E-03 |
| ELANE | Neutrophil elastase | 2.53 | 2.5E-03 |
| GNAI2 | Guanine nucleotide-binding protein G | 2.74 | 2.5E-03 |
| IGHV3-7 | Immunoglobulin heavy variable 3-7 | 2.33 | 2.5E-03 |
| GSTP1 | Glutathione S-transferase P | 1.92 | 2.6E-03 |
| MYH9 | Myosin-9 | 1.69 | 2.7E-03 |
| PYCARD | Apoptosis-associated speck-like protein containing a CARD | 2.54 | 2.8E-03 |
| ARPC3 | Actin-related protein 2/3 complex subunit 3 | 2.87 | 2.8E-03 |
| C1QC | Complement C1q subcomponent subunit C | 2.58 | 2.9E-03 |
| IGKV4-1 | Immunoglobulin kappa variable 4-1 | 1.95 | 2.9E-03 |
| DBI | Acyl-CoA-binding protein | 3.37 | 3.0E-03 |
| H2BC12 | Histone H2B type 1-K | 2.29 | 3.0E-03 |
| SUMO3 | Small ubiquitin-related modifier 3 | 1.81 | 3.0E-03 |
| FAU | 40S ribosomal protein S30 | 1.71 | 3.1E-03 |
| RPL8 | 60S ribosomal protein L8 | 2.59 | 3.1E-03 |
| TPT1 | Translationally-controlled tumor protein | 2.30 | 3.2E-03 |
| AZU1 | Azurocidin | 3.16 | 3.2E-03 |
| PFN1 | Profilin-1 | 2.01 | 3.3E-03 |
| C1QA | Complement C1q subcomponent subunit A | 2.12 | 3.3E-03 |
| TUBB | Tubulin beta chain | 2.19 | 3.3E-03 |
| HNRNPD | Heterogeneous nuclear ribonucleoprotein D0 | 2.41 | 3.5E-03 |
| TPD52L2 | Tumor protein D54 | 2.39 | 3.6E-03 |
| TUBB2A | Tubulin beta-2A chain | 1.76 | 3.7E-03 |
| TAGLN2 | Transgelin-2 | 2.58 | 3.7E-03 |
| SERPINF1 | Pigment epithelium-derived factor | 2.53 | 4.0E-03 |
| WDR1 | WD repeat-containing protein 1 | 1.61 | 4.1E-03 |
| HBA1 | Hemoglobin subunit alpha | 16.60 | 4.3E-03 |
| ARPC2 | Actin-related protein 2/3 complex subunit 2 | 2.23 | 4.6E-03 |
| ITIH2 | Inter-alpha-trypsin inhibitor heavy chain H2 | 1.57 | 4.6E-03 |
| RPS14 | 40S ribosomal protein S14 | 2.10 | 4.8E-03 |
| RAN | GTP-binding nuclear protein Ran | 1.68 | 4.8E-03 |

**[Table C-9-4]**

| Gene name | Protein names | Fold change | p-value |
|---|---|---|---|
| H1-5 | Histone H1.5 | 3.31 | 5.0E-03 |
| CTSG | Cathepsin G | 2.34 | 5.2E-03 |
| H3C1 | Histone H3.1 | 1.98 | 5.5E-03 |
| SUB1 | Activated RNA polymerase II transcriptional coactivator p15 | 1.87 | 5.5E-03 |
| MYL6 | Myosin light polypeptide 6 | 2.55 | 5.7E-03 |
| IGKV1-5 | Immunoglobulin kappa variable 1-5 | 1.60 | 5.7E-03 |
| RP1BL | Ras-related protein Rap-1b-like protein | 1.75 | 5.8E-03 |
| ACTB | Actin, cytoplasmic 1 | 2.09 | 5.9E-03 |
| ANXA1 | Annexin A1 | 1.96 | 5.9E-03 |
| TUBB4B | Tubulin beta-4B chain | 1.52 | 6.2E-03 |
| YWHAE | 14-3-3 protein epsilon | 1.57 | 6.6E-03 |
| YWHAH | 14-3-3 protein eta | 1.73 | 6.9E-03 |
| PPIB | Peptidyl-prolyl cis-trans isomerase B | 1.53 | 7.5E-03 |
| NME2 | Nucleoside diphosphate kinase B | 2.05 | 7.8E-03 |
| IGKV3-11 | Immunoglobulin kappa variable 3-11 | 2.04 | 7.8E-03 |
| CAMP | Cathelicidin antimicrobial peptide | 2.43 | 7.8E-03 |
| RAC2 | Ras-related C3 botulinum toxin substrate 2 | 3.28 | 8.0E-03 |
| SRSF3 | Serine/arginine-rich splicing factor 3 | 2.15 | 8.0E-03 |
| GPI | Glucose-6-phosphate isomerase | 1.61 | 8.2E-03 |
| AGT | Angiotensinogen | 2.00 | 8.5E-03 |
| MIF | Macrophage migration inhibitory factor | 2.44 | 9.2E-03 |
| PYGL | Glycogen phosphorylase, liver form | 3.88 | 0.010 |
| TACSTD2 | Tumor-associated calcium signal transducer 2 | 2.23 | 0.010 |
| IGHV3-33 | Immunoglobulin heavy variable 3-33 | 1.64 | 0.010 |
| RPL6 | 60S ribosomal protein L6 | 2.71 | 0.010 |
| LGALS1 | Galectin-1 | 2.13 | 0.010 |
| PLS3 | Plastin-3 | 1.80 | 0.010 |
| RETN | Resistin | 3.17 | 0.011 |
| MACROH2A1 | Core histone macro-H2A.1 | 3.38 | 0.011 |
| IGKV3-20 | Immunoglobulin kappa variable 3-20 | 2.22 | 0.011 |
| EPS8L1 | Epidermal growth factor receptor kinase substrate 8-like protein 1 | 1.83 | 0.011 |
| CORO1A | Coronin-1A | 1.59 | 0.011 |
| RPS19 | 40S ribosomal protein S19 | 2.32 | 0.011 |

**[Table C-9-5]**

| Gene name | Protein names | Fold change | p-value |
|---|---|---|---|
| ANXA6 | Annexin A6 | 2.26 | 0.012 |
| PON1 | Serum paraoxonase/arylesterase 1 | 3.88 | 0.012 |
| APOA2 | Apolipoprotein A-II | 3.16 | 0.012 |
| ARHGDIB | Rho GDP-dissociation inhibitor 2 | 2.07 | 0.013 |
| MYL12B | Myosin regulatory light chain 12B | 2.19 | 0.013 |
| HSPA1A | Heat shock 70 kDa protein 1A | 1.75 | 0.013 |
| BTF3 | Transcription factor BTF3 | 1.54 | 0.013 |
| AKR1A1 | Aldo-keto reductase family 1 member A1 | 1.63 | 0.013 |
| UGP2 | UTP--glucose-1-phosphate uridylyltransferase | 1.70 | 0.013 |
| LCP1 | Plastin-2 | 1.63 | 0.014 |
| LCN2 | Neutrophil gelatinase-associated lipocalin | 2.33 | 0.014 |
| UBE2N | Ubiquitin-conjugating enzyme E2 N | 1.64 | 0.014 |
| COTL1 | Coactosin-like protein | 4.01 | 0.014 |
| RALY | RNA-binding protein Raly | 1.55 | 0.015 |
| DEFA3 | Neutrophil defensin 3 | 2.23 | 0.015 |
| NAMPT | Nicotinamide phosphoribosyltransferase | 2.28 | 0.015 |
| IGHG2 | Immunoglobulin heavy constant gamma 2 | 1.69 | 0.015 |
| H1-3 | Histone H1.3 | 2.82 | 0.016 |
| ALDH3A1 | Aldehyde dehydrogenase, dimeric NADP-preferring | 2.32 | 0.016 |
| C1S | Complement C1s subcomponent | 2.23 | 0.016 |
| ACTR2 | Actin-related protein 2 | 1.92 | 0.016 |
| TNNI3K | Serine/threonine-protein kinase TNNI3K | 2.00 | 0.016 |
| AFM | Afamin | 4.46 | 0.017 |
| ASPRV1 | Retroviral-like aspartic protease 1 | 1.81 | 0.017 |
| CAPZA1 | F-actin-capping protein subunit alpha-1 | 1.94 | 0.018 |
| MPO | Myeloperoxidase | 1.60 | 0.018 |
| CANX | Calnexin | 1.96 | 0.018 |
| CBR1 | Carbonyl reductase [NADPH] 1 | 3.01 | 0.019 |
| DNAJB1 | DnaJ homolog subfamily B member 1 | 1.93 | 0.019 |
| RTCB | RNA-splicing ligase RtcB homolog | 1.56 | 0.019 |
| CAPG | Macrophage-capping protein | 1.77 | 0.020 |
| H1-0 | Histone H1.0 | 2.42 | 0.020 |
| RPL4 | 60S ribosomal protein L4 | 2.23 | 0.020 |

**[Table C-9-6]**

| Gene name | Protein names | Fold change | p-value |
|---|---|---|---|
| TRIM29 | Tripartite motif-containing protein 29 | 1.54 | 0.020 |
| EFNA1 | Ephrin-A1 | 1.72 | 0.020 |
| HNRNPK | Heterogeneous nuclear ribonucleoprotein K | 1.59 | 0.021 |
| CALR | Calreticulin | 2.53 | 0.021 |
| IGLV1-51 | Immunoglobulin lambda variable 1-51 | 1.51 | 0.022 |
| RPS6 | 40S ribosomal protein S6 | 1.56 | 0.023 |
| LPO | Lactoperoxidase | 5.16 | 0.024 |
| TMSL3 | Thymosin beta-4-like protein 3 | 2.89 | 0.024 |
| SERPINA4 | Kallistatin | 1.98 | 0.025 |
| EFHD2 | EF-hand domain-containing protein D2 | 2.55 | 0.026 |
| SEPTIN8 | Septin-8 | 2.03 | 0.026 |
| RAB27A | Ras-related protein Rab-27A | 2.10 | 0.027 |
| RPS23 | 40S ribosomal protein S23 | 2.96 | 0.027 |
| RPS9 | 40S ribosomal protein S9 | 1.54 | 0.028 |
| YWHAG | 14-3-3 protein gamma | 1.53 | 0.028 |
| TMED5 | Transmembrane emp24 domain-containing protein 5 | 1.65 | 0.030 |
| HNRNPR | Heterogeneous nuclear ribonucleoprotein R | 2.20 | 0.030 |
| HK3 | Hexokinase-3 | 3.24 | 0.030 |
| SBSN | Suprabasin | 5.57 | 0.030 |
| SRSF2 | Serine/arginine-rich splicing factor 2 | 2.00 | 0.030 |
| LDHA | L-lactate dehydrogenase A chain | 1.66 | 0.031 |
| IGHV3-30 | Immunoglobulin heavy variable 3-30 | 2.49 | 0.031 |
| LRG1 | Leucine-rich alpha-2-glycoprotein | 1.50 | 0.033 |
| SEPTIN9 | Septin-9 | 1.91 | 0.035 |
| RPL12 | 60S ribosomal protein L12 | 1.73 | 0.035 |
| CCT6A | T-complex protein 1 subunit zeta | 2.13 | 0.037 |
| RPL18A | 60S ribosomal protein L18a | 1.71 | 0.037 |
| THBS1 | Thrombospondin-1 | 2.04 | 0.038 |
| C7 | Complement component C7 | 3.69 | 0.040 |
| DAG1 | Dystroglycan | 1.70 | 0.040 |
| APOC1 | Apolipoprotein C-I | 1.56 | 0.041 |
| RPL10A | 60S ribosomal protein L10a | 1.57 | 0.042 |

**[Table C-9-7]**

| Gene name | Protein names | Fold change | p-value |
|---|---|---|---|
| ITGB2 | Integrin beta-2 | 2.17 | 0.043 |
| CA2 | Carbonic anhydrase 2 | 2.27 | 0.044 |
| RPS25 | 40S ribosomal protein S25 | 1.83 | 0.044 |
| RAB1B | Ras-related protein Rab-1B | 2.03 | 0.048 |
| PSMD14 | 26S proteasome non-ATPase regulatory subunit 14 | 2.67 | 0.048 |
| PSME2 | Proteasome activator complex subunit 2 | 1.77 | 0.048 |
| RPL5 | 60S ribosomal protein L5 | 1.89 | 0.049 |
| BPI | Bactericidal permeability-increasing protein | 1.69 | 0.050 |

**[Table C-10-1]**

| Gene name | Protein names | Fold change | p-value |
|---|---|---|---|
| RAD9B | Cell cycle checkpoint control protein RAD9B | 0.04 | 4.0E-05 |
| FLG2 | Filaggrin-2 | 0.51 | 1.3E-04 |
| DHX36 | ATP-dependent DNA/RNA helicase DHX36 | 0.27 | 1.3E-03 |
| MGST2 | Microsomal glutathione S-transferase 2 | 0.62 | 2.8E-03 |
| GSDMA | Gasdermin-A | 0.64 | 4.2E-03 |
| TPP1 | Tripeptidyl-peptidase 1 | 0.66 | 5.5E-03 |
| F5 | Coagulation factor V | 0.71 | 6.1E-03 |
| KRT77 | Keratin, type II cytoskeletal 1b | 0.63 | 6.1E-03 |
| STS | Steryl-sulfatase | 0.48 | 6.3E-03 |
| MYH1 | Myosin-1 | 0.35 | 8.0E-03 |
| PLD3 | 5'-3' exonuclease PLD3 | 0.67 | 8.6E-03 |
| SCGB2A2 | Mammaglobin-A | 0.52 | 9.3E-03 |
| PSMB4 | Proteasome subunit beta type-4 | 0.55 | 0.010 |
| CCAR2 | Cell cycle and apoptosis regulator protein 2 | 0.45 | 0.011 |
| PSMB3 | Proteasome subunit beta type-3 | 0.67 | 0.011 |
| PSMA1 | Proteasome subunit alpha type-1 | 0.69 | 0.014 |
| DHRS11 | Dehydrogenase/reductase SDR family member 11 | 0.53 | 0.014 |
| POM121 | Nuclear envelope pore membrane protein POM 121 | 0.47 | 0.019 |
| HSPE1 | 10 kDa heat shock protein, mitochondrial | 0.65 | 0.020 |
| FBXO6 | F-box only protein 6 | 0.69 | 0.022 |
| GART | Trifunctional purine biosynthetic protein adenosine-3 | 0.66 | 0.023 |
| DCD | Dermcidin | 0.58 | 0.023 |
| CRNN | Cornulin | 0.59 | 0.024 |
| SYNGR2 | Synaptogyrin-2 | 0.66 | 0.026 |
| PHB2 | Prohibitin-2 | 0.72 | 0.028 |
| DLD | Dihydrolipoyl dehydrogenase, mitochondrial | 0.75 | 0.032 |
| ME1 | NADP-dependent malic enzyme | 0.59 | 0.033 |
| IDH2 | Isocitrate dehydrogenase [NADP], mitochondrial | 0.63 | 0.035 |
| IMPA2 | Inositol monophosphatase 2 | 0.65 | 0.039 |
| HMGA1 | High mobility group protein HMG-I/HMG-Y | 0.55 | 0.040 |
| KRT15 | Keratin, type I cytoskeletal 15 | 0.65 | 0.040 |
| PLTP | Phospholipid transfer protein | 0.67 | 0.040 |
| SFPQ | Splicing factor, proline- and glutamine-rich | 0.50 | 0.042 |

**[Table C-10-2]**

| Gene name | Protein names | Fold change | p-value |
|---|---|---|---|
| GMPR2 | GMP reductase 2 | 0.71 | 0.043 |
| ZNF236 | Zinc finger protein 236 | 0.28 | 0.046 |
| TIMP2 | Metalloproteinase inhibitor 2 | 0.48 | 0.048 |
| ZNF292 | Zinc finger protein 292 | 0.71 | 0.049 |

### Example C-3 Construction of discriminant model for detecting childhood atopic dermatitis

### (Data used)

In order to approximate the quantitative data on the proteins obtained in Example C-1 to normal distribution, the unnormalized peak intensity was used as protein quantitative values, and Log₂ (Abundance + 1) values were calculated by the conversion of a value of each protein quantitative value divided by the sum of the quantitative values of all the detected proteins to a logarithmic value to base 2. The obtained Log₂ (Abundance + 1) values were used in the construction of machine learning models. 475 proteins which produced a calculated quantitative value without missing values in 75% or more (29 or more subjects) of all the test subjects were extracted as analysis objects in the same manner as in Example C-1, and used as analysis objects.

### 3-1 Construction of discriminant model using differentially expressed protein

### 1) Selection of feature protein

127 proteins whose expression statistically significantly differed in the children with AD compared with the healthy children (Tables C-11-1 to C-11-4) were identified among the 475 proteins. These proteins were selected as feature proteins, and quantitative data thereon was used as features.

### 2) Model construction

The Log₂ (Abundance + 1) values of the 127 proteins were used as explanatory variables, and the healthy children and the children with AD (the presence or absence of AD) were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the error rate was 18.42% in the model using the 127 proteins as feature proteins.

**[Table C-11-1]**

| Gene name | Protein name | Fold change | p-value | Regulation |
|---|---|---|---|---|
| LGALS7 | Galectin-7 | 4.38 | 1.9E-05 | UP |
| SERPINB4 | Serpin B4 | 3.10 | 4.6E-05 | UP |
| TAGLN2 | Transgelin-2 | 2.41 | 2.3E-04 | UP |
| IGHG3 | Immunoglobulin heavy constant gamma 3 | 2.40 | 8.1E-04 | UP |
| RECQL | ATP-dependent DNA helicase Q1 | 2.36 | 1.1E-03 | UP |
| RPL22 | 60S ribosomal protein L22 | 2.31 | 7.7E-04 | UP |
| RPL26 | 60S ribosomal protein L26 | 2.26 | 6.0E-04 | UP |
| EEF1A1 | Elongation factor 1-alpha 1 | 2.13 | 3.4E-04 | UP |
| SERPINB5 | Serpin B5 | 2.07 | 8.2E-04 | UP |
| APOH | Beta-2-glycoprotein 1 | 2.05 | 1.0E-03 | UP |
| LMNA | Prelamin-A/C | 2.01 | 9.4E-04 | UP |
| HSPA5 | Endoplasmic reticulum chaperone BiP | 1.69 | 8.7E-04 | UP |
| CLEC3B | Tetranectin | 1.67 | 1.2E-03 | UP |
| SPRR2D | Small proline-rich protein 2D | 3.37 | 1.4E-03 | UP |
| SERPINB3 | Serpin B3 | 2.28 | 1.5E-03 | UP |
| CAP1 | Adenylyl cyclase-associated protein 1 | 2.10 | 1.6E-03 | UP |
| IGHG1 | Immunoglobulin heavy constant gamma 1 | 2.17 | 1.6E-03 | UP |
| ALDOA | Fructose-bisphosphate aldolase A | 1.58 | 1.7E-03 | UP |
| SFN | 14-3-3 protein sigma | 2.57 | 2.0E-03 | UP |
| DYNLL1 | Dynein light chain 1, cytoplasmic | 1.57 | 2.0E-03 | UP |
| APOA2 | Apolipoprotein A-II | 2.87 | 2.1E-03 | UP |
| S100A10 | Protein S100-A10 | 2.21 | 2.2E-03 | UP |
| SPRR2F | Small proline-rich protein 2F | 2.60 | 2.2E-03 | UP |
| RPS11 | 40S ribosomal protein S11 | 3.34 | 2.4E-03 | UP |
| DSC3 | Desmocollin-3 | 2.15 | 2.5E-03 | UP |
| POF1B | Protein POF1B | 3.87 | 2.9E-03 | UP |
| APOA1 | Apolipoprotein A-I | 2.98 | 2.9E-03 | UP |
| HNRNPA2B1 | Heterogeneous nuclear ribonucleoproteins A2/B1 | 2.72 | 3.0E-03 | UP |
| VDAC1 | Voltage-dependent anion-selective channel protein 1 | 2.07 | 3.1E-03 | UP |
| S100A7 | Protein S100-A7 | 2.63 | 3.2E-03 | UP |
| KLK6 | Kallikrein-6 | 1.75 | 3.2E-03 | UP |
| S100A8 | Protein S100-A8 | 1.53 | 3.2E-03 | UP |
| VTN | Vitronectin | 2.14 | 3.8E-03 | UP |
| HSPB1 | Heat shock protein beta-1 | 1.82 | 4.1E-03 | UP |
| KLK13 | Kallikrein-13 | 2.50 | 4.4E-03 | UP |
| PLG | Plasminogen | 2.48 | 4.5E-03 | UP |

**[Table C-11-2]**

| Gene name | Protein name | Fold change | p-value | Regulation |
|---|---|---|---|---|
| ECM1 | Extracellular matrix protein 1 | 2.39 | 4.5E-03 | UP |
| EIF5A | Eukaryotic translation initiation factor 5A-1 | 1.77 | 4.6E-03 | UP |
| PGAM1 | Phosphoglycerate mutase 1 | 1.70 | 4.7E-03 | UP |
| SBSN | Suprabasin | 1.68 | 5.3E-03 | UP |
| MYH14 | Myosin-14 | 2.60 | 5.7E-03 | UP |
| WFDC5 | WAP four-disulfide core domain protein 5 | 2.18 | 6.4E-03 | UP |
| ASPRV1 | Retroviral-like aspartic protease 1 | 3.59 | 6.6E-03 | UP |
| LY6G6C | Lymphocyte antigen 6 complex locus protein G6c | 1.56 | 8.5E-03 | UP |
| AHNAK | Neuroblast differentiation-associated protein AHNAK | 2.96 | 8.6E-03 | UP |
| AMBP | Protein AMBP | 2.11 | 9.0E-03 | UP |
| IL36G | Interleukin-36 gamma | 2.19 | 9.3E-03 | UP |
| NCCRP1 | F-box only protein 50 | 1.92 | 9.4E-03 | UP |
| YWHAZ | 14-3-3 protein zeta/delta | 1.71 | 9.9E-03 | UP |
| RPL30 | 60S ribosomal protein L30 | 1.70 | 0.010 | UP |
| H1-5 | Histone H1.5 | 4.94 | 0.011 | UP |
| PI3 | Elafin | 2.32 | 0.011 | UP |
| HLA-DRB1 | HLA class II histocompatibility antigen, DRB1 beta chain | 2.58 | 0.012 | UP |
| EIF4A2 | Eukaryotic initiation factor 4A-II | 2.84 | 0.013 | UP |
| PLEC | Plectin | 1.84 | 0.013 | UP |
| P4HB | Protein disulfide-isomerase | 2.11 | 0.013 | UP |
| VIM | Vimentin | 1.95 | 0.014 | UP |
| GPLD1 | Phosphatidylinositol-glycan-specific phospholipase D | 1.82 | 0.015 | UP |
| F2 | Prothrombin | 2.41 | 0.015 | UP |
| CAPG | Macrophage-capping protein | 2.43 | 0.016 | UP |
| TF | Serotransferrin | 2.34 | 0.017 | UP |
| MYL6 | Myosin light polypeptide 6 | 2.04 | 0.017 | UP |
| PDIA3 | Protein disulfide-isomerase A3 | 1.95 | 0.018 | UP |
| CLIC1 | Chloride intracellular channel protein 1 | 1.77 | 0.017 | UP |
| GDI2 | Rab GDP dissociation inhibitor beta | 1.70 | 0.018 | UP |
| ARF6 | ADP-ribosylation factor 6 | 1.67 | 0.017 | UP |
| SNRPD3 | Small nuclear ribonucleoprotein Sm D3 | 1.54 | 0.018 | UP |
| S100A11 | Protein S100-A11 | 1.67 | 0.019 | UP |
| GPI | Glucose-6-phosphate isomerase | 2.92 | 0.021 | UP |
| FABP5 | Fatty acid-binding protein 5 | 2.09 | 0.020 | UP |
| H2AC4 | Histone H2A type 1-B/E | 2.03 | 0.021 | UP |
| RAN | GTP-binding nuclear protein Ran | 1.75 | 0.021 | UP |

**[Table C-11-3]**

| Gene name | Protein name | Fold change | p-value | Regulation |
|---|---|---|---|---|
| GC | Vitamin D-binding protein | 1.70 | 0.021 | UP |
| CDH23 | Cadherin-23 | 1.79 | 0.022 | UP |
| LGALSL | Galectin-related protein | 1.69 | 0.022 | UP |
| LDHA | L-lactate dehydrogenase A chain | 2.62 | 0.025 | UP |
| FGG | Fibrinogen gamma chain | 2.21 | 0.024 | UP |
| PFN1 | Profilin-1 | 2.04 | 0.024 | UP |
| DSP | Desmoplakin | 1.67 | 0.025 | UP |
| AHSG | Alpha-2-HS-glycoprotein | 2.39 | 0.025 | UP |
| EEF2 | Elongation factor 2 | 2.20 | 0.025 | UP |
| WFDC12 | WAP four-disulfide core domain protein 12 | 1.87 | 0.025 | UP |
| ALB | Serum albumin | 1.90 | 0.026 | UP |
| PKM | Pyruvate kinase PKM | 1.88 | 0.026 | UP |
| CALR | Calreticulin | 1.84 | 0.026 | UP |
| YWHAG | 14-3-3 protein gamma | 1.75 | 0.027 | UP |
| DCD | Dermcidin | 1.53 | 0.027 | UP |
| PPIA | Peptidyl-prolyl cis-trans isomerase A | 1.54 | 0.027 | UP |
| KLK7 | Kallikrein-7 | 1.73 | 0.028 | UP |
| PPL | Periplakin | 1.52 | 0.028 | UP |
| KLK10 | Kallikrein-10 | 1.60 | 0.028 | UP |
| ORM1 | Alpha-1-acid glycoprotein 1 | 2.00 | 0.029 | UP |
| MUCL1 | Mucin-like protein 1 | 1.93 | 0.031 | UP |
| MIF | Macrophage migration inhibitory factor | 1.52 | 0.031 | UP |
| SCGB1D2 | Secretoglobin family 1D member 2 | 2.26 | 0.032 | UP |
| EIF6 | Eukaryotic translation initiation factor 6 | 1.56 | 0.032 | UP |
| MYH9 | Myosin-9 | 1.87 | 0.033 | UP |
| SERPINA3 | Alpha-1-antichymotrypsin | 1.75 | 0.034 | UP |
| EPPK1 | Epiplakin | 3.50 | 0.035 | UP |
| CP | Ceruloplasmin | 2.72 | 0.035 | UP |
| FLNB | Filamin-B | 1.66 | 0.035 | UP |
| HSD17B4 | Peroxisomal multifunctional enzyme type 2 | 1.61 | 0.035 | UP |
| GM2A | Ganglioside GM2 activator | 1.56 | 0.039 | UP |
| RPL15 | 60S ribosomal protein L15 | 1.82 | 0.040 | UP |
| MNDA | Myeloid cell nuclear differentiation antigen | 2.17 | 0.040 | UP |
| RPL31 | 60S ribosomal protein L31 | 1.62 | 0.043 | UP |
| CFL1 | Cofilin-1 | 1.83 | 0.045 | UP |
| GBA | Lysosomal acid glucosylceramidase | 1.66 | 0.046 | UP |

**[Table C-11-4]**

| Gene name | Protein name | Fold change | p-value | Regulation |
|---|---|---|---|---|
| H1-3 | Histone H1.3 | 1.92 | 0.048 | UP |
| ARHGDIB | Rho GDP-dissociation inhibitor 2 | 1.80 | 0.048 | UP |
| SCGB2A2 | Mammaglobin-A | 1.82 | 0.049 | UP |
| APCS | Serum amyloid P-component | 1.77 | 0.049 | UP |
| ANXA3 | Annexin A3 | 1.83 | 0.049 | UP |
| ERP29 | Endoplasmic reticulum resident protein 29 | 1.58 | 0.050 | UP |
| DDX10 | Probable ATP-dependent RNA helicase DDX10 | 0.42 | 9.5E-03 | DOWN |
| SERPINB13 | Serpin B13 | 0.62 | 5.6E-03 | DOWN |
| DDX10 | Probable ATP-dependent RNA helicase DDX10 | 0.42 | 9.E-03 | DOWN |
| POLR3A | DNA-directed RNA polymerase III subunit RPC1 | 0.45 | 0.011 | DOWN |
| JCHAIN | Immunoglobulin J chain | 0.69 | 0.028 | DOWN |
| LTF | Lactotransferrin | 0.45 | 0.030 | DOWN |
| SAMD4A | Protein Smaug homolog 1 | 0.46 | 0.030 | DOWN |
| LCN15 | Lipocalin-15 | 0.14 | 0.033 | DOWN |
| LYZ | Lysozyme C | 0.63 | 0.040 | DOWN |
| PRR4 | Proline-rich protein 4 | 0.51 | 0.040 | DOWN |
| BST1 | ADP-ribosyl cyclase/cyclic ADP-ribose hydrolase 2 | 0.68 | 0.041 | DOWN |
| SCGB2A1 | Mammaglobin-B | 0.40 | 0.042 | DOWN |
| LACRT | Extracellular glycoprotein lacritin | 0.57 | 0.046 | DOWN |
| LCN1 | Lipocalin-1 | 0.42 | 0.048 | DOWN |

### 3-2 Construction of discriminant model using protein with high variable importance in random forest

### 1) Selection of feature protein

The Log₂ (Abundance + 1) values of the 475 proteins were used as explanatory variables, and the healthy children and the children with AD (the presence or absence of AD) were used as objective variables. Random forest algorithm was designated as a method in the "caret" package of R language, the number of variables (mtry value) for use in the construction of one decision tree was tuned into the optimum value. The random forest algorithm was carried out using the mtry value determined by tuning, and top 140 proteins of variable importance based on Gini coefficient were calculated (Tables C-12-1 to C-12-4). These 140 proteins and all the 475 proteins used in the selection of feature proteins were used as feature proteins, and quantitative data thereon was used as features.

### 2) Model construction

The Log₂ (Abundance + 1) values of the 140 proteins or all the 475 proteins were used as explanatory variables, and the healthy children and the children with AD (the presence or absence of AD) were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an estimate error rate (OOB error rate) was calculated. As a result, the error rate was 28.95% when all the 475 proteins were used as feature proteins, whereas the error rate was 7.89% when the top 140 proteins of variable importance were used as feature proteins.

**[Table C-12-1]**

| Rank | Gene name | Protein name | Mean Decrease Gini |
|---|---|---|---|
| 1 | KLK6 | Kallikrein-6 | 0.140 |
| 2 | H1-5 | Histone H1.5 | 0.112 |
| 3 | RPL29 | 60S ribosomal protein L29 | 0.111 |
| 4 | EIF4A2 | Eukaryotic initiation factor 4A-II | 0.108 |
| 5 | MYL6 | Myosin light polypeptide 6 | 0.106 |
| 6 | POF1B | Protein POF1B | 0.102 |
| 7 | LCN2 | Neutrophil gelatinase-associated lipocalin | 0.099 |
| 8 | YWHAG | 14-3-3 protein gamma | 0.095 |
| 9 | HNRNPA2B1 | Heterogeneous nuclear ribonucleoproteins A2/B1 | 0.094 |
| 10 | S100A11 | Protein S100-A11 | 0.091 |
| 11 | IL36G | Interleukin-36 gamma | 0.091 |
| 12 | MNDA | Myeloid cell nuclear differentiation antigen | 0.090 |
| 13 | SERPINB4 | Serpin B4 | 0.090 |
| 14 | RAB1A | Ras-related protein Rab-1A | 0.088 |
| 15 | PGAM1 | Phosphoglycerate mutase 1 | 0.087 |
| 16 | CLEC3B | Tetranectin | 0.085 |
| 17 | PLEC | Plectin | 0.084 |
| 18 | MYH14 | Myosin-14 | 0.084 |
| 19 | LDHA | L-lactate dehydrogenase A chain | 0.083 |
| 20 | LGALS7 | Galectin-7 | 0.083 |
| 21 | NME1 | Nucleoside diphosphate kinase A | 0.083 |
| 22 | ERP29 | Endoplasmic reticulum resident protein 29 | 0.083 |
| 23 | LACRT | Extracellular glycoprotein lacritin | 0.082 |
| 24 | CFB | Complement factor B | 0.081 |
| 25 | H2AC4 | Histone H2A type 1-B/E | 0.079 |
| 26 | LGALSL | Galectin-related protein | 0.079 |
| 27 | HSPA5 | Endoplasmic reticulum chaperone BiP | 0.078 |
| 28 | SERPINB3 | Serpin B3 | 0.078 |
| 29 | AMBP | Protein AMBP | 0.078 |
| 30 | PFN1 | Profilin-1 | 0.075 |
| 31 | PSMB5 | Proteasome subunit beta type-5 | 0.073 |
| 32 | DSC3 | Desmocollin-3 | 0.072 |
| 33 | TF | Serotransferrin | 0.072 |
| 34 | GCA | Grancalcin | 0.072 |
| 35 | ACTB | Actin, cytoplasmic 1 | 0.071 |
| 36 | KRT23 | Keratin, type I cytoskeletal 23 | 0.069 |

**[Table C-12-2]**

| Rank | Gene name | Protein name | Mean Decrease Gini |
|---|---|---|---|
| 37 | IGHG1 | Immunoglobulin heavy constant gamma 1 | 0.069 |
| 38 | ORM1 | Alpha-1-acid glycoprotein 1 | 0.069 |
| 39 | SCGB1D2 | Secretoglobin family 1D member 2 | 0.068 |
| 40 | RECQL | ATP-dependent DNA helicase Q1 | 0.068 |
| 41 | RPL26 | 60S ribosomal protein L26 | 0.068 |
| 42 | GSN | Gelsolin | 0.068 |
| 43 | FGA | Fibrinogen alpha chain | 0.067 |
| 44 | APOH | Beta-2-glycoprotein 1 | 0.067 |
| 45 | CP | Ceruloplasmin | 0.066 |
| 46 | TKT | Transketolase | 0.066 |
| 47 | FLNB | Filamin-B | 0.065 |
| 48 | PSMB1 | Proteasome subunit beta type-1 | 0.065 |
| 49 | GBA | Lysosomal acid glucosylceramidase | 0.065 |
| 50 | RPL30 | 60S ribosomal protein L30 | 0.065 |
| 51 | ASPRV1 | Retroviral-like aspartic protease 1 | 0.064 |
| 52 | GPI | Glucose-6-phosphate isomerase | 0.064 |
| 53 | APOA1 | Apolipoprotein A-I | 0.064 |
| 54 | MMGT1 | Membrane magnesium transporter 1 | 0.064 |
| 55 | KLK13 | Kallikrein-13 | 0.063 |
| 56 | H2AC11 | Histone H2A type 1 | 0.063 |
| 57 | RPS27A | Ubiquitin-40S ribosomal protein S27a | 0.063 |
| 58 | KNG1 | Kininogen-1 | 0.063 |
| 59 | FGB | Fibrinogen beta chain | 0.062 |
| 60 | HSPB1 | Heat shock protein beta-1 | 0.062 |
| 61 | H4C1 | Histone H4 | 0.061 |
| 62 | SCEL | Sciellin | 0.061 |
| 63 | SBSN | Suprabasin | 0.061 |
| 64 | VTN | Vitronectin | 0.061 |
| 65 | FABP5 | Fatty acid-binding protein 5 | 0.061 |
| 66 | RPL22 | 60S ribosomal protein L22 | 0.060 |
| 67 | APOA2 | Apolipoprotein A-II | 0.059 |
| 68 | SPRR1B | Cornifin-B | 0.059 |
| 69 | MSLN | Mesothelin | 0.059 |
| 70 | RARRES1 | Retinoic acid receptor responder protein 1 | 0.059 |
| 71 | CBR1 | Carbonyl reductase [NADPH] 1 | 0.058 |
| 72 | MYL12B | Myosin regulatory light chain 12B | 0.058 |

**[Table C-12-3]**

| Rank | Gene name | Protein name | Mean Decrease Gini |
|---|---|---|---|
| 73 | ENO1 | Alpha-enolase | 0.058 |
| 74 | ITGAM | Integrin alpha-M | 0.058 |
| 75 | ANXA2 | Annexin A2 | 0.058 |
| 76 | PDIA3 | Protein disulfide-isomerase A3 | 0.057 |
| 77 | DSP | Desmoplakin | 0.057 |
| 78 | SLURP2 | Secreted Ly-6/uPAR domain-containing protein 2 | 0.057 |
| 79 | DYNLL1 | Dynein light chain 1, cytoplasmic | 0.057 |
| 80 | LYZ | Lysozyme C | 0.057 |
| 81 | SERPINB5 | Serpin B5 | 0.056 |
| 82 | LAMP2 | Lysosome-associated membrane glycoprotein 2 | 0.056 |
| 83 | LCN15 | Lipocalin-15 | 0.056 |
| 84 | PLG | Plasminogen | 0.056 |
| 85 | DSC1 | Desmocollin-1 | 0.056 |
| 86 | CAPG | Macrophage-capping protein | 0.055 |
| 87 | PSMA1 | Proteasome subunit alpha type-1 | 0.055 |
| 88 | YWHAZ | 14-3-3 protein zeta/delta | 0.055 |
| 89 | MUC5AC | Mucin-5AC | 0.055 |
| 90 | JCHAIN | Immunoglobulin J chain | 0.055 |
| 91 | ELANE | Neutrophil elastase | 0.055 |
| 92 | PCBP1 | Poly(rC)-binding protein 1 | 0.054 |
| 93 | TPM3 | Tropomyosin alpha-3 chain | 0.054 |
| 94 | S100A10 | Protein S100-A10 | 0.054 |
| 95 | IGHG3 | Immunoglobulin heavy constant gamma 3 | 0.053 |
| 96 | LTF | Lactotransferrin | 0.053 |
| 97 | ALB | Serum albumin | 0.053 |
| 98 | RAB10 | Ras-related protein Rab-10 | 0.053 |
| 99 | CRISP3 | Cysteine-rich secretory protein 3 | 0.053 |
| 100 | VSIG10L | V-set and immunoglobulin domain-containing protein 10-like | 0.053 |
| 101 | WFDC5 | WAP four-disulfide core domain protein 5 | 0.053 |
| 102 | CPNE3 | Copine-3 | 0.052 |
| 103 | CTSG | Cathepsin G | 0.052 |
| 104 | VIM | Vimentin | 0.052 |
| 105 | RPSA | 40S ribosomal protein SA | 0.052 |
| 106 | ANXA3 | Annexin A3 | 0.052 |
| 107 | IGHM | Immunoglobulin heavy constant mu | 0.052 |
| 108 | MDH2 | Malate dehydrogenase, mitochondrial | 0.052 |

**[Table C-12-4]**

| Rank | Gene name | Protein name | Mean Decrease Gini |
|---|---|---|---|
| 109 | APCS | Serum amyloid P-component | 0.052 |
| 110 | CARD18 | Caspase recruitment domain-containing protein 18 | 0.052 |
| 111 | CAP1 | Adenylyl cyclase-associated protein 1 | 0.051 |
| 112 | AZGP1 | Zinc-alpha-2-glycoprotein | 0.051 |
| 113 | NPC2 | NPC intracellular cholesterol transporter 2 | 0.051 |
| 114 | KRT13 | Keratin, type I cytoskeletal 13 | 0.051 |
| 115 | TGM1 | Protein-glutamine gamma-glutamyltransferase K | 0.050 |
| 116 | JUP | Junction plakoglobin | 0.050 |
| 117 | EVPL | Envoplakin | 0.050 |
| 118 | GDI2 | Rab GDP dissociation inhibitor beta | 0.050 |
| 119 | RPL14 | 60S ribosomal protein L14 | 0.050 |
| 120 | SPRR2F | Small proline-rich protein 2F | 0.050 |
| 121 | KRT15 | Keratin, type I cytoskeletal 15 | 0.050 |
| 122 | PRDX2 | Peroxiredoxin-2 | 0.050 |
| 123 | PNP | Purine nucleoside phosphorylase | 0.050 |
| 124 | S100A6 | Protein S100-A6 | 0.049 |
| 125 | PGK1 | Phosphoglycerate kinase 1 | 0.049 |
| 126 | CKMT1A | Creatine kinase U-type, mitochondrial | 0.049 |
| 127 | AHNAK | Neuroblast differentiation-associated protein AHNAK | 0.048 |
| 128 | A2M | Alpha-2-macroglobulin | 0.048 |
| 129 | PRSS27 | Serine protease 27 | 0.048 |
| 130 | CALR | Calreticulin | 0.048 |
| 131 | TALDO1 | Transaldolase | 0.048 |
| 132 | CASP14 | Caspase-14 | 0.048 |
| 133 | KLK9 | Kallikrein-9 | 0.048 |
| 134 | HSPE1 | 10 kDa heat shock protein, mitochondrial | 0.047 |
| 135 | S100A14 | Protein S100-A14 | 0.047 |
| 136 | HLA-DPB1 | HLA class II histocompatibility antigen, DP beta 1 chain | 0.047 |
| 137 | B2M | Beta-2-microglobulin | 0.047 |
| 138 | PKM | Pyruvate kinase PKM | 0.047 |
| 139 | RNASE3 | Eosinophil cationic protein | 0.046 |
| 140 | KRTAP2-3 | Keratin-associated protein 2-3 | 0.046 |

### 3-3 Construction of discriminant model using feature protein extracted by Boruta method

### 1) Selection of feature protein

The Log₂ (Abundance + 1) values of the 475 proteins were used as explanatory variables, and the healthy children and the children with AD (the presence or absence of AD) were used as objective variables. Algorithm in the "Boruta" package of R language was carried out. The maximum number of trials was set to 1,000, and 35 proteins which attained a p value of less than 0.01 were extracted (Table C-13) and used as feature proteins. Quantitative data on these proteins was used as features.

### 2) Model construction

The Log₂ (Abundance + 1) values of the 35 proteins were used as explanatory variables, and the healthy children and the children with AD (the presence or absence of AD) were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the error rate was 10.53% in the model using the 35 proteins as feature proteins.

**[Table C-13]**

| Gene name | Protein name |
|---|---|
| LGALS7 | Galectin-7 |
| SERPINB4 | Serpin B4 |
| TAGLN2 | Transgelin-2 |
| IGHG3 | Immunoglobulin heavy constant gamma 3 |
| RECQL | ATP-dependent DNA helicase Q1 |
| RPL22 | 60S ribosomal protein L22 |
| RPL26 | 60S ribosomal protein L26 |
| EEF1A1 | Elongation factor 1-alpha 1 |
| SERPINB5 | Serpin B5 |
| CLEC3B | Tetranectin |
| SPRR2D | Small proline-rich protein 2D |
| SERPINB3 | Serpin B3 |
| CAP1 | Adenylyl cyclase-associated protein 1 |
| IGHG1 | Immunoglobulin heavy constant gamma 1 |
| ALDOA | Fructose-bisphosphate aldolase A |
| APOA2 | Apolipoprotein A-II |
| SPRR2F | Small proline-rich protein 2F |
| RPS11 | 40S ribosomal protein S11 |
| DSC3 | Desmocollin-3 |
| POF1B | Protein POF1B |
| KLK13 | Kallikrein-13 |
| AMBP | Protein AMBP |
| PLEC | Plectin |
| F2 | Prothrombin |
| H2AC4 | Histone H2A type 1-B/E |
| PFN1 | Profilin-1 |
| ORM1 | Alpha-1-acid glycoprotein 1 |
| MNDA | Myeloid cell nuclear differentiation antigen |
| CORO1A | Coronin-1A |
| KNG1 | Kininogen-1 |
| ANXA2 | Annexin A2 |
| TPM3 | Tropomyosin alpha-3 chain |
| RPL29 | 60S ribosomal protein L29 |
| RARRES1 | Retinoic acid receptor responder protein 1 |
| LCN15 | Lipocalin-15 |

### Example C-4 Construction of discriminant model for detecting adult atopic dermatitis

### (Data used)

In order to approximate the quantitative data on the proteins obtained in Example C-2 to normal distribution, the unnormalized peak intensity was used as protein quantitative values, and Log₂ (Abundance + 1) values were calculated by the conversion of a value of each protein quantitative value divided by the sum of the quantitative values of all the detected proteins to a logarithmic value to base 2. The obtained Log₂ (Abundance + 1) values were used in the construction of machine learning models. 985 proteins which produced a calculated quantitative value without missing values in 75% or more (31 or more subjects) of all the test subjects (except for 3 subjects, the protein quantitative data from whom did not follow normal distribution) were extracted in the same manner as in Example C-2, and used as analysis objects.

### 4-1 Construction of discriminant model using differentially expressed protein

### 1) Selection of feature protein

220 proteins whose expression statistically differed in the AD patients compared with the healthy subjects (Tables C-14-1 to C-14-7) were identified among the 985 proteins. These proteins were selected as feature proteins, and quantitative data thereon was used as features.

### 2) Model construction

The Log₂ (Abundance + 1) values of the 220 proteins were used as explanatory variables, and the healthy subjects and the AD patients (the presence or absence of AD) were selected as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the error rate was 24.39% in the model using the 220 proteins as feature proteins.

**[Table C-14-1]**

| Gene name | Protein name | Fold change | p-value | Regulation |
|---|---|---|---|---|
| LGALS3 | Galectin-3 | >1000 | - | UP |
| SERPINB1 | Leukocyte elastase inhibitor | 1.92 | 4.0E-06 | UP |
| HMGB2 | High mobility group protein B2 | 2.57 | 1.5E-05 | UP |
| GC | Vitamin D-binding protein | 2.49 | 2.5E-05 | UP |
| TF | Serotransferrin | 2.47 | 2.8E-05 | UP |
| ITIH4 | Inter-alpha-trypsin inhibitor heavy chain H4 | 3.11 | 3.0E-05 | UP |
| ALB | Serum albumin | 2.62 | 3.5E-05 | UP |
| HPX | Hemopexin | 2.20 | 3.5E-05 | UP |
| TTR | Transthyretin | 3.20 | 3.9E-05 | UP |
| SERPINA1 | Alpha-1-antitrypsin | 1.67 | 6.0E-05 | UP |
| VTN | Vitronectin | 2.39 | 7.6E-05 | UP |
| APOA1 | Apolipoprotein A-I | 3.36 | 1.2E-04 | UP |
| APOB | Apolipoprotein B-100 | 2.78 | 1.4E-04 | UP |
| IGHV1-46 | Immunoglobulin heavy variable 1-46 | 2.16 | 1.5E-04 | UP |
| MSN | Moesin | 2.66 | 1.9E-04 | UP |
| CFB | Complement factor B | 2.63 | 1.9E-04 | UP |
| EZR | Ezrin | 1.54 | 2.0E-04 | UP |
| ERP29 | Endoplasmic reticulum resident protein 29 | 2.84 | 2.0E-04 | UP |
| PLG | Plasminogen | 1.91 | 2.2E-04 | UP |
| KV310 | Ig kappa chain V-III region VH | 2.96 | 2.2E-04 | UP |
| CP | Ceruloplasmin | 2.18 | 2.5E-04 | UP |
| AMBP | Protein AMBP | 1.86 | 2.7E-04 | UP |
| FN1 | Fibronectin | 2.46 | 3.0E-04 | UP |
| F2 | Prothrombin | 2.84 | 3.1E-04 | UP |
| DDX55 | ATP-dependent RNA helicase DDX55 | 2.34 | 3.2E-04 | UP |
| PPIA | Peptidyl-prolyl cis-trans isomerase A | 2.88 | 3.3E-04 | UP |
| PRDX6 | Peroxiredoxin-6 | 2.31 | 3.9E-04 | UP |
| H2AZ1 | Histone H2A.Z | 1.81 | 4.2E-04 | UP |
| A2M | Alpha-2-macroglobulin | 3.22 | 4.3E-04 | UP |
| AHSG | Alpha-2-HS-glycoprotein | 3.20 | 4.5E-04 | UP |
| IGHG3 | Immunoglobulin heavy constant gamma 3 | 1.77 | 4.8E-04 | UP |
| A1BG | Alpha-1B-glycoprotein | 1.71 | 5.0E-04 | UP |
| ITIH1 | Inter-alpha-trypsin inhibitor heavy chain H1 | 3.20 | 5.3E-04 | UP |
| FGG | Fibrinogen gamma chain | 1.96 | 5.4E-04 | UP |

**[Table C-14-2]**

| Gene name | Protein name | Fold change | p-value | Regulation |
|---|---|---|---|---|
| C4BPA | C4b-binding protein alpha chain | 2.80 | 5.5E-04 | UP |
| SERPINF2 | Alpha-2-antiplasmin | 1.77 | 5.5E-04 | UP |
| GSN | Gelsolin | 1.78 | 5.8E-04 | UP |
| CEACAM5 | Carcinoembryonic antigen-related cell adhesion molecule 5 | 1.77 | 6.0E-04 | UP |
| HRG | Histidine-rich glycoprotein | 1.85 | 6.1E-04 | UP |
| CFH | Complement factor H | 2.04 | 6.5E-04 | UP |
| SERPIND1 | Heparin cofactor 2 | 2.22 | 7.2E-04 | UP |
| KNG1 | Kininogen-1 | 2.53 | 7.4E-04 | UP |
| P4HB | Protein disulfide-isomerase | 2.30 | 8.0E-04 | UP |
| VIM | Vimentin | 2.80 | 9.0E-04 | UP |
| SERPINB5 | Serpin B5 | 1.89 | 9.9E-04 | UP |
| RNASE3 | Eosinophil cationic protein | 4.33 | 9.9E-04 | UP |
| MMP9 | Matrix metalloproteinase-9 | 3.88 | 1.0E-03 | UP |
| G6PD | Glucose-6-phosphate 1-dehydrogenase | 2.71 | 1.0E-03 | UP |
| C3 | Complement C3 | 2.70 | 1.0E-03 | UP |
| IGHG1 | Immunoglobulin heavy constant gamma 1 | 1.76 | 1.1E-03 | UP |
| ORM1 | Alpha-1-acid glycoprotein 1 | 2.80 | 1.1E-03 | UP |
| SERPING1 | Plasma protease C1 inhibitor | 5.91 | 1.2E-03 | UP |
| CFL1 | Cofilin-1 | 1.95 | 1.3E-03 | UP |
| H4C1 | Histone H4 | 2.44 | 1.3E-03 | UP |
| FGB | Fibrinogen beta chain | 2.49 | 1.3E-03 | UP |
| HMGB1 | High mobility group protein B1 | 4.45 | 1.4E-03 | UP |
| C4A | Complement C4-A | 1.63 | 1.5E-03 | UP |
| GPT | Alanine aminotransferase 1 | 2.89 | 1.6E-03 | UP |
| IGKC | Immunoglobulin kappa constant | 2.64 | 1.7E-03 | UP |
| FGA | Fibrinogen alpha chain | 2.41 | 1.7E-03 | UP |
| APCS | Serum amyloid P-component | 2.08 | 1.8E-03 | UP |
| PGAM1 | Phosphoglycerate mutase 1 | 2.30 | 1.9E-03 | UP |
| PDIA3 | Protein disulfide-isomerase A3 | 2.55 | 1.9E-03 | UP |
| CDC42 | Cell division control protein 42 homolog | 2.01 | 2.0E-03 | UP |
| HBB | Hemoglobin subunit beta | 8.71 | 2.1E-03 | UP |
| ELANE | Neutrophil elastase | 2.53 | 2.5E-03 | UP |
| GNAI2 | Guanine nucleotide-binding protein G | 2.74 | 2.5E-03 | UP |

**[Table C-14-3]**

| Gene name | Protein name | Fold change | p-value | Regulation |
|---|---|---|---|---|
| IGHV3-7 | Immunoglobulin heavy variable 3-7 | 2.33 | 2.5E-03 | UP |
| GSTP1 | Glutathione S-transferase P | 1.92 | 2.6E-03 | UP |
| MYH9 | Myosin-9 | 1.69 | 2.7E-03 | UP |
| PYCARD | Apoptosis-associated speck-like protein containing a CARD | 2.54 | 2.8E-03 | UP |
| ARPC3 | Actin-related protein 2/3 complex subunit 3 | 2.87 | 2.8E-03 | UP |
| C1QC | Complement C1q subcomponent subunit C | 2.58 | 2.9E-03 | UP |
| IGKV4-1 | Immunoglobulin kappa variable 4-1 | 1.95 | 2.9E-03 | UP |
| DBI | Acyl-CoA-binding protein | 3.37 | 3.0E-03 | UP |
| H2BC12 | Histone H2B type 1-K | 2.29 | 3.0E-03 | UP |
| RPL8 | 60S ribosomal protein L8 | 2.59 | 3.1E-03 | UP |
| TPT1 | Translationally-controlled tumor protein | 2.30 | 3.2E-03 | UP |
| AZU1 | Azurocidin | 3.16 | 3.2E-03 | UP |
| PFN1 | Profilin-1 | 2.01 | 3.3E-03 | UP |
| TUBB | Tubulin beta chain | 2.19 | 3.3E-03 | UP |
| HNRNPD | Heterogeneous nuclear ribonucleoprotein D0 | 2.41 | 3.5E-03 | UP |
| TPD52L2 | Tumor protein D54 | 2.39 | 3.6E-03 | UP |
| TAGLN2 | Transgelin-2 | 2.58 | 3.7E-03 | UP |
| SERPINF 1 | Pigment epithelium-derived factor | 2.53 | 4.0E-03 | UP |
| WDR1 | WD repeat-containing protein 1 | 1.61 | 4.1E-03 | UP |
| HBAL | Hemoglobin subunit alpha | 16.60 | 4.3E-03 | UP |
| ARPC2 | Actin-related protein 2/3 complex subunit 2 | 2.23 | 4.6E-03 | UP |
| ITIH2 | Inter-alpha-trypsin inhibitor heavy chain H2 | 1.57 | 4.6E-03 | UP |
| RPS14 | 40S ribosomal protein S14 | 2.10 | 4.8E-03 | UP |
| RAN | GTP-binding nuclear protein Ran | 1.68 | 4.8E-03 | UP |
| H1-5 | Histone H1.5 | 3.31 | 5.0E-03 | UP |
| CTSG | Cathepsin G | 2.34 | 5.2E-03 | UP |
| H3C1 | Histone H3.1 | 1.98 | 5.5E-03 | UP |
| SUB1 | Activated RNA polymerase II transcriptional coactivator p15 | 1.87 | 5.5E-03 | UP |
| MYL6 | Myosin light polypeptide 6 | 2.55 | 5.7E-03 | UP |
| IGKV1-5 | Immunoglobulin kappa variable 1-5 | 1.60 | 5.7E-03 | UP |
| RP1BL | Ras-related protein Rap-1b-like protein | 1.75 | 5.8E-03 | UP |
| ACTB | Actin, cytoplasmic 1 | 2.09 | 5.9E-03 | UP |

**[Table C-14-4]**

| Gene name | Protein name | Fold change | p-value | Regulation |
|---|---|---|---|---|
| ANXA1 | Annexin A1 | 1.96 | 5.9E-03 | UP |
| TUBB4B | Tubulin beta-4B chain | 1.52 | 6.2E-03 | UP |
| YWHAE | 14-3-3 protein epsilon | 1.57 | 6.6E-03 | UP |
| YWHAH | 14-3-3 protein eta | 1.73 | 6.9E-03 | UP |
| PPIB | Peptidyl-prolyl cis-trans isomerase B | 1.53 | 7.5E-03 | UP |
| NME2 | Nucleoside diphosphate kinase B | 2.05 | 7.8E-03 | UP |
| IGKV3-11 | Immunoglobulin kappa variable 3-11 | 2.04 | 7.8E-03 | UP |
| CAMP | Cathelicidin antimicrobial peptide | 2.43 | 7.8E-03 | UP |
| RAC2 | Ras-related C3 botulinum toxin substrate 2 | 3.28 | 8.0E-03 | UP |
| SRSF3 | Serine/arginine-rich splicing factor 3 | 2.15 | 8.0E-03 | UP |
| GPI | Glucose-6-phosphate isomerase | 1.61 | 8.2E-03 | UP |
| AGT | Angiotensinogen | 2.00 | 8.5E-03 | UP |
| MIF | Macrophage migration inhibitory factor | 2.44 | 9.2E-03 | UP |
| PYGL | Glycogen phosphorylase, liver form | 3.88 | 9.8E-03 | UP |
| IGHV3-33 | Immunoglobulin heavy variable 3-33 | 1.64 | 9.9E-03 | UP |
| RPL6 | 60S ribosomal protein L6 | 2.71 | 0.010 | UP |
| PLS3 | Plastin-3 | 1.80 | 0.010 | UP |
| MACROH2A1 | Core histone macro-H2A.1 | 3.38 | 0.011 | UP |
| IGKV3-20 | Immunoglobulin kappa variable 3-20 | 2.22 | 0.011 | UP |
| CORO1A | Coronin-1A | 1.59 | 0.011 | UP |
| RPS19 | 40S ribosomal protein S19 | 2.32 | 0.011 | UP |
| ANXA6 | Annexin A6 | 2.26 | 0.012 | UP |
| PON1 | Serum paraoxonase/arylesterase 1 | 3.88 | 0.012 | UP |
| APOA2 | Apolipoprotein A-II | 3.16 | 0.012 | UP |
| ARHGDIB | Rho GDP-dissociation inhibitor 2 | 2.07 | 0.013 | UP |
| MYL12B | Myosin regulatory light chain 12B | 2.19 | 0.013 | UP |
| HSPA1A | Heat shock 70 kDa protein 1A | 1.75 | 0.013 | UP |
| BTF3 | Transcription factor BTF3 | 1.54 | 0.013 | UP |
| AKR1A1 | Aldo-keto reductase family 1 member A1 | 1.63 | 0.013 | UP |
| UGP2 | UTP--glucose-1-phosphate uridylyltransferase | 1.70 | 0.013 | UP |
| LCP1 | Plastin-2 | 1.63 | 0.014 | UP |
| LCN2 | Neutrophil gelatinase-associated lipocalin | 2.33 | 0.014 | UP |
| UBE2N | Ubiquitin-conjugating enzyme E2 N | 1.64 | 0.014 | UP |
| COTL1 | Coactosin-like protein | 4.01 | 0.014 | UP |

**[Table C-14-5]**

| Gene name | Protein name | Fold change | p-value | Regulation |
|---|---|---|---|---|
| RALY | RNA-binding protein Raly | 1.55 | 0.015 | UP |
| DEFA3 | Neutrophil defensin 3 | 2.23 | 0.015 | UP |
| NAMPT | Nicotinamide phosphoribosyltransferase | 2.28 | 0.015 | UP |
| IGHG2 | Immunoglobulin heavy constant gamma 2 | 1.69 | 0.015 | UP |
| H1-3 | Histone H1.3 | 2.82 | 0.016 | UP |
| ALDH3A1 | Aldehyde dehydrogenase, dimeric NADP-preferring | 2.32 | 0.016 | UP |
| C1S | Complement C1s subcomponent | 2.23 | 0.016 | UP |
| ACTR2 | Actin-related protein 2 | 1.92 | 0.016 | UP |
| TNNI3K | Serine/threonine-protein kinase TNNI3K | 2.00 | 0.016 | UP |
| AFM | Afamin | 4.46 | 0.017 | UP |
| ASPRV1 | Retroviral-like aspartic protease 1 | 1.81 | 0.017 | UP |
| CAPZA1 | F-actin-capping protein subunit alpha-1 | 1.94 | 0.018 | UP |
| MPO | Myeloperoxidase | 1.60 | 0.018 | UP |
| CANX | Calnexin | 1.96 | 0.018 | UP |
| CBR1 | Carbonyl reductase [NADPH] 1 | 3.01 | 0.019 | UP |
| DNAJB1 | DnaJ homolog subfamily B member 1 | 1.93 | 0.019 | UP |
| CAPG | Macrophage-capping protein | 1.77 | 0.020 | UP |
| H1-0 | Histone H1.0 | 2.42 | 0.020 | UP |
| RPL4 | 60S ribosomal protein L4 | 2.23 | 0.020 | UP |
| TRIM29 | Tripartite motif-containing protein 29 | 1.54 | 0.020 | UP |
| EFNA1 | Ephrin-A1 | 1.72 | 0.020 | UP |
| HNRNPK | Heterogeneous nuclear ribonucleoprotein K | 1.59 | 0.021 | UP |
| CALR | Calreticulin | 2.53 | 0.021 | UP |
| IGLV1-51 | Immunoglobulin lambda variable 1-51 | 1.51 | 0.022 | UP |
| RPS6 | 40S ribosomal protein S6 | 1.56 | 0.023 | UP |
| LPO | Lactoperoxidase | 5.16 | 0.024 | UP |
| TMSL3 | Thymosin beta-4-like protein 3 | 2.89 | 0.024 | UP |
| EFHD2 | EF-hand domain-containing protein D2 | 2.55 | 0.026 | UP |
| SEPTIN8 | Septin-8 | 2.03 | 0.026 | UP |
| RPS9 | 40S ribosomal protein S9 | 1.54 | 0.028 | UP |
| YWHAG | 14-3-3 protein gamma | 1.53 | 0.028 | UP |
| TMED5 | Transmembrane emp24 domain-containing protein 5 | 1.65 | 0.030 | UP |
| HNRNPR | Heterogeneous nuclear ribonucleoprotein R | 2.20 | 0.030 | UP |
| SBSN | Suprabasin | 5.57 | 0.030 | UP |

**[Table C-14-6]**

| Gene name | Protein name | Fold change | p-value | Regulation |
|---|---|---|---|---|
| SRSF2 | Serine/arginine-rich splicing factor 2 | 2.00 | 0.030 | UP |
| LDHA | L-lactate dehydrogenase A chain | 1.66 | 0.031 | UP |
| IGHV3-30 | Immunoglobulin heavy variable 3-30 | 2.49 | 0.031 | UP |
| LRG1 | Leucine-rich alpha-2-glycoprotein | 1.50 | 0.033 | UP |
| RPL12 | 60S ribosomal protein L12 | 1.73 | 0.035 | UP |
| CCT6A | T-complex protein 1 subunit zeta | 2.13 | 0.037 | UP |
| RPL18A | 60S ribosomal protein L18a | 1.71 | 0.037 | UP |
| THBS1 | Thrombospondin-1 | 2.04 | 0.038 | UP |
| C7 | Complement component C7 | 3.69 | 0.040 | UP |
| RPL10A | 60S ribosomal protein L10a | 1.57 | 0.042 | UP |
| ITGB2 | Integrin beta-2 | 2.17 | 0.043 | UP |
| CA2 | Carbonic anhydrase 2 | 2.27 | 0.044 | UP |
| RPS25 | 40S ribosomal protein S25 | 1.83 | 0.044 | UP |
| RAB1B | Ras-related protein Rab-1B | 2.03 | 0.048 | UP |
| PSMD14 | 26S proteasome non-ATPase regulatory subunit 14 | 2.67 | 0.048 | UP |
| RPL5 | 60S ribosomal protein L5 | 1.89 | 0.049 | UP |
| BPI | Bactericidal permeability-increasing protein | 1.69 | 0.050 | UP |
| FLG2 | Filaggrin-2 | 0.51 | 1.3E-04 | DOWN |
| DHX36 | ATP-dependent DNA/RNA helicase DHX36 | 0.27 | 1.3E-03 | DOWN |
| MGST2 | Microsomal glutathione S-transferase 2 | 0.62 | 2.8E-03 | DOWN |
| GSDMA | Gasdermin-A | 0.64 | 4.2E-03 | DOWN |
| TPP1 | Tripeptidyl-peptidase 1 | 0.66 | 5.5E-03 | DOWN |
| F5 | Coagulation factor V | 0.71 | 6.1E-03 | DOWN |
| KRT77 | Keratin, type II cytoskeletal 1b | 0.63 | 6.1E-03 | DOWN |
| STS | Steryl-sulfatase | 0.48 | 6.3E-03 | DOWN |
| MYH1 | Myosin-1 | 0.35 | 8.0E-03 | DOWN |
| PLD3 | 5'-3' exonuclease PLD3 | 0.67 | 8.6E-03 | DOWN |
| SCGB2A2 | Mammaglobin-A | 0.52 | 9.3E-03 | DOWN |
| PSMB4 | Proteasome subunit beta type-4 | 0.55 | 0.010 | DOWN |
| CCAR2 | Cell cycle and apoptosis regulator protein 2 | 0.45 | 0.011 | DOWN |
| PSMB3 | Proteasome subunit beta type-3 | 0.67 | 0.011 | DOWN |
| PSMA1 | Proteasome subunit alpha type-1 | 0.69 | 0.014 | DOWN |
| DHRS11 | Dehydrogenase/reductase SDR family member 11 | 0.53 | 0.014 | DOWN |
| POM121 | Nuclear envelope pore membrane protein POM 121 | 0.47 | 0.019 | DOWN |

**[Table C-14-7]**

| Gene name | Protein name | Fold change | p-value | Regulation |
|---|---|---|---|---|
| HSPE1 | 10 kDa heat shock protein, mitochondrial | 0.65 | 0.020 | DOWN |
| FBXO6 | F-box only protein 6 | 0.69 | 0.022 | DOWN |
| GART | Trifunctional purine biosynthetic protein adenosine-3 | 0.66 | 0.023 | DOWN |
| DCD | Dermcidin | 0.58 | 0.023 | DOWN |
| CRNN | Cornulin | 0.59 | 0.024 | DOWN |
| SYNGR2 | Synaptogyrin-2 | 0.66 | 0.026 | DOWN |
| PHB2 | Prohibitin-2 | 0.72 | 0.028 | DOWN |
| DLD | Dihydrolipoyl dehydrogenase, mitochondrial | 0.75 | 0.032 | DOWN |
| ME1 | NADP-dependent malic enzyme | 0.59 | 0.033 | DOWN |
| IDH2 | Isocitrate dehydrogenase [NADP], mitochondrial | 0.63 | 0.035 | DOWN |
| IMPA2 | Inositol monophosphatase 2 | 0.65 | 0.039 | DOWN |
| HMGA1 | High mobility group protein HMG-I/HMG-Y | 0.55 | 0.040 | DOWN |
| KRT15 | Keratin, type I cytoskeletal 15 | 0.65 | 0.040 | DOWN |
| PLTP | Phospholipid transfer protein | 0.67 | 0.040 | DOWN |
| SFPQ | Splicing factor, proline- and glutamine-rich | 0.50 | 0.042 | DOWN |
| GMPR2 | GMP reductase 2 | 0.71 | 0.043 | DOWN |
| ZNF236 | Zinc finger protein 236 | 0.28 | 0.046 | DOWN |
| TIMP2 | Metalloproteinase inhibitor 2 | 0.48 | 0.048 | DOWN |
| ZNF292 | Zinc finger protein 292 | 0.71 | 0.049 | DOWN |

### 4-2 Construction of discriminant model using protein with high variable importance in random forest

### 1) Selection of feature protein

The Log₂ (Abundance + 1) values of the 985 proteins were used as explanatory variables, and the healthy subjects and the AD patients (the presence or absence of AD) were used as objective variables. Random forest algorithm was designated as a method in the "caret" package of R language, the number of variables (mtry value) for use in the construction of one decision tree was tuned into the optimum value. The random forest algorithm was carried out using the mtry value determined by tuning, and top 110 proteins of variable importance based on Gini coefficient were calculated (Tables C-15-1 to C-15-4). These 110 proteins and all the 985 proteins used in the selection of feature proteins were used as feature proteins, and quantitative data thereon was used as features.

### 2) Model construction

The Log₂ (Abundance + 1) values of the 110 proteins or all the 985 proteins were used as explanatory variables, and the healthy subjects and the AD patients (the presence or absence of AD) were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an estimate error rate (OOB error rate) was calculated. As a result, the error rate was 29.27% when all the 985 proteins were used as feature proteins, whereas the error rate was 12.20% when the top 110 proteins of variable importance were used as feature proteins.

**[Table C-15-1]**

| Ran k | Gene name | Protein name | Mean Decreas e Gini |
|---|---|---|---|
| 1 | SERPINB1 | Leukocyte elastase inhibitor | 0.565 |
| 2 | SERPINC1 | Antithrombin-!!! | 0.505 |
| 3 | KLKB1 | Plasma kallikrein | 0.396 |
| 4 | TTR | Transthyretin | 0.388 |
| 5 | DHX36 | ATP-dependent DNA/RNA helicase DHX36 | 0.373 |
| 6 | ITIH4 | Inter-alpha-trypsin inhibitor heavy chain H4 | 0.370 |
| 7 | GC | Vitamin D-binding protein | 0.360 |
| 8 | ALB | Serum albumin | 0.346 |
| 9 | F5 | Coagulation factor V | 0.332 |
| 10 | SERPING 1 | Plasma protease C1 inhibitor | 0.286 |
| 11 | DDX55 | ATP-dependent RNA helicase DDX55 | 0.262 |
| 12 | HP | Haptoglobin | 0.251 |
| 13 | IGHV1-46 | Immunoglobulin heavy variable 1-46 | 0.251 |
| 14 | EZR | Ezrin | 0.243 |
| 15 | VTN | Vitronectin | 0.238 |
| 16 | AHSG | Alpha-2-HS-glycoprotein | 0.213 |
| 17 | EPX | Eosinophil peroxidase | 0.211 |
| 18 | HPX | Hemopexin | 0.206 |
| 19 | PPIA | Peptidyl-prolyl cis-trans isomerase A | 0.197 |
| 20 | TF | Serotransferrin | 0.194 |
| 21 | KNG1 | Kininogen-1 | 0.176 |
| 22 | HMGB2 | High mobility group protein B2 | 0.171 |
| 23 | FN1 | Fibronectin | 0.157 |
| 24 | OPRPN | Opiorphin prepropeptide | 0.156 |
| 25 | CFB | Complement factor B | 0.155 |
| 26 | TASOR2 | Protein TASOR 2 | 0.151 |
| 27 | NDUFB6 | NADH dehydrogenase [ubiquinone] 1 beta subcomplex subunit 6 | 0.148 |
| 28 | CDC42 | Cell division control protein 42 homolog | 0.148 |
| 29 | PLG | Plasminogen | 0.139 |
| 30 | HNRNPD | Heterogeneous nuclear ribonucleoprotein D0 | 0.133 |

**[Table C-15-2]**

| Rank | Gene name | Protein name | Mean Decrease Gini |
|---|---|---|---|
| 31 | CCT3 | T-complex protein 1 subunit gamma | 0.129 |
| 32 | SERBP1 | Plasminogen activator inhibitor 1 RNA-binding protein | 0.125 |
| 33 | ORM1 | Alpha-1-acid glycoprotein 1 | 0.123 |
| 34 | PGAM1 | Phosphoglycerate mutase 1 | 0.122 |
| 35 | PDIA6 | Protein disulfide-isomerase A6 | 0.118 |
| 36 | GLRX | Glutaredoxin-1 | 0.117 |
| 37 | TPD52L2 | Tumor protein D54 | 0.116 |
| 38 | MSN | Moesin | 0.115 |
| 39 | PRDX6 | Peroxiredoxin-6 | 0.111 |
| 40 | AMBP | Protein AMBP | 0.111 |
| 41 | HMGA1 | High mobility group protein HMG-I/HMG-Y | 0.108 |
| 42 | IMPA2 | Inositol monophosphatase 2 | 0.103 |
| 43 | ASPRV1 | Retroviral-like aspartic protease 1 | 0.100 |
| 44 | PSMA1 | Proteasome subunit alpha type-1 | 0.098 |
| 45 | WDR1 | WD repeat-containing protein 1 | 0.095 |
| 46 | GARS1 | Glycine--tRNA ligase | 0.092 |
| 47 | ME1 | NADP-dependent malic enzyme | 0.090 |
| 48 | KRT25 | Keratin, type I cytoskeletal 25 | 0.089 |
| 49 | KRT77 | Keratin, type II cytoskeletal 1b | 0.088 |
| 50 | PSMB4 | Proteasome subunit beta type-4 | 0.087 |
| 51 | GSN | Gelsolin | 0.086 |
| 52 | PLS3 | Plastin-3 | 0.084 |
| 53 | FLG2 | Filaggrin-2 | 0.082 |
| 54 | CPQ | Carboxypeptidase Q | 0.080 |
| 55 | IGKV3-20 | Immunoglobulin kappa variable 3-20 | 0.079 |
| 56 | ELANE | Neutrophil elastase | 0.078 |
| 57 | KRT79 | Keratin, type II cytoskeletal 79 | 0.075 |
| 58 | RPL18A | 60S ribosomal protein L18a | 0.074 |
| 59 | APOA1 | Apolipoprotein A-I | 0.073 |
| 60 | TIMP1 | Metalloproteinase inhibitor 1 | 0.073 |

**[Table C-15-3]**

| Rank | Gene name | Protein name | Mean Decrease Gini |
|---|---|---|---|
| 61 | HBB | Hemoglobin subunit beta | 0.070 |
| 62 | KLK10 | Kallikrein-10 | 0.068 |
| 63 | H4C1 | Histone H4 | 0.068 |
| 64 | ARPC3 | Actin-related protein 2/3 complex subunit 3 | 0.066 |
| 65 | CTSA | Lysosomal protective protein | 0.066 |
| 66 | ALDH3A1 | Aldehyde dehydrogenase, dimeric NADP-preferring | 0.065 |
| 67 | POF1B | Protein POF1B | 0.064 |
| 68 | CFL1 | Cofilin-1 | 0.063 |
| 69 | TPP1 | Tripeptidyl-peptidase 1 | 0.063 |
| 70 | HM13 | Minor histocompatibility antigen H13 | 0.062 |
| 71 | CP | Ceruloplasmin | 0.061 |
| 72 | MMP9 | Matrix metalloproteinase-9 | 0.060 |
| 73 | LRG1 | Leucine-rich alpha-2-glycoprotein | 0.060 |
| 74 | ITIH1 | Inter-alpha-trypsin inhibitor heavy chain H1 | 0.059 |
| 75 | KV310 | Ig kappa chain V-III region VH | 0.058 |
| 76 | SERPINA1 | Alpha-1-antitrypsin | 0.057 |
| 77 | APOB | Apolipoprotein B-100 | 0.055 |
| 78 | DDB1 | DNA damage-binding protein 1 | 0.054 |
| 79 | F2 | Prothrombin | 0.053 |
| 80 | HSPA9 | Stress-70 protein, mitochondrial | 0.051 |
| 81 | TAGLN2 | Transgelin-2 | 0.051 |
| 82 | RPL13 | 60S ribosomal protein L13 | 0.050 |
| 83 | IGHG3 | Immunoglobulin heavy constant gamma 3 | 0.050 |
| 84 | ACP5 | Tartrate-resistant acid phosphatase type 5 | 0.049 |
| 85 | AGRN | Agrin | 0.048 |
| 86 | MTAP | S-methyl-5'-thioadenosine phosphorylase | 0.048 |
| 87 | CRISPLD2 | Cysteine-rich secretory protein LCCL domain-containing 2 | 0.047 |
| 88 | PSMB2 | Proteasome subunit beta type-2 | 0.047 |
| 89 | ANXA11 | Annexin A11 | 0.046 |
| 90 | SCGB2A2 | Mammaglobin-A | 0.046 |

**[Table C-15-4]**

| Rank | Gene name | Protein name | Mean Decrease Gini |
|---|---|---|---|
| 91 | MAST4 | Microtubule-associated serine/threonine-protein kinase 4 | 0.044 |
| 92 | SERPINF1 | Pigment epithelium-derived factor | 0.043 |
| 93 | ATP5PO | ATP synthase subunit O, mitochondrial | 0.043 |
| 94 | E!F3! | Eukaryotic translation initiation factor 3 subunit I | 0.043 |
| 95 | CCT6A | T-complex protein 1 subunit zeta | 0.042 |
| 96 | RP1BL | Ras-related protein Rap-1b-like protein | 0.042 |
| 97 | RPS16 | 40S ribosomal protein S16 | 0.042 |
| 98 | DNAAF1 | Dynein assembly factor 1, axonemal | 0.042 |
| 99 | RANBP1 | Ran-specific GTPase-activating protein | 0.042 |
| 100 | KRT15 | Keratin, type I cytoskeletal 15 | 0.041 |
| 101 | APOH | Beta-2-glycoprotein 1 | 0.039 |
| 102 | REEP5 | Receptor expression-enhancing protein 5 | 0.039 |
| 103 | RPL7 | 60S ribosomal protein L7 | 0.039 |
| 104 | ATP1B1 | Sodium/potassium-transporting ATPase subunit beta-1 | 0.039 |
| 105 | CASP14 | Caspase-14 | 0.039 |
| 106 | RAN | GTP-binding nuclear protein Ran | 0.038 |
| 107 | MIF | Macrophage migration inhibitory factor | 0.038 |
| 108 | RDH12 | Retinol dehydrogenase 12 | 0.038 |
| 109 | C3 | Complement C3 | 0.037 |
| 110 | RPL8 | 60S ribosomal protein L8 | 0.037 |

### 4-3 Construction of discriminant model using feature extracted by Boruta method

### 1) Selection of feature

The Log₂ (Abundance + 1) values of the 985 proteins were used as explanatory variables, and the healthy subject and the AD patients (the presence or absence of AD) were used as objective variables. Algorithm in the "Boruta" package of R language was carried out. The maximum number of trials was set to 1,000, and 24 proteins which attained a p value of less than 0.01 were extracted (Table C-16) and used as feature proteins. Quantitative data on these proteins was used as features.

### 2) Model construction

The Log₂ (Abundance + 1) values of the 24 proteins were used as explanatory variables, and the healthy subject and the AD patients (the presence or absence of AD) were used as objective variables. Random forest algorithm was designated as a method in the "caret" package of R language, and the number of variables (mtry value) for use in the construction of one decision tree was tuned into the optimum value. The random forest algorithm was carried out using the mtry value determined by tuning, and an OOB error rate was calculated. As a result, the error rate was 19.51% in the model using the 24 proteins as feature proteins.

**[Table C-16]**

| Gene name | Protein name |
|---|---|
| VTN | Vitronectin |
| FN1 | Fibronectin |
| ALB | Serum albumin |
| ITIH4 | Inter-alpha-trypsin inhibitor heavy chain H4 |
| EZR | Ezrin |
| HPX | Hemopexin |
| GC | Vitamin D-binding protein |
| DDX55 | ATP-dependent RNA helicase DDX55 |
| TTR | Transthyretin |
| SERPING1 | Plasma protease C1 inhibitor |
| AHSG | Alpha-2-HS-glycoprotein |
| PLG | Plasminogen |
| KNG1 | Kininogen-1 |
| SERPINB1 | Leukocyte elastase inhibitor |
| EPX | Eosinophil peroxidase |
| IGHV1-46 | Immunoglobulin heavy variable 1-46 |
| PPIA | Peptidyl-prolyl cis-trans isomerase A |
| PRDX6 | Peroxiredoxin-6 |
| KLKB1 | Plasma kallikrein |
| SERPINC1 | Antithrombin-III |
| OPRPN | Opiorphin prepropeptide |
| NDUFB6 | NADH dehydrogenase [ubiquinone] 1 beta subcomplex subunit 6 |
| DHX36 | ATP-dependent DNA/RNA helicase DHX36 |
| FLG2 | Filaggrin-2 |

A total of 418 proteins (Tables C-1-1 to C-1-13 described above) obtained in the analysis of these Examples C-1 to C-4 were examined for the number of articles reporting their relation to AD by text mining (Elsevier). By the mining, 147 proteins were reported in 4 or less articles related to AD, and confirmed to be free from description about relation to AD (Tables C-2-1 to C-2-5 described above). These 147 proteins are novel markers for detecting AD.

### Example D-1 Identification of AD-related protein in child SSL and expression analysis of SerpinB4 protein

### 1) Test subject and SSL collection

23 healthy children (from 6 months to 5 years old, male and female) (healthy group) and 16 children with atopic dermatitis (children with AD) (from 6 months to 5 years old, male and female) (AD group) were selected as test subjects. For the recruiting of the children with AD, children with AD who satisfied the UKWP criteria (The UK Working Party; Br J Dermatol, 131: 406-416 (1994)) under parent's judgement were gathered, and patients from whom a parent's consent was obtained by informed consent were selected. A dermatologist performed systemic skin observation and interview as to the selected children with AD, and diagnosed AD on the basis of Guidelines for the Management of Atopic Dermatitis (see The Japanese Journal of Dermatology, 128 (12): 2431-2502, 2018). Among the children with AD who were thus diagnosed with AD, children who manifested symptoms such as mild or higher AD-like eczema or dryness on the face were selected as test subjects on the basis of the eczema area and severity index (EASI; Exp Dermatol, 10: 11-18 (2001)). The selected 16 subjects of the AD group included 9 mild subjects (mild AD group) and 7 moderate subjects (moderate AD group) based on EAST scores.

Sebum was collected from each site of the whole face (including an eruption site for the children with AD) and the whole back (including no eruption site for the children with AD) of each test subject using an oil blotting film (5 × 8 cm, made of polypropylene, 3M Company). The oil blotting film was transferred to a glass vial and preserved at -80°C for approximately 1 month until use in protein extraction.

### 2) Protein preparation

The oil blotting film of the above section 1) was cut into an appropriate size, and protein precipitates were obtained using QIAzol Lysis Reagent (Qiagen N.V.) in accordance with the attached protocol. Proteins were dissolved from the obtained protein precipitates with a solubilizing solution using MPEX PTS Reagent (GL Sciences Inc.) in accordance with the attached protocol, and then digested with trypsin. The obtained digested solution was dried under reduced pressure (35°C) and then dissolved in an aqueous solution containing 0.1% (v/v) formic acid and 2% (v/v) acetonitrile to prepare a peptide solution. Peptide concentrations in the solution were measured using a microplate reader (Corona Electric Co., Ltd.) in accordance with the protocol of Pierce(TM) Quantitative Fluorometric Peptide Assay (Thermo Fisher Scientific, Inc.). Quantitative values of proteins were calculated by LC-MS/MS analysis with constant concentrations of peptide solutions. Peptide solutions from one specimen of the back among the healthy children and one specimen of the face among the children with AD were excluded from LC-MS/MS analysis because a necessary amount of peptides could not be obtained.

### 3) LC-MS/MS analysis and data analysis

Each sample peptide solution obtained in the above section 2) was analyzed by LC-MS/MS under conditions of the following Table D-1.

**[Table D-1]**

| | System and parameter |
|---|---|
| LC | nanoAcquity UPLC (Waters) |
| Trap column | nanoEase Xbridge BEH 130 C18, 0.3 mm × 50 mm, 5 µm |
| Column | nanoAcquity BEH 130 C18, 0.1 mm × 100 mm, 1.7 µm, 40°C |
| Solution A | 0.1% (v/v) Formic acid, water |
| Solution B | 0.1% (v/v) Formic acid, 80% (v/v) acetonitrile |
| Flow rate | 0.4-0.5 µL/min |
| Injection volume | 4 µL |
| Gradient | Sol.B 5% (0-5 min) -> Sol.B 50% (125 min) -> Sol. B 95% (126-150 min) |
| MS system | Q-Exactive plus (Thermo Fisher Scientific) |
| Collision | HCD |
| Top N MSMS | 15 |
| Detection | nanoESI, Positive polarty, Spray voltage: 1,800V, |
| Capillary temp | 250°C |

The spectral data obtained by LC-MS/MS analysis was analyzed using Proteome Discoverer ver. 2.2 (Thermo Fisher Scientific, Inc.). For human-derived protein identification, a reference database was Swiss Prot and was searched using Mascot database search (Matrix Science) with Taxonomy set to Homo sapiens. In the search, Enzyme was set to Trypsin; Missed cleavage was set to 2; Dynamic modifications were set to Oxidation (M), Acetyl (N-term), and Acetyl (Protein N-term); and Static Modifications were set to Carbamidomethyl (C). Peptides which satisfied a false discovery rate (FDR) of p < 0.01 were to be searched for. The identified proteins were subjected to label free quantification (LFQ) based on precursor ions. Protein abundance was calculated from the peak intensity of precursor ions derived from the peptides, and peak intensity equal to or lower than a detection limit was regarded as a missing value. In order to correct experimental bias, the protein abundance was normalized by the total peptide amount method, and protein abundance ratios were calculated by the summed abundance based method, p values which indicate the significance of difference in abundance among groups were calculated using ANOVA (individual based, t study). Among the identified human-derived proteins, proteins having a false discovery rate (FDR) of 0.1 or more were excluded from analysis. Prism 8 ver. 3.0 was used in diagram drawing and statistical processing given below. A Log₂ (Abundance + 1) value was calculated by the conversion of a value of the unnormalized protein abundance divided by the sum of the abundance values of all the human-derived proteins to a logarithmic value to base 2, and used as each protein quantitative value.

### 4) Expression analysis (eruption site)

First, 533 proteins which produced calculated abundance without missing values in 75% or more test subjects in either the healthy group or the AD group were extracted as analysis objects by the analysis of human-derived proteins contained in SSL collected from the face (including an eruption site for the AD group). 116 proteins whose abundance ratio was increased to 1.5 times or more (p ≤ 0.05) in the AD group compared with the healthy group were identified, and included SerpinB4 protein. Figure 1 shows a plot of the quantitative value (Log₂ (Abundance + 1)) of SerpinB4 protein in SSL derived from the face of each test subject of the healthy group and the AD group. It was found that the expression level of SerpinB4 protein in SSL collected from the eruption sites (face) of the AD group was statistically significantly increased as compared with the healthy group (face) (Student's t-test, P < 0.001).

15 AD patients except for one subject excluded from LC-MS/MS analysis were divided into a mild AD group (9 subjects) and a moderate AD group (6 subjects). Figure 2 shows a plot of the quantitative value (Log₂ (Abundance + 1)) of SerpinB4 protein in SSL derived from the face of each test subject of the healthy group, the mild AD group, and the moderate AD group. It was found that the expression level of SerpinB4 protein in SSL collected from the eruption sites (face) of the mild AD group and the moderate AD group was statistically significantly increased as compared with the healthy group (face), and increased in a stepwise fashion depending on severity (Tukey's test, P < 0.05 or P < 0.001).

### 5) Expression analysis (non-eruption site)

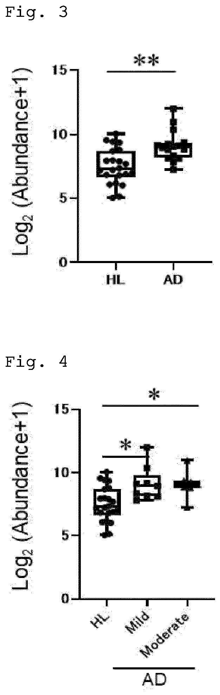

Next, 894 proteins which produced calculated abundance without missing values in 75% or more test subjects in either the healthy group or the AD group were extracted as analysis objects by the analysis of SSL-derived proteins collected from the back including no eruption. 135 proteins whose abundance ratio was increased to 1.5 times or more (p ≤ 0.05) in the AD group compared with the healthy group were identified, and included SerpinB4 protein. Figure 3 shows a plot of the quantitative value (Log₂ (Abundance + 1)) of SerpinB4 protein in SSL derived from the back of each test subject of the healthy group and the AD group. It was found that the expression level of SerpinB4 protein in SSL collected from the non-eruption sites (back) of the AD group was statistically significantly increased as compared with the healthy group (back) (Student's t-test, P < 0.01).

16 AD patients were divided into a mild AD group (9 subjects) and a moderate AD group (7 subjects). Figure 4 shows a plot of the quantitative value (Log₂ (Abundance + 1)) of SerpinB4 protein in SSL derived from the back of each test subject of the healthy group, the mild AD group, and the moderate AD group. It was found that the expression level of SerpinB4 protein in SSL collected from the non-eruption sites (back) of the mild AD group and the moderate AD group was statistically significantly increased as compared with the healthy group (back) (Tukey's test, P < 0.05).

### 6) ROC analysis

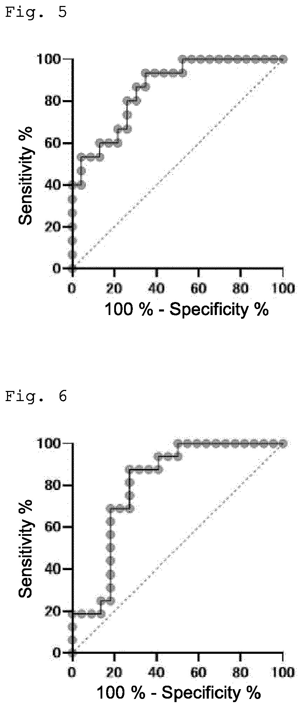

ROC curves were prepared (Figures 5 and 6) using the quantitative value (Log₂ (Abundance + 1)) of SerpinB4 protein in SSL collected from the face (eruption sites for the AD group) and the back (non-eruption sites for the AD group) of each test subject of the healthy group and the AD group. For SerpinB4 protein in SSL collected from the face (eruption sites for the AD group) an area under the ROC curve was 0.86 and a p value was 0.0002 which was significant, indicating the effectiveness of the detection of childhood atopic dermatitis using the SerpinB4 protein expression level in SSL as an index. The detection accuracy of AD using a cutoff value of 7.76 based on the Youden index was sensitivity of 93.33% and specificity of 65.22% (Figure 5). On the other hand, for SerpinB4 protein in SSL collected from the back (non-eruption sites for the AD group), an area under the ROC curve was 0.80 and a p value was 0.0016 which was significant, also indicating the effectiveness of the detection of childhood atopic dermatitis using the SerpinB4 protein expression level in SSL at a non-eruption site as an index. The detection accuracy of AD using a cutoff value of 8.05 based on the Youden index was sensitivity of 87.50% and specificity of 72.73% (Figure 6).

### Comparative Example D-1 Expression analysis of AD-related RNA in child SSL

### 1) RNA preparation and sequencing

SSL-derived RNA of test subjects was extracted from a nucleic acid-containing fraction obtained in the process of extracting proteins from the oil blotting film containing SSL collected from the face (eruption sites for the AD group) in Example D-1. On the basis of the extracted RNA, cDNA was synthesized through reverse transcription at 42°C for 90 minutes using Superscript VILO cDNA Synthesis kit (Life Technologies Japan Ltd.). The primers used for reverse transcription reaction were random primers attached to the kit. A library containing DNA derived from 20802 genes was prepared by multiplex PCR from the obtained cDNA. The multiplex PCR was performed using Ion AmpliSeq Transcriptome Human Gene Expression Kit (Life Technologies Japan Ltd.) under conditions of [99°C, 2 min → (99°C, 15 sec -> 62°C, 16 min) × 20 cycles → 4°C, hold]. The obtained PCR product was purified with Ampure XP (Beckman Coulter Inc.), followed by buffer reconstitution, primer sequence digestion, adaptor ligation, purification, and amplification to prepare a library. The prepared library was loaded on Ion 540 Chip and sequenced using Ion S5/XL system (Life Technologies Japan Ltd.).

### 2) Data analysis

### i) Data used

Data (read count values) on the expression level of RNA derived from the test subjects measured in the above section 1) was normalized by use of DESeq2. Log₂(Normalized count + 1) was calculated from the normalized count values and used in RNA expression analysis.

### ii) RNA expression analysis

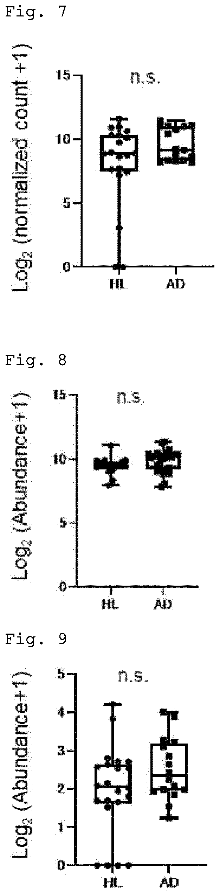

Figure 7 shows a plot of the expression level (Log₂ (Normalized count + 1)) of SerpinB4 RNA from each test subject of the healthy group and the AD group. No significant increase in SerpinB4 RNA expression level was observed in the AD group compared with the healthy group. Specifically, it was found from Example D-1 and this example that no significant increase in the expression level of SerpinB4 RNA in SSL was observed in the AD group, whereas the expression level of SerpinB4 protein was significantly increased in the AD group, indicating that the expression of SerpinB4 in SSL is inconsistent between the protein and the RNA.

### Comparative Example D-2 Expression analysis of SerpinB4 protein in adult SSL

### 1) Test subject and SSL collection

18 healthy subjects (from 20 to 59 years old, male) (healthy group) and 26 atopic dermatitis patients (AD patients) (from 20 to 59 years old, male) (AD group) were selected as test subjects. A consent was obtained from the test subjects by informed consent. The test subjects of the AD group were AD patients each diagnosed with mild or moderate atopic dermatitis when a dermatologist comprehensively assessed severity on five scales "minor", "mild", "moderate", "severe" and "most severe" on the day of the test as to the face. Sebum was collected from the whole face (including an eruption site for the AD patients) of each test subject using an oil blotting film (5 × 8 cm, made of polypropylene, 3M Company). The oil blotting film was transferred to a vial and preserved at -80°C for approximately 1 month until use in protein extraction.

### 2) Protein preparation

Peptide solution preparation and peptide concentration measurement were performed by the same procedures as in Example D-1 except that the peptide solution was obtained using EasyPep(TM) Mini MS Sample Prep Kit (Thermo Fisher Scientific, Inc.) instead of MPEX PTS Reagent (GL Sciences Inc.) in accordance with the attached protocol.

### 3) LC-MS/MS analysis and data analysis

Protein analysis and data analysis were conducted using the same conditions and procedures as in Example D-1.

### 4) Results

Among the identified proteins, proteins having a false discovery rate (FDR) of 0.1 or more were excluded from analysis. 1075 proteins which produced calculated protein abundance without missing values in 75% or more test subjects in either the healthy group or the AD group were extracted as analysis objects. One AD patient for whom many missing values were observed in the protein abundance was excluded from analysis. 205 proteins whose abundance ratio was increased to 1.5 time or more (p ≤ 0.05) were obtained in the AD group compared with the healthy group, but did not include SerpinB4 protein. Figure 8 shows a plot of the quantitative value (Log₂ (Abundance + 1)) of SerpinB4 protein from each test subject of the healthy group and the AD group. According to the previous report, it has been reported that SerpinB4 protein concentrations in blood are elevated in pediatric and adult AD patients (Non Patent Literature 7). On the other hand, it was found from the results of Example D-1 and this example that the expression level of SerpinB4 protein in SSL was increased in childhood AD but was not increased in adult AD, demonstrating that the expression of SerpinB4 in SSL is not necessarily consistent with its difference in blood.

### Comparative Example D-3 Expression analysis of known AD-related protein in child SSL

According to the previous reports, it has been reported that: the level of interleukin-18 (IL-18) protein is increased in the blood of children with childhood AD compared with healthy children; and the level of SerpinB12 protein is decreased in the stratum corneum of children with childhood AD compared with healthy children (Non Patent Literatures 5 and 8). In this example, the expression of IL-18 protein and SerpinB12 protein was analyzed in the child SSL collected in Example D-1.

Figure 9 shows a plot of the quantitative value (Log₂ (Abundance + 1)) of IL-18 protein in SSL collected from the back (non-eruption sites for the AD group) of each test subject of the healthy group and the AD group. No significant difference in the expression level of IL-18 protein was observed between the healthy group and the AD group. IL-18 protein was not identified in the face (eruption sites for the AD group).

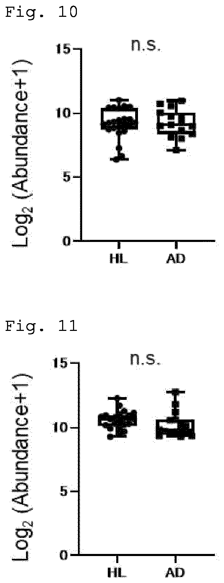

Figures 10 or 11 each show a plot of the quantitative value (Log₂ (Abundance + 1)) of SerpinB12 protein in SSL collected from the face (eruption sites for the AD group) or the back (non-eruption sites for the AD group) of each test subject of the healthy group and the AD group. No significant difference at any of the sites was observed between the healthy group and the AD group.

Much still remains to be elucidated about the presence or absence and behavior of the expression of various proteins in SSL. For example, as shown in Comparative Example D-1, the expression level of a protein contained in SSL is not necessarily consistent with the expression level of RNA encoding the protein. These facts mean that the expression behavior of various proteins in SSL is difficult to estimate. Furthermore, the results of these experiments demonstrated that the expression behavior of a protein in SSL is not necessarily consistent with that in blood or in the stratum corneum. As shown in Figures 9 to 11, IL-18 protein and SerpinB12 protein reportedly related to AD exhibit no relation to AD in SSL, unlike blood or the stratum corneum. The previous report has not clearly showed whether SerpinB4 protein in the stratum corneum of children is related to AD (Non Patent Literature 8). SerpinB4 protein in blood has heretofore been reported as a marker for pediatric and adult AD (Non Patent Literature 6). Nonetheless, as shown in Comparative Example D-2, SerpinB4 protein in SSL exhibits no relation to adult AD. The results of these experiments indicate that the expression of SerpinB4 protein in SSL or its relation to AD cannot be estimated.

These previous findings on proteins in SSL and the results of Example D-1 and Comparative Examples D-1 to D-3 indicate that the technique of using SerpinB4 protein in SSL as a childhood AD marker, provided by the present invention, is totally unexpected and is not readily findable.

## Claims

1. A method for detecting adult atopic dermatitis in an adult test subject, comprising a step of measuring an expression level of at least one gene selected from the group of 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 or an expression product thereof in a biological sample collected from the test subject.

2. The method according to claim 1, wherein the expression level of the gene or the expression product thereof is measured as an expression level of mRNA.

3. The method according to claim 1 or 2, wherein the gene or the expression product thereof is RNA contained in skin surface lipids of the test subject.

4. The method according to any one of claims 1 to 3, wherein the presence or absence of adult atopic dermatitis is evaluated by comparing the measurement value of the expression level with a reference value of the gene or the expression product thereof.

5. The method according to any one of claims 1 to 4, wherein the presence or absence of adult atopic dermatitis in the test subject is evaluated by the following steps: preparing a discriminant which discriminates between an adult atopic dermatitis patient and an adult healthy subject by using measurement values of an expression level of the gene or the expression product thereof derived from an adult atopic dermatitis patient and an expression level of the gene or the expression product thereof derived from an adult healthy subject as teacher samples; substituting the measurement value of the expression level of the gene or the expression product thereof obtained from the biological sample collected from the test subject into the discriminant; and comparing the obtained results with a reference value.

6. The method according to claim 5, wherein expression levels of all the genes of the group of 17 genes or expression products thereof are measured.

7. The method according to claim 5 or 6, wherein expression levels of the at least one gene selected from the group of 17 genes as well as at least one gene selected from the group of 245 genes shown in the following Table A-a except for the 17 genes, or expression products thereof are measured.
**[Table A-a]**
| | | | | | | |
|---|---|---|---|---|---|---|
| ACAT1 | CDS1 | FABP7 | HMHA1 | MTSS1 | PSMA5 | SSH1 |
| ACO1 | CEP76 | FABP9 | IL17RA | MVP | PSMB4 | ST6GALNAC2 |
| ADAP2 | CETN2 | FAM108B1 | IL2RB | MYO6 | PTPN18 | TCHHL1 |
| AKAP17A | CHMP4C | FAM120A | ILF3 | NCOR2 | RAB11FIP5 | TEX2 |
| AKT1 | CISD1 | FAM190B | ISCA1 | NCS1 | RABL6 | TGFB1 |
| ANXA1 | COBLL1 | FAM26E | ITPRIPL2 | NDUFA4 | RAC1 | THBD |
| APOBR | COPS2 | FBXL17 | KIAA0146 | NIPSNAP3A | RAI14 | TM7SF2 |
| ARHGAP23 | COX6A1 | FBXL18 | KIAA0513 | NMRK1 | RASA4CP | TMC5 |
| ARHGAP24 | COX7B | FBXL6 | KLK5 | NPEPL1 | RB1CC1 | TMEM165 |
| ARHGAP29 | CREG1 | FBXO32 | KRT23 | NPR1 | RGS19 | TMEM222 |
| ARHGAP4 | CRISPLD2 | FDFT1 | KRT25 | NPR2 | RHOC | TMPRSS11E |
| ARL8A | CRTC2 | FIS1 | KRT71 | NR1D1 | RNPEPL1 | TNRC18 |
| ARRDC4 | CRY2 | FMN1 | LCE1D | NUDT16 | RORC | TPGS2 |
| ATOX1 | CSNK1G2 | FOSB | LCE2C | OAT | RPS6KB2 | TSTD1 |
| ATP12A | CSTB | FOXQ1 | LENG9 | OGFR | RRM1 | TTC39B |
| ATP5A1 | CTBP1 | FURIN | LEPREL1 | PADI1 | SAP30BP | TWSG1 |
| ATPIF1 | CTDSP1 | GABARAPL2 | LMNA | PALD1 | SCARB2 | TYK2 |
| ATXN7L3B | CTSB | GDE1 | LOC146880 | PARP4 | SFN | U2AF2 |
| BAX | CTSL2 | GIGYF1 | LOC152217 | PCDH1 | SH3BGRL2 | UNC13D |
| BCKDHB | CXCL16 | GLRX | LRP8 | PCSK7 | SHC1 | UQCRQ |
| BCRP3 | CYTH2 | GNA15 | LY6D | PCTP | SIRT6 | USP38 |
| BSG | DBNDD2 | GNB2 | LYNX1 | PDZK1 | SKP1 | VHL |
| C15orf23 | DBT | GPD1 | MAN2A2 | PHB | SLC12A9 | VOPP1 |
| C16orf70 | DGKA | GPNMB | MAPK3 | PINK1 | SLC25A16 | VPS4B |
| C17orf107 | DHX32 | GRASP | MAPKBP1 | PLAA | SLC25A33 | WBSCR16 |
| C19orf71 | DNASE1L1 | GRN | MARK2 | PLEKHG2 | SLC2A4RG | WDR26 |
| C1QB | DOPEY2 | GSDMA | MAZ | PLP2 | SLC31A1 | XKRX |
| C2CD2 | DPYSL3 | GSE1 | MECR | PMVK | SMAP2 | XPO5 |
| C4orf52 | DSTN | GTF2H2 | MEMO1 | PNPLA1 | SMARCD1 | ZC3H15 |
| CAMP | DUSP16 | HADHA | MINK1 | POLD4 | SNORA71C | ZC3H18 |
| CAPN1 | DYNLL1 | HBP1 | MIR548I1 | PPA1 | SNORA8 | ZFP36L2 |
| CARD18 | EFHD2 | HINT3 | MKNK2 | PPBP | SNORD17 | ZMIZ1 |
| CCDC88B | EHBP1L1 | HLA-B | MLL2 | PPP1R12C | SPDYE7P | ZNF335 |
| CCND3 | EIF1AD | HMGCL | MLL4 | PPP1R9B | SPINK5 | ZNF664 |
| CDK9 | EMP3 | HMGCS1 | MLLT11 | PRSS8 | SRF | ZNF706 |

8. The method according to claim 5 or 6, wherein expression levels of the at least one gene selected from the group of 17 genes as well as at least one gene selected from the group of 123 genes shown in the following Tables A-1-1 to A-1-3, 150 genes shown in the following Tables A-3-1 to A-3-4 or 45 genes shown in the following Table A-4 except for the 17 genes, or expression products thereof are measured.

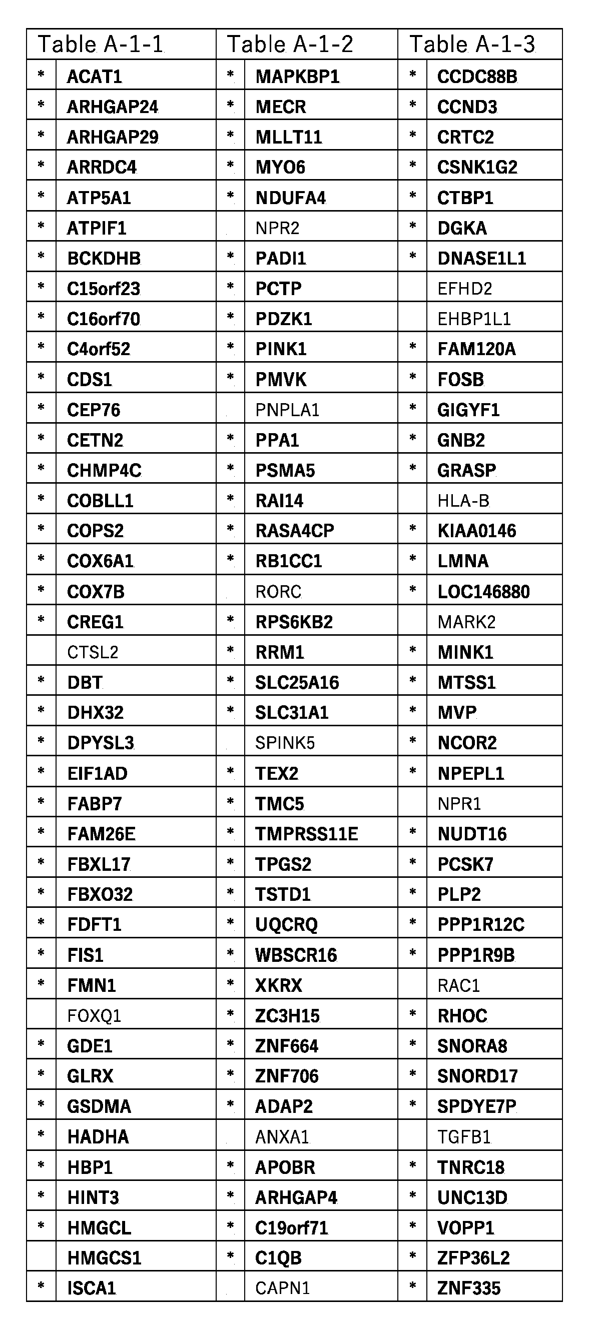

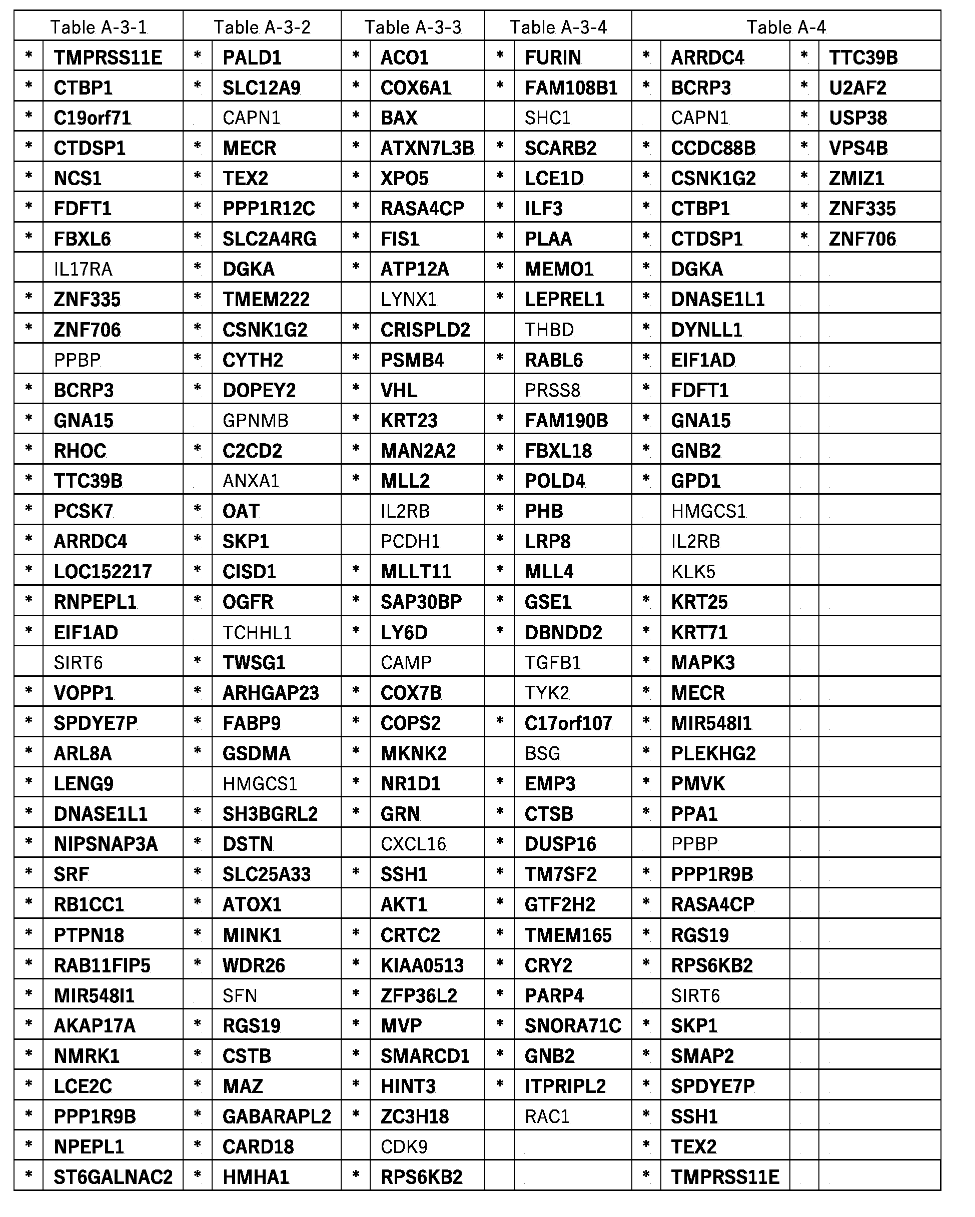

9. The method according to claim 7 or 8, wherein expression levels of the at least one gene selected from the group of 17 genes as well as at least one gene selected from the groups of 107, 127 and 39 genes shown in the following tables except for the 17 genes, or expression products thereof are measured.
<107 genes (indicated by boldface with * added in Tables A -1-1 to A-1-3)>
| | | | | | | |
|---|---|---|---|---|---|---|
| ACAT1 | COX6A1 | GSDMA | PPA1 | XKRX | FAM120A | PPP1R12C |
| ARHGAP24 | COX7B | HADHA | PSMA5 | ZC3H15 | FOSB | PPP1R9B |
| ARHGAP29 | CREG1 | HBP1 | RAI14 | ZNF664 | GIGYF1 | RHOC |
| ARRDC4 | DBT | HINT3 | RASA4CP | ZNF706 | GNB2 | SNORA8 |
| ATP5A1 | DHX32 | HMGCL | RB1CC1 | ADAP2 | GRASP | SNORD17 |
| ATPIF1 | DPYSL3 | ISCA1 | RPS6KB2 | APOBR | KIAA0146 | SPDYE7P |
| BCKDHB | EIF1AD | MAPKBP1 | RRM1 | ARHGAP4 | LMNA | TNRC18 |
| C15orf23 | FABP7 | MECR | SLC25A16 | C19orf71 | LOC146880 | UNC13D |
| C16orf70 | FAM26E | MLLT11 | SLC31A1 | C1QB | MINK1 | VOPP1 |
| C4orf52 | FBXL17 | MYO6 | TEX2 | CCDC88B | MTSS1 | ZFP36L2 |
| CDS1 | FBXO32 | NDUFA4 | TMC5 | CCN D3 | MVP | ZNF335 |
| CEP76 | FDFT1 | PADI1 | TMPRSS11E | CRTC2 | NCOR2 | |
| CETN2 | FIS1 | PCTP | TPGS2 | CSNK1G2 | NPEPL1 | |
| CHMP4C | FMN1 | PDZK1 | TSTD1 | CTBP1 | NUDT16 | |
| COBLL1 | GDE1 | PINK1 | UQCRQ | DGKA | PCSK7 | |
| COPS2 | GLRX | PMVK | WBSCR16 | DNASE1L1 | PLP2 | |
<127 genes (indicated by boldface with * added in Tables A -3-1 to A-3-4)>
| | | | | | | |
|---|---|---|---|---|---|---|
| TMPRSS11E | SPDYE7P | TEX2 | SLC25A33 | PSMB4 | HINT3 | DBNDD2 |
| CTBP1 | ARL8A | PPP1R12C | ATOX1 | VHL | ZC3H18 | C17orf107 |
| C19orf71 | LENG9 | SLC2A4RG | MINK1 | KRT23 | RPS6KB2 | EMP3 |
| CTDSP1 | DNASE1L1 | DGKA | WDR26 | MAN2A2 | FURIN | CTSB |
| NCS1 | NIPSNAP3A | TMEM222 | RGS19 | MLL2 | FAM108B1 | DUSP16 |
| FDFT1 | SRF | CSNK1G2 | CSTB | MLLT11 | SCARB2 | TM7SF2 |
| FBXL6 | RB1CC1 | CYTH2 | MAZ | SAP30BP | LCE1D | GTF2H2 |
| ZNF335 | PTPN18 | DOPEY2 | GABARAPL2 | LY6D | ILF3 | TMEM165 |
| ZNF706 | RAB11FIP5 | C2CD2 | CARD18 | COX7B | PLAA | CRY2 |
| BCRP3 | MIR548I1 | OAT | HMHA1 | COPS2 | MEMO1 | PARP4 |
| GNA15 | AKAP17A | SKP1 | ACO1 | MKNK2 | LEPREL1 | SNORA71C |
| RHOC | NMRK1 | CISD1 | COX6A1 | NR1D1 | RABL6 | GNB2 |
| TTC39B | LCE2C | OGFR | BAX | GRN | FAM190B | ITPRIPL2 |
| PCSK7 | PPP1R9B | TWSG1 | ATXN7L3B | SSH1 | FBXL18 | |
| ARRDC4 | NPEPL1 | ARHGAP23 | XPO5 | CRTC2 | POLD4 | |
| LOC152217 | ST6GALNAC2 | FABP9 | RASA4CP | KIAA0513 | PHB | |
| RNPEPL1 | PALD1 | GSDMA | FIS1 | ZFP36L2 | LRP8 | |
| EIF1AD | SLC12A9 | SH3BGRL2 | ATP12A | MVP | MLL4 | |
| VOPP1 | MECR | DSTN | CRISPLD2 | SMARCD1 | GSE1 | |
<39 genes (indicated by boldface with * added in Table A-4 ) >
| | | | | | | |
|---|---|---|---|---|---|---|
| ARRDC4 | DGKA | GNB2 | MIR548I1 | RGS19 | TEX2 | ZMIZ1 |
| BCRP3 | DNASE1L1 | GPD1 | PLEKHG2 | RPS6KB2 | TMPRSS11E | ZNF335 |
| CCDC88B | DYNLL1 | KRT25 | PMVK | SKP1 | TTC39B | ZNF706 |
| CSNK1G2 | EIF1AD | KRT71 | PPA1 | SMAP2 | U2AF2 | |
| CTBP1 | FDFT1 | MAPK3 | PPP1R9B | SPDYE7P | USP38 | |
| CTDSP1 | GNA15 | MECR | RASA4CP | SSH1 | VPS4B | |

10. A test kit for detecting adult atopic dermatitis, the kit being used in the method according to any one of claims 1 to 9, and comprising an oligonucleotide which specifically hybridizes to the gene or a nucleic acid derived therefrom, or an antibody which recognizes an expression product of the gene.

11. A marker for detecting adult atopic dermatitis comprising at least one gene selected from the group of 210 genes shown in the following Table A-b or an expression product thereof.
**[Table A-b]**
| | | | | | | |
|---|---|---|---|---|---|---|
| ACAT1 | CEP76 | FABP7 | HMGCL | MLLT11 | PSMA5 | ST6GALNAC2 |
| ACO1 | CETN2 | FABP9 | HMHA1 | MTSS1 | PSMB4 | TEX2 |
| ADAP2 | CHMP4C | FAM108B1 | ILF3 | MVP | PTPN18 | TM7SF2 |
| AKAP17A | CISD1 | FAM120A | ISCA1 | MYO6 | RAB11FIP5 | TMC5 |
| APOBR | COBLL1 | FAM190B | ITPRIPL2 | NCOR2 | RABL6 | TMEM165 |
| ARHGAP23 | COPS2 | FAM26E | KIAA0146 | NCS1 | RAI14 | TMEM222 |
| ARHGAP24 | COX6A1 | FBXL17 | KIAA0513 | NDUFA4 | RASA4CP | TMPRSS11E |
| ARHGAP29 | COX7B | FBXL18 | KRT23 | NIPSNAP3A | RB1CC1 | TNRC18 |
| ARHGAP4 | CREG1 | FBXL6 | KRT25 | NMRK1 | RGS19 | TPGS2 |
| ARL8A | CRISPLD2 | FBXO32 | KRT71 | NPEPL1 | RHOC | TSTD1 |
| ARRDC4 | CRTC2 | FDFT1 | LCE1D | NR1D1 | RNPEPL1 | TTC39B |
| ATOX1 | CRY2 | FIS1 | LCE2C | NUDT16 | RPS6KB2 | TWSG1 |
| ATP12A | CSNK1G2 | FMN1 | LENG9 | OAT | RRM1 | U2AF2 |
| ATP5A1 | CSTB | FOSB | LEPREL1 | OGFR | SAP30BP | UNC13D |
| ATPIF1 | CTBP1 | FURIN | LMNA | PADI1 | SCARB2 | UQCRQ |
| ATXN7L3B | CTDSP1 | GABARAPL2 | LOC146880 | PALD1 | SH3BGRL2 | USP38 |
| BAX | CTSB | GDE1 | LOC152217 | PARP4 | SKP1 | VHL |
| BCKDHB | CYTH2 | GIGYF1 | LRP8 | PCSK7 | SLC12A9 | VOPP1 |
| BCRP3 | DBNDD2 | GLRX | LY6D | PCTP | SLC25A16 | VPS4B |
| C15orf23 | DBT | GNA15 | MAN2A2 | PDZK1 | SLC25A33 | WBSCR16 |
| C16orf70 | DGKA | GNB2 | MAPK3 | PHB | SLC2A4RG | WDR26 |
| C17orf107 | DHX32 | GPD1 | MAPKBP1 | PINK1 | SLC31A1 | XKRX |
| C19orf71 | DNASE1L1 | GRASP | MAZ | PLAA | SMAP2 | XPO5 |
| C1QB | DOPEY2 | GRN | MECR | PLEKHG2 | SMARCD1 | ZC3H15 |
| C2CD2 | DPYSL3 | GSDMA | MEMO1 | PLP2 | SNORA71C | ZC3H18 |
| C4orf52 | DSTN | GSE1 | MINK1 | PMVK | SNORA8 | ZFP36L2 |
| CARD18 | DUSP16 | GTF2H2 | MIR548I1 | POLD4 | SNORD17 | ZMIZ1 |
| CCDC88B | DYNLL1 | HADHA | MKNK2 | PPA1 | SPDYE7P | ZNF335 |
| CCND3 | EIF1AD | HBP1 | MLL2 | PPP1R12C | SRF | ZNF664 |
| CDS1 | EMP3 | HINT3 | MLL4 | PPP1R9B | SSH1 | ZNF706 |

12. The marker for detecting adult atopic dermatitis according to claim 11, wherein the marker is at least one gene selected from the group of 17 genes consisting of MECR, RASA4CP, ARRDC4, EIF1AD, FDFT1, ZNF706, TEX2, TMPRSS11E, RPS6KB2, CTBP1, ZNF335, DGKA, PPP1R9B, SPDYE7P, DNASE1L1, GNB2 and CSNK1G2 or an expression product thereof.

13. A method for detecting childhood atopic dermatitis in a child test subject, comprising a step of measuring an expression level of at least one gene selected from the group of 7 genes consisting of IMPDH2, ERI1, FBXW2, STK17B, TAGLN2, AMICA1 and HNRNPA1 or an expression product thereof in a biological sample collected from the test subject.

14. The method for detecting childhood atopic dermatitis according to claim 13, wherein the method comprises at least measuring an expression level of a gene selected from the group of 3 genes consisting of IMPDH2, ERI1 and FBXW2 or an expression product thereof.

15. The method according to claim 13 or 14, wherein the expression level of the gene or the expression product thereof is measured as an expression level of mRNA.

16. The method according to any one of claims 13 to 15, wherein the gene or the expression product thereof is RNA contained in skin surface lipids of the test subject.

17. The method according to any one of claims 13 to 16, wherein the presence or absence of childhood atopic dermatitis is evaluated by comparing the measurement value of the expression level with a reference value of the gene or the expression product thereof.

18. The method according to any one of claims 13 to 16, wherein the presence or absence of childhood atopic dermatitis in the test subject is evaluated by the following steps: preparing a discriminant which discriminates between a child with atopic dermatitis and a healthy child by using measurement values of an expression level of the gene or the expression product thereof derived from a child with atopic dermatitis and an expression level of the gene or the expression product thereof derived from a healthy child as teacher samples; substituting the measurement value of the expression level of the gene or the expression product thereof obtained from the biological sample collected from the test subject into the discriminant; and comparing the obtained results with a reference value.

19. The method according to claim 18, wherein expression levels of all the genes of the group of 7 genes or expression products thereof are measured.

20. The method according to claim 18 or 19, wherein expression levels of the at least one gene selected from the group of 7 genes as well as at least one gene selected from the group of 100 genes shown in the following Tables B-3-1 to B-3-3 or 9 genes shown in the following Table B-4 except for the 7 genes, or expression products thereof are measured.
| **Table B-3-1** | | **Table 8-3-2** | | **Table B-3-3** | | **Table B-4** | |
|---|---|---|---|---|---|---|---|
| **^{∗}** | **AMICAL** | **^{∗}** | **U2AF1** | **^{∗}** | **PLIN2** | | CCL17 |
| **^{∗}** | **FBXW2** | **^{∗}** | **VPS13C** | **^{∗}** | **CDC42EP1** | | PYCARD |
| | PYCARD | **^{∗}** | **SNX8** | **^{∗}** | **CCM2** | **^{∗}** | **IMPDH2** |
| **^{∗}** | **STK17B** | **^{∗}** | **NBPF10** | **^{∗}** | **RN F24** | **^{∗}** | **ERI1** |
| | DNAJB11 | **^{∗}** | **ZNF430** | **^{∗}** | **SRPK2** | **^{∗}** | **FBXW2** |
| **^{∗}** | **ERI1** | **^{∗}** | **SPEN** | **^{∗}** | **LST1** | **^{∗}** | **STK17B** |
| ^{∗} | ECH1 | ^{∗} | **CIB1** | ^{∗} | YPEL2 | ^{∗} | TAGLN2 |
| ^{∗} | MED14 | ^{∗} | TMEM33 | ^{∗} | **INF2** | ^{∗} | AMICA1 |
| ^{∗} | HYOU1 | ^{∗} | NPEPPS | ^{∗} | AMD1 | ^{∗} | HNRNPA1 |
| | MAP1LC3B2 | ^{∗} | SEC24D | | ITGAM | | |
| | IL7R | ^{∗} | SLC7A11 | ^{∗} | **IMPDH2** | | |
| ^{∗} | CTDSP1 | ^{∗} | **ARHGDIB** | ^{∗} | CAPG | | |
| ^{∗} | USP16 | ^{∗} | C10orf128 | ^{∗} | VKORC1 | | |
| ^{∗} | HNRNPA1 | ^{∗} | HNRNPUL1 | ^{∗} | ACSL4 | | |
| | CCL17 | ^{∗} | TXN2 | ^{∗} | CDC123 | | |
| ^{∗} | UBE2R2 | | CISH | ^{∗} | SCARNA7 | | |
| ^{∗} | SDHD | ^{∗} | YWHAG | ^{∗} | RNASET2 | | |
| | AREG | ^{∗} | GPT2 | ^{∗} | RLF | | |
| ^{∗} | **TXN DC17** | ^{∗} | KIAA0930 | ^{∗} | C6orf62 | | |
| ^{∗} | FBXW4 | ^{∗} | LAMTOR4 | ^{∗} | SLC39A8 | | |
| ^{∗} | FBP1 | ^{∗} | CRCP | ^{∗} | ARHGAP9 | | |
| ^{∗} | FAM100B | ^{∗} | CLEC4A | ^{∗} | NDUFS7 | | |
| ^{∗} | PDIA3P | ^{∗} | STT3A | ^{∗} | SEC61G | | |
| ^{∗} | ZNF91 | ^{∗} | CRISPLD2 | | SCAP | | |
| ^{∗} | RBM17 | ^{∗} | DEFB4B | ^{∗} | TMEM214 | | |
| ^{∗} | PRPF38B | ^{∗} | CD93 | ^{∗} | USF2 | | |
| ^{∗} | ATP5H | ^{∗} | PLIN3 | | | | |
| ^{∗} | BAX | ^{∗} | USMG5 | | | | |
| ^{∗} | ALYREF | ^{∗} | LOC285359 | | | | |
| ^{∗} | HK2 | ^{∗} | SLC20A1 | | | | |
| ^{∗} | PRMT1 | ^{∗} | MSL1 | | | | |
| ^{∗} | CTSC | ^{∗} | SLC11A2 | | | | |
| ^{∗} | SNRPD1 | ^{∗} | KHDRBS1 | | | | |
| ^{∗} | TAGLN2 | ^{∗} | ABHD8 | | | | |
| ^{∗} | CYTIP | ^{∗} | CORO1B | | | | |
| ^{∗} | CASS4 | ^{∗} | ZFAND2A | | | | |
| ^{∗} | SNORA6 | | DOK2 | | | | |

21. The method according to claim 18 or 19, wherein expression levels of the at least one gene selected from the group of 7 genes as well as at least one gene selected from the group of 371 genes shown in the following Tables B-1-1 to B-1-9 except for the 7 genes, or expression products thereof are measured.

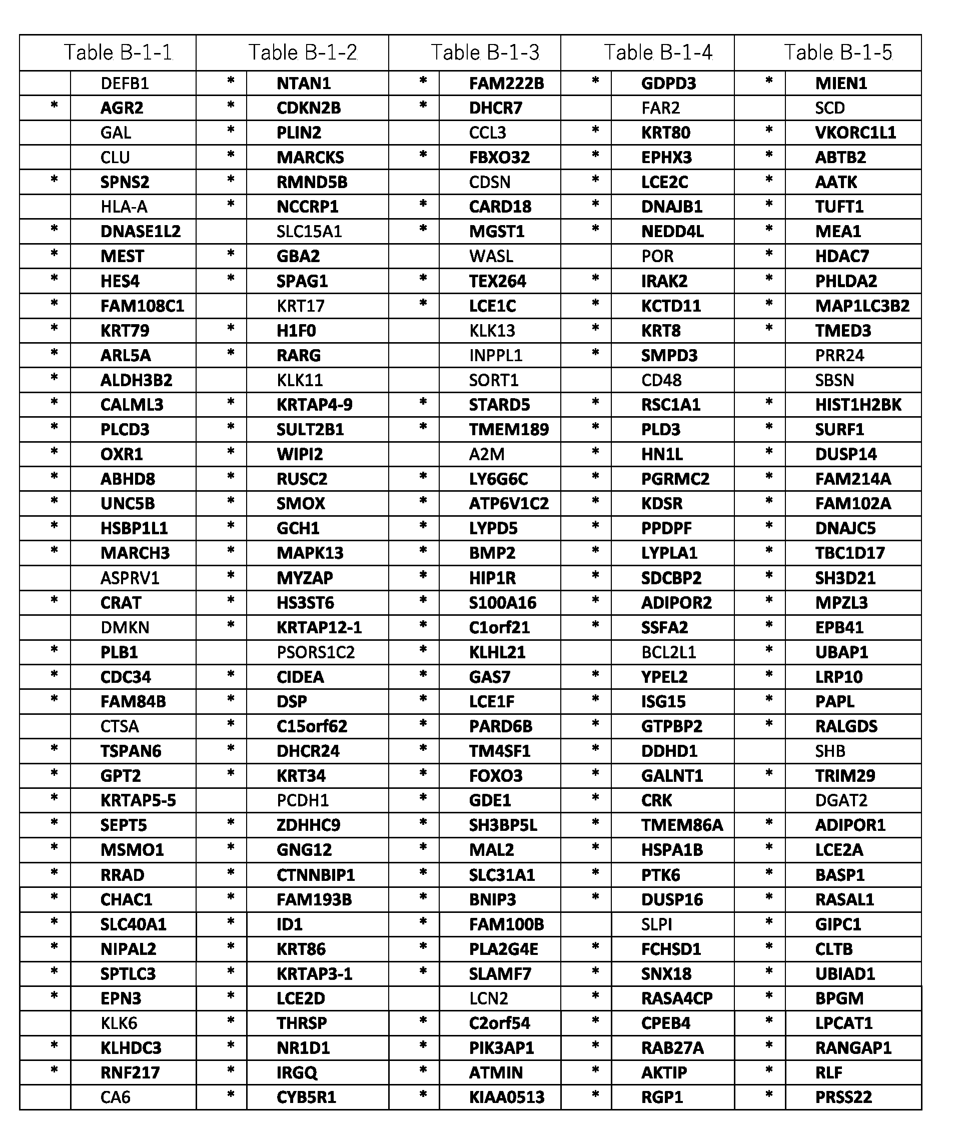

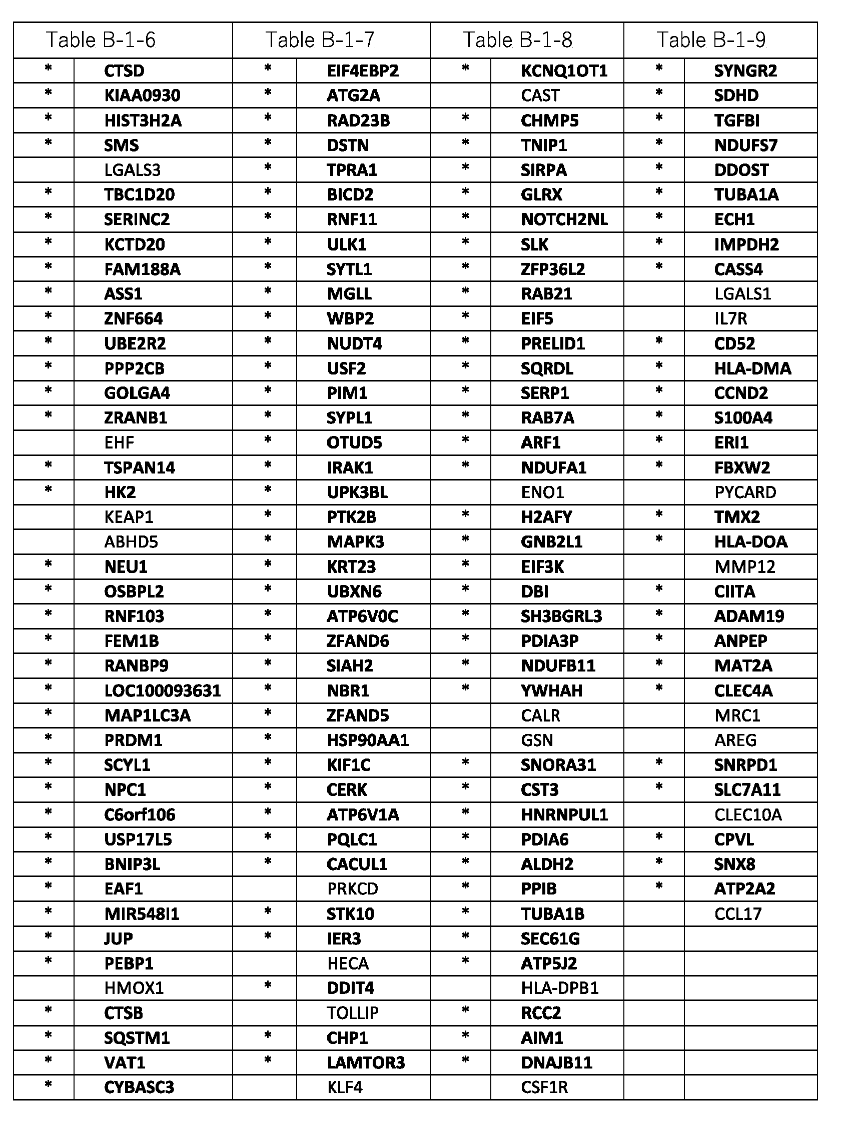

22. The method according to claim 21, wherein expression levels of the at least one gene selected from the group of 7 genes as well as at least one gene selected from the following group of 25 genes or expression products thereof are measured:
ABHD8, GPT2, PLIN2, FAM100B, YPEL2, MAP1LC3B2, RLF, KIAA0930, UBE2R2, HK2, USF2, PDIA3P, HNRNPUL1, SEC61G, DNAJB11, SDHD, NDUFS7, ECH1, CASS4, IL7R, CLEC4A, AREG, SNRPD1, SLC7A11 and SNX8.

23. A test kit for detecting childhood atopic dermatitis, the kit being used in the method according to any one of claims 13 to 22, and comprising an oligonucleotide which specifically hybridizes to the gene or a nucleic acid derived therefrom, or an antibody which recognizes an expression product of the gene.

24. A marker for detecting childhood atopic dermatitis comprising at least one gene selected from the group of 383 genes shown in the following Tables B-b-1 and B-b-2 or an expression product thereof.
**[Table B-b-1]**
| | | | | | |
|---|---|---|---|---|---|
| AGR2 | H1F0 | LY6G6C | KDSR | TBC1D17 | LOC100093631 |
| SPNS2 | RARG | ATP6V1C2 | PPDPF | SH3D21 | MAP1LC3A |
| DNASE1L2 | KRTAP4-9 | LYPDS | LYPLA1 | MPZL3 | PRDM1 |
| MEST | SULT2B1 | BMP2 | SDCBP2 | EPB41 | SCYL1 |
| HES4 | WIPI2 | HIP1R | ADIPOR2 | UBAP1 | NPC1 |
| FAM108C1 | RUSC2 | S100A16 | SSFA2 | LRP10 | C6orf106 |
| KRT79 | SMOX | Clorf21 | ISG15 | PAPL | USP17L5 |
| ARLSA | GCH1 | KLHL21 | GTPBP2 | RALGDS | BNIP3L |
| ALDH3B2 | MAPK13 | GAS7 | DDHD1 | TRIM29 | EAF1 |
| CALML3 | MYZAP | LCE1F | GALNT1 | ADIPOR1 | MIR548I1 |
| PLCD3 | HS3ST6 | PARD6B | CRK | LCE2A | JUP |
| OXR1 | KRTAP12-1 | TM4SF1 | TMEM86A | BASP1 | PEBP1 |
| UNCSB | CIDEA | FOXO3 | HSPA1B | RASAL1 | CTSB |
| HSBP1L1 | DSP | GDE1 | PTK6 | GIPC1 | SQSTM1 |
| MARCH3 | C15orf62 | SH3BP5L | DUSP16 | CLTB | VAT1 |
| CRAT | DHCR24 | MAL2 | FCHSD1 | UBIAD1 | CYBASC3 |
| PLB1 | KRT34 | SLC31A1 | SNX18 | BPGM | EIF4EBP2 |
| CDC34 | ZDHHC9 | BNIP3 | RASA4CP | LPCAT1 | ATG2A |
| FAM84B | GNG12 | PLA2G4E | CPEB4 | RANGAP1 | RAD23B |
| TSPAN6 | CTNNBIP1 | SLAM F7 | RAB27A | PRSS22 | DSTN |
| KRTAP5-5 | FAM 193 B | C2orf54 | AKTIP | CTSD | TPRA1 |
| SEPTS | ID1 | PIK3AP1 | RGP1 | HIST3H2A | BICD2 |
| MSMO1 | KRT86 | ATMIN | MIEN1 | SMS | RNF11 |
| RRAD | KRTAP3-1 | KIAA0513 | VKORC1L1 | TBC1D20 | ULK1 |
| CHAC1 | LCE2D | GDPD3 | ABTB2 | SERINC2 | SYTL1 |
| SLC40A1 | THRSP | KRT80 | AATK | KCTD20 | MGLL |
| NIPAL2 | NR1D1 | EPHX3 | TUFT1 | FAM188A | WBP2 |
| SPTLC3 | IRGQ | LCE2C | MEA | ASS1 | NUDT4 |
| EPN3 | CYB5R1 | DNAJB1 | HDAC7 | ZNF664 | PIM1 |
| KLH DC3 | FAM222B | NEDD4L | PHLDA2 | PPP2CB | SYPL1 |
| RNF217 | DHCR7 | IRAK2 | TMED3 | GOLGA4 | OTUDS |
| NTAN1 | FBXO32 | KCTD11 | PRR24 | ZRANB1 | IRAK1 |
| CDKN2B | CARD18 | KRT8 | HIST1H2BK | TSPAN14 | UPK3BL |
| MARCKS | MGST1 | SMPD3 | SURF1 | NEU1 | PTK2B |
| RMND5B | TEX264 | RSC1A1 | DUSP14 | OSBPL2 | MAPK3 |
| NCCRP1 | LCE1C | PLD3 | FAM214A | RNF103 | KRT23 |
| GBA2 | STARDS | HN1L | FAM102A | FEM1B | UBXN6 |
| SPAG1 | TMEM189 | PGRMC2 | DNAJCS | RANBP9 | ATP6V0C |
| ZFAND6 | SNORA31 | SEC61G | SEC24D | STK17B | H2AFY |
| SIAH2 | CST3 | DNAJB11 | ARHGDIB | HNRNPA1 | GNB2L1 |
| NBR1 | PDIA6 | SDHD | C10orf128 | TAGLN2 | EIF3K |
| ZFANDS | ALDH2 | NDUFS7 | TXN2 | TNIP1 | DBI |
| HSP90AA1 | PPIB | ECH1 | YWHAG | SIRPA | SH3BGRL3 |
| KIF1C | TUBA1B | CASS4 | LAMTOR4 | GLRX | NDUFB11 |
| CERK | ATP5J2 | CLEC4A | CRCP | NOTCH2NL | YWHAH |
| ATP6V1A | RCC2 | SNRPD1 | STT3A | SLK | TMX2 |
| PQLC1 | AIM1 | SLC7A11 | CRISPLD2 | ZFP36L2 | HLA-DOA |
| CACUL1 | SYNGR2 | SNX8 | DEFB4B | RAB21 | CIITA |
| STK10 | TGFBI | IMPDH2 | CD93 | EIF5 | ADAM 19 |
| IER3 | DDOST | ERI1 | PLIN3 | PRELID1 | ANPEP |
| DDIT4 | TUBA1A | FBXW2 | USMGS | SQRDL | MAT2A |
| CHP1 | CD52 | MED14 | LOC285359 | SERP1 | CPVL |
| LAMTOR3 | HLA-DMA | HYOU1 | SLC20A1 | RAB7A | ATP2A2 |
| KCNQ1OT1 | CCND2 | CTDSP1 | MSL1 | ARF1 | ABHD8 |
| CHMP5 | S100A4 | USP16 | SLC11A2 | NDUFA1 | GPT2 |
**[Table B-b-2]**
| | | |
|---|---|---|
| PLIN2 | TXNDC17 | CAPG |
| FAM100B | FBXW4 | VKORC1 |
| YPEL2 | FBP1 | ACSL4 |
| MAP1LC3B2 | ZNF91 | CDC123 |
| RLF | RBM17 | SCARNA7 |
| KIAA0930 | PRPF38B | RNASET2 |
| UBE2R2 | ATPSH | C6orf62 |
| HK2 | BAX | SLC39A8 |
| USF2 | ALYREF | ARHGAP9 |
| PDIA3P | PRMT1 | TMEM214 |
| HNRNPUL1 | CTSC | AMICA1 |
| KHDRBS1 | CYTIP | |
| CORO1B | SNORA6 | |
| ZFAND2A | U2AF1 | |
| CDC42EP1 | VPS13C | |
| CCM2 | NBPF10 | |
| RNF24 | ZNF430 | |
| SRPK2 | SPEN | |
| LST1 | CIB1 | |
| INF2 | TMEM33 | |
| AMD1 | NPEPPS | |

25. The marker according to claim 24, wherein the marker is at least one gene selected from the group of 7 genes consisting of IMPDH2, ERI1, FBXW2, STK17B, TAGLN2, AMICA1 and HNRNPA1 or an expression product thereof.

26. The marker according to claim 24, wherein the marker is at least one gene selected from the group of 23 genes consisting of ABHD8, GPT2, PLIN2, FAM100B, YPEL2, MAP1LC3B2, RLF, KIAA0930, UBE2R2, HK2, USF2, PDIA3P, HNRNPUL1, SEC61G, DNAJB11, SDHD, NDUFS7, ECH1, CASS4, CLEC4A, SNRPD1, SLC7A11 and SNX8 or an expression product thereof.

27. A method for preparing a protein marker for detecting atopic dermatitis, comprising collecting at least one protein selected from the group consisting of proteins shown in the following Table C-1 from skin surface lipids collected from a test subject.
**[Table C-1]**
| | | | | | | | | | | | | |
|---|---|---|---|---|---|---|---|---|---|---|---|---|
| A1BG | AZU1 | CLIC1 | EF4A2 | GPLD1 | HSPB1 | KRT13 | MPO | PLTP | RECQL | S100A10 | SPRR2F | VTN |
| A2M | B2M | CORO1A | EIF5A | GPT | HSFE1 | KRT15 | MSLN | PNP | REEP5 | S100A11 | SRSF2 | WDR1 |
| ACP5 | BPI | OOTL1 | EF6 | GSDMA | IDH2 | KRT23 | MSN | POF1B | RETN | S100A14 | SRSF3 | WFDC12 |
| ACTB | BST1 | CP | ELANE | GSN | IGHG1 | KRT25 | MTAP | POLR3A | RNASE3 | S100A6 | STS | WFDC5 |
| ACTR2 | BTF3 | CPNE3 | ENO1 | GSTP1 | IGHG2 | KRT77 | MUC5AC | POM121 | RP1BL | S100A7 | SUB1 | YWHAE |
| AFM | C1QA | CPQ | EPPK1 | H1-0 | IGHG3 | KRT79 | MUCL1 | PON1 | RPL10A | S100A8 | SUMO3 | YWHAG |
| AGRN | CLQC | CRISP3 | EPS8L1 | HI-3 | IGHG4 | KRTAP2-3 | MYH1 | PPI4 | FPL12 | SAMD4A | SYNGR2 | YWHAH |
| AGT | C1S | CRISPLD2 | EPX | HI-5 | IGHM | KV310 | MYH14 | PPIB | RPL13 | SBSN | TACSTD2 | YWHAZ |
| AHNAK | C3 | CRNN | ERP29 | H2AC11 | IGHV1-46 | LACRT | MYH9 | PPL | RPL14 | SCEL | TAGLN2 | ZNF236 |
| AHSG | C4A | CTSA | EVPL | H2AC4 | IGHV3-30 | LAMP2 | MYL12B | PRDX2 | RPL15 | SCGB1D2 | TALD01 | ZNF292 |
| AKR1A1 | C4BPA | CTSG | EZR | H2AZ1 | IGHV3-33 | LCN1 | MYL6 | PRDX6 | RPL18A | SCGB2A1 | TASOR2 | |
| ALB | C7 | DAG1 | F2 | H2BC12 | IGHV3-7 | LCN15 | NAMPT | PRR4 | RPL22 | SCGB2A2 | TF | |
| ALDH3A1 | CA2 | DEI | F5 | H3C1 | IGKC | LCN2 | NAPA | PRSS27 | RPL26 | SEPN8 | TGM1 | |
| ALDOA | CALR | DCD | FABP5 | H4C1 | IGKV1-5 | LCP1 | NCCRP1 | PSMA1 | RPL29 | SEPTIN9 | THBS1 | |
| AMBP | CAMP | DDB1 | FAU | HBA1 | IGKV3-11 | LDHA | NDUFB6 | PSMB1 | RPL30 | SERBP1 | TIMP1 | |
| ANXA1 | GANX | DDX10 | FBXO6 | HBB | IGKV3-20 | LGALS1 | NME1 | PSMB2 | IR.31 | SERPNA1 | TIMP2 | |
| ANXA11 | CAP1 | DOX55 | FGA | FK3 | IGKV4-1 | LGALS3 | NME2 | PSMB3 | RPL4 | SEFPNA3 | TKT | |
| ANXA2 | CAPG | DEFA3 | FGB | HLA-DPB1 | IGLV1-51 | LG4LS7 | NPC2 | PSMB4 | RPL5 | SERPNA4 | TMED5 | |
| ANXA3 | GAPZA1 | DERA | FGG | HLA-DRB1 | L36G | LGALSL | OPRPN | PSMB5 | RPL6 | SERPNB1 | TMSL3 | |
| ANXA6 | CARD18 | DHRS11 | FLG2 | HM13 | MPA2 | LMNA | ORM1 | PSMD14 | RPL7 | SERPNB13 | TNN3K | |
| APCS | GASP14 | DHX36 | FLNB | FWGA1 | ITGAM | LPO | P4HB | PSME2 | RPL8 | SERPNB3 | TPD52L2 | |
| APOA1 | C8R1 | DLD | FN1 | FMGB1 | ITGB2 | LRG1 | PCBP1 | PYCARD | RPS11 | SERPNB4 | TPM3 | |
| APOA2 | CCAR2 | DNAAF1 | G6PD | HMGB2 | ITH1 | LTF | PDIA3 | PYGL | RPS13 | SEPNB5 | TPP1 | |
| APOB | CCT3 | DNAB1 | GARS1 | HNRNPA2B1 | ITH2 | LY6G6C | PDIA6 | RAB10 | RPS14 | SERPINC1 | TPT1 | |
| APOC1 | CCT6A | DSC1 | GART | HNRNPD | ITIH4 | LYZ | PFN1 | RAB1A | RPS16 | SERPIND1 | TRIM29 | |
| APOH | CDC42 | DSC3 | GBA | HNRNPK | JCHAIN | MACROH2A1 | PGAM1 | RAB1B | RPS17 | SERPINF1 | TTR | |
| ARF6 | CDH23 | DSP | GC | HNRNPR | JLP | MAST4 | PGK1 | RAB27A | RPS19 | SERPINF2 | TUBB | |
| ARHGDB | CEACAM5 | DYNLL1 | GCA | I-P | KLK10 | MDH2 | PHB2 | RAC2 | RPS23 | SERPING1 | TUBB2A | |
| ARPC2 | CFB | ECM1 | GDI2 | HPX | KLK13 | ME1 | PI3 | RAD9B | RPS25 | SFN | TUBB4B | |
| ARPC3 | CFH | EEF1A1 | GLRX | HRG | KLK6 | MGST2 | PKM | RALY | RPS27A | SFPQ | UBE2N | |
| ASPHV1 | CFI | EEF2 | GM2A | HSD17B4 | KLK7 | MIF | PLD3 | RAN | RPS6 | SLURP2 | UGP2 | |
| ATP1B1 | CFL1 | EFHD2 | GMPR2 | HSPA1A | KLK9 | MMGT1 | PLEC | RANBP1 | RPS9 | SNRPD3 | VDAC1 | |
| ATP5PO | CKMT1A | EFNA1 | GNAI2 | HSPA5 | KLKB1 | MMP9 | PLG | RARRES1 | RPSA | SPRR1B | VIM | |
| AZGP1 | CLEC3B | EF3 | GPI | HSPA9 | KNG1 | MNDA | PLS3 | RDH12 | RTCB | SPRR2D | VSIG10L | |

28. A method for detecting atopic dermatitis in a test subject, comprising detecting at least one protein selected from the group consisting of proteins shown in the following Table C-2 from skin surface lipids collected from the test subject.
**[Table C-2]**
| | | | | | | | | | | | | |
|---|---|---|---|---|---|---|---|---|---|---|---|---|
| A1BG | AZU1 | CLIC1 | EIF4A2 | GPLD1 | HSPB1 | KRT13 | MPO | PLTP | RECQL | S100A10 | SPRR2F | VTN |
| A2M | B2M | CORO1A | EIF5A | GPT | HSPE1 | KRT15 | MSLN | PNP | REEP5 | S100A11 | SRSF2 | WDR1 |
| ACP5 | BPI | COTL1 | EIF6 | GSDMA | IDH2 | KRT23 | MSN | POF1B | RETN | S100A14 | SRSF3 | WFDC12 |
| ACTB | BST1 | CP | ELANE | GSN | IGHG1 | KRT25 | MTAP | POLR3A | RNASE3 | S100A8 | STS | WFDC5 |
| ACTR2 | BTF3 | CPNE3 | ENO1 | GSTP1 | IGHG2 | KRT77 | MUC5AC | POM121 | RP1BL | S100A7 | SUB1 | YWHAE |
| AFM | C1QA | CPQ | EPPK1 | H1-0 | IGHG3 | KRT79 | MUCL1 | PON1 | RPL10A | S100A8 | SUMO3 | YWHAG |
| AGRN | C1QC | CRISP3 | EPS8L1 | H1-3 | IGHG4 | KRTAP2-3 | MYH1 | PPIA | RPL12 | SAMD4A | SYNGR2 | YWHAH |
| AGT | C1S | CRISPLD2 | EPX | H1-5 | IGHM | KV310 | MYH14 | PPIB | RPL13 | SBSN | TACSTD2 | YWHAZ |
| AHNAK | C3 | CRNN | ERP29 | H2AC11 | IGHV1-46 | LACRT | MYH9 | PPL | RPL14 | SCEL | TAGLN2 | ZNF236 |
| AHSG | C4A | CTSA | EVPL | H2AC4 | IGHV3-30 | LAMP2 | MYL12B | PRDX2 | RPL15 | SCGB1D2 | TALDO1 | ZNF292 |
| AKR1A1 | C4BPA | CTSG | EZR | H2AZ1 | IGHV3-33 | LCN1 | MYL6 | PRDX6 | RPL18A | SCGB2A1 | TASOR2 | |
| ALB | C7 | DAG1 | F2 | H2BC12 | IGHV3-7 | LCN15 | NAMPT | PRR4 | RPL22 | SCGB2A2 | TF | |
| ALDH3A1 | CA2 | DBI | F5 | H3C1 | IGKC | LCN2 | NAPA | PRSS27 | RPL26 | SEPTINB | TGM1 | |
| ALDOA | CALR | DCD | FABP5 | H4C1 | IGKV1-5 | LCP1 | NCCRP1 | PSMA1 | RPL29 | SEPTIN9 | THBS1 | |
| AMBP | CAMP | DDB1 | FAU | HBA1 | IGKV3-11 | LDHA | NDUFB6 | PSMB1 | RPL30 | SERBP1 | TIMP1 | |
| ANXA1 | CANX | DDX10 | FBXO6 | HBB | IGKV3-20 | LGALS1 | NME1 | PSMB2 | RPL31 | SERPINA1 | TIMP2 | |
| ANXA11 | CAP1 | DDX55 | FGA | HK3 | IGKV4-1 | LGALS3 | NME2 | PSMB3 | RPL4 | SERPINA3 | TKT | |
| ANXA2 | CAPG | DEFA3 | FGB | HLA-DPB1 | IGLV1-51 | LGALS7 | NPC2 | PSMB4 | RPL5 | SERPINA4 | TMED5 | |
| ANXA3 | CAPZA1 | DERA | FGG | HLA-DRB1 | IL36G | LGALSL | OPRPN | PSMB5 | RPL6 | SERPINB1 | TMSL3 | |
| ANXA6 | CARD18 | DHRS11 | FLG2 | HM13 | IMPA2 | LMNA | ORM1 | PSMD14 | RPL7 | SERPINB13 | TNNI3K | |
| APCS | CASP14 | DHX36 | FLNB | HMGA1 | ITGAM | LPO | P4HB | PSME2 | RPL8 | SERPINB3 | TPD52L2 | |
| APOA1 | CBR1 | DLD | FN1 | HMGB1 | ITGB2 | LRG1 | PCBP1 | PYCARD | RPS11 | SERPINB4 | TPM3 | |
| APOA2 | CCAR2 | DNAAF1 | G6PD | HMGB2 | ITIH1 | LTF | PDIA3 | PYGL | RPS13 | SERPINB5 | TPP1 | |
| APOB | CCT3 | DNAJB1 | GARS1 | HNRNPA2B1 | ITIH2 | LY6G6C | PDIA6 | RAB10 | RPS14 | SERPINC1 | TPT1 | |
| APOC1 | CCT6A | DSC1 | GART | HNRNPD | ITIH4 | LYZ | PFN1 | RAB1A | RPS16 | SERPIND1 | TRIM29 | |
| APOH | CDC42 | DSC3 | GBA | HNRNPK | JCHAIN | MACROH2A1 | PGAM1 | RAB1B | RPS17 | SERPINF1 | TTR | |
| ARF6 | CDH23 | DSP | GC | HNRNPR | JUP | MAST4 | PGK1 | RAB27A | RPS19 | SERPINF2 | TUBB | |
| ARHGDIB | CEACAM5 | DYNLL1 | GCA | HP | KLK10 | MDH2 | PHB2 | RAC2 | RPS23 | SERPING1 | TUBB2A | |
| ARPC2 | CFB | ECM1 | GDI2 | HPX | KLK13 | ME1 | PI3 | RAD9B | RPS25 | SFN | TUBB4B | |
| ARPC3 | CFH | EEF1A1 | GLRX | HRG | KLK6 | MGST2 | PKM | RALY | RPS27A | SFPQ | UBE2N | |
| ASPRV1 | CFI | EEF2 | GM2A | HSD17B4 | KLK7 | MIF | PLD3 | RAN | RPS6 | SLURP2 | UGP2 | |
| ATP1B1 | CFL1 | EFHD2 | GMPR2 | HSPA1A | KLK9 | MMGT1 | PLEC | RANBP1 | RPS9 | SNRPD3 | VDAC1 | |
| ATP5PO | CKMT1A | EFNA1 | GNAI2 | HSPA5 | KLKB1 | MMP9 | PLG | RARRES1 | RPSA | SPRR1B | VIM | |
| AZGP1 | CLEC3B | EIF3I | GPI | HSPA9 | KNG1 | MNDA | PLS3 | RDH12 | RTCB | SPRR2D | VSIG10L | |

29. The method according to claim 27 or 28, wherein the at least one protein is at least one protein selected from the group consisting of proteins shown in the following Table C-3.
**[Table C-3]**
| | | | | |
|---|---|---|---|---|
| CCAR2 | LGALSL | RPS25 | SRSF3 | DHRS11 |
| CKMT1A | KV310 | RPS27A | SUB1 | HNRNPA2B1 |
| DDX10 | ATP5PO | SAMD4A | TRIM29 | ITIH1 |
| DDX55 | DERA | SEPTIN8 | TUBB4B | LACRT |
| DYNLL1 | PRR4 | SEPTIN9 | CPQ | PRSS27 |
| EIF3I | AKR1A1 | SERBP1 | FLNB | PSMB2 |
| EIF5A | BTF3 | SFPQ | RPS9 | PSME2 |
| GMPR2 | CCT6A | SNRPD3 | RPLB | RPS16 |
| H1-0 | CPNE3 | TAGLN2 | A1BG | CAP1 |
| H2AC4 | DNAAF1 | TMSL3 | ARHGDIΘ | CTSA |
| HNRNPR | EIF4A2 | TNNI3K | CDH23 | DLD |
| 1GKV3-11 | EPSBL1 | ZNF292 | EIF6 | |
| IGLV1-51 | ERP29 | WDR1 | FBXO6 | |
| IMPA2 | GART | ARPC3 | HSD17B4 | |
| KRTAP2-3 | GDI2 | BST1 | IGHV3-30 | |
| MMGT1 | HM13 | CAPZA1 | IGHV3-33 | |
| MYH14 | IGHV1-46 | CCT3 | IGHV3-7 | |
| RAD9B | IGKV1-5 | COTL1 | ITIH2 | |
| REEP5 | IGKV4-1 | CRISPLD2 | LCN15 | |
| RP1BL | MAST4 | GPLD1 | LY6G6C | |
| RPL6 | MDH2 | IGKV3-20 | PLD3 | |
| RTCB | MYH1 | MACROH2A1 | POF1B | |
| SYNGR2 | NCCRP1 | MYL6 | PSMA1 | |
| TASOR2 | PCBP1 | NDUFB6 | RPL15 | |
| TMED5 | POM121 | PDIA6 | RPL30 | |
| TPD52L2 | PSMB3 | PGAM1 | RPL31 | |
| VSIG10L | RAB10 | POLR3A | RPS17 | |
| ZNF236 | RAB1B | PSMB1 | TUBB2A | |
| GARS1 | RECQL | PSMB5 | HK3 | |
| H3C1 | RPL10A | PSMD14 | MTAP | |
| H1-5 | RPL12 | RAB1A | RALY | |
| H2AZ1 | RPL29 | RANBP1 | RPL4 | |
| H2AC11 | RPS14 | RDH12 | RPL7 | |
| H2BC12 | RPS23 | RPL14 | TPP1 | |

30. The method according to claim 27 or 28, wherein the at least one protein is at least one protein selected from the group consisting of proteins shown in the following Table C-4.
**[Table C-4]**
| | |
|---|---|
| H1-5 | PSMA1 |
| MYL6 | ELANE |
| POF1B | IGHG3 |
| LCN2 | ALB |
| YWHAG | CTSG |
| PGAM1 | VIM |
| LDHA | APCS |
| ERP29 | KRT15 |
| CFB | A2M |
| AMBP | CALR |
| PFN1 | CASP14 |
| TF | HSPE1 |
| ACTB | RNASE3 |
| IGHG1 | CORO1A |
| ORM1 | TAGLN2 |
| GSN | F2 |
| FGA | P4HB |
| APOH | RAN |
| CP | GC |
| ASPRV1 | FGG |
| GPI | AHSG |
| APOA1 | DCD |
| KNG1 | PPIA |
| FGB | KLK10 |
| H4C1 | MIF |
| SBSN | MYH9 |
| VTN | CFL1 |
| APOA2 | H1-3 |
| CBR1 | ARHGDIB |
| MYL12B | SCGB2A2 |
| PDIA3 | CA2 |
| SERPINB5 | |
| PLG | |
| CAPG | |

31. The method according to claim 27 or 28, wherein the test subject is a child, and the at least one protein is at least one protein selected from the group consisting of proteins shown in the following Tables C-5-1 and C-5-2.
**[Table C-5-1]**
| | | | |
|---|---|---|---|
| LGALS7 | HSPB1 | CLIC1 | RPS13 |
| SERPINB4 | KLK13 | GDI2 | SERPINA3 |
| TAGLN2 | PLG | ARF6 | EPPK1 |
| IGHG3 | ECM1 | SNRPD3 | CP |
| RECQL | EIF5A | S100A11 | FLNB |
| RPL22 | PGAM1 | FABP5 | HSD17B4 |
| RPL26 | SBSN | H2AC4 | GM2A |
| EEF1A1 | MYH14 | RAN | RPL15 |
| SERPINB5 | WFDC5 | GC | MNDA |
| APOH | ASPRV1 | CDH23 | RPL31 |
| LMNA | CA2 | LGALSL | CFL1 |
| HSPA5 | IGHG4 | LDHA | GBA |
| CLEC3B | LY6G6C | FGG | H1-3 |
| SPRR2D | AHNAK | PFN1 | ARHGDIB |
| SERPINB3 | AMBP | DSP | SCGB2A2 |
| CAP1 | IL36G | AHSG | APCS |
| IGHG1 | NCCRP1 | EEF2 | ANXA3 |
| ALDOA | YWHAZ | WFDC12 | ERP29 |
| SFN | RPL30 | ALB | |
| DYNLL1 | H1-5 | PKM | |
| APOA2 | P13 | CALR | |
| S100A10 | HLA-DRB1 | YWHAG | |
| SPRR2F | EIF4A2 | DCD | |
| RPS11 | PLEC | PPIA | |
| DSC3 | P4HB | KLK7 | |
| POF1B | VIM | PPL | |
| APOA1 | GPLD1 | KLK10 | |
| HNRNPA2B1 | F2 | ORM1 | |
| VDAC1 | CAPG | MUCL1 | |
| S100A7 | TF | MIF | |
| KLK6 | MYL6 | SCGB1D2 | |
| S100A8 | PDIA3 | EIF6 | |
| VTN | | MYH9 | |
**[Table C-5-2]**
| |
|---|
| SERPINB13 |
| POLR3A |
| JCHAIN |
| LTF |
| SAMD4A |
| LCN15 |
| LYZ |
| PRR4 |
| BST1 |
| SCGB2A1 |
| LACRT |
| LCN1 |

32. The method according to claim 27 or 28, wherein the test subject is an adult, and the at least one protein is at least one protein selected from the group consisting of proteins shown in the following Tables C-6-1 and C-6-2.
**[Table C-6-1]**
| | | | | | | |
|---|---|---|---|---|---|---|
| LGALS3 | A1BG | CDC42 | H1-5 | ANXA6 | TRIM29 | ITGB2 |
| SERPINB1 | ITIH1 | HBB | CTSG | PON1 | EFNA1 | CA2 |
| HMGB2 | FGG | RPS17 | H3C1 | APOA2 | HNRNPK | RPS25 |
| GC | C4BPA | ELANE | SUB1 | ARHGDIB | CALR | RAB1B |
| TF | SERPINF2 | GNAI2 | MYL6 | MYL12B | IGLV1-51 | PSMD14 |
| ITIH4 | GSN | IGHV3-7 | IGKV1-5 | HSPA1A | RPS6 | PSME2 |
| ALB | CEACAM5 | GSTP1 | RP1BL | BTF3 | LPO | RPL5 |
| HPX | HRG | MYH9 | ACTB | AKR1A1 | TMSL3 | BPI |
| TTR | CFH | PYCARD | ANXA1 | UGP2 | SERPINA4 | |
| DERA | SERPIND1 | ARPC3 | TUBB4B | LCP1 | EFHD2 | |
| SERPINA1 | KNG1 | C1QC | YWHAE | LCN2 | SEPTIN8 | |
| VTN | P4HB | IGKV4-1 | YWHAH | UBE2N | RAB27A | |
| APOA1 | VIM | DBI | PPIB | COTL1 | RPS23 | |
| NAPA | SERPINB5 | H2BC12 | NME2 | RALY | RPS9 | |
| APOB | RNASE3 | SUMO3 | IGKV3-11 | DEFA3 | YWHAG | |
| IGHV1-46 | MMP9 | FAU | CAMP | NAMPT | TMED5 | |
| MSN | G6PD | RPL8 | RAC2 | IGHG2 | HNRNPR | |
| CFB | C3 | TPT1 | SRSF3 | H1-3 | HK3 | |
| EZR | IGHG1 | AZU1 | GPI | ALDH3A1 | SBSN | |
| ERP29 | ORM1 | PFN1 | AGT | C1S | SRSF2 | |
| PLG | SERPING1 | C1QA | MIF | ACTR2 | LDHA | |
| CP | CFL1 | TUBB | PYGL | TNNI3K | IGHV3-30 | |
| KV310 | H4C1 | HNRNPD | TACSTD2 | AFM | LRG1 | |
| AMBP | FGB | TPD52L2 | IGHV3-33 | ASPRV1 | SEPTIN9 | |
| FN1 | HMGB1 | TUBB2A | RPL6 | CAPZA1 | RPL12 | |
| F2 | C4A | TAGLN2 | LGALS1 | MPO | CCT6A | |
| DDX55 | CFI | SERPINF1 | PLS3 | CANX | RPL18A | |
| PPIA | GPT | WDR1 | RETN | CBR1 | THBS1 | |
| PRDX6 | IGKC | HBA1 | MACROH2A1 | DNAJB1 | C7 | |
| H2AZ1 | FGA | ARPC2 | IGKV3-20 | RTCB | DAG1 | |
| A2M | APCS | ITIH2 | EPS8L1 | CAPG | APOC1 | |
| AHSG | PGAM1 | RPS14 | CORO1A | H1-0 | RPL10A | |
| IGHG3 | PDIA3 | RAN | RPS19 | RPL4 | | |
**[Table C-6-2]**
| | |
|---|---|
| RAD9B | FBXO6 |
| FLG2 | GART |
| DHX36 | DCD |
| MGST2 | CRNN |
| GSDMA | SYNGR2 |
| TPP1 | PHB2 |
| F5 | DLD |
| KRT77 | ME1 |
| STS | IDH2 |
| MYH1 | IMPA2 |
| PLD3 | HMGA1 |
| SCGB2A2 | KRT15 |
| PSMB4 | PLTP |
| CCAR2 | SFPQ |
| PSMB3 | GMPR2 |
| PSMA1 | ZNF236 |
| DHRS11 | TIMP2 |
| POM121 | ZNF292 |
| HSPE1 | |

33. The method according to claim 31, wherein
the at least one protein is at least one protein selected from the group consisting of proteins shown in Table C-5-1, and
the method comprises detecting the test subject as having atopic dermatitis when a concentration of the at least one protein is increased as compared with a healthy children group.

34. The method according to claim 31, wherein
the at least one protein is at least one protein selected from the group consisting of proteins shown in Table C-5-2, and
the method comprises detecting the test subject as having atopic dermatitis when a concentration of the at least one protein is decreased as compared with a healthy children group.

35. The method according to claim 32, wherein
the at least one protein is at least one protein selected from the group consisting of proteins shown in Table C-6-1, and
the method comprises detecting the test subject as having atopic dermatitis when a concentration of the at least one protein is increased as compared with a healthy adult group.

36. The method according to claim 32, wherein
the at least one protein is at least one protein selected from the group consisting of proteins shown in Table C-6-2, and
the method comprises detecting the test subject as having atopic dermatitis when a concentration of the at least one protein is decreased as compared with a healthy adult group.

37. The method according to any one of claims 28 to 32, wherein the method comprises detecting AD on the basis of a prediction model constructed with a concentration of the at least one protein as an explanatory variable and the presence or absence of AD as an objective variable.

38. The method according to any one of claims 27 to 37, further comprising collecting skin surface lipids from the test subject.

39. A protein marker for detecting atopic dermatitis comprising at least one protein selected from the group consisting of proteins shown in the following Table C-7.
**[Table C-7]**
| | | | | |
|---|---|---|---|---|
| CCAR2 | LGALSL | RPS25 | SRSF3 | DHRS11 |
| CKMT1A | KV310 | RPS27A | SUB1 | HNRNPA2B1 |
| DDX10 | ATP5PO | SAMD4A | TRIM29 | ITIH1 |
| DDX55 | DERA | SEPTIN8 | TUBB4B | LACRT |
| DYNLL1 | PRR4 | SEPTIN9 | CPQ | PRSS27 |
| EIF3I | AKR1A1 | SERBP1 | FLNB | PSMB2 |
| EIF5A | BTF3 | SFPQ | RPS9 | PSME2 |
| GMPR2 | CCT6A | SNRPD3 | RPLB | RPS16 |
| H1-0 | CPNE3 | TAGLN2 | A1BG | CAP1 |
| H2AC4 | DNAAF1 | TMSL3 | ARHGDIΘ | CTSA |
| HNRNPR | EIF4A2 | TNNI3K | CDH23 | DLD |
| 1GKV3-11 | EPSBL1 | ZNF292 | EIF6 | |
| IGLV1-51 | ERP29 | WDR1 | FBXO6 | |
| IMPA2 | GART | ARPC3 | HSD17B4 | |
| KRTAP2-3 | GDI2 | BST1 | IGHV3-30 | |
| MMGT1 | HM13 | CAPZA1 | IGHV3-33 | |
| MYH14 | IGHV1-46 | CCT3 | IGHV3-7 | |
| RAD9B | IGKV1-5 | COTL1 | ITIH2 | |
| REEP5 | IGKV4-1 | CRISPLD2 | LCN15 | |
| RP1BL | MAST4 | GPLD1 | LY6G6C | |
| RPL6 | MDH2 | IGKV3-20 | PLD3 | |
| RTCB | MYH1 | MACROH2A1 | POF1B | |
| SYNGR2 | NCCRP1 | MYL6 | PSMA1 | |
| TASOR2 | PCBP1 | NDUFB6 | RPL15 | |
| TMED5 | POM121 | PDIA6 | RPL30 | |
| TPD52L2 | PSMB3 | PGAM1 | RPL31 | |
| VSIG10L | RAB10 | POLR3A | RPS17 | |
| ZNF236 | RAB1B | PSMB1 | TUBB2A | |
| GARS1 | RECQL | PSMB5 | HK3 | |
| H3C1 | RPL10A | PSMD14 | MTAP | |
| H1-5 | RPL12 | RAB1A | RALY | |
| H2AZ1 | RPL29 | RANBP1 | RPL4 | |
| H2AC11 | RPS14 | RDH12 | RPL7 | |
| H2BC12 | RPS23 | RPL14 | TPP1 | |

40. A method for detecting childhood atopic dermatitis in a child test subject, comprising a step of measuring an expression level of SerpinB4 protein in skin surface lipids collected from the test subject.

41. The method according to claim 40, further comprising detecting the presence or absence of childhood atopic dermatitis, or a degree of progression thereof by comparing the measurement value of the expression level of SerpinB4 protein with a reference value.

42. The method according to claim 41, wherein the detection of the degree of progression of childhood atopic dermatitis is detection of mild or moderate atopic dermatitis.

43. The method according to any one of claims 40 to 42, wherein the child is a 0- to 5-year-old child.

44. The method according to any one of claims 40 to 43, further comprising collecting skin surface lipids from the test subject.

45. A test kit for detecting childhood atopic dermatitis, the kit being used in the method according to any one of claims 40 to 44, and comprising an antibody which recognizes SerpinB4 protein.
